(19) Europäisches Patentamt
European Patent Office
Office européen des brevets

(11) EP 1 983 336 A1

(12) **EUROPEAN PATENT APPLICATION**
published in accordance with Art. 158(3) EPC

(43) Date of publication:
**22.10.2008 Bulletin 2008/43**

(21) Application number: **07707744.4**

(22) Date of filing: **30.01.2007**

(51) Int Cl.:
***G01N 25/18*** *(2006.01)*

(86) International application number:
**PCT/JP2007/051526**

(87) International publication number:
**WO 2007/086585 (02.08.2007 Gazette 2007/31)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.01.2006 JP 2006020873**
**28.02.2006 JP 2006052744**
**28.02.2006 JP 2006052745**
**28.03.2006 JP 2006088416**

(71) Applicant: **Mitsui Mining and Smelting Co., Ltd.**
**Shinagawa-ku,**
**Tokyo 141-8584 (JP)**

(72) Inventors:
- **YAMAGISHI, Kiyoshi**
  **Ageo-shi, Saitama 3620021 (JP)**
- **NAKAMURA, Toshimi**
  **Ageo-shi, Saitama 3620021 (JP)**
- **YANAGI, Kiyotaka**
  **Ageo-shi, Saitama 3620021 (JP)**
- **KUBOTA, Akiko**
  **Ageo-shi, Saitama 3620021 (JP)**

(74) Representative: **Hall, Matthew Benjamin**
**Frank B. Dehn & Co.**
**St Bride's House**
**10 Salisbury Square**
**London**
**EC4Y 8JD (GB)**

(54) **FLUID IDENTIFICATION DEVICE AND FLUID IDENTIFICATION METHOD**

(57) There is provided by the invention a fluid identification device capable of being mounted in single device constitution on a tank irrespective of the type of the tank. The fluid identification device of the invention is **characterized in that** a fluid detection part which is equipped with a temperature detector for fluid detection and a heating element disposed in the vicinity of the temperature detector for fluid detection and which is disposed on the identification target fluid side, a fluid detection circuit connected to the fluid detection part and an identification operation part which performs identification of an identification target fluid based on the output of the fluid detection circuit are contained in an container.

[Fig. 1]

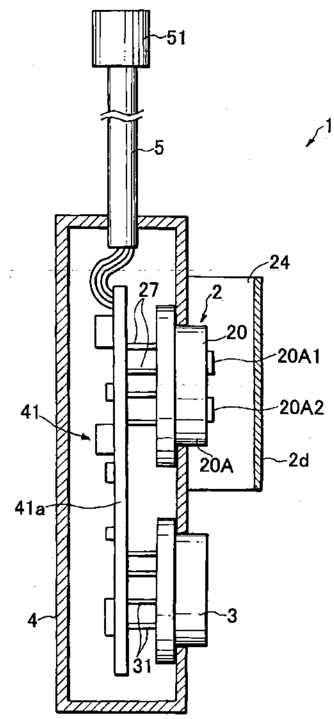


EP 1 983 336 A1

## Description

### TECHNICAL FIELD

The present invention relates to a fluid identification device for performing identification of identification target fluids, e.g., hydrocarbon type liquids, such as gasoline, naphtha, light oil and heavy oil, alcohol type liquids, such as ethanol and methanol, urea aqueous solution, gasses and powders, utilizing thermal properties of the fluids, specifically performing various identification, such as fluid type identification, concentration identification, identification of presence of fluid, fluid temperature identification, flow rate identification, fluid leak identification, fluid level identification and identification of quantity of ammonia generated, and also relates to a fluid identification method using the fluid identification device.

The fluid identification device of the invention and the fluid identification method using the fluid identification device can be utilized to judge, for example, whether a liquid that is sprayed, as a urea aqueous solution having a given concentration, onto an exhaust gas purification catalyst in order to decompose nitrogen oxide (NOx) in a system for purifying an exhaust gas exhausted from an internal-combustion engine of an automobile or the like is truly a urea aqueous solution of a given concentration.

### BACKGROUND ART

In internal-combustion engines of automobiles, fossil fuels such as gasoline and light oil are burned. In the exhaust gas generated by the burning, environmental pollution substances, such as unburned carbon monoxide (CO), unburned hydrogen carbide (HC), sulfur oxide (SOx) and nitrogen oxide (NOx), are contained together with water, carbon dioxide and the like. Particularly for purposes of environmental protection and prevention of living environmental pollution, various countermeasures have been taken in recent years in order to purify automobile exhaust gas.

As one of such countermeasures, use of an exhaust gas purification catalyst device can be mentioned. This is intended to make the exhaust gas harmless by disposing an exhaust gas purifying three-element catalyst somewhere in the exhaust system and decomposing CO, HC, NOx and the like by oxidation-reduction reaction. In order to continuously maintain decomposition of NOx in the catalyst device, a urea aqueous solution is sprayed onto the catalyst just from the upstream side of the catalyst device of the exhaust system. In order to enhance the effect of decomposition of NOx, this urea aqueous solution needs to have a urea concentration in the specific range, and particularly, a urea concentration of 32.5% is considered to be optimum.

The urea aqueous solution is contained in a urea aqueous solution tank mounted on the automobile, and a change of concentration of the urea aqueous solution with time sometimes occurs. Further, heterogeneity of a concentration distribution sometimes occurs locally in the tank. The urea aqueous solution that is supplied to a spray nozzle from the tank through a supply pipe by means of a pump is generally withdrawn from an outlet near the bottom of the tank, and therefore, in order to enhance efficiency of the catalyst device, it is important that the urea aqueous solution present in this region has a given urea concentration.

Further, there is actually a possibility of introducing a liquid other than the urea aqueous solution into the urea aqueous solution tank by mistake. In such a case, in order to allow the catalyst device to fulfill its function, it is necessary to rapidly detect that the liquid is a liquid other than the urea aqueous solution of a given urea concentration and to give a warning.

By the way, the present inventors have already proposed, in Japanese Patent Laid-Open Publication No. 153561/1999 (patent document 1, see particularly paragraphs [0042] to [0049]), a fluid identification method comprising allowing a heating element to generate heat by electrical conduction, heating a temperature detector with the heat, giving thermal influence on the heat transfer from the heating element to the temperature detector by the identification target fluid, and identifying the type of the identification target fluid based on an electrical output corresponding to electrical resistance of the temperature detector, wherein the electrical conduction to the heating element is carried out periodically.

In this fluid identification method, however, it is necessary to periodically carry out electrical conduction to the heating element (with multiple pulse), so that the identification takes a time and it is difficult to instantly identify the fluid. In this method, it is possible to carry out fluid identification by the representative value in the case of, for example, substances having considerably different properties, such as water, air and oil, but it is difficult to carry out the above-mentioned identification of urea concentration of the urea solution accurately and rapidly.

For such a purpose, in Japanese Patent Laid-Open Publication No. 337969/2005 (patent document 2), a liquid type identification device having an indirect-heated liquid type detection part comprising a heating element and a temperature detector, a liquid temperature detection part for detecting the temperature of the measuring target liquid, and an identification sensor part disposed facing a passage of the measuring target liquid is described as a liquid type identification device for judging whether the measuring target liquid is a given one or not. This liquid type identification device is equipped with an identification operation part having functions of applying a single pulse voltage to the heating element of the indirect-heated liquid type detection part to allow the heating element to generate heat and performing identification of the measuring target liquid based on an output of a liquid type detection circuit comprising the temperature detector of the indirect-heated liquid type detection part and the liquid temperature detection part.

Patent document 1: Japanese Patent Laid-Open Publication No. 153561/1999
Patent document 2: Japanese Patent Laid-Open Publication No. 337969/2005
Patent document 3: Japanese Patent Laid-Open Publication No. 118566/1999
Patent document 4: Japanese Patent Laid-Open Publication No. 185522/2003

DISCLOSURE OF THE INVENTION

PROBLEM TO BE SOLVED BY THE INVENTION

In the liquid type identification device described in the patent document 2, however, the upper end of the supporting part is connected to the mounting part for mounting the device onto the tank opening, and to the lower end of the supporting part is fixed the identification sensor part. The identification operation part is disposed in the vicinity of the mounting part, that is, the identification operation part is disposed apart from the identification sensor part.

In such a device, therefore, there is a manufacturing disadvantage that it is requested to prepare a supporting part of different length according to the size and the type (e.g., model) of the tank.

It is an object of the present invention to provide a fluid identification device capable of being mounted in single device constitution on a tank irrespective of the type of the tank. It is another object of the invention to provide a small-sized fluid identification device.

It is a further object of the invention to provide a fluid identification device capable of exhibiting stable identification performance over a long period of time.

It is a further object of the invention to provide a fluid identification device capable of performing identification with enhanced accuracy.

It is a further object of the invention to provide a fluid identification device capable of exhibiting stable identification performance over a long period of time.

In the liquid type identification device described in the patent document 2, further, the liquid type detection part is disposed above the liquid temperature detection part in the identification sensor part. That is to say, the liquid type detection part and the liquid temperature detection part are disposed apart from each other in the extending direction of the supporting part, namely, in the vertical direction.

On the other hand, as a flow meter [flow rate sensor] (or a flow velocity meter [flow velocity sensor]) to measure flow rate or flow velocity of various fluids, particularly liquids, various types have been used in the past, and for the reason of facilitation of cost reduction, a thermal process (particularly indirect-heated) flow meter has been utilized.

As the indirect-heated flow meter, there is used a flow meter wherein a sensor chip obtained by laminating a thin film heating element and a thin film temperature detector onto a substrate through an insulating layer utilizing thin film technique is disposed so as to enable heat transfer between the sensor chip and the fluid in a pipe.

By virtue of electrical conduction to the heating element, the temperature detector is heated to thereby change electrical properties of the temperature detector, e.g., value of electrical resistance. The change of the electrical resistance value (based on temperature rise of the temperature detector) varies according to the flow rate (flow velocity) of the fluid flowing in the pipe.

The reason is that a part of the quantity of heat generated by the heating element is transferred into the fluid, and the quantity of heat diffused into the fluid varies according to the flow rate (flow velocity) of the fluid, and correspondingly thereto, the quantity of heat supplied to the temperature detector varies to thereby change the electrical resistance value of the temperature detector.

The change of the electrical resistance value of the temperature detector varies also by the temperature of the fluid. On this account, a temperature detection element for temperature compensation has been incorporated into the electrical circuit for measuring the change of the electrical resistance value of the temperature detector, and the change of the flow rate measured value due to the fluid temperature has been made as small as possible.

Such an indirect-heated flow meter using a thin film element is described in, for example, Japanese Patent Laid-Open Publication No. 118566/1999 (patent document 3). In this flow meter, an electrical circuit (detection circuit) including a bridge circuit is used to obtain an electrical output corresponding to the flow rate of the fluid.

In such a flow meter as above, if the thermal properties of fluids are different, the outputs of the detection circuit differ from one another even if the actual flow rates are the same, so that on the assumption that the type of the fluid, whose flow rate is to be measured, is already known, the output of the detection circuit is generally converted to the fluid flow rate value using a calibration curve of the fluid.

Recently, however, there has been carried out flow rate identification wherein a dispense supply source containing dispensed fluid is used as a source of supply of a fluid whose flow rate is to be measured and plural dispense supply sources of the same fluid are replaced with one another in order.

For example, in synthesis of high-purity reagents, synthesis of medicines and chemical analysis, a small portable container containing a raw material fluid or a reagent fluid is connected to a reactor or an analytical instrument through a measuring part, and with measuring a flow rate at the measuring part, the raw material fluid or the reagent fluid is supplied to the reactor, in some cases.

For replenishing the raw material fluid or the reagent fluid, the empty portable container is replaced with a new portable container filled with the raw material fluid or the reagent fluid, and the new portable container is connected to the reactor or the analytical instrument.

Also in the case where a medical fluid is injected into a living body, the medical fluid is dispensed into packs in a portable quantity, and the medical fluid pack is connected to, for example, a blood vessel of a living body through a measuring part, and with measuring a flow rate at the measuring part, the medical fluid is injected into the living body. For replenishing the medical fluid, the empty pack is replaced with a new pack filled with the medical fluid, and the new pack is connected to the living body.

In such a case of injection of the medical fluid into the living body, use of the dispense supply source is of practical great advantage, but on the other side, there is a possibility of connecting a supply source containing a fluid other than the necessary fluid by mistake in the replacement of the fluid supply source.

If fluid supply is carried out without knowledge of the mistake in the above case, accurate measurement of flow rate cannot be carried out because of difference in thermal properties between the necessary fluid and the fluid actually supplied, and besides, supply of the wrong fluid causes failure in production or analysis, or medical accident.

Then, the present invention is intended to avoid flow of such a wrong fluid, and it is an object of the present invention to provide a fluid identification device which has a simple constitution and can be used as a thermal process flow meter having a function of judging whether the fluid is a necessary one or not.

On the other hand, fuel oils or various liquid chemicals are stored in tanks. For example, a centralized oil supply system in an apartment house has been proposed in recent years, and in this system, fuel kerosene is supplied to each apartment from the centralized kerosene tank through a pipe.

The tank is sometimes cracked because of deterioration with time, and in this case, the in-tank liquid leaks out of the tank. In order to prevent ignition explosion or environmental pollution or generation of poisonous gas, it is important to detect such a situation at once and to properly cope with it.

In Japanese Patent Laid-Open Publication No. 185522/2003 (patent documents 4), there is disclosed, as a device to detect leak of an in-tank liquid as soon as possible, a device which has a measuring tube where a liquid in a tank is introduced and a measuring fine tube located under the measuring tube and which detects extremely small liquid surface fluctuation, i.e., liquid level change, of the in-tank liquid by measuring a flow rate of the liquid in the measuring fine tube by means of a sensor installed in the measuring fine tube.

In this leak detection device, an indirect-heated flow meter is used as the sensor installed in the measuring fine tube. In this flow meter, the heating element is allowed to generate heat by electrical conduction and a part of the quantity of heat generated is allowed to be absorbed by the liquid, and utilizing a phenomenon that the quantity of heat absorbed by the liquid varies depending on the flow rate of the liquid, influence of the heat absorption is detected as a change of electrical property value, e.g., resistance value, due to the temperature change of the temperature detector.

In the indirect-heated flow meter used in the leak detection device described in the patent document 4, however, the change of electrical circuit output against the change of flow rate becomes small in the region where the flow rate value is an extremely small value such as 1 mml/hr or less, so that the error of the flow rate measured value tends to become larger. On this account, there is limitation on the enhancement of accuracy of leak detection.

By the way, if an external commercial power source is used as a power source for the electrical conduction to the heating element in the above leak detection, it becomes necessary to lead a power source wiring from the outside to the sensor of the leak detection device. In the use of such a power source wiring for a long period of time, there is a possibility of occurrence of leak of electricity at the wiring intake of the structure part of the leak detection device. In the case where the liquid is combustible or electrically conductive, the leak of electricity sometimes causes ignition of the liquid adhering to the structure part of the leak detection device or causes a short circuit.

Particularly in the case of a combustible or electrically conductive liquid, it is preferable from the above viewpoints to use a battery housed in the structure part of the leak detection device as the power source for the heating element of the sensor. In this case, in order to perform leak detection over the longest period without replacing the battery, it is desirable to reduce power consumption of the leak detection device.

Further, the leak of the in-tank liquid can be detected based on the magnitude of liquid level change. For the measurement of the liquid level change, a pressure sensor can be employed. The liquid level detection by the pressure sensor is carried out by converting the detected liquid pressure to the depth from the liquid surface to the pressure sensor, so that the specific gravity of the measuring target liquid participates in the conversion.

Therefore, when the leak detection is carried out on only a measuring target liquid whose specific gravity is constant (e.g., water), a liquid level value can be obtained at once based on the output of the pressure sensor using the specific gravity value of the liquid having been inputted into the conversion program in advance, and based on the resulting liquid level value, leak detection can be carried out accurately.

In the case where the measuring target liquid is a mixed composition of many organic compounds such as a fuel oil (gasoline, naphtha, kerosene, light oil or heavy oil), however, there are present a greet number of, for example, kerosenes having different compound constitutions depending on the kerosene distillation conditions in the refining process and therefore having different specific gravities (e.g., specific gravity difference: 0.05), though they are fuel oils of the same type.

Accordingly, in the case of leak detection of, for example, kerosene contained in the tank, even if a specific gravity of standard kerosene is used as a liquid specific gravity value in the conversion program for converting the liquid pressure to the liquid level in the leak detection device, an error due to conversion occurs when the kerosene actually contained in the tank has a specific gravity different from that of standard kerosene.

If the residue of kerosene in the tank is decreased, kerosene is newly replenished, and there are various kerosenes as those used for the replenishment. Therefore, the specific gravity of kerosene in the tank varies each time of replenishment in some cases. In the leak detection of a mixed composition such as kerosene, consequently, measurement of liquid level is frequently accompanied by such a conversion error as above, and the accuracy of leak detection tends to lower.

Accordingly, it is an object of the present invention to provide a device for detecting leak of an in-tank liquid, which exhibits high detection accuracy when leak of the in-tank liquid is detected by the change of a liquid level measured by a pressure sensor.

It is another object of the invention to provide a fluid identification device employable as a device for detecting leak of an in-tank liquid, which can detect leak of a very slight amount of a liquid.

It is a further object of the invention to provide a fluid identification device employable as a leak detection device capable of continuously performing leak detection and capable of reducing power consumption.

On the other hand, in the fluid identification method described in the patent document 1 (Japanese Patent Laid-Open Publication No. 153561/1999), it is possible to perform discrimination between fluids having considerably different properties, such as water, air and oil, by their representative values. However, this method cannot be said to be sufficient for performing accurate and rapid discrimination in the application of the method to discrimination between different types of the same fluid such as the aforesaid gasoline.

Further, in the case where this method is applied to identification of the type of gasoline loaded on movable bodies such as automobiles, another technical problem takes place.

That is to say, in the above case, the inclination angle (inclination) of the automobile to the vertical direction (direction of gravity) is not always kept constant. For example, when the automobile is stopped on a slope, the inclination angle becomes larger as compared with the case of stopping on a flat place, and according to this, the identification device is inclined at various angles, whereby thermal influence given by gasoline that is an identification target fluid to the heat transfer from the heating element to the temperature detector varies, and accuracy of identification is sometimes lowered.

Further, during moving of the automobile, forced flow of gasoline around the heat transfer member is sometimes caused by an external factor, whereby thermal influence given by the identification target fluid to the heat transfer from the heating element to the temperature detector varies, and accuracy of identification is sometimes lowered.

Accordingly, it is an object of the present invention to provide a fluid identification device employable as a gasoline type identification device capable of identifying the type of gasoline accurately and rapidly even if the identification device is inclined at various angles or is being moved.

In order that the material constitution of a fuel may be made constant and the optimum combustion conditions should not vary, it is considered to use components of fossil fuels, i.e., hydrocarbons, such as pentane, cyclohexane and octane, and alcohols, such as methanol and alcohol, singly or as a mixture of at least about two kinds. The fuels of this type are broadly divided into hydrocarbon type fuels and alcohol type fuels.

However, if such various fuels are used in parallel in the city, there is a fear of supplying a fuel other than the given fuel to a fuel tank by mistake in the refueling. If the fuel supplied to the internal-combustion engine is different from the given one, the output efficiency of the engine is markedly lowered, so that occurrence of such a situation must be avoided.

On this account, also on the automobile side, it is desirable to actually detect the type of the fuel supplied from the fuel tank to the internal-combustion engine and to identify the fuel type as the given one.

In the case where the type of the fuel detected is similar to the given one, it is desirable to optimize the combustion conditions of the internal-combustion engine according to the detected type of the fuel.

That is to say, it is desirable that the type of the fuel actually supplied to the internal-combustion engine is identified, and according the identification result, the combustion conditions of the internal-combustion engine are properly determined to realize a preferred combustion state (i.e., combustion state where output torque of the internal-combustion engine is increased to thereby reduce the quantity of an incomplete combustion product in the exhaust gas) corresponding to the type of the fuel that is actually subjected to combustion.

As described above, the hydrocarbon type fuels and the alcohol type fuels greatly differ from each other in combustion properties and physical properties, so that it is necessary to judge first which group the measuring target liquid (fuel) belongs to. Moreover, it is preferable to judge what the measuring target liquid (fuel) is like in the hydrocarbon type fuels or the alcohol type fuels.

In the fluid identification method described in the patent document 1 (Japanese Patent Laid-Open Publication No. 153561/1999), however, identification can be carried out by the representative value in the case of fluids having considerably different properties, such as water, air and oil, but accurate and rapid discrimination between such hydrocarbon type liquids and alcohol type liquids as above cannot be carried out sufficiently favorably.

Accordingly, it is an object of the present invention to provide an identification device capable of performing discrimination between the hydrocarbon type liquids and the alcohol type liquids employable particularly as fuels, accurately, rapidly and easily.

It is another object of the invention to provide an identification device capable of performing judging what the measuring target liquid is like in the hydrocarbon type liquids or the alcohol type liquids accurately, rapidly and easily.

On the other hand, in the case of the thermal process sensor of indirect-heated type described in the patent document 1 (Japanese Patent Laid-Open Publication No. 153561/1999), if the measuring target fluid is a liquid, air or the like dissolved in the liquid is vaporized by temperature rise to form bubbles, and the bubbles sometimes adhere to the outer surface of the sensor.

In the case where the measuring target fluid is a liquid similarly to the above, if the liquid is contained in a tank and if a free surface of the liquid is present in the tank, the liquid surface waves by oscillation of the in-tank liquid, and a gas such as air in contact with the liquid surface is involved in the liquid and remains as bubbles in the liquid. These bubbles sometimes adhere to the outer surface of the sensor.

Especially in the case of a urea aqueous solution in the tank mounted on the automobile, vigorous oscillation due to an external force is repeated during traveling of the automobile, and therefore, adhesion of bubbles onto the outer surface of the sensor is conspicuous.

If the bubbles adhere to the sensor, heat generated by the heating element of the sensor is not favorably transferred to the liquid through the heat transfer member, and besides, heat transfer from the liquid to the temperature detector through the heat transfer member is not favorably carried out. If the heat transfer between the sensor and the measuring target liquid is not carried out normally as above, the concentration measured value of the measuring target liquid has a large error, and there is a fear of marked lowering of measuring reliability.

Accordingly, it is an object of the present invention to provide a fluid identification device sensor module employable as a thermal process sensor which has been reduced in adhesion of bubbles to the sensor outer surface and can enhance measuring accuracy especially when the measuring target is an aqueous liquid, and to provide a fluid identification device employable as a measuring device using the sensor module.

It is another object of the invention to provide a fluid identification method employable as a liquid level detection method that is intended to detect a liquid level with high accuracy by making corrections based on the density of the liquid in the detection of liquid level using a pressure sensor, and to provide a fluid identification device employable as a liquid level detection device.

On the other hand, in order to continuously maintain decomposition of NOx in the exhaust gas purification catalyst device, a urea aqueous solution is sprayed onto the catalyst just from the upstream side of the catalyst device of the exhaust system, as previously described, and particularly, a urea concentration of 32.5% is considered to optimum.

In this case, however, the urea aqueous solution has a relatively high solidifying point, and the urea aqueous solution having a urea concentration of 32.5% freezes at -11°C. Therefore, in the extremely cold districts, such as Wakkanai in Japan, Alaska, the surroundings of the Great Lakes, Canada and Russia, decomposition of NOx by the above-mentioned exhaust gas purification catalyst system cannot be continuously maintained.

On this account, in for example U.S.A., a mixed solution obtained by mixing a urea aqueous solution with an ammonium formate solution is used instead of a urea aqueous solution in the exhaust gas purification catalyst device, whereby the solidifying point, namely, freezing temperature, is lowered.

By the way, in the case of such a mixed solution, 20% by weight of urea, 26% by weight of ammonium formate and 54% by weight of $H_2O$ are preferable in order that the reduction reaction may undergo on the upstream side of the catalyst device efficiently without solidifying the mixed solution, but as it is, any means to grasp such a preferred mixing ratio has not been provided.

Accordingly, it is an object of the present invention to provide a device for measuring a quantity of ammonia generated and a method for measuring a quantity of ammonia generated, by which the concentration of a mixed solution of a urea aqueous solution and an ammonium formate solution in a urea tank and the quantity of ammonia generated can be accurately and rapidly grasped when the mixed solution is used instead of a urea aqueous solution in the exhaust gas purification catalyst device, and as a result, the mixed solution can be maintained in a given concentration, and consequently the quantity of NOx in the exhaust gas can be remarkably deceased by reduction.

MEANS TO SOLVE THE PROBLEM

The present invention has been made to solve such problems associated with the prior art and to attain such objects as described above, and the identification sensor module containing, in an container,
a fluid detection part which has a temperature detector for fluid detection and is disposed on the identification target fluid side,
a fluid detection circuit connected to the fluid detection part, and
an identification operation part which performs identification of an identification target fluid based on an output of the fluid detection circuit.
The identification sensor module of the present invention is an identification sensor module containing, in a container,
a fluid detection part which has a temperature detector for fluid detection and a heating element disposed in the vicinity of the temperature detector for fluid detection and which is disposed on the identification target fluid side,
a fluid detection circuit connected to the fluid detection part, and
an identification operation part which performs identification of an identification target fluid based on an output of the fluid detection circuit,
wherein the fluid detection part has a chip for fluid detection comprising the temperature detector for fluid detection and a lead one end of which is electrically connected to the chip for fluid detection and the other end of which is connected to a substrate of the fluid detection circuit, and
the lead has a spring structure.
In the identification sensor module of the invention, the other end of the lead connected to a substrate of the fluid detection circuit is constructed so as to penetrate the substrate from the front surface to the back surface and so as to project from the back surface.
In the fluid detection part of the identification sensor module of the invention,
the chip for fluid detection is embedded in a synthetic resin mold, and
one end of the lead is connected to the synthetic resin mold.
In the identification sensor module of the invention, the spring structure of the lead is constructed so as to bias the synthetic resin mold toward the inner surface of the container.
In the identification sensor module of the invention, the spring structure comprises a plate spring.
In the identification sensor module of the invention, the lead has a first linear portion close to the one end, a second linear portion located on the extension of the first linear portion and close to the other end, and a bent portion located between the first linear portion and the second linear portion.
The identification sensor module of the present invention is an identification sensor module containing, in an container,
a fluid detection part having a temperature detector for fluid detection,
a fluid detection circuit comprising the temperature detector for fluid detection, and
an identification operation part which performs identification of an identification target fluid based on an output of the fluid detection circuit,
wherein the container is provided with a protruded portion which protrudes toward the outside of the container correspondingly to the position at which the fluid detection part is disposed.
In the identification sensor module of the invention, the shape of the protruded portion is any one of circular, elliptical, elongated circular and rectangular shapes.
In the identification sensor module of the invention, a depressed portion is formed on the interior side of the container correspondingly to the protruded portion.
In the identification sensor module of the invention, the shape of the depressed portion is any one of circular, elliptical, elongated circular and rectangular shapes.
In the identification sensor module of the invention, the fluid detection part is disposed in contact with the inner surface of the depressed portion.
In the identification sensor module of the invention, the depth of the depressed portion is larger than a smaller dimension between dimensions of the depressed portion in the direction crossing the depth direction at right angles.
In the identification sensor module of the invention, the fluid detection part has a chip for fluid detection comprising the temperature detector for fluid detection, a synthetic resin mold in which the chip for fluid detection is embedded, and a lead one end of which is connected to the synthetic resin mold and is electrically connected to the chip for fluid detection, and
the synthetic resin mold is disposed in the depressed portion.
In the identification sensor module of the invention, the fluid detection part is disposed in such a manner that the chip for fluid detection is in contact with the inner surface of the depressed portion.

In the identification sensor module of the invention, the chip for fluid detection is in contact with the side surface of the depressed portion.

In the identification sensor module of the invention, the other end of the lead is connected to a substrate of the fluid detection circuit, and the lead has a spring structure which biases the synthetic resin mold toward the bottom surface of the depressed portion.

In the identification sensor module of the invention, the spring structure of the lead is constructed so as to bias the synthetic resin mold toward the bottom surface of the depressed portion.

In the identification sensor module of the invention, the spring structure comprises a plate spring.

In the identification sensor module of the invention, the lead has:

a first linear portion close to the one end,
a second linear portion located on the extension of the first linear portion and close to the other end, and
a bent portion located between the first linear portion and the second linear portion.

In the identification sensor module of the invention, the fluid detection part has a temperature detector for fluid detection and a heating element disposed in the vicinity of the temperature detector for fluid detection.

The identification sensor module of the present invention comprises a fluid detection part which has a temperature detector for fluid detection and a heating element disposed in the vicinity of the temperature detector for fluid detection and which is disposed on the identification target fluid side and a fluid temperature detection part which has a temperature detector for fluid temperature detection, said fluid detection part and said fluid temperature detection part being disposed so as to be side by side apart from each other at a given distance in the horizontal direction to the fluid level of an identification target fluid.

In the identification sensor module of the invention, to the fluid detection part is connected a fluid detection circuit, and an identification operation part which performs identification of an identification target fluid based on an output of the fluid detection circuit is installed.

In the identification sensor module of the invention, a difference in position in the vertical direction between the fluid detection part and the fluid temperature detection part which are disposed so as to be side by side apart from each other at a given distance in the horizontal direction is not more than 2 mm.

In the identification sensor module of the invention, the fluid detection part is disposed in contact with the container on the identification target fluid side in such a manner that the fluid detection part is not exposed from the container on the identification target fluid side.

In the identification sensor module of the invention, the fluid detection part is disposed in such a manner that at least a part of the fluid detection part is exposed from the container on the identification target fluid side.

In the identification sensor module of the invention, the exposed portion of the fluid detection part is covered with a hydrophilic film or a filter.

In the identification sensor module of the invention, the container is constituted of a container main body located on the identification target fluid side and a cap located on the opposite side to the identification target fluid side.

In the identification sensor module of the invention, the container main body is made of a metal.

In the identification sensor module of the invention, a part of the fluid detection circuit and the identification operation part are incorporated into an IC.

In the identification sensor module of the invention, the fluid detection part is constructed by embedding a thin film chip for fluid detection, which is obtained by forming a temperature detector for fluid detection made of a thin film on a chip substrate, in a synthetic resin mold so that one surface of the thin film chip may be exposed, and
the thin film chip for fluid detection is disposed so that the one surface thereof may be located on the identification target fluid side of the container.

In the identification sensor module of the invention, a fluid temperature detection part having a temperature detector for fluid temperature detection is contained in the container,
the fluid detection circuit is connected to the temperature detector for fluid temperature detection, and
the fluid temperature detection part is disposed on the identification target fluid side.

In the identification sensor module of the invention, the fluid temperature detection part is disposed in contact with the container on the identification target fluid side in such a manner that the fluid temperature detection part is not exposed from the container on the identification target fluid side.

In the identification sensor module of the invention, the fluid temperature detection part is disposed in such a manner that at least a part of the fluid temperature detection part is exposed from the container on the identification target fluid side.

In the identification sensor module of the invention, the exposed portion of the fluid temperature detection part is covered with a hydrophilic film or a filter.

In the identification sensor module of the invention, the fluid temperature detection part is constructed by embedding a thin film chip for fluid temperature detection, which is obtained by forming a temperature detector for fluid temperature detection made of a thin film on a chip substrate, in a synthetic resin mold so that one surface of the thin film chip may be exposed, and
the thin film chip for fluid temperature detection is disposed so that the one surface thereof may be located on the identification target fluid side of the container.

The fluid identification device of the present invention has any one of the above-mentioned identification sensor modules.

In the fluid identification device of the invention, a measuring target fluid inlet part whose both ends are open is formed through the region close to the fluid detection part and the fluid temperature detection part, and a difference in position in the vertical direction between the fluid detection part and the fluid temperature detection part in a measuring target fluid inlet passage is not more than 2 mm.

In the fluid identification device of the invention, the identification sensor module is installed in a waterproof case, and
the identification sensor module is disposed in such a manner that the container on the fluid detection part side is exposed from the waterproof case on the identification target fluid side.

In the fluid identification device of the invention, the identification sensor module is disposed in such a manner that the container on the fluid detection part side protrudes from the waterproof case on the identification target fluid side.

In the fluid identification device of the invention, the container is constituted of a container main body located on the identification target fluid side and a cap located on the opposite side to the identification target fluid side, and a joint between the container main body and the cap is disposed inside the waterproof case.

In the fluid identification device of the invention, the waterproof case has a cover member that covers the container on the identification target fluid side, and inside the cover member, a passage of the identification target fluid is formed.

In the fluid identification device of the invention, the waterproof case is equipped with a fluid level sensor' module for detecting a fluid level of the identification target fluid.

In the fluid identification device of the invention, a power circuit part is encased in the waterproof case.

In the fluid identification device of the invention, a waterproof wiring is extended from the waterproof case.

In the fluid identification device of the invention, the identification of the identification target fluid is at least one of fluid type identification, concentration identification, identification of presence of fluid, identification of fluid temperature, flow rate identification, detection of leak of fluid and fluid level identification.

In the fluid identification device of the invention, the identification target fluid is any one of a hydrocarbon type liquid, an alcohol type liquid and a urea aqueous solution.

The fluid identification device of the invention is constructed so that detection of a flow rate of the fluid may be performed based on an electrical property value of the temperature detector for fluid detection and an electrical property value of the temperature detector for fluid temperature detection, and identification of a fluid type may be performed by measuring electrical conductivity between the fluid detection part and the fluid temperature detection part.

The fluid identification device of the invention is constructed so as to perform identification of a fluid type of the identification target fluid by:

disposing the fluid identification device in a fluid type identification chamber,
applying a pulse voltage to the heating element of the fluid detection part for a given period of time to heat the identification target fluid, which is temporarily staying in the fluid type identification chamber, by means of the heating element of the fluid detection part, and
identifying the fluid type of the identification target fluid by a voltage output difference corresponding to the temperature difference between the initial temperature of the temperature detector for fluid detection and the peak temperature thereof.

The fluid identification device of the invention is a fluid identification device having:

a main passage through which the identification target fluid flows,
a sub-passage diverged from the main passage,
the fluid identification device installed in the sub-passage,
a sub-passage on-off valve which is installed in the sub-passage and controls flow of the identification target fluid to the fluid identification device, and
a control device to control the fluid identification device and the sub-passage on-off valve,

wherein the control device is constructed so as to perform control in such a manner that:

in the identification of the identification target fluid, the control device closes the sub-passage on-off valve to allow the identification target fluid to temporarily stay in the fluid identification device and identifies the identification target fluid, and
in the detection of a flow rate of the identification target fluid, the control device opens the sub-passage on-off valve to allow the identification target fluid to flow to the fluid identification device and detects the flow rate of the identification target fluid.

The fluid identification device of the invention has:

a fluid identification detection chamber in which the identification target fluid is allowed to temporarily stay,
the identification sensor module of the fluid identification device, which is placed in the fluid identification detection chamber, and
a flow control plate which is placed in the fluid identification detection chamber and surrounds the identification sensor module.

The fluid identification device of the invention is constructed so as to perform identification of a concentration of the identification target fluid by:

disposing the fluid identification device in a fluid type identification chamber,
applying a pulse voltage to the heating element of the fluid detection part for a given period of time to heat the identification target fluid, which is temporarily staying in the fluid type identification chamber, by means of the heating element of the fluid detection part, and
identifying the concentration of the identification target fluid by a voltage output difference corresponding to the temperature difference between the initial temperature of the temperature detector for fluid detection and the peak temperature thereof.

The fluid identification device of the invention is placed in a measuring fine tube at the lower end of which the identification target fluid in a tank is introduced or discharged, and performs detection of leak of the identification target fluid from the tank by:

obtaining an output corresponding to a difference between temperatures detected by the temperature detector for fluid detection in the fluid detection part and the temperature detector for fluid temperature detection in the fluid temperature detection part,
detecting a specific gravity of the identification target fluid in the tank based on a flow rate-corresponding value corresponding to a flow rate of the identification target fluid that is calculated using the above-obtained output,
measuring a fluid level of the identification target fluid by the fluid level sensor module using the resulting specific gravity value, and detecting leak of the identification target fluid from the tank based on the magnitude of a time change rate of the fluid level.

The fluid identification device of the invention is constructed so as to perform detection of a fluid level of the identification target fluid by:

detecting a fluid pressure of the identification target fluid by the fluid level sensor module and calculating a temporary fluid level value based on the fluid pressure on the assumption that the identification target fluid is a fluid of a given density,
applying a pulse voltage to the heating element of the fluid detection part for a given period of time to identify a concentration of the identification target fluid by means of the heating element of the fluid detection part,
obtaining a density value of the identification target fluid based on the relationship between the identified concentration and the density of the identification target fluid, and
calculating the fluid level of the identification target fluid based on the temporary fluid level value and the density value.

The device for measuring a quantity of ammonia generated according to the present invention is a device equipped with any one of the above-mentioned fluid identification devices and for measuring a quantity of ammonia generated from an identification target liquid comprising urea water, ammonium formate water or mixed water thereof, wherein the quantity of ammonia generated is measured by:

using a sensor equipped with a heating element and a temperature detector disposed in the vicinity of the heating element,

applying a pulse voltage to the heating element for a given period of time to heat the measuring target liquid by means of the heating element,
then, by an electrical output corresponding to electrical resistance of the temperature detector, measuring a thermal conductivity-corresponding output value which is an electrical output of the temperature detector dependent on the thermal conductivity of the measuring target liquid,
measuring a density-corresponding output value which is an electrical output dependent on the density of the measuring target liquid, using a differential pressure sensor,
calculating a urea concentration X% by weight and an ammonium formate concentration Y% by weight of the measuring target liquid, from the relationship between the thermal conductivity-corresponding output value and the density-corresponding output value,
calculating a urea quantity A and an ammonium formate quantity B contained in the measuring target liquid, from the concentration and the quantity of the measuring target liquid, and
determining the quantity of ammonia generated, by the following formula:

$$\text{Quantity of ammonia generated} = X \times A + Y \times B$$

The device for measuring a quantity of ammonia generated according to the present invention is a device equipped with any one of the above-mentioned fluid identification devices and for measuring a quantity of ammonia generated from an identification target liquid comprising urea water, ammonium formate water or mixed water thereof, wherein the quantity of ammonia generated is measured by:

using a sensor equipped with a heating element and a temperature detector disposed in the vicinity of the heating element,
applying a pulse voltage to the heating element for a given period of time to heat the measuring target liquid by means of the heating element,
then, by an electrical output corresponding to electrical resistance of the temperature detector, measuring a thermal conductivity-corresponding output value which is an electrical output of the temperature detector dependent on the thermal conductivity of the measuring target liquid,
measuring a density-corresponding output value which is an electrical output dependent on the density of the measuring target liquid, using a differential pressure sensor,
calculating a urea concentration X% by weight and an ammonium formate concentration Y% by weight of the measuring target liquid, from the relationship between the thermal conductivity-corresponding output value and the density-corresponding output value,
calculating a urea quantity A and an ammonium formate quantity B contained in the measuring target liquid, from the concentration and the quantity of the measuring target liquid, and
determining the quantity of ammonia generated, by the following formula:

$$\text{Quantity of ammonia generated} = X \times A + Y \times B$$

The method for measuring a quantity of ammonia generated according to the present invention is a method for measuring a quantity of ammonia generated from an identification target liquid comprising urea water, ammonium formate water or mixed water thereof, using any one of the above-mentioned fluid identification devices, and comprises:

using a sensor equipped with a heating element and a temperature detector disposed in the vicinity of the heating element,
applying a pulse voltage to the heating element for a given period of time to heat the measuring target liquid by means of the heating element,
then, by an electrical output corresponding to electrical resistance of the temperature detector, measuring a thermal conductivity-corresponding output value which is an electrical output of the temperature detector dependent on the thermal conductivity of the measuring target liquid, and measuring a kinematic viscosity-corresponding output value which is an electrical output of the temperature detector dependent on the kinematic viscosity of the measuring target liquid,
calculating a urea concentration X% by weight and an ammonium formate concentration Y% by weight of the measuring target liquid, from the relationship between the thermal conductivity-corresponding output value and the kinematic viscosity-corresponding output value,
calculating a urea quantity A and an ammonium formate quantity B contained in the measuring target liquid, from the concentration and the quantity of the measuring target liquid, and
determining the quantity of ammonia generated, by the following formula:

$$\text{Quantity of ammonia generated} = X \times A + Y \times B$$

The method for measuring a quantity of ammonia generated according to the present invention is a method for measuring a quantity of ammonia generated from an identification target liquid comprising urea water, ammonium formate water or mixed water thereof, using any one of the above-mentioned fluid identification devices, and comprises:

using a sensor equipped with a heating element and a temperature detector disposed in the vicinity of the heating element,
applying a pulse voltage to the heating element for a given period of time to heat the measuring target liquid by means of the heating element,
then, based on an electrical output corresponding to electrical resistance of the temperature detector, measuring a thermal conductivity-corresponding output value which is an electrical output of the temperature detector dependent on the thermal conductivity of the measuring target liquid,
measuring a density-corresponding output value which is an electrical output dependent on the density of the measuring target liquid, using a differential pressure sensor,
calculating a urea concentration X% by weight and an ammonium formate concentration Y% by weight of the measuring target liquid, from the relationship between the thermal conductivity-corresponding output value and the density-corresponding output value,
calculating a urea quantity A and an ammonium formate quantity B contained in the measuring target liquid, from the concentration and the quantity of the measuring target liquid, and
determining the quantity of ammonia generated, by the following formula:

$$\text{Quantity of ammonia generated} = X \times A + Y \times B$$

The fluid identification method of the present invention comprises performing identification of an identification target fluid using any one of the above-mentioned fluid identification devices.

EFFECT OF THE INVENTION

According to the present invention, the fluid identification device is provided with an identification sensor module containing a fluid detection part, a fluid detection circuit and an identification operation part in a container. The container is installed in a waterproof case, and the container is allowed to protrude from the waterproof case, and further, the fluid detection part is disposed in contact with the inner surface of the container. Therefore, it is possible to mount the fluid identification device in single device constitution on a tank irrespective of the type of the tank, and moreover, miniaturization of the device is also possible.
According to the present invention, the fluid identification device is provided with an identification sensor module containing a fluid detection part, a fluid detection circuit and an identification operation part in a container. The fluid detection part is disposed in contact with the inner surface of the container, and the fluid detection part is equipped with a chip for fluid detection, a synthetic resin mold and a lead, and further, the lead has a spring structure which biases the synthetic resin mold toward the inner surface of the container. Therefore, it is possible to mount the fluid identification device in single device constitution on a tank irrespective of the type of the tank, and miniaturization of the device is also possible. Moreover, stable identification performance can be exhibited over a long period of time.
According to the present invention, the fluid identification device is provided with an identification sensor module containing a fluid detection part, a fluid detection circuit and an identification operation part in a container. The container is provided with a protruded portion and a depressed portion which corresponds to the protruded portion and is on the interior side of the container, and the fluid detection part is disposed in contact with the inner surface of the depressed portion.
Therefore, it is possible to mount the fluid identification device in single device constitution on a tank irrespective of the type of the tank, and miniaturization of the device is also possible. Moreover, identification with enhanced accuracy is possible.

According to the present invention, the fluid detection part and the fluid temperature detection part are disposed so as to be side by side apart from each other at a given distance in the horizontal direction to the fluid level of the measuring target fluid. Therefore, even if heterogeneity of a temperature distribution of the fluid occurs, the difference between the temperature detector of the fluid detection part and the temperature detector of the fluid temperature detection part becomes negligibly small, and on this account, the accuracy of the fluid type identification is hardly lowered. By virtue of this, accurate identification can be carried out.

Further, if the difference in position in the vertical direction between the fluid detection part and the fluid temperature detection part is not more than 2 mm, more accurate identification can be carried out.

According to the present invention, further, the exposed portion of the fluid detection part and the exposed portion of the fluid temperature detection part are covered with a hydrophilic film or a filter, and therefore, especially when the measuring target is an aqueous liquid, adhesion of bubbles to the outer surface of the identification sensor module is reduced. Moreover, because of covering with the filter, the identification target fluid hardly suffers forced flow due to an external factor. Consequently, an identification sensor module capable of enhancing measuring accuracy and a fluid identification device using the sensor module can be provided.

According to the present invention, furthermore, the waterproof case has a cover member that covers the container on the identification target fluid side, and inside the cover member, a passage of the identification target fluid is formed. Therefore, forced flow due to an external factor is hardly formed in the identification target fluid around the heat transfer member, such as gasoline, and a change in thermal influence of the identification target fluid such as gasoline on the heat transfer from the heating element to the temperature detector is small independent of inclination of the identification device. Hence, accuracy in the identification of a type of the identification target fluid such as gasoline can be enhanced.

According to the present invention, furthermore, identification of fluid type is carried out by measuring conductivity of the fluid between the temperature detector for fluid detection which is used for the detection of flow rate and the temperature detector for fluid temperature detection. Therefore, it can be prevented by a simple constitution that a fluid having apparently different conductivity from that of the identification target fluid that is a measuring target is allowed to flow by mistake.

According to the present invention, furthermore, the fluid identification device is constructed so as to perform identification of fluid type of the identification target fluid by applying a pulse voltage to the heating element of the fluid detection part for a given period of time to heat the identification target fluid, which is temporarily staying in the fluid type identification chamber, by means of the heating element of the fluid detection part, and identifying the fluid type of the identification target fluid by a voltage output difference corresponding to the temperature difference between the initial temperature of the temperature detector for fluid detection and the peak temperature thereof. Therefore, mere application of a pulse voltage for a given period of time is enough, and hence, it is possible to identify the type and the distillation property of the identification target fluid accurately and rapidly by heating for a short period of time without heating the identification target fluid such as light oil to its flash point.

That is to say, a correlation between the kinematic viscosity of the identification target fluid and the sensor output is utilized, natural convection is utilized, and an application voltage of 1 pulse is utilized. Therefore, it is possible to identify the type of the identification target fluid accurately and rapidly.

According to the present invention, furthermore, in the case of identification of the identification target fluid, such as fluid type detection or concentration detection, the identification of the identification target fluid can be accurately and rapidly carried out by closing the sub-passage on-off valve to allow the identification target fluid to temporarily stay in the fluid identification device.

On the other hand, in the case of detection of flow rate of the identification target fluid, the flow rate of the identification target fluid can be detected by opening the sub-passage on-off valve to allow the identification target fluid to flow to the fluid identification device.

Accordingly, simultaneously with detection of flow rate of the fluid, detection of, for example, fluid type and fluid concentration can be carried out accurately and rapidly, and besides, by the use of one fluid identification device, detection of fluid type, fluid concentration and the like can be carried out simultaneously with detection of flow rate of the fluid. Therefore, this identification system is compact, and if it is applied to an automobile system, the whole system can be made compact.

According to the present invention, when the identification target fluid is allowed to temporarily stay in the fluid identification detection chamber, flow of the identification target fluid in the fluid identification detection chamber is inhibited by the flow control plate, and flow of the identification target fluid that is present around the identification sensor module surrounded by the flow control plate and located in the flow control plate stops instantly.

Accordingly, in the fluid identification by the identification sensor module, flow of the identification target fluid does not occur, and disorder of the identification target fluid attributable to oscillation does not occur. Therefore, influence of the identification target fluid on the detection of, for example, fluid type and fluid concentration can be prevented, and accurate identification of the identification target fluid is possible.

Moreover, because the fluid identification detection chamber is installed, the quantity of the identification target fluid staying is increased, and therefore, in the detection of liquid type and concentration of the identification target fluid, accurate detection can be carried out without being influenced by the surrounding conditions such as external temperature.

Hence, in the case of application of the device to identification of fluids for automobiles such as gasoline and light oil, the liquid type and the concentration of the identification target fluid can be instantly detected by stopping a pump of gasoline or the like when the automobile is stopped for, for example, waiting for a signal, and after the detection, the automobile can be started by starting the pump. Therefore, any trouble is not given to traveling of the automobile.

According to the present invention, furthermore, identification of concentration of the identification target fluid is carried out by applying a pulse voltage to the heating element of the fluid detection part for a given period of time to heat the identification target fluid, which is temporarily staying in the fluid type identification chamber, by means of the heating element of the fluid detection part and identifying the concentration of the fluid by a voltage output difference corresponding to the temperature difference between the initial temperature of the temperature detector for fluid detection and the peak temperature thereof. Thus, mere application of a pulse voltage for a given period of time is enough, and therefore, concentration of the identification target fluid, such as a urea concentration of a urea solution, can be accurately and rapidly identified by heating for a short period of time.

That is to say, a relationship between the kinematic viscosity of the identification target fluid and the output of the sensor is utilized, natural convection is utilized, and an application voltage of 1 pulse is utilized. Therefore, it is possible to accurately and rapidly identify the urea concentration of the urea solution.

According to the present invention, furthermore, the voltage output difference can be accurately obtained based on the mean value of values of a given number of sampling times, and therefore, it is possible to accurately and rapidly identify the concentration of the identification target fluid.

According to the present invention, furthermore, the fluid identification device is placed in a measuring fine tube at the lower end of which the identification target fluid in a tank is introduced or discharged, and detection of leak of the identification target fluid is carried out by obtaining an output corresponding to a difference between temperatures detected by the temperature detector for fluid detection in the fluid detection part and the temperature detector for fluid temperature detection in the fluid temperature detection part, detecting a specific gravity of the identification target fluid in the tank based on the flow rate-corresponding value corresponding to the flow rate of the identification target fluid that is calculated using the obtained output, measuring a fluid level of the identification target fluid by the fluid level sensor module using the resulting specific gravity value, and detecting leak of the identification target fluid from the tank based on the magnitude of the time change rate of the fluid level. Therefore, when leak of the in-tank fluid is detected by variation of the fluid level measured by the fluid level sensor module, detection accuracy can be enhanced irrespective of specific gravity of the in-tank liquid.

According to the present invention, furthermore, detection of a fluid level of the identification target fluid is carried out by detecting a fluid pressure of the identification target fluid by the fluid level sensor module, calculating a temporary fluid level value based on the fluid pressure on the assumption that the identification target fluid is a fluid of a given density, applying a pulse voltage to the heating element of the fluid detection part for a given period of time to heat the identification target fluid by means of the heating element of the fluid detection part, identifying a concentration of the identification target fluid by a voltage output difference corresponding to the temperature difference between the initial temperature of the temperature detector for fluid detection and the peak temperature thereof, obtaining a density value of the identification target fluid based on the relationship between the identified concentration and the density of the identification target fluid, and calculating the fluid level of the identification target fluid based on the temporary fluid level value and the density value. Therefore, high-accuracy fluid level detection prevented from occurrence of detection error due to the density of the fluid becomes possible.

According to the present invention, furthermore, measurement of a quantity of ammonia generated is carried out by:

using a sensor equipped with a heating element and a temperature detector disposed in the vicinity of the heating element,
applying a pulse voltage to the heating element for a given period of time to heat the measuring target liquid by means of the heating element,
then, by an electrical output corresponding to electrical resistance of the temperature detector, measuring a thermal conductivity-corresponding output value which is an electrical output of the temperature detector dependent on the thermal conductivity of the measuring target liquid, and measuring a kinematic viscosity-corresponding output value which is an electrical output of the temperature detector dependent on the kinematic viscosity of the measuring target liquid,
calculating a urea concentration X% by weight and an ammonium formate concentration Y% by weight of the measuring target liquid, from the relationship between the thermal conductivity-corresponding output value and the kinematic viscosity-corresponding output value,
calculating a urea quantity A and an ammonium formate quantity B contained in the measuring target liquid, from the concentration and the quantity of the measuring target liquid, and
determining the quantity of ammonia generated, by the following formula:

$$\text{Quantity of ammonia generated} = X \times A + Y \times B$$

Therefore, there can be provided a device for measuring a quantity of ammonia generated and a method for measuring a quantity of ammonia generated, by the use of which the concentration of a mixed solution of a urea aqueous solution and an ammonium formate solution in a urea tank and the quantity of ammonia generated can be accurately and rapidly grasped when the mixed solution is used instead of a urea aqueous solution in the exhaust gas purification catalyst device, and as a result, the mixed solution can be maintained in a given concentration, and the quantity of NOx in the exhaust gas can be remarkably decreased by reduction.

BRIEF DESCRIPTION OF THE DRAWINGS

Fig. 1 is a schematic sectional view showing an embodiment wherein a liquid type identification device is used as an example of the fluid identification device of the present invention.
Fig. 2 is a schematic sectional view of an identification sensor module of the liquid type identification device of Fig. 1.
Fig. 3 is a schematic sectional view showing a liquid type detection part of the liquid type identification device of Fig. 1.
Fig. 4 is schematic sectional view showing a situation in which the liquid type identification device of Fig. 1 is used.
Fig. 5 is an exploded perspective view of a thin film chip of an indirect-heated liquid type detection part.
Fig 6 is a constitutional view of a circuit for liquid type identification.
Fig. 7 is a view showing a relationship between a single pulse voltage P applied to a heating element and a sensor output Q.
Fig. 8 is a view indicating that a liquid type-corresponding first voltage value of a sugar aqueous solution having a sugar concentration of a certain range is present in the region of a liquid type-corresponding first voltage value V01 obtained from a urea aqueous solution having a urea concentration of a given range.
Fig. 9 is a view showing liquid type-corresponding first voltage values V01 and liquid type-corresponding second voltage values V02 of a urea aqueous solution, a sugar aqueous solution and water, which are expressed in relative values given when the voltage value of a urea aqueous solution having a urea concentration of 30% is 1,000.
Fig. 10 is a view showing examples of first calibration curves.
Fig. 11 is a view showing examples of second calibration curves.
Fig. 12 is a view showing examples of liquid temperature-corresponding output values T.
Fig. 13 is a graph schematically indicating that the criterion for identifying a given liquid by a combination of a liquid type-corresponding first voltage value V01 and a liquid type-corresponding second voltage value V02 varies according to the temperature.
Fig. 14 is a flow chart showing a liquid type identification process.
Fig. 15 is a group of sectional views showing an embodiment wherein a fluid identification device using a cover member 2d is applied to a gasoline identification device.
Fig. 16 is a graph showing a change of a gasoline type-corresponding voltage value V0 obtained when an inclination angle of a measuring part is changed in the fluid identification device of the embodiment (having cover member 2d) of Fig. 15.
Fig. 17 is a graph showing a change of a gasoline type-corresponding voltage value V0 obtained when an inclination angle is changed in a comparative embodiment.
Fig. 18 is a constitutional schematic view of a series circuit that is used instead of a parallel circuit of Fig. 6 in an embodiment.
Fig. 19 is a graph showing examples of outputs A (i.e., results of liquid temperature detection) obtained when a change-over switch shown in Fig. 18 is connected to the a side.
Fig. 20 is a sectional view of an identification sensor module of another embodiment of the present invention.
Fig. 21 shows an identification sensor module of the embodiment of Fig. 20; Fig. 21(A) is a schematic view showing the interior of the identification sensor module of Fig. 20; and Fig. 21(B) is a partial enlarged sectional view of the identification sensor module viewed along the A direction of Fig. 20.

Fig. 22 shows an identification sensor module of another embodiment of the present invention; Fig. 22(A) is a perspective view of the identification sensor module of the another embodiment of the present invention; and Fig. 22(B) is a schematic view showing a situation in which the identification sensor module of Fig. 22(A) is installed.
Fig. 23 is a vertical sectional view of the identification sensor module of Fig. 22 viewed along the B direction.
Fig. 24 is a graph to explain another embodiment using the fluid identification device of the present invention.
Fig. 25 is a graph to explain another embodiment using the fluid identification device of the present invention.
Fig. 26 is a circuit constitutional view of an embodiment wherein the fluid identification device of the present invention is used as a flow meter.
Fig. 27 is a schematic sectional view of an embodiment of a liquid type identification device for light oil of the present invention.
Fig. 28 is a graph showing a time-voltage relationship illustrating a liquid type identification method using the liquid type identification device for light oil of the present invention.
Fig. 29 is a graph showing a relationship between a kinematic viscosity and a sensor output.
Fig. 30 is a graph showing a relationship between a kinematic viscosity and a distillation temperature.
Fig. 31 is a graph showing a relationship between a sensor output and a distillation temperature.
Fig. 32 is a graph showing calibration curves illustrating a liquid type identification method using the liquid type identification device for light oil of the present invention.
Fig. 33 is a graph showing distillation properties of a light oil.
Fig. 34 is a schematic view of another embodiment wherein the fluid identification device of the present invention is used as a flow rate/liquid type detection device.
Fig. 35 is a graph showing calibration curves illustrating a flow rate detection method using the flow rate/liquid type detection device of Fig. 34.
Fig. 36 is an exploded perspective view of the whole of another embodiment wherein the fluid identification device of the present invention is used as a liquid type detection device.
Fig. 37 is an exploded perspective view of a liquid type detection chamber of the liquid type detection device of Fig. 36.
Fig. 38 is a schematic view to explain detection in the liquid type detection chamber of the liquid type detection device of Fig. 36.
Fig. 39 is a schematic view of an embodiment wherein a urea concentration identification device for urea solution of the present invention is applied to an automobile system.
Fig. 40 is a partial broken perspective view to explain an embodiment wherein the fluid identification device of the present invention is used as a leak detection device for in-tank liquid.
Fig. 41 is a partly omitted sectional view of the leak detection device of the above embodiment.
Fig. 42 is a timing view showing a relationship between a voltage Q applied to a thin film heating element from a pulse voltage generation circuit and a voltage output S of a leak detection circuit.
Fig. 43 is a view showing a specific example of a relationship between a voltage Q applied to a thin film heating element and a voltage output S of a leak detection circuit.
Fig. 44 is a view showing specific examples of relationships between a liquid level change rate and an integrated value $\int(S0-S)dt$.
Fig. 45 is a view showing a specific example of a relationship between a liquid level change rate and a time change rate P' of a liquid level-corresponding output.
Fig. 46 is a flow chart of specific gravity detection.
Fig. 47 is an exploded perspective view showing another embodiment wherein the fluid identification device of the present invention is used as a liquid level detection device.
Fig. 48 is a partly omitted sectional view of Fig. 47.
Fig. 49 is a view showing a situation in which the fluid identification device of the present invention is installed in a tank.
Fig. 50 is a flow chart showing a process of liquid level detection by a microcomputer.
Fig. 51 is a group of schematic sectional views each of which shows a protruded portion, a depressed portion and the vicinity of a liquid type detection part.
Fig. 52 is a group of schematic sectional views each of which shows a protruded portion, a depressed portion and the vicinity of a liquid type detection part.
Fig. 53 is a schematic perspective view showing an embodiment of an identification sensor module.
Fig. 54 is a schematic sectional perspective view of the embodiment of Fig. 53.
Fig. 55 is a schematic sectional view showing a protruded portion, a depressed portion and the vicinity of a liquid type detection part.
Fig. 56 is a schematic sectional view of another identification sensor module of a liquid type identification device.
Fig. 57 is a schematic sectional view showing another liquid type detection part of a liquid type identification device.
Fig. 58 is a schematic perspective view showing a lead of a liquid type identification device.
Fig. 59 is a group of schematic views showing modification examples of leads.

Fig. 60 is a group of schematic views showing modification examples of leads.

Fig. 61 is a schematic view showing a modification example of a lead.

Fig. 62 is a schematic perspective view showing a modification example of a liquid type detection part.

Fig. 63 is a schematic sectional view showing a fluid identification device.

Fig. 64 is a schematic sectional view showing an embodiment wherein the fluid identification device of the present invention is used as a device for measuring a quantity of ammonia generated.

Fig. 65 is a view showing examples of a first and a second calibration curves.

Fig. 66 is a schematic sectional view showing another embodiment wherein the fluid identification device of the present invention is used as a device for measuring a quantity of ammonia generated, and is a schematic sectional view showing a device for measuring a quantity of ammonia generated in the case where a differential pressure sensor 300 is installed in addition to an identification sensor module 2.

Fig. 67 is a view showing examples of a third and a fourth calibration curves.

Description of symbols

1: liquid type identification device
2: identification sensor module
2a: base
2b: O-ring
2c: O-ring
2d: cover member
3: liquid level sensor module
4: waterproof case
4a: mounting part
5: waterproof wiring
6: sensor holder .
6a: sensor loading hole
7: fixing screw
8: filter holder
9: filter
11: flow rate/liquid type detection sensor device
12: liquid type identification device main body
12a: filter
12b: filter cover
13: resistor
13a: sensor holder
13b: measuring fine tube
14: change-over switch
15: circuit container part
15a: leak detection control part
16: cap
16a: air passage
17: wiring
18: light oil inlet passage
20: container
20A: container main body
20A1: protruded portion
20A2: protruded portion
20B: container cap
21: liquid type detection part
21a: thin film chip for liquid type detection (thin film chip)
21a1: chip substrate
21a2: temperature detector
21a3: layer insulating film
21a4: heating element
21a5: heating element electrode

21a6: protective film
21a7: electrode pad
21c: metallic fin
21d: bonding wire
21e: outer electrode terminal (lead)
22: liquid temperature detection part
22a2: temperature detector
22c: metallic fin
22e: outer electrode terminal
23: synthetic resin mold
23a: synthetic resin
24: measuring target liquid inlet passage
25: liquid type detection circuit board
26: custom IC
27: terminal pin
31: terminal pin
32: temperature detector for flow rate detection
32a: temperature detector for temperature compensation
33: thin film heating element
36: fin
40: measuring part
41: power circuit part
41a: circuit board
42: flow rate detection part
44: fin plate for flow rate detection
44a: fin plate for fluid temperature detection
49: electrode terminal
49a: electrode terminal
50: hydrophilic film
51: connector
54: light oil exit port
62: resistor
63: voltmeter
64: resistor
65: A/D converter
66: resistor
68: bridge circuit
70: differential amplification circuit
71: liquid temperature detection amplifier
72: microcomputer
73: bridge circuit
74: switch
75: differential amplification circuit
76: output buffer circuit
77: integration circuit
78: V/F conversion circuit
79: temperature compensation type quartz oscillator
80: reference frequency generation circuit
81: transistor
82: pulse counter
83: microcomputer
84: memory
85: display part
90: power circuit
92: resistor
94: variable resistor
100: tank
101: wall member

110: liquid supply pump for urea water
116: catalyst device
130: urea solution supply mechanism
132: urea solution tank
133: first temperature sensor
134: second temperature sensor
135: heater
136: third temperature sensor
137: pressure sensor
138: on-off vale
138a: valve body
140: NOx sensor
142: NOx sensor
200: ASIC substrate
202: packing
204: connector
206: fixing member
208: fixing screw
210: packing
212: identification target fluid
300: differential pressure sensor
300a: first inlet
300b: second inlet
301: terminal pin
400: light oil liquid type identification chamber
402: opening for liquid type identification sensor
404: liquid type identification sensor
405: liquid type identification sensor heater
406: liquid type detection sensor heater
408: lead electrode
410: liquid temperature sensor
412: mold resin
416: check valve
417: main passage on-off valve
418: orifice
419: sensor control device
420: flow rate/liquid type detection device
422: main passage
424: sub-passage
426: sub-passage on-off valve
428: ECU
430: liquid type detection device
432: liquid type detection device main body
434: liquid type detection chamber
436: first passage
438: second passage
440: fluid entry passage
442: fluid exit port
444: cap member for liquid type detection chamber
446: opening for liquid type detection sensor
448: liquid type detection sensor
450: circuit board member
452: outer cap member
454a: mounting flange
454b: mounting flange
456: flow control plate
458: plate member
460: side plate member

462: side plate member
464: cover plate member
466: fluid inlet
468: fluid outlet
470: sidewall
472: liquid temperature sensor
473: liquid temperature sensor heater
474: lead electrode
480: automobile system
482: urea concentration identification device
490: tank
492: metering port
494: liquid injection port
496: top plate
498: liquid supply port
500: side plate
502: bottom plate
504: leak detection device
506: liquid inlet-outlet part
508: flow rate measuring part
510: liquid reservoir part
512: sheathing tube
520: liquid tank for urea water
522: opening
523: liquid level detection device
524: inlet pipe
526: outlet pipe
528: identification sensor part
530: pressure sensor
532: supporting part
540: circuit board
542: cap member
544: wiring
546: wiring
548: wiring
550: connector
560: bent portion
562: bent portion
564: first linear portion
566: second linear portion
568: depressed portion
570: depressed portion

## BEST MODE FOR CARRYING OUT THE INVENTION

Modes for carrying out the present invention (embodiments of the present invention) are described in more detail hereinafter referring to the drawings.

Fig. 1 is a schematic sectional view showing an embodiment wherein a liquid type identification device is used as an example of the fluid identification device of the invention; Fig. 2 is a schematic sectional view of an identification sensor module of the device; and Fig. 3 is a schematic sectional view showing a liquid type detection part of the device. Fig. 4 is a schematic sectional view showing a situation in which the liquid type identification device of this embodiment is used. In this embodiment, a urea aqueous solution having a urea concentration of a given range is supposed to be a given liquid.

As shown in Fig. 4, the liquid type identification device 1 is mounted on a wall member 11 for constituting a dosing pipe unit disposed inside a tank 100 of a urea aqueous solution for NOx decomposition, said tank constituting an exhaust gas purification system loaded on, for example, an automobile. This mounting can be carried out by screwing or banding. As shown in Fig. 1 and Fig. 4, the liquid type identification device 1 has an identification sensor module 2, a liquid level sensor module 3, a waterproof case 4 and a waterproof wiring 5.

As shown in Fig. 2, the identification sensor module 2 comprises an indirect-heated liquid type detection part 21, a liquid temperature detection part 22, a liquid type detection circuit board 25 and a custom IC (ASIC) 26, which are contained in a container 20. The container 20 comprises an integral main body 20A made of an anticorrosive material, e.g., a metal such as stainless steel, and an integral cap 20B similarly made of a metal such as stainless steel, said main body 20A and said cap 20B being joined to each other. This joining can be carried out by, for example, caulking. As shown in Fig. 1, the joint between the main body 20A and the cap 20B of the container is present inside the waterproof case 4.

On the bottom (in Fig. 1 and Fig. 2, the right hand side portion; in Fig. 3, the lower side portion) of the container main body 20A, two protruded portions 20A1 and 20A2 are formed, as shown in Fig. 2, and in depressed portions on the container interior side corresponding to these protruded portions, the liquid type detection part 21 and the liquid temperature detection part 22 are disposed, respectively.
The liquid type detection part 21 and the liquid temperature detection part 22 are disposed apart from each other at a given distance in the longitudinal direction.

The liquid type detection part 21 and the liquid temperature detection part 22 may be disposed so as to be side by side apart from each other at a given distance in the horizontal direction (in Fig. 1 and Fig. 4, the perpendicular direction to the paper surface is the horizontal direction; in Fig. 63, the transverse direction on the paper surface is the horizontal direction; in Fig. 2, the longitudinal direction on the paper surface is the horizontal direction). The liquid type identification device 1 is installed in the urea aqueous solution tank 100 in such a manner as shown in Fig. 4. That is to say, the liquid type detection part 21 and the liquid temperature detection part 22 may be disposed so as to be side by side apart from each other at a given distance in the horizontal direction, like the relationship between the protruded portions 20A2 and 20A1 in Fig. 63.

The liquid type detection part 21 and the liquid temperature detection part 22 are disposed apart from each other at a given distance in the horizontal direction, as described above, but regarding their relationship in the vertical direction, they may be slightly shifted in the vertical direction.

That is to say, the liquid type detection part 21 and the liquid temperature detection part 22 have only to be held by a holding means so that a difference in position in the vertical direction between them may be not more than 2 mm, preferably not more than 1 mm, most preferably 0 mm. The holding means comprises the container 20, the waterproof case 4 and a mounting member for mounting the waterproof case 4 onto the tank 100 in which the measuring target fluid is to be contained. The mounting member used herein is a means to mount the waterproof case onto the wall member 101, such as the screwing means or the banding means. The mounting member may be any other means provided that the difference in position in the vertical direction between the liquid type detection part 21 and the liquid temperature detection part 22 becomes not more than 2 mm, preferably not more than 1 mm, most preferably 0 mm, when the waterproof case 4 is mounted onto the tank 100. That is to say, in the present invention, the liquid type detection part 21 and the liquid temperature detection part 22 have only to be disposed so as to be side by side apart from each other at a given distance in the horizontal direction to the fluid level of the measuring target fluid. In the present invention, further, the difference in position (height) in the vertical direction between the liquid type detection part 21 and the liquid temperature detection part 22 is most preferably zero, but if the difference is not more than 2 mm, the later-described effect of the present invention can be obtained.

As shown in Fig. 3, the liquid type detection part 21 is formed by embedding a liquid type detection thin film chip 21a, which is obtained by forming a liquid type detection temperature detector made of a thin film on a chip substrate as described later, in a synthetic resin mold 23 in such a manner that one surface of the thin film chip is exposed. The synthetic resin mold 23 is composed of, for example, an epoxy resin. The exposed surface (in Fig. 2, surface on the right hand side; in Fig. 3, surface on the lower side) of the thin film chip 21a of the liquid type detection part is in contact with the inner surface of the depressed portion of the container main body 20A.

Fig. 51 is a group of schematic sectional views each of which shows the protruded portion 20A1, the depressed portion 568 and the vicinity of the liquid type detection part 21. In Fig. 51, however, the shape of a lead 21e is different from that shown in Fig. 3. In Fig. 51, (a) shows a longitudinal section, and (b) shows a transverse section. Using Fig. 51, the dimensional relationship between the depressed portion 568 and the synthetic resin mold 23 is described.

As shown in this figure, regarding the planar dimensions of the depressed portion 568, the width is X, and the length is Y. The height of the depressed portion 568 is Z. Regarding the planar dimensions of the synthetic resin mold 23, the width is X', and the length is Y'. The height of the synthetic resin mold 23 is Z'. The synthetic resin mold 23 is disposed on the bottom surface of the depressed portion 568, and therefore, $X' \leq X$ and $Y' \leq Y$. Z' may be smaller than Z, or may be equal to Z, or may be larger than Z. In Fig. 2 and Fig. 3, a case where Z' is larger than Z is shown, and in Fig. 51, a case where Z' is smaller than Z is shown.

Examples of these dimensions are given below.
X: 5 mm to 30 mm
Y: 5 mm to 30 mm
Z: 2 mm to 10 mm

X': 4 mm to 15 mm
Y': 4 mm to 15 mm
Z': 2 mm to 5 mm

In the present embodiment, the liquid type detection part 21 is disposed in contact with the inner surface of the depressed portion 568 of the container main body 20A, and the depressed portion 568 is formed correspondingly to the protruded portion 20A1, as described above.

On this account, the surface area of the container main body 20A contributing to heat exchange between the liquid type detection thin film chip 21a and the urea aqueous solution US through the container main body 20A is increased. Therefore, efficiency of the heat exchange is enhanced, and the identification accuracy is improved.

In order to further enhance efficiency of the heat exchange, a synthetic resin mold having as high thermal conductivity as possible is preferably used as the synthetic resin mold 23. For such a purpose, a synthetic resin mold containing a filler such as glass fiber can be used as the synthetic resin mold 23.

By virtue of this, the synthetic resin mold 23 can be made to have a thermal conductivity of, for example, about 0.7 to 1.2 W/m·K, and the synthetic resin mold 23 can be also used as a heat transfer route for the heat exchange between the liquid type detection thin film chip 21a and the urea aqueous solution US through the container main body 20A.

In the present embodiment, the depressed portion 568 is formed correspondingly to the protruded portion 20A1, but whether the depressed portion 568 is formed or not can be properly selected. From the viewpoint of enhancing the efficiency of the heat exchange as above, however, the depressed portion 568 is preferably formed correspondingly to the protruded portion 21A1.

The shape of the protruded portion 20A1 is not limited to a rectangular parallelepiped, and for example, the transverse section may be circular, elliptic, elongated circular, or pentagonal or higher polygonal. The tip of the protruded portion 20A1 may be slanted.

The shape of the depressed portion 568 is not limited to a rectangular parallelepiped, and for example, the transverse section may be circular, elliptic, elongated circular, or pentagonal or higher polygonal. The bottom surface of the depressed portion 568 may be slanted against the side surface.

The shape of the protruded portion 20A1 and the shape of the depressed portion 568 may be different from each other, and their combination can be properly selected.

Fig. 52 is a group of schematic sectional views each of which shows the protruded portion 20A1, the depressed portion 568 and the vicinity of the liquid type detection part 21. In Fig. 52, (a) shows a longitudinal section, and (b) shows a transverse section.

In this figure, the contact mode of the synthetic resin mold 23 with the depressed portion 568 is different from that shown in Fig. 2, Fig. 3 and Fig. 51. That is to say, in the mode shown in Fig. 2, Fig. 3 and Fig. 51, the liquid type detection part 21 is disposed so that the liquid type detection chip 21a may be in contact with the bottom surface of the depressed portion 568.

On the other hand, in the mode shown in Fig. 52, the liquid type detection part 21 is disposed so that the liquid type detection chip 21a may be in contact with the side surface of the depressed portion 568.

Also in the embodiment of Fig. 52, regarding the planar dimensions of the depressed portion 568, the width is X, and the length is Y. The height of the depressed portion 568 is Z. Regarding the planar dimensions of the synthetic resin mold 23, the width is X', and the length is Y'. The height of the synthetic resin mold 23 is Z'. The synthetic resin mold 23 is disposed on the longitudinal side surface of the depressed portion 568, and therefore, X'≤X and Y'≤Y. Z' may be smaller than Z, or may be equal to Z, or may be larger than Z. In Fig. 52, a case where Z' is smaller than Z is shown.

Examples of these dimensions are given below.

X: 2 mm to 10 mm
Y: 10 mm to 20 mm
Z: 10 mm to 30 mm
X': 2 mm to 5 mm
Y': 5 mm to 15 mm
Z': 4 mm to 10 mm

In the depressed portion 568, the depth Z is preferably larger than X that is a smaller dimension (i.e., dimension in the transverse direction crossing the longitudinal side surface at right angles) between the planer dimensions. By virtue of this, the protrusion quantity of the protruded portion 20A1 and further the surface area thereof are much more increased. Therefore, the efficiency of the heat exchange between the liquid type detection thin film chip 21a and the urea aqueous solution US through the container main body 20A can be enhanced, and the identification accuracy is improved.

Fig. 5 is an exploded perspective view of a liquid type detection thin film chip 21a of an indirect-heated liquid type detection part 21. The liquid type detection thin film chip 21a consists of a chip substrate 21a1 made of $Al_2O_3$, a liquid type detection temperature detector 21a2 made of Pt, a layer insulating film 21a3 made of $SiO_2$, a heating element 21a4 made of $TaSiO_2$, a heating element electrode 21a5 made of Ni, a protective film 21a6 made of $SiO_2$ and an electrode

pad 21a7 made of Ti/Au, which are properly laminated in order. The liquid type detection temperature detector 21a2 is formed in a zigzag pattern though the pattern is not shown in the figure.

The electrode pad 21a7 connected to the liquid type detection temperature detector 21a2 and to the heating element electrode 21a5 is connected to an outer electrode terminal 21e through a bonding wire 21d, as shown in Fig. 3.

As shown in Fig. 3, one end (in Fig. 3, end on the lower side) of the lead 21e is connected to the synthetic resin mold 23, that is, comes into the synthetic resin mold 23, and is electrically connected to the liquid type detection chip 21a through the bonding wire 21d. The other end (in Fig. 3, end on the upper side) of the lead 21e is connected to a liquid type detection circuit board 25, that is, fixed to the liquid type detection circuit board 25.

The end of the lead 21e connected to the liquid type detection circuit board 25 penetrates the liquid type detection circuit board 25 from the front surface side to the back surface side (in Fig. 3, from the lower side to the upper side of the liquid type detection circuit board 25) and projects from the back surface side. The projecting end is connected to the liquid type detection circuit board 25 at its back surface side (in Fig. 3, upper side) by, for example, soldering.

In Fig. 3, the end of the lead 21e is connected to the back surface of the liquid type detection circuit board 25, but it is possible to connect it to the front surface or both surfaces thereof.

However, in the case where the lead 21a has a spring structure as described later, a burden on the end of the lead 21e occurs, and therefore, it is preferable to connect the end of the lead to the back surface of the liquid type detection circuit board 25 also for the purpose of preventing short circuit.

By virtue of this, not only mechanical connection but also necessary electrical connection to the circuit formed in the liquid type detection circuit board 25 is made.

Also the liquid temperature detection part 22 can have the same constitution as that of the liquid type detection part 21. In this part, however, only the temperature detector (temperature detector for liquid temperature detection in the liquid temperature detection part 22) is allowed to function without allowing the heating element to function. In Fig. 2, the outer electrode terminal of the liquid temperature detection part 22 is designated by a symbol 22e. As the liquid temperature detection part 22, one provided with no heating element may be used differently from the liquid type detection part.

As shown in Fig. 2, the outer electrode terminal 21e of the liquid type detection part 21 and the outer electrode terminal 22e of the liquid temperature detection part 22 are each connected to the circuit of the liquid type detection circuit board 25. The liquid type detection circuit board 25 is equipped with a terminal pin 27. The terminal pin 27 penetrates the container cap 20B and extends out of the container. As described later, the custom IC 26 is constituted of a part of the liquid type detection circuit and an identification operation part.

Fig. 53 is a schematic perspective view showing another embodiment of the identification sensor module. Fig. 54 is a schematic sectional perspective view of the embodiment. Fig. 55 is a schematic sectional view showing a protruded portion 20A1, a depressed portion 568 and the vicinity of a liquid type detection part 21 of the embodiment. In Fig. 55, however, the shape of one end of the lead 21a is simplified and illustrated linearly.

In this embodiment, both of main surfaces of the synthetic resin mold 23 (i.e., surface where the liquid type detection thin film chip 21a is exposed and surface on the opposite side) are in contact with the side surfaces of the depressed portion 568.

That is to say, the dimension of the synthetic resin mold 23 in the direction crossing both the main surfaces at right angles is almost equal to the dimension of the depressed portion 568. The dimension of the synthetic resin mold 23 in the direction crossing the paper surface of Fig. 55 at right angles is slightly smaller than the dimension of the depressed portion 56. The height of the synthetic resin mold 23 is slightly smaller than the height of the depressed portion 568.

In the present embodiment, the synthetic resin mold is in such a form as nearly fills the interior of the depressed portion 568, as described above. By virtue of this, the heat exchange between the liquid type detection thin film chip 21a and the urea aqueous solution US is carried out through the container main body 20A and further through most of the synthetic resin mold 23. Therefore, efficiency of the heat exchange is enhanced, and the identification accuracy is further improved.

The liquid level sensor module 3 comprises a hitherto publicly known pressure sensor, and the sensor module detects a water pressure received from an in-tank liquid and outputs its detection signal (corresponding to liquid level) from a terminal pin 31.

As shown in Fig. 1, a power circuit part 41 is disposed in the waterproof case 4, and the power circuit part 41 is supported by a supporting means that is not shown in the figure. The power circuit part 41 comprises a circuit board 41a and a necessary circuit element mounted thereon, and forms, for example, a direct current 5V suitable for driving each circuit of the liquid type identification device 1 based on, for example, a direct current 24V supplied from an external power source. To the circuit of the circuit board 41a, the terminal pin 27 of the identification sensor module 2 and the terminal pin 31 of the liquid level sensor module 3 are connected.

As shown in Fig. 1 and Fig. 4, the waterproof case 4 is provided with a cover member 2d so that the container main body 20A that protrudes from the waterproof case may be surrounded by the cover member. By virtue of the cover member 2d, there is formed a measuring target liquid inlet passage 24 whose upper and lower ends are open and which extends in the longitudinal direction (vertical direction) through a region that is close to the liquid type detection part 21 and the liquid temperature detection part 22 and is located outside the container main body 20A. That is to say, the liquid type detection part 21 and the liquid temperature detection part 22 are disposed so that the difference in position in the direction of the measuring target liquid inlet passage 24 (vertical direction) between the liquid type detection part 21 and the liquid temperature detection part 22 may be not more than 2 mm, preferably not more than 1 mm, particularly preferably 0 mm.

The waterproof wiring 5 extends upward from the waterproof case 4 and penetrates a top plate of the tank 100, and its end is located outside the tank. The end of the waterproof wiring 5 is provided with a connector 51 for making connection to an external circuit. The waterproof wiring 5 includes a feeder to the power circuit part 41 and output signal lines respectively from the identification sensor module 2 and the liquid level sensor module 3 through the circuit board 41a.

In Fig. 6, constitution of a circuit for the liquid type identification in the present embodiment is shown. A bridge circuit (liquid type detection circuit) 68 is formed from the temperature detector 21a2 of the indirect-heated liquid type detection part 21, the temperature detector 22a2 of the liquid temperature detection part 22, and two resistors 64, 66. The output of the bridge circuit 68 is inputted into a differential amplifier 70, and the output of the differential amplifier (also referred to as "liquid type detection circuit output" or "sensor output") is inputted, through an A/D converter (not shown), into a microcomputer (MICMPTR) 72 that constitutes an identification operation part.

Into the microcomputer 72, a liquid temperature-corresponding output value corresponding to a temperature of the measuring target liquid is inputted from the temperature detector 22a2 of the liquid temperature detection part 22 through a liquid temperature detection amplifier 71. On the other hand, from the microcomputer 72, a heater control signal to control switching of a switch 74 located on an electrical conduction route to the heating element 21a4 of the indirect-heated liquid type detection part 21 is outputted.

In the present embodiment, the part surrounded with an alternate long and short dash line in Fig. 6 forms the custom IC 26.

In Fig. 6, the switch 74 is described as a switch that performs mere switching, for simplification, but in the formation of the custom IC 26, it is possible that plural voltage application routes capable of applying voltages different from one another are formed and any one of the voltage application routes is selected for the heater control. By virtue of this, the range of selection of properties of the heating element 21a4 of the liquid type detection part 21 can be greatly widened. That is to say, a voltage that is optimum for the identification can be applied according to the properties of the heating element 21a4. Further, because application of plural voltages different from one another can be carried out in the heater control, the types of the identification target liquids can be increased.

In Fig. 6, the resistors 64, 66 are described as resistors whose resistance value is constant, for simplification, but in the formation of the custom IC 26, it is possible that resistors 64, 66 whose resistance value is variable are formed and the resistance values of the resistors 64, 66 are properly changed in the identification. In the formation of the custom IC 26, similarly to the above, it is possible that the differential amplifier 70 and the liquid temperature detection amplifier 71 capable of being controlled in their properties are formed and the properties of the amplifiers are properly changed in the identification.

By virtue of this, optimum properties of the liquid type detection circuit can be easily set. Therefore, variability of identification properties, which occurs based on the individual variability of the liquid type detection part 21 and the liquid temperature detection part 22 in manufacturing and the individual variability of the custom IC 26 in manufacturing, can be reduced, and the manufacturing yield is enhanced.

In the above embodiment, the lead 21e is linear and is connected to the circuit board 25, but the shape of the lead is not limited thereto and may be, for example, the following one.

As shown in Fig. 56, Fig. 57 and Fig. 58, the lead 21e in another embodiment is in the form of a batten, and the lead 21e has a first linear portion 564 close to one end and a second linear portion 566 close to the other end and further has a bent portion 560 located between the first linear portion 564 and the second linear portion 566. The bent portion 560 has a V-shaped section, as shown in the figure.

The term "linear" used herein means linear in the longitudinal direction in Fig. 56, Fig. 57 and Fig. 58, and the second linear portion 566 is located on the extension of the first linear portion 564.

In Fig. 58, the boundary between a portion of the lead 21e inserted in the synthetic resin mold 23 and a portion thereof that is not inserted in the synthetic resin mold 23 is designated by a symbol 21e1, and the boundary between a portion of the lead 21e fixed to the liquid type detection circuit board 25 and a portion thereof that is not fixed is designated by a symbol 21e2. In the lead 21e, the portion between these two boundaries 21e1, 21e2 forms a spring structure, and in particular, the bent portion 560 forms a main body of the spring structure.

This spring structure functions as a compression plate spring, that is, the lead 21e is held in a state where it is compressed longitudinally between the synthetic resin mold 23 and the liquid type detection circuit board 25 (i.e., in a state where the longitudinal dimension of the bent portion is shortened), whereby the synthetic resin mold 23 is biased toward the inner surface of the container 20 (specifically, inner surface of the container main body 20A).

Therefore, even if the longitudinal distance between the synthetic resin mold 23 and the liquid type detection circuit board 25 is changed by the environmental conditions such as temperature change, the contact of the synthetic resin mold 23, particularly the exposed surface of the liquid type detection thin film chip 21a embedded therein, with the inner surface of the container main body 20A can be surely maintained. Hence, the later-described liquid type identification operations can be stably maintained over a long period of time.

In particular, deformation of the spring is mainly made by the bent portion 560, while the first linear portion 564 and the second linear portion 566 are on the same straight line and are hardly deformed. Therefore, on the synthetic resin mold 23, only a substantially downward force acts, and bending moment does not act. On this account, a fear of damaging the synthetic resin mold is further reduced.

As a material of the lead 21e, a material having electrical conductivity necessary for an electrode terminal and capable of realizing desired spring properties is used. Examples of such materials include Cu-Ni alloy and phosphor bronze. The thickness of the lead 21e is, for example, 0.05 to 0.3 mm.

Fig. 59, Fig. 60 and Fig. 61 show modification examples of the leads 21e. In these figures, portions between the two boundaries 21e1, 21e2, which constitute the spring structure, are only shown.

In Fig. 59, (a) shows an example of the lead whose bent portion 560 has a U-shaped section, (b) shows an example of the lead whose bent portion 560 is bent more gently as a whole than the above bent portion 560 and has a V-shaped section, and (c) shows an example of the lead whose bent portion is bent gently as a whole and has a U-shaped section.

In Fig. 60, (a) shows an example of the lead in which the bending direction of the bent portion 560 is opposite to that of Fig. 58, (b) shows an example of the lead in which the bending direction of the bent portion 560 is opposite to that of Fig. 59(a), (c) shows an example of the lead in which the bending direction of the bent portion 560 is opposite to that of Fig. 59(b), and (d) shows an example of the lead in which the bending direction of the bent portion 560 is opposite to that of Fig. 59(c).

In the above embodiment and the modification examples thereof, the number of bending times (i.e., number of folding times) is 1, but the number of bending times may be a plural number. In this case, the lead can be, for example, a lead in which the bent portion shown in Fig. 58 and the bent portion shown in Fig. 60(a) are alternately disposed in the longitudinal direction.

Fig. 61 shows an example of the lead 21e which comprises a linear member and whose bent portion 560 is in the form of a coil. In this modification example, the spring structure functions as a compression coil spring.

Fig. 62 is a schematic perspective view showing a modification example of the liquid type detection part 21. In this modification example, the direction of a batten of the batten lead 21a and the bending direction of the bent portion 560 differ from those of the above embodiment.

The liquid temperature detection part 22 can have the same constitution as that of the liquid type detection part 21, and particularly by the use of a lead having a spring structure that biases the synthetic resin mold toward the inner surface of the container as the lead for constituting the liquid temperature detection part 22, the below-described liquid type identification operations can be more stably maintained over a long period of time.

Next, the liquid type identification operations in the present embodiment are described.

When the measuring target liquid US is introduced into the tank 100, the measuring target liquid inlet passage 24 that is formed by the cover member 2d covering the identification sensor module 2 is also filled with the urea aqueous solution US. The measuring target liquid US in the tank 100 and in the urea aqueous solution inlet passage 24 does not substantially flow.

By a heater control signal outputted from the microcomputer 72 to the switch 74, the switch 74 is closed for a given period of time (e.g., 8 seconds) to apply a single pulse voltage P of a given height (e.g., 10 V) to the heating element 21a4 and thereby allow the heating element to generate heat. In this case, the output voltage (sensor output) Q of the differential amplifier 70 gradually increases during the voltage application to the heating element 21a4 and gradually decreases after completion of the voltage application to the heating element 21a4, as shown in Fig. 7.

In the microcomputer 72, sampling of a sensor output is carried out a given number of times (e.g., 256 times) for a given period of time (e.g., 0.1 second) before the beginning of the voltage application to the heating element 21a4, and an operation to determine their mean value is carried out to obtain a mean initial voltage value V1. This mean initial voltage value V1 corresponds to an initial temperature of the temperature detector 21a2.

As shown in Fig. 7, after the lapse of a first period of time (e.g., not more than 1/2 of the single pulse application time and specifically 0.5 to 3 seconds; in Fig. 7, 2 seconds) that is a relatively short period of time from the beginning of the voltage application to the heating element, specifically, immediately before the lapse of the first period of time, sampling of a sensor output is carried out a given number of times (e.g., 256 times), and an operation to determine their mean value is carried out to obtain a mean first voltage value V2. This mean first voltage value V2 corresponds to a first temperature of the temperature detector 21a2 after the lapse of the first period of time from the beginning of the single pulse application. Then, a difference V01 (=V2-V1) between the mean initial voltage value V1 and the mean first voltage value V2 is obtained as a liquid type-corresponding first voltage value.

As shown in Fig. 7, after the lapse of a second period of time (e.g., single pulse application time; in Fig. 7, 8 seconds) that is a relatively long period of time from the beginning of the voltage application to the heating element, specifically, immediately before the lapse of the second period of time, sampling of a sensor output is carried out a given number of times (e.g., 256 times), and an operation to determine their mean value is carried out to obtain a mean second voltage value V3. This mean second voltage value V3 corresponds to a second temperature of the temperature detector 21a2 after the lapse of the second period of time from the beginning of the single pulse application. Then, a difference V02 (=V3-V1) between the mean initial voltage value V1 and the mean second voltage value V3 is obtained as a liquid type-corresponding second voltage value.

By the way, a part of heat generated by the heating element 21a4 based on such a single pulse voltage application as above is transferred to the temperature detector 21a2 through the measuring target liquid. In this heat transfer, there are mainly two modes which differ from each other depending on the time from the beginning of the pulse application. That is to say, in the first stage of a relatively short period of time (e.g., 3 seconds, particularly 2 seconds) from the beginning of the pulse application, the heat transfer is mainly governed by conduction (on this account, the liquid type-corresponding first voltage value V01 is influenced mainly by thermal conductivity of the liquid).

On the other hand, in the second stage after the first stage, the heat transfer is mainly governed by natural convection (on this account, the liquid type-corresponding second voltage value V02 is influenced mainly by kinematic viscosity of the liquid). The reason is that in the second stage, there occurs natural convection due to the measuring target liquid that is heated in the first stage, and thereby the ratio of heat transfer is increased.

As previously described, an optimum concentration (% by weight, the same shall apply hereinafter) of the urea aqueous solution used in the exhaust gas purification system is considered to be 32.5%. Therefore, the allowable range of the urea concentration of the urea aqueous solution to be contained in the urea aqueous solution tank 100 can be determined to be, for example, 32.5%±5%. This allowance ±5% is properly changeable, if desired. That is to say, in this embodiment, a urea aqueous solution having a urea concentration of 32.5%±5% is defined as a given liquid.

The liquid type-corresponding first voltage value V01 and the liquid type-corresponding second voltage value V02 vary according as the urea concentration of the urea aqueous solution varies. Therefore, a range (given range) of the liquid type-corresponding first voltage value V01 and a range (given range) of the liquid type-corresponding second voltage value V02 each of which corresponds to the urea aqueous solution having a urea concentration range of 32.5%±5% are present.

By the way, even if the measuring target liquid is a liquid other than the urea aqueous solution, an output in the given range of the liquid type-corresponding first voltage value V01 and an output in the given range of the liquid type-corresponding second voltage value V02 are sometimes obtained depending on the concentration of the liquid. That is to say, even if the liquid type-corresponding first voltage value V01 and the liquid type-corresponding second voltage value V02 are in the respective given ranges, the liquid is not always the given urea aqueous solution. For example, in the range of the liquid type-corresponding first voltage value V01 obtained from the urea aqueous solution having a given urea concentration range of 32.5%+5% (that is, in the range of 32.5%+5% in terms of a sensor display concentration value), a liquid type-corresponding first voltage value of a sugar aqueous solution having a sugar concentration range of about 25%±3% is present, as shown in Fig. 8.

However, a liquid type-corresponding second voltage value V02 obtained from the sugar aqueous solution having the above sugar concentration range is far from the range of the liquid type-corresponding second voltage value V02 obtained from the urea aqueous solution having the given urea concentration range. That is to say, the liquid type-corresponding first voltage value V01 of the sugar aqueous solution having a sugar concentration range of 15% to 35% including a sugar concentration range of about 25%±3% and that of the urea aqueous solution having the given urea concentration range sometimes overlap, but the liquid type-corresponding second voltage value V02 of the sugar aqueous solution greatly differs from that of the urea aqueous solution having the given urea concentration range, as shown in Fig. 9.

In Fig. 9, both of the liquid type-corresponding first voltage value V01 and the liquid type-corresponding second voltage value V02 are expressed in relative values given when those of the urea aqueous solution having a urea concentration of 30% are each 1,000. Thus, the sugar aqueous solution can be surely discriminated from the given liquid based on the criterion that the measuring target liquid is the given liquid only when both of the liquid type-corresponding first voltage value V01 and the liquid type-corresponding second voltage value V02 are in the respective given ranges.

In the present invention, identification of liquid type is carried out utilizing a phenomenon that the relationship between the liquid type-corresponding first voltage value V01 and the liquid type-corresponding second voltage value V02 varies depending on the type of the solution, as described above. That is to say, the liquid type-corresponding first voltage value V01 and the liquid type-corresponding second voltage value V02 are influenced by liquid properties that are different from each other, i.e., thermal conductivity and kinematic viscosity, and the relationship therebetween varies depending on the type of the solution. Therefore, such liquid type identification as above becomes possible. By narrowing the given range of the urea concentration, accuracy of identification can be further enhanced.

In the present invention, identification of liquid type is carried out utilizing a phenomenon that the relationship between the liquid type-corresponding first voltage value V01 and the liquid type-corresponding second voltage value V02 varies depending on the type of the solution, as described above. That is to say, the liquid type-corresponding first voltage value V01 and the liquid type-corresponding second voltage value V02 are influenced by liquid properties that are different from each other, i.e., thermal conductivity and kinematic viscosity, and the relationship between them varies depending on the type of the solution. Therefore, such liquid type identification as above becomes possible. By narrowing the given range of the urea concentration, accuracy of the identification can be further enhanced.

That is to say, in the embodiment of the invention, with respect to several urea aqueous solutions having known urea concentrations (reference urea aqueous solutions), a first calibration curve showing a relationship between a temperature and the liquid type-corresponding first voltage value V01 and a second calibration curve showing a relationship between a temperature and the liquid type-corresponding second voltage value V02 are obtained in advance, and these calibration curves are stored in a memory means of the microcomputer 72. Examples of the first and the second calibration curves are shown in Fig. 10 and Fig. 11, respectively. In these examples, calibration curves of the reference urea aqueous solutions having a urea concentration c1 (e.g., 27.5%) and a urea concentration c2 (e.g., 37.5%) are prepared.

As shown in Fig. 10 and Fig. 11, the liquid type-corresponding first voltage value V01 and the liquid type-corresponding second voltage value V02 depend on temperature, and therefore, when the measuring target liquid is identified using these calibration curves, a liquid temperature-corresponding output value T to be inputted from the temperature detector 22a2 of the liquid temperature detection part 22 through the liquid temperature detection amplifier 71 is also used. Examples of the liquid temperature-corresponding output values T are shown in Fig. 12. Such a calibration curve is also stored in the memory means of the microcomputer 72.

In the measurement of the liquid type-corresponding first voltage value V01, first, using the calibration curve of Fig. 12, a temperature value is obtained from the liquid temperature-corresponding output value T obtained with respect to the measuring target liquid. The resulting temperature value is designated by t. Then, from the first calibration curves of Fig. 10, liquid type-corresponding first voltage values V01(c1;t), V01(c2;t) corresponding to the temperature value t are obtained.

Then, cx of the liquid type-corresponding first voltage value V01(cx;t) obtained with respect to the measuring target liquid is determined by a proportional operation using the liquid type-corresponding first voltage values V01(c1; t), V01(c2;t) of the calibration curves. That is to say, cx is determined by the following formula (1) using V01(cx;t), V01 (c1;t) and V01(c2;t).

$$cx = c1+(c2-c1)[V01(cx;t)-V01(c1;t)]/[V01(c2;t)-V01(c1;t)] \qquad (1)$$

In the measurement of the liquid type-corresponding second voltage value V02, similarly to the above, from the second calibration curves of Fig. 11, liquid type-corresponding second voltage values V02(c1;t), V02(c2;t) corresponding to the temperature value t obtained with respect to the measuring target liquid are obtained. Then, cy of the liquid type-corresponding second voltage value V02(cy;t) obtained with respect to the measuring target liquid is determined by a proportional operation using the liquid type-corresponding second voltage values V02(c1;t), V02(c2;t) of the calibration curves.

That is to say, cy is determined by the following formula (2) using V01(cy;t), V01(c1;t) and V01(c1;t).

$$cy = c1+(c2-c1)[V02(cy;t)-V02(c1;t)]/[V02(c2;t)-V02(c1;t)] \qquad (2)$$

If calibration curves obtained by using the liquid temperature-corresponding output value T instead of the temperature are used as the first and the second calibration curves of Fig. 10 and Fig. 11, storing of the calibration curve of Fig. 12 and conversion using it can be omitted.

As described above, with respect to each of the liquid type-corresponding first voltage value V01 and the liquid type-corresponding second voltage value V02, a given range of its change with temperature can be determined. When c1 is 27.5% and c2 is 37.5% as above, the region between the two calibration curves in each of Fig. 10 and Fig. 11 corresponds to the given liquid (i.e., urea aqueous solution having a urea concentration of 32.5%±5%).

Fig. 13 is a graph schematically indicating that the criterion for identifying a given liquid by a combination of the liquid type-corresponding first voltage value V01 and the liquid type-corresponding second voltage value V02 varies according to the temperature. As the temperature rises to t1, t2, t3, the region judged to be a region of the given liquid moves to AR(t1), AR(t2), AR(t3).

Fig. 14 is a flow chart showing a process of liquid type identification by a microcomputer 72.

First, prior to pulse voltage application to the heating element 21a4 by means of heater control, N=1 is enclosed in the microcomputer (S1), and then sensor outputs are sampled to obtain a mean initial voltage value V1 (S2). Then, heater control is performed, and after the lapse of the first period of time from the beginning of the voltage application to the heating element 21a4, sensor outputs are sampled to obtain a mean first voltage value V2 (S3). Then, an operation of V2-V1 is performed to obtain a liquid type-corresponding first voltage value V01 (S4). Then, after the lapse of the second period of time from the beginning of the voltage application to the heating element 21a4, sensor outputs are sampled to obtain a mean second voltage value V3 (S5). Then, an operation of V3-V1 is performed to obtain a liquid type-corresponding second voltage value V02 (S6).

Next, making reference to the temperature value t obtained with respect to the measuring target liquid, whether the conditions that the liquid type-corresponding first voltage value V01 is in the given range at that temperature and the liquid type-corresponding second voltage value V02 is in the given range at that temperature are satisfied or not is judged (S7). In the case where at least one of the liquid type-corresponding first voltage value V01 and the liquid type-corresponding second voltage value V02 is judged to be not in the given range (NO) in S7, whether the enclosed value N is 3 or not is judged (S8). In the case where N is judged to be not 3 (that is, the present measuring routine is not the third time and is specifically the first time or the second time) (NO) in S8, the enclosed value N is increased by 1 (S9), and the step is returned to S2.

On the other hand, in the case where N is judged to be 3 (that is, the present measuring routine is the third time) (YES) in S8, the measuring target liquid is judged to be not the given one (S10).

On the other hand, in the case where both of the liquid type-corresponding first voltage value V01 and the liquid type-corresponding second voltage value V02 are judged to be in the respective given ranges (YES) in S7, the measuring target liquid is judged to be the given one (S11).

In the present embodiment, S11 is followed by calculation of a urea concentration of the urea aqueous solution (S12). This concentration calculation can be carried out using the aforesaid formula (1), based on the output of the liquid temperature detection part 22, i.e., the temperature value t obtained with respect to the measuring target liquid, and the liquid type-corresponding first voltage value V01 and the first calibration curve of Fig. 10. The concentration calculation may be carried out using the aforesaid formula (2), based on the output of the liquid temperature detection part 22, i.e., the temperature value t obtained with respect to the measuring target liquid, and the liquid type-corresponding second voltage value V02 and the second calibration curve of Fig. 11.

In such a manner as above, identification of liquid type can be carried out accurately and rapidly. This routine of the liquid type identification can be properly performed at the time of starting of automobile engine or periodically or on demand from the driver or automobile (later-described ECU) side or at the time of key-OFF of automobile, and whether the liquid in the urea tank is a urea aqueous solution of a given urea concentration or not can be watched in a desired manner.

A signal indicating the resulting liquid type (signal indicating whether the liquid is a given one or not and further indicating a urea concentration in the case of the given liquid (urea aqueous solution of a given urea concentration)) is outputted to an output buffer circuit 76 shown in Fig. 6 through a D/A converter (not shown), and from the output buffer circuit, the signal is outputted, as an analogue output, to a main computer (ECU, not shown) that performs control of combustion in automobile engine, through the terminal pin 27, the power circuit board 41a and the waterproof wiring 5. An analogue output voltage value corresponding to the liquid temperature is also outputted to the main computer (ECU) through the same route.

On the other hand, a signal indicating the liquid type can be taken out as a digital output and inputted into equipments that perform displaying, alarming and other operations through the same route, when needed.

Further, when lowering of the temperature of the urea aqueous solution down to a temperature in the vicinity of its freezing temperature (about -13°C) is detected based on the liquid temperature-corresponding output value T inputted from the liquid temperature detection part 22, warning can be given.

The above liquid type identification utilizes natural convection and utilizes a principle that the kinematic viscosity of the measuring target liquid such as a urea aqueous solution and the sensor output have a correlation. In order to enhance accuracy of the liquid type identification, it is preferable that forced flow due to an external factor should not occur in the measuring target liquid around the container main body 20A wherein heat transfer is made between the liquid type detection part 21 and the measuring target liquid and between the liquid temperature detection part 22 and the measuring target liquid, and from this viewpoint, use of a cover member 2d, particularly a cover member designed so as to form a measuring target liquid inlet passage in the longitudinal direction, is preferable. The cover member 2d functions also as a protective member for preventing contact with foreign matters.

In the above embodiment, a urea aqueous solution having a given urea concentration is used as the given fluid, but in the invention, the given liquid may be an aqueous solution using a substance other than urea as a solute or another liquid.

In the above embodiment, a measuring target liquid is used as the identification target fluid, but as described later, various identification, such as fluid type identification, concentration identification, identification of presence of fluid, identification of fluid temperature, flow rate identification, identification of fluid leak, identification of fluid level, and identification of quantity of ammonia generated, can be carried out with respect to various identification target fluids, for example, hydrocarbon type liquids, such as gasoline, naphtha, kerosene, light oil and heavy oil, alcohol type liquids, such as methanol and methanol, urea aqueous solution, and fluids, such as gases and powders, utilizing thermal properties of the fluids.

The embodiment shown in Fig. 15 is an embodiment wherein the fluid identification device using the cover member 2d is applied to a gasoline identification device.

In this case, it is enough for the gasoline type identification to utilize natural convection and utilize a principle that the kinematic viscosity of gasoline and the sensor output have a correlation, similarly to the above embodiment. In order to enhance accuracy of the gasoline type identification, it is preferable that occurrence of forced flow of gasoline around a gasoline type detection fin 21c and a liquid temperature detection fin 22c, said forced flow being due to an external factor, should be suppressed to the lowest, and from this viewpoint, use of a cover member 2d, particularly a cover member designed so as to form a gasoline inlet passage in the vertical direction, is preferable. The cover member 2d also functions as a protective member for preventing contact with foreign matters.

In the case where the inclination angle of the measuring part 40 particularly to the vertical direction of the identification sensor module 2 is changed, the cover member 2d also fulfills a function of more enhancing accuracy of the gasoline type identification as compared with the case where a cover member is not present. That is to say, in the case where the cover member is not present, the mode of transfer of heat generated by the heating element to the temperature detector by means of the aforesaid natural convection is greatly changed with the change of the inclination angle, and therefore, the change of the gasoline type-corresponding voltage value V0 of the same gasoline becomes large. On this account, the inclination angle range in which confusion with the output value of gasoline of another type does not occur is relatively narrowed.

In contrast therewith, in the case where the cover member 2d is present, the mode of transfer of heat generated by the heating element to the temperature detector by means of the aforesaid natural convection is changed small with the change of the inclination angle (that is, natural convection is always formed mainly along the gasoline inlet passage in the cover member 2d), and therefore, the change of the gasoline type-corresponding voltage value V0 of the same gasoline becomes small. On this account, the inclination angle range in which confusion with the output value of gasoline of another type does not occur is relatively wide.

In Fig. 16, a change of the gasoline type-corresponding voltage value V0 obtained when the inclination angle of the measuring part is changed in the fluid identification device of the embodiment (having cover member 2d) of Fig. 15 is shown, and in Fig. 17, a change of the gasoline type-corresponding voltage value V0 obtained when the inclination angle of the measuring part is changed in the same device of the above embodiment of the invention except that the cover member is removed (comparative embodiment) is shown. As gasoline, two kinds of gasoline different in T50 [sample 1 (T50=99°C) and sample 2 (T50=87°C)] were used, and as the inclination directions, two kinds of directions, i.e., X direction shown in Fig. 15(a) and Y direction shown in Fig. 15(b), were used.

In the comparative embodiment, the gasoline type-corresponding voltage value V0 of the sample 1 and that of the sample 2 sometimes overlap in the inclination angle $\theta$ range of +30°, as shown in Fig. 17. In the embodiment of the invention, however, the gasoline type-corresponding voltage value V0 of the sample 1 and that of the sample 2 do not overlap in the inclination angle $\theta$ range of $\pm$30°, as shown in Fig. 16. From this, it can be seen that by providing the cover member 2d, gasoline type identification of high accuracy in the wide range of inclination angle becomes possible.

Fig. 18 is a constitutional schematic view of a series circuit that is used in the embodiment instead of the parallel circuit of Fig. 6. The same constitutional parts as in the circuit of Fig. 6 are given the same reference numerals, and detailed descriptions thereof are omitted.

In Fig. 18, the numeral 13 designates a resistor, and the numeral 14 designates a change-over switch for liquid temperature detection. As shown in this figure, voltage at the both ends of the liquid temperature detection part is outputted as an output A.

In this embodiment, when the change-over switch is connected to the a side, an output A for the liquid temperature detection is obtained. On the other hand, in the identification of liquid type or the like, by connecting the change-over switch to the b side, an output A for the liquid type identification is obtained. Based on the same principle as in the above embodiment, the liquid type identification and the liquid temperature detection can be both carried out.

Fig. 19 is a graph showing examples of the outputs A (i.e., results of liquid temperature detection) obtained when the change-over switch 14 shown in Fig. 18 is connected to the a side. The liquid temperature and the output A have a corresponding relation of 1:1. This property can be measured in advance, and therefore, from the value of the resulting output A, the liquid temperature can be detected.

Fig. 20 is a sectional view of an identification sensor module of another embodiment of the invention; Fig. 21(A) is a schematic view showing the interior of the identification sensor module of Fig. 20; and Fig. 21(B) is a partial enlarged sectional view of the identification sensor module viewed along the A direction of Fig. 20.

As shown in Fig. 20 and Fig. 21, the metallic fins 21c, 22c are partly exposed from the resin mold 23 to form exposed surface portions, and on the exposed surface portions, a hydrophilic film 50 is formed. Also on the surface of the resin mold 23 located around the exposed surface portions of the metallic fins 21c, 22c, a hydrophilic film 50 is preferably formed. That is to say, the hydrophilic film 50 is formed over the exposed surface portions of the metallic fins 21c, 22c and the surface of the resin mold 23 around the exposed surface portions.

The hydrophilic film 50 is, for example, a silicon oxide film. The thickness of the silicon oxide film 50 is, for example, 0.01 μm to 1 μm. The silicon oxide film 50 has excellent adhesion to both of the metallic fins 21c, 22c and the resin mold 23 and has high film strength. The surface of the silicon oxide film 50 has higher hydrophilicity than any of the surfaces of the metallic fins 21c, 22c and the resin mold 23.

The degree of hydrophilicity can be indicated by a water contact angle, and a substance having a water contact angle of not more than about 40° is generally considered to be hydrophilic. The silicon oxide film 50 can be made to have a water contact angle of not more than 40°C, and the silicon oxide film 50 exhibits hydrophilicity. In the present invention, the water contact angle of the hydrophilic film 50 is preferably not more than 35°, more preferably not more than 30°, still more preferably not more than 25°, particularly preferably not more than 20°.

The silicon oxide film 50 can be formed by, for example, sputtering, CVD (chemical vapor deposition) or application of a coating material. Of these, the sputtering and the CVD take a long time for the actual treatment. In these methods, moreover, it is difficult to form a film of large thickness, and the apparatus for the film formation becomes large. In contrast therewith, application of a coating material has many practical advantages such that the treatment is easy and the actual treatment time is relatively short apart from the time for leaving to stand. As the coating material, a coating material containing an organosilicon compound and undergoing a reaction after application to form a silicon oxide film is employable.

Such a coating material is, for example, a coating material containing polysilazane such as perhydropolysilazane, a silane coupling agent that is added when necessary, an organic solvent and a palladium catalyst or an amine catalyst that is used when necessary (e.g., Aquamica (registered trademark) available from Clariant Japan Co., Ltd.). Specific examples of the coating material application step and its relevant steps around it are as follows.

(1) Ethanol cleaning step (for removing stain from the surface portion to be coated with a coating material)
(2) Xylene cleaning step (for degreasing the surface portion)
(3) Drying step (for removing water content from the surface portion, about 100°C, about 1 hour)
(4) Coating material application step (by spray coating, coating by brush or waste, flow coating, dip coating, etc.)
(5) Heating step (for removing solvent and for conversion to silicon oxide, 125 to 200°C, about 1 hour)
(6) Heating moistening step (for conversion to silicon oxide, 50 to 90°C, 80 to 95%, about 3 hours)
(7) Cooling step

In addition to the cleaning step using ethanol or xylene as a cleaning solvent, there can be mentioned a cleaning step using an organic solvent, such as acetone, isopropyl alcohol or hexane, as a cleaning solvent.

In the heating step and the heating moistening step, the coating material undergoes the following conversion reaction with water in the surrounding atmosphere (water that naturally exists or water given by moistening) to form a silicon oxide film.

$$-(-SiH_2NH-)-+2H_2O \rightarrow -(-SiO_2-)-+NH_3+2H_2$$

By virtue of the heating moistening step, the heating temperature of the heating step can be lowered. For example, if the heating moistening step is not carried out, the heating temperature in the heating step is about 250°C.

The thickness of the silicon oxide film thus formed is, for example, 0.01 μm to 1 μm, as previously described. If the thickness is too large, peeling is liable to occur. On the other hand, if the thickness is too small, it becomes difficult to maintain hydrophilicity over a long period of time. The thickness is preferably 0.05 μm to 0.8 μm.

Fig. 22(A) is a perspective view of an identification sensor module of another embodiment of the invention; Fig. 22(B) is a schematic view showing a situation in which the identification sensor module of Fig. 22(A) is installed; and Fig. 23 is a vertical sectional view of the identification sensor module of Fig. 22 viewed along the B direction.

As shown in Fig. 22 and Fig. 23, the identification sensor module 2 of this embodiment is constructed so as to be in a closed state, and has a container 20 into which the identification target fluid 212 does not penetrate. In the container 20, a sensor holder 6 is disposed on the identification target fluid side and installed so as to make the container 20 be in a closed state.

As shown in Fig. 23, at a sensor loading hole 6a formed at the center of the sensor holder 6, a liquid type detection thin film chip 21a is embedded in the synthetic resin mold 23, similarly to the embodiment of Fig. 3.

The liquid type detection thin film chip 21a is constructed so that it may protrude at the end of the synthetic resin mold 23 toward the identification target fluid 212 side and the protruded portion on the identification target fluid 212 side may be covered with the synthetic resin mold 23a.

As shown in Fig. 22(B), the identification sensor module 2 of this embodiment is fixed by tightening a fixing screw 208 against a waterproof case 4 through a fixing member 206. In Fig 22(B), the numeral 210 designates a packing.

To the sensor holder 6, a filter holder 8 is fixed through a fixing screw 7, and to the filter holder 8, a filter 9 having a nearly U-shaped section is fixed so as to surround a metallic fin 21c. In this figure, the numeral 200 designates an ASIC substrate, the numeral 202 designates a packing, and the numeral 204 designates a connector.

The filter is not specifically restricted, and examples of materials of the filters include resins, such as polyethylene and polyester, and ceramics, such as zirconia and alumina. Any of porous bodies of these materials is employable for the filter. The exposed portion of the identification sensor module 2 is covered with the hydrophilic film or the filter. Therefore, especially when the measuring target is an aqueous liquid, adhesion of bubbles to the outer surface of the identification sensor module 2 is reduced. Moreover, the identification target fluid hardly suffers forced flow due to an external factor, and enhancement of measuring accuracy becomes possible.

Fig. 24 and Fig. 25 are each a graph to explain another embodiment using the fluid identification device of the invention.

By the way, in order that the material composition of a fuel may be made constant and the optimum combustion conditions should not vary, it is considered to use hydrocarbons that are components of the fossil fuel, such as pentane, cyclohexane and octane, or alcohols, such as methanol and ethanol, singly or as a mixture of at most two kinds. Such fuels are broadly divided into hydrocarbon type fuels and alcohol type fuels.

Next, a fluid identification method using the hydrocarbon type fuel or the alcohol type fuel is described.

Similarly to the aforesaid embodiment of Fig. 7, in the microcomputer 72, sampling of a sensor output is carried out a given number of times (e.g., 256 times) for a given period of time (e.g., 0.1 second) before the beginning of the voltage application to the heating element 21a4, as shown in Fig. 6, and an operation to determine their mean value is carried out to obtain a mean initial voltage value V1. This mean initial voltage value V1 corresponds to an initial temperature of the temperature detector 21a2.

As shown in Fig. 7, after the lapse of a first period of time (e.g., not more than 1/2 of the single pulse application time and specifically 0.5 to 1.5 seconds; in Fig. 7, 1 second) that is a relatively short period of time from the beginning of the voltage application to the heating element, specifically, immediately before the lapse of the first period of time, sampling of a sensor output is carried out a given number of times (e.g., 256 times), and an operation to determine their mean value is carried out to obtain a mean first voltage value V2. This mean first voltage value V2 corresponds to a first temperature of the temperature detector 21a2 after the lapse of the first period of time from the beginning of the single pulse application. Then, a difference V01 (=V2-V1) between the mean initial voltage value V1 and the mean first voltage value V2 is obtained as a liquid type-corresponding first voltage value.

As shown in Fig. 7, after the lapse of a second period of time (e.g., single pulse application time; in Fig. 7, 4 seconds) that is a relatively long period of time from the beginning of the voltage application to the heating element, specifically, immediately before the lapse of the second period of time, sampling of a sensor output is carried out a given number of times (e.g., 256 times), and an operation to determine their mean value is carried out to obtain a mean second voltage value V3. This mean second voltage value V3 corresponds to a second temperature of the temperature detector 21a2 after the lapse of the second period of time from the beginning of the single pulse application. Then, a difference V02 (=V3-V1) between the mean initial voltage value V1 and the mean second voltage value V3 is obtained as a liquid type-corresponding second voltage value.

By the way, a part of heat generated by the heating element 21a4 based on such a single pulse voltage application as above is transferred to the temperature detector 21a2 through the measuring target liquid. In this heat transfer, there are mainly two modes which differ from each other depending on the period of time from the beginning of pulse application. That is to say, in the first stage of a relatively short period of time (e.g., 1.5 seconds) from the beginning of the pulse application, the heat transfer is mainly governed by conduction.

In contrast therewith, in the second stage after the first stage, the heat transfer is mainly governed by natural convection. The reason is that in the second stage, there occurs natural convection due to the measuring target liquid that is heated in the first stage, and thereby the ratio of heat transfer is increased.

In the heat transfer due to conduction in the first stage, thermal conductivity of the measuring target liquid greatly participates, and in the heat transfer due to natural convection in the second stage, kinematic viscosity of the measuring target liquid greatly participates. With respect to several measuring target liquids belonging to the hydrocarbon type liquids and the alcohol type liquids (hydrocarbon type liquids: cyclohexane, pentane, octane, toluene and o-xylene; alcohol type liquids: methanol, ethanol and propanol), the relationship between the liquid type-corresponding first voltage value V01 (=V2-V1) obtained by the device of the present embodiment under the conditions of the first period of time of 1.5 seconds and the thermal conductivity of the measuring target liquid is shown in Fig. 24. Further, with respect to the same measuring target liquids, the relationship between the liquid type-corresponding second voltage value V02 (=V3-V1) obtained by the device of the present embodiment under the conditions of the second period of time of 5 seconds and the kinematic viscosity of the measuring target liquid is shown in Fig. 25.

From Fig. 24, it can be seen that there is a considerable correlation between the liquid type-corresponding first voltage value V01 and the thermal conductivity of the measuring target liquids and that the alcohol type liquids are located in the region in which the liquid type-corresponding first voltage value V01 is smaller than the boundary value Vs and the hydrocarbon type liquids are located in the region in which the liquid type-corresponding first voltage value V01 is larger than the boundary value Vs. From Fig. 25, it can be seen that there is a considerable correlation between the liquid type-corresponding second voltage value V02 and the kinematic viscosity of the hydrocarbon type liquids and between the liquid type-corresponding second voltage value V02 and the kinematic viscosity of the alcohol type liquids.

Fig. 26 is a circuit constitutional view of an embodiment using the fluid identification device of the invention as a flow meter.

A stabilized direct current fed from a power circuit 90 is fed to a bridge circuit (detection circuit) 73. The bridge circuit 73 comprises a flow rate detection temperature detector 32a, a temperature compensation temperature detector 32b, a resistor 92 and a variable resistor 94. Electric potentials Va, Vb at the points a, b of the bridge circuit 73 are inputted into a differential amplification circuit 75 of variable amplification factor. The output of the differential amplification circuit 75 is inputted into an integration circuit 77.

On the other hand, the output of the power circuit 90 is fed to a thin film heating element 33 through an electric field-effect transistor 81 for controlling an electric current fed to the thin film heating element 33. That is to say, in a flow rate detection part 42, the thin film temperature detector 32a is influenced by heat absorption of the detection target fluid through a fin plate 44, based on heat generation of the thin film heating element 33, and performs temperature detection.

As a result of the temperature detection, a difference between the electric potentials Va, Vb at the points a, b of the bridge circuit 73 shown in Fig. 26 is obtained.

The value of (Va-Vb) varies as the temperature of the flow rate detection temperature detector 32a varies according to the flow rate of the fluid. By properly setting a resistance value of the variable resistor 94 in advance, the value of (Va-Vb) can be made zero in the case of a desired flow rate of the fluid that becomes a reference flow rate. At the reference flow rate, the output of the differential amplification circuit 75 is zero, and the output of the integration circuit 77 becomes constant (value corresponding to the reference flow rate). The output of the integration circuit 77 has been adjusted in its level so that the minimum value may become 0 V.

The output of the integration circuit 77 is inputted into a V/F conversion circuit 78, and herein, a pulse signal of a frequency (e.g., maximum of $5\times10^{-5}$) corresponding to the voltage signal is formed. The pulse width (time width) of this pulse signal is constant (e.g., desired value of 1 to 10 microseconds). For example, when the output of the integration circuit 77 is 1 V, a pulse signal of a frequency of 0.5 kHz is outputted, and when the output of the integration circuit 77 is 4 V, a pulse signal of a frequency of 2 kHz is outputted.

The output of the V/F conversion circuit is fed to a gate of the transistor 81. Through the transistor 81 into the gate of which the pulse signal has been inputted as above, an electric current flows to the thin film heating element 33.

Accordingly, to the thin film heating element 33, a divided voltage of the output voltage of the power circuit 90 is applied in the form of pulse at a frequency corresponding to the output of the integration circuit 77, and an electric current intermittently flows through the thin film heating element 33, whereby the thin film heating element 33 generates heat. The frequency of the V/F conversion circuit 78 is set by a high-accuracy clock that is set based on oscillation of a temperature compensation type quartz oscillator 79 in a reference frequency generation circuit 80.

The pulse signal outputted from the V/F conversion circuit 78 is counted by a pulse counter 82. Based on the result (pulse frequency) of pulse counting using the frequency generated by the reference frequency generation circuit 80 as a reference, a microcomputer 83 performs conversion to the corresponding flow rate (instantaneous flow rate), and integrates the flow rate concerning time to determine an integrated flow rate.

The conversion to the flow rate is carried out using a calibration curve of a necessary fluid relating to the flow rate detection, said calibration curve having been stored in a memory 84 in advance. That is to say, a data table obtained by measuring pulse frequency that is outputted from the pulse counter 82 on each flow rate of the fluid has been stored as a calibration curve in the memory 84. The microcomputer 83 specifies, as a measured value, the flow rate value on the calibration curve corresponding to the pulse frequency outputted from the pulse counter 82.

The instantaneous flow rate value and the integrated flow rate value obtained as above are not only displayed by a display part 85 but also transmitted outside through a telephone line or a communication line composed of another network. If desired, the data of the instantaneous flow rate and the integrated flow rate can be stored in the memory 84.

If the flow rate of the fluid is increased or decreased, polarity (dependent on positiveness or negativeness of resistance-temperature property of the flow detection temperature detector 32a) and intensity of the output of the differential amplification circuit 75 vary according to the value of (Va-Vb), and correspondingly to this, the output of the integration circuit 77 is changed. The velocity of the change of the output of the integration circuit 77 can be controlled by setting an amplification factor of the differential amplification circuit 75. By these integration circuit 77 and differential amplification circuit 75, response property of the control system is determined.

If the flow rate of the fluid is increased, the temperature of the flow rate detection temperature detector 32a lowers. Therefore, such an output of the integration circuit 77 as increases the quantity of heat generated by the thin film heating element 33 (that is, such an output as increases the pulse frequency), namely, a higher voltage value, is obtained, and at the time this output of the integration circuit becomes a voltage corresponding to the flow rate of the fluid, the bridge circuit 73 becomes in an equilibrium state.

On the other hand, if the flow rate of the fluid is decreased, the temperature of the flow rate detection temperature detector 32a rises. Therefore, such an output of the integration circuit 77 as decreases the quantity of heat generated by the thin film heating element 33 (that is, such an output as decreases the pulse frequency), namely, a lower voltage value, is obtained, and at the time this output of the integration circuit becomes a voltage corresponding to the flow rate of the fluid, the bridge circuit 73 becomes in an equilibrium state.

That is to say, in the control system of the present embodiment, the frequency (corresponding to the quantity of heat) of the pulse electric current fed to the thin film heating element 33 is set so that the bridge circuit 73 may become in an equilibrium state, and such an equilibrium state (response of control system) can be realized in not more than 0.1 second.

On the other hand, the output of the power circuit 90 is fed to a fluid temperature detection fin plate 44a through a resistor 62 of a high resistance value (e.g., about 10 kΩ) and an electrode terminal 49a, and a flow rate detection fin plate 44 is earthed through an electrode terminal 49. To both ends of the resistor 62, a voltmeter 63 is connected.

The voltage measured by the voltmeter 63 corresponds to the intensity of an electric current that flows through the fluid between the two fin plates 44, 44a when the fluid is introduced into the measuring part, and this corresponds to a resistance value of the fluid between the fin plates 44, 44a. Thus, a circuit for the measurement of electrical conductivity between the flow rate detection fin plate 44 and the fluid temperature detection fin plate 44a is constructed including these fin plates.

The output of the voltmeter 63 is inputted into the microcomputer 83 through an A/D converter 65. In the memory 84, data of the output value range (referred to as "applicable range" hereinafter) of the voltmeter 63 on the necessary measuring target fluid, said range being to be measured by the conductivity measuring circuit, have been stored.

This applicable range can be properly set by introducing the necessary measuring target fluid into the measuring part, actually measuring the output value of the voltmeter 63 of the conductivity measuring circuit and taking a necessary detection error range into account.

For example, in the conductivity measuring circuit capable of setting a range of 3.2 to 3.6 V as the applicable range of a physiological salt solution that is the necessary measuring target fluid, the output value of the voltmeter 63 on the city water is 0.8 to 1.2 V, and the output value of the voltmeter 63 on an alcohol or acetone is not more than 0.05 V. Therefore, if such a fluid that is not the necessary measuring target fluid is introduced by mistake, discrimination from the necessary measuring target fluid is possible.

In this embodiment, prior to detection of the flow rate of the fluid, the fluid is introduced into the measuring part of the flow meter, then flow of the fluid is stopped, and in this state, conductivity (specifically, output voltage value of the voltmeter 63) of the fluid in the measuring part is measured.

Then, the microcomputer 83 judges whether the voltage value inputted from the A/D converter 65 belongs to the applicable range having been stored in the memory 84 or not (that is, within the applicable range or out of the applicable range).

In the case where the output voltage value of the voltmeter 63 is judged to be within the applicable range, the fluid introduced into the measuring part is regarded as the given measuring target fluid, then the fluid is successively supplied into the measuring part from the fluid supply source, and the fluid is allowed to flow with performing such flow rate detection as above.

Contrary to the above, in the case where the output voltage value of the voltmeter 63 is judged to be out of the applicable range, successive supply of the fluid into the measuring part from the fluid supply source is not carried out, and warning display can be given by a display part 85 or warning sound can be given by a warning means (not shown).

In the present embodiment, accordingly, by performing the above-mentioned fluid identification each time the fluid supply source is replaced, the fluid having an apparently different conductivity (which is not the necessary measuring target fluid apparently) from that of the necessary measuring target fluid can be prevented from flowing by mistake.

According to the above embodiment, further, a pulse signal formed by the V/F conversion circuit 78 is used for the measurement of flow rate, and the errors of this pulse signal due to temperature change can be sufficiently reduced easily. Consequently, the errors of the flow rate value and the integrated flow rate value obtained based on the pulse frequency can be reduced. In this embodiment, furthermore, control of electrical conduction to the thin film heating element 33 is made by ON-OFF of the pulse signal formed by the V/F conversion circuit 78. Therefore, control errors due to temperature change occur very rarely.

In this embodiment, moreover, a fine chip including a thin film heating element and a thin film temperature detector is used as the flow rate detection part. Therefore, such a high-speed response as above can be realized, and excellent accuracy can be obtained in the flow rate measurement.

In this embodiment, moreover, the temperature of the flow rate detection temperature detector 32a around the thin film heating element 33 is maintained almost constant irrespective of the flow rate of the detection target fluid. Therefore, deterioration of the flow rate sensor unit with time hardly occurs, and occurrence of ignition explosion of a combustible detection target fluid can be prevented.

According to the flow meter of the invention, identification of a fluid is carried out by measuring electrical conductivity of the fluid between the flow rate detection heat transfer member that is used for flow rate detection and the fluid temperature detection heat transfer member, as described above. Therefore, flow of a fluid having apparently different conductivity from that of the necessary measuring target fluid by mistake can be prevented by a simple constitution.

As shown in Fig. 27, the liquid type identification device 1 of the invention for light oil has a liquid type identification device main body 12 and has a first passage 436 and a second passage 438 which are formed inside the liquid type identification device main body 12.

This liquid type identification device is constructed so that the light oil may be introduced through a light oil inlet 18, pass through the first passage 436 and temporarily stay in a light oil liquid type identification chamber 400, as indicated by arrows in Fig. 27. At the upper part of the light oil liquid type identification chamber 400, an opening 402 for liquid type identification sensor, in the form of an approximate track, is formed.

To the opening 402 for liquid type identification sensor, a liquid type identification sensor 404 is fitted, as shown in Fig. 27.

In Fig. 27, the numeral 36 designates a fin, the numeral 54 designates a light oil exit port, the numeral 408 designates a lead electrode, the numeral 410 designates a liquid temperature sensor, and the numeral 412 designates a mold resin.

In a preferred embodiment, differences in properties of light oils can be discriminated by allowing a heater of the liquid type identification sensor heater 405 to generate heat at 50 to 400 mW, preferably 250 mW, and measuring a change of the temperature of the liquid temperature sensor after 1 to 50 seconds, preferably after 10 seconds, in terms of a voltage output difference V0.

That is to say, it is apparent from the graph in Fig. 28 that voltage output differences V0 after 10 seconds differ from one another as described below.
Gas oil A in Japan proper: 1.20 V
Standard light oil B in Europe: 1.21 V
Standard light oil C in U.S.A.: 1.20 V
Standard light oil D in Sweden: 1.18 V

Accordingly, it is possible to identify liquid type and to recognize distillation property of light oil based on the data having been stored in advance in a computer constituting an identification control part.

The above-mentioned liquid type identification method for a light oil utilizes natural convection and utilizes a principle that the kinematic viscosity of a light oil and the sensor output have a correlation.

That is to say, as shown in Fig. 29, there is a correlation between the kinematic viscosity and the sensor output, and as shown in Fig. 30, there is a correlation also between the kinematic viscosity and the distillation temperature. As a result, there is a correlation between the sensor output and the distillation temperature, as shown in Fig. 31. In the liquid type identification device of the invention, identification of liquid type of a light oil and recognition of distillation property of a light oil can be carried out utilizing the above relationships, as described above.

In order to carry out the identification of liquid type of a light oil and the recognition of distillation property of a light oil more accurately and more rapidly, the following method has only to be carried out.

That is to say, as shown in Fig. 32, calibration curve data (i.e., correlation between voltage output difference and temperature) of given reference light oils such as a light oil A in Japan proper that is heaviest (hardly vaporized) and a standard light oil D in Sweden that is lightest (easily vaporized) are obtained and stored in a computer constituting the identification control part, in advance.

Based on the calibration curve data, a proportional operation is carried out in the computer, and by the voltage output difference V0 obtained on the identification target light oil, identification of the light oil type is performed.

Specifically, similarly to the above embodiment, the liquid type voltage output Vout regarding the voltage output difference V at the measuring temperature T of the identification target light oil is corrected correlatively with the output voltage regarding the voltage output difference at the measuring temperature of the given threshold reference light oil (in this embodiment, light oil A in Japan proper and standard light oil D in Sweden), though this is not shown in the figure.

That is to say, based on the calibration curve data, a voltage output difference V0-A of the light oil A in Japan proper, a voltage output difference V0-D of the standard light oil D in Sweden and a voltage output difference V0-S of the identification target light oil are obtained at the temperature T, though this is not shown in the figure.

Then, by obtaining the liquid type voltage output Vout of the identification target light oil so that the liquid type output of the threshold reference light oil may become the given voltage, that is, in this embodiment, the liquid type output of the light oil A in Japan proper may become 3.5 V and the liquid type output of the standard light oil D in Sweden may become 0.5 V, the liquid type voltage output can be allowed to have a correlation with the light oil property.

By comparing the liquid type voltage output Vout of the identification target light oil with the data having been stored in advance in the computer based on the calibration curve data, it becomes possible to perform identification of a liquid type of the light oil accurately and rapidly (instantly).

It is known that in such a liquid type identification method for light oil, the distillation property of the light oil shown in Fig. 33 exhibits a correlation more in the case of distillation property of T30 to T70, and such a case is desirable.

The liquid type identification device 1 for light oil having the above constitution can be applied to an automobile system (not shown).

In the automobile system, the liquid type identification device 10 for light oil has only to be installed in a light oil tank or on the upstream side of a light oil pump.

By the liquid type identification device 1 for light oil, identification of a liquid type of a light oil present in the light oil tank or on the upstream side or the downstream side of the light oil pump is carried out, then according to the resulting type of the light oil, control of the control device is carried out, and ignition timing can be controlled by the ignition timing control device.

That is to say, for example, when the liquid is identified as the standard light oil D in Sweden that is light (easily vaporized), the ignition timing is controlled to be hastened. On the other hand, when the liquid is identified as the light oil A in Japan proper that is heavy (hardly vaporized), the ignition timing is controlled to be delayed.

By the above control, torque is not decreased even at the time of engine starting at which particularly engine and a catalyst device have not been warmed. Moreover, the quantity of HC in the exhaust gas can be reduced, and the fuel consumption can be improved.

By the liquid type identification device 1 for light oil, further, identification of a liquid type of a light oil present in the light oil tank or on the upstream side or the downstream side of the light oil pump is carried out, then according to the resulting type of the light oil, control of the control device is carried out, and compression ratio of the light oil can be controlled by the light oil compression control device.

That is to say, for example, when the liquid is identified as the standard light oil D in Sweden that is light (easily vaporized), the compression ratio is controlled to be lowered. On the other hand, when the liquid is identified as the light oil A in Japan proper that is heavy (hardly vaporized), the compression ratio is controlled to be raised.

By the above control, torque is not decreased even at the time of engine starting at which particularly engine and a catalyst device have not been warmed. Moreover, the quantity of HC in the exhaust gas can be reduced, and the fuel consumption can be improved.

Fig. 34 is a schematic view of another embodiment wherein the fluid identification device of the invention is used as a flow rate/liquid type detection device, and Fig. 35 is a graph showing calibration curves illustrating a flow rate detection method using the flow rate/liquid type detection device of Fig. 34.

In Fig. 34, the numeral 420 designates the whole of the flow rate/liquid type detection device of the invention. The flow rate/liquid type detection device 420 has a main passage 422 through which a detection target fluid, such as gasoline, a light oil or a urea solution, flows. Further, a sub-passage 424 diverged from the main passage 422 is installed.

The sub-passage 424 is provided with a flow rate/liquid type detection sensor device 11, and on the upstream side thereof, a sub-passage on-off valve to control flow of the detection target fluid to the flow rate/liquid type detection sensor device 11 is installed. The sub-passage 424 is further provided with a check valve 416 on the downstream side of the flow rate/liquid type detection sensor device 11.

On the other hand, the main passage 422 is provided with a main passage on-off valve 417 for controlling flow of the detection target fluid to the main passage, and is further provided with an orifice 418 on the downstream side of the main passage on-off valve.

Further, a sensor control device 419 including a communication device to control the flow rate/liquid type detection sensor device 11, the sub-passage on-off valve 426 and the main passage on-off valve 417 is installed. In the case where this detection device is applied to an automobile, ECU (electronic circuit unit) 418 is connected to the sensor control device 419.

In this case, the sub-passage on-off valve 426 and the main passage on-off valve 417 are not specifically restricted, and for example, electromagnetic valves are adoptable.

The orifice 418 is not specifically restricted either, and for example, a flange tap orifice, a variable orifice, an orifice equipped with plural fine tubes, or the like is adoptable.

The flow rate/liquid type detection device 420 having the above constitution is operated in the following manner.

In performance of any one or both of liquid type detection and concentration detection of the detection target fluid, by the control of the sensor control device 419 (or ECU 428), the sub-passage on-off valve 426 is opened and then closed to allow the detection target fluid to temporarily stay in the flow rate/liquid type detection sensor device 11, and any one or both of the liquid type detection and the concentration detection are carried out.

On the other hand, in the detection of a flow rate of the detection target fluid, by the control of the sensor control device 419 (or ECU 428), the sub-passage on-off valve 426 is opened to allow the detection target fluid to flow into the flow rate/liquid type detection sensor device 11, and in this state, the flow rate is detected.

In this case, if the flow rate of the detection target fluid is low, the sensor control device 419 (or ECU 428) controls so that the main passage on-off valve 417 may be closed, and if the flow rate of the detection target fluid is high, the sensor control device 419 (or ECU 428) controls so that the main passage on-off valve 417 may be opened.

That is to say, when the flow rate of the detection target fluid is low, the main passage on-off valve 417 is closed to allow the detection target fluid to flow into the sub-passage 424, whereby a flow rate of the fluid necessary for the detection in the flow rate/liquid type detection sensor device 11 can be secured.

Contrary to the above, when the flow rate of the detection target fluid is high, the main passage on-off valve 417 is opened to allow the detection target fluid to flow into the main passage 422, whereby the flow rate of the fluid that flows in the sub-passage 424 is decreased, and a flow rate of the fluid necessary for the detection in the flow rate/liquid type detection sensor device 11 can be secured.

Accordingly, it is possible to cope with a case where the dynamic range of the flow rate is wide, and the sensitivity range is widened.

By disposing the check valve 416 on the downstream side of the flow rate/liquid type detection sensor device 11 in the sub-passage 424, back flow can be inhibited even if pulsating flow occurs depending on the type of a pump that is a liquid feed device for feeding the fluid or the type of a driving system to form back flow.

Thus, the back flow of the fluid in the flow rate/liquid type detection sensor device 11 can be prevented, and therefore, in the detections of liquid type, concentration and flow rate, these detections can be carried out accurately and rapidly without being influenced by the back flow of the fluid.

Further, because the orifice 418 is disposed in the main passage 422, pressure loss in the main passage 422 is small, and in the case where the fluid hardly flows in the sub-passage 424, the pressure loss in the main passage 422 can be increased by the orifice 418, whereby the fluid having a constant flow rate necessary for the detection can be allowed to flow into the sub-passage 424, and detection can be surely carried out.

In this state, the voltage output Vout of the detection target fluid is obtained in the same manner as in the aforesaid liquid type detection, and by comparing the resulting value with the data having been stored in the computer based on such calibration curve data regarding the previously measured flow rate as shown in Fig. 35, detection of flow rate of gasoline can be carried out accurately and rapidly (instantly).

Fig. 36 is an exploded perspective view of the whole of another embodiment wherein the fluid identification device of the invention is used as a liquid type detection device. Fig. 37 is an exploded perspective view of a liquid type detection chamber of the liquid type detection device of Fig. 36. Fig. 38 is a schematic view to explain detection in the liquid type detection chamber of the liquid type detection device of Fig. 36.

In Fig. 36, the numeral 430 designates the whole of the liquid type detection device of the invention. The liquid type detection device 430 has a liquid type detection device main body 432 in the form of an approximate box, in which the detection target fluid, such as gasoline, a light oil or a urea solution, flows.

As shown in Fig. 36, inside the liquid type detection device main body 432, a liquid type detection chamber 434 in the form of a nearly round tube is installed. The liquid type detection device main body 432 further has a first passage 436 and a second passage 438.

The first passage 436 is connected to a fluid entry port 440 formed in the liquid type detection chamber 434. The second passage 438 is connected to a fluid exit port 442 formed in the liquid type detection chamber 434.

This liquid type detection device is constructed so that the fluid having been introduced into the liquid type detection device main body 432 may flow through the first passage 436, pass through the fluid entry port 440 and temporarily stay in the liquid type detection chamber 434, as indicated by an arrow in Fig. 37.

On the upper part of the liquid type detection chamber 434, a cap member 444 for liquid type detection chamber is mounted, and in the cap member 444 for liquid type detection chamber, an opening 446 for liquid type detection sensor, in the form of an approximate track, is formed.

To the opening 446 for liquid type detection sensor, a liquid type detection sensor 448 is fitted.

In Fig. 37, the numeral 472 designates a liquid temperature sensor, the numeral 473 designates a liquid temperature sensor heater, and the numeral 474 designates a lead electrode.

As shown in Fig. 36, the liquid type detection sensor 448 is provided with a circuit board member 450 and an outer cap member 452 covering the circuit board member. In Fig. 37, the circuit board member 450 and the outer cap member 452 are omitted for the convenience of description.

In Fig. 36, 454a and 454b are mounting flanges installed on the liquid type detection device main body 432 and for mounting the liquid type detection device 430 on, for example, an automobile.

On the other hand, in the liquid type detection chamber 434, a flow control plate 456 is formed on the inside of the cap member 444 for liquid type detection chamber so as to surround the liquid type detection sensor 448 that protrudes into the liquid type detection chamber 434

This flow control plate 456 is made up by a plate member 458 having a nearly U-shaped section, and this plate member 458 has a pair of side plate members 460, 462 enclosing the liquid type detection sensor 448 at the both sides and extending from the fluid entry port 440 to the fluid exit port 442 in the liquid type detection chamber 434 and has a cover plate member 464 joined to the side plate members 460, 462.

In the flow control plate 456, a fluid inlet 466 facing the fluid entry port 440 of the liquid type detection chamber 434 and a fluid outlet facing the fluid exit port 442 of the liquid type detection chamber 434 are formed.

The fluid entry port 440 of the liquid type detection chamber 434 and the fluid inlet 466 of the flow control plate 456 are apart from each other by a given distance L1, and the fluid exit port 442 of the liquid type detection chamber 434 and the fluid outlet 468 of the flow control plate 456 are apart from each other by a given distance L2.

By virtue of the above constitution, when introduction of the detection target fluid into the liquid type detection device main body 432 is stopped to allow the detection target fluid to temporarily stay in the liquid type detection chamber 434, the flow of the detection target fluid in the liquid detection chamber 434 is inhibited by the flow control plate 456, and the flow of the detection target fluid around the liquid type detection sensor 448 that is surrounded by the flow control plate 456 and located inside the flow control plate 456 stops instantly.

That is to say, the detection target fluid flows from the fluid entry port 440 of the liquid detection chamber 434 to the inside of the flow control plate 456 (i.e., portion surrounded by the flow control plate 456) through the fluid inlet 466 of the flow control plate 456 and surely enters the surroundings of the liquid type detection sensor 448 located inside the flow control plate 456, and as a result, detection of liquid type and concentration of the detection target fluid can be carried out by the liquid type detection senor 448.

After the detection of liquid type and concentration of the detection target fluid is carried out by the liquid type detection senor 448, the detected fluid can be surely discharged from the fluid exit port 442 of the liquid type detection chamber 434 through the fluid outlet 448 of the flow control plate 456. Therefore, accurate detection of a detection target fluid can be successively carried out.

Accordingly, when the liquid type and the concentration are detected by the liquid type detection sensor 448, flow of the detection target fluid is not formed, and disorder of the detection target fluid attributable to oscillation does not occur. As a result, influence on the detection of liquid type and concentration of the detection target fluid can be prevented, and accurate measurements of liquid type and concentration of the detection target fluid can be carried out.

Moreover, because the liquid type detection chamber 434 is installed, the quantity of the detection target fluid that stays is increased, and consequently, in the detection of liquid type and concentration of the detection target fluid, accurate detection can be carried out without being influenced by the surrounding conditions such as external temperature.

Accordingly, in the case of application to fluids for automobiles such as gasoline and light oil, the liquid type and the concentration of the detection target fluid can be instantly detected by stopping a pump of gasoline or the like when the automobile is stopped for, for example, waiting for a signal, and after the detection, the automobile can be started by starting the pump, so that any trouble is not given to traveling of the automobile.

In the above detection, further, air introduced into the detection target fluid can be surely exhausted from the fluid exit port 442 of the liquid type detection chamber 434 through the fluid outlet 468 of the flow control plate 456 in this detection, as indicated by the arrows B in Fig. 38, and therefore, air does not stay around the liquid type detection sensor 448. As a result, influence on the detection can be prevented, and accurate detection can be carried out.

Furthermore, the fluid entry port 440 of the liquid type detection chamber 434 and the fluid inlet 466 of the flow control plate 456 are apart from each other by a given distance L1, and as indicated by the arrows A in Fig. 38, the air introduced into the detection target fluid moves to the outside of the flow control plate 456 from the gap between them and is exhausted outside from the fluid exit port 442 of the liquid type detection chamber 434.

Therefore, air does not enter the inside of the flow control plate 456, and air does not stay around the liquid type detection sensor 448. As a result, influence on the detection can be prevented, and accurate detection can be carried out.

Moreover, even if air enters the inside of the flow control plate 456, the air can be surely exhausted from the fluid exit port 442 of the liquid type detection chamber 434 through the fluid outlet 468 of the flow control plate 456, as indicated by the arrow C in Fig. 38. Therefore, air does not stay around the liquid type detection sensor 448. As a result, influence on the detection can be prevented, and accurate detection can be carried out.

Moreover, the liquid type detection chamber 434 is in the form of a nearly round tube and its sidewall in the vicinity of the fluid exit port 442 is nearly arcuate, and therefore, air introduced into the detection target fluid is led to the inside of the fluid exit port 442 of the liquid type detection chamber 434 along the nearly arcuate sidewall 470 of the liquid type detection chamber 434 and exhausted, as indicated by the arrows B in Fig. 38.

Therefore, air does not stay in the vicinity of the fluid exit port 442 of the liquid type detection chamber 434, and air does not stay around the liquid type detection sensor 448. As a result, influence on the detection can be prevented, and accurate detection can be carried out.

In order to exert such effects, each of the given distances L1, L2 shown in Fig. 38 is desired to be in the range of 1.5 mm to 5 mm, preferably 2 mm to 3.5 mm. A distance L3 between the pair of side plate members 460, 462 of the flow control plate 456 and the liquid type detection sensor 448 is desired to be in the range of 5 mm to 10 mm, preferably 6 mm to 8 mm.

The size of the liquid type detection chamber 434 is not specifically restricted.

Although the material to constitute the liquid type detection chamber 434 is not specifically restricted, metals such as stainless steel (e.g., SUS304), synthetic resins such as polyacetal (POM), fiber-reinforced resins such as FRP, etc. are employable.

Although the material to constitute the flow control plate 456 is not specifically restricted either, metals such as stainless steel (e.g., SUS304), synthetic resins such as polyacetal (POM), fiber-reinforced resins such as FRP, ceramics, etc. are employable.

The liquid type detection device 430 of the invention has a circuit constitution similar to the aforesaid circuit constitution shown in the embodiment of Fig. 6.

Although the circuit constitution of this embodiment is not shown in the figure, a liquid type detection liquid temperature sensor of a liquid type detection sensor heater 406 of the liquid type detection sensor 448, and the liquid temperature sensor 472 are connected through two resistances to form a bridge circuit, similarly to the aforesaid circuit constitution of Fig. 6. An output of the bridge circuit is connected to an input of an amplifier, and an output of the amplifier is connected to an input of a computer that constitutes a detection control part.

The voltage applied to the heater of the liquid type detection sensor heater 406 is controlled by the computer.

In the liquid type detection device 430 of the above constitution, detection of liquid type of, for example, gasoline is carried out in the following manner.

First, by the control of the control device (not shown), the detection target fluid is introduced into the liquid type detection device main body 432, and thereby, the detection target fluid is allowed to flow into the liquid type detection chamber 434 through the first passage 436 and the fluid entry port 440. Thereafter, flow of the detection target fluid is stopped to allow the fluid to temporarily stay in the liquid type detection chamber 434.

When introduction of the detection target fluid into the liquid type detection device main body 432 is stopped to allow the detection target fluid to temporarily stay in the liquid type detection chamber 434 as above, flow of the detection target fluid in the liquid type detection chamber 434 is inhibited by the flow control plate 456, and thereby, flow of the detection target fluid around the liquid type detection sensor 448 that is surrounded by the flow control plate 456 and located inside the flow control plate 456 stops instantly.

The fluid identification device of the invention can be used for a method for identifying urea concentration of a urea solution. Also in this case, natural convection is utilized, and a principle that the kinematic viscosity of urea and the sensor output have a correlation is utilized, similarly to the above embodiment.

Fig. 39 is a schematic view of an embodiment wherein the urea concentration identification device 482 for urea solution, which has the above constitution, is applied to an automobile system 480.

In Fig. 39, the numeral 130 designates a urea solution supply mechanism, and each of the numerals 140, 142 designates a NOx sensor.

In the automobile system 480, the urea concentration identification device 482 for urea solution is disposed inside a urea solution tank 132 or on the upstream side of a urea pump.

By the urea concentration identification device 482 for urea solution, identification of urea concentration of a urea solution present inside the urea solution tank 132 or on the upstream or downstream side (in this embodiment, upstream side is adopted for the convenience of description) of the urea pump is carried out, and in order that the reduction reaction may efficiently proceed on the upstream side of a catalyst device 116 without solidification of the urea solution, the urea solution to be sprayed to the upstream side of the catalyst device 116 is made to have a constant urea concentration of, for example, 32.5% by weight and a constant H2O concentration of, for example, 67.5% by weight.

Thus, the urea solution in the urea tank can be maintained in a given urea concentration, and therefore, the quantity of NOx in the exhaust gas can be markedly decreased by reduction.

The embodiment of the present invention is described below referring to the drawings.

Fig. 40 is a partial broken perspective view to explain an embodiment wherein the fluid identification device of the invention is used as a leak detection device for in-tank liquid, and Fig. 41 is a partly omitted sectional view of the leak detection device of this embodiment.

A tank 490 has a top plate 496 provided with a metering port 492 and a liquid injection port 494 used for injecting a liquid into the tank, a side plate 500 provided with a liquid supply port 498 used for supplying a liquid to the outside of the tank from the tank, and a bottom plate 502. As shown in Fig. 40, in the tank 490, a liquid L (combustible liquid composed of a mixed composition of many organic compounds, e.g., gasoline, light oil, kerosene or the like) is contained. The symbol LS designates a surface of the liquid.

The leak detection device 504 passes through the metering port 492 formed in the top plate 496 of the tank 490, partially enters the tank 490 and is disposed in the vertical direction as a whole. The leak detection device 504 has a liquid inlet-outlet part 506, a flow rate measuring part 508, a liquid reservoir part 510, a cap 16 and a circuit container part 15. The liquid inlet-outlet part 506, the flow rate measuring part 508 and the liquid reservoir part 510 are located inside the tank 490, and the position of the liquid surface LS is within a range of a height of the liquid reservoir part 510.

The flow rate measuring part 508 and the liquid reservoir part 510 comprise a sheathing tube 512 extending over them in the vertical direction.

As shown in Fig. 41, in the flow rate measuring part 508, a sensor holder 13a is disposed in the sheathing tube 512, and by the sensor holder, a measuring fine tube 13b is fixed and held in the vertical direction. The measuring fine tube 13b is equipped with a first temperature sensor 133, a heater 135 and a second temperature sensor 134 which are disposed in this order from the upper side. The heater 135 is disposed at a position equally distant from the first temperature sensor 133 and from the second temperature sensor 134.

Because the sensor holder 13a is covered outside with the sheathing tube 512, the first temperature sensor 133, the heater 135 and the second temperature sensor 134 are protected from corrosion with the liquid L. The measuring fine tube 13b functions as a passage of the liquid between the liquid reservoir part 510 and the liquid inlet-outlet part 506. The first temperature sensor 133, the heater 135 and the second temperature sensor 134 constitute a flow rate sensor part for measuring a flow rate of the liquid in the measuring fine tube 13b.

The flow rate measuring part 508 is provided with a pressure sensor 137 that is fitted to the sensor holder 13a in the vicinity of the lower end of the measuring fine tube 13b. The pressure sensor 137 is a sensor to measure a liquid level of the in-tank liquid L, and for example, a piezo element or a pressure detection element of condenser type is employable. The pressure sensor 137 outputs an electrical signal corresponding to the liquid level of the liquid, such as a voltage signal.

In the liquid inlet-outlet part 506, a filter 12a is fixed to the lower part of the sensor holder 13a by means of a filter cover 12b, as shown in Fig. 41. The filter 12a has a function of removing foreign matters floating on or deposited in the liquid in the tank, such as sludge, to introduce only the liquid into the liquid reservoir part 510 through the measuring fine tube 13b. The sidewall of the filter cover 12b is provided with an opening, and the liquid L in the tank 490 is introduced into the measuring fine tube 13b through the filter 12a of the liquid inlet-outlet part 506.

The liquid reservoir part 510 is located above the flow rate measuring part 508, has a space G surrounded by the sheathing tube 512 and is constructed so that the liquid introduced through the measuring fine tube 13b may be reserved in the space G. The sensor holder 13a is equipped with a third temperature sensor 136 for measuring the temperature of the liquid in the space G.

To the upper part of the sheathing tube 512, the cap 16 is fixed, and in the cap, an air passage 16a for making connection between the inside of the liquid reservoir part 510 and the tank interior space outside the detection device is formed. In the cap 16, an on-off valve 138 to make the air passage 16a be in an open state or a closed state is disposed.

A valve body 138a of the on-off valve is vertically movable, and when the valve body is located at the lowest position, the air passage 16a is in a closed state (the on-off valve is in an open state), and when it is located at the position higher than that, the air passage 16a is in an open state (the on-off value is in a closed state).

The cap 16 is provided with a circuit container part 15, and in the circuit container part, a leak detection control part 15a is contained. In the sheathing tube 512, a guide tube Pg extending so as to connect the top of the sensor holder 13a to the cap 16 is disposed, and a wiring 17 to connect the first temperature sensor 133, the heater 135, the second temperature sensor 134, the pressure sensor 137 and the third temperature sensor 136 in the flow rate measuring part 508 to the leak detection control part 15a passes through the guide tube Pg. The on-off valve 138 is connected to the leak detection control part 15a.

The sheathing tube 512 in the liquid reservoir part 510 constitutes the measuring tube of the invention. By setting the sectional area of the measuring fine tube 13b to a value sufficiently smaller than the sectional area of the sheathing tube 512 (except sectional area of the guide tube Pg), e.g., not less than 1/50 and not more than 1/100, further not more than 1/300, liquid flow that enables measurement of a flow rate even if a slight change of liquid level due to a slight leak of liquid occurs can be formed in the measuring fine tube 13b.

The sheathing tube 512, the sensor holder 13a, the filter cover 12b, the cap 16 and the guide tube Pg are each preferably composed of a metal having a thermal expansion coefficient approximate to that of the material constituting the tank 490, and more preferably, they are each composed of the same metal as that of the tank 490, such as cast iron or stainless steel.

If the above leak detection device 504 is set to the metering port 492 of the tank 490 while the air passage 16a is made to be in an open state by the on-off valve 138, the position of the liquid surface LS of the in-tank liquid L is within a range of a height of the liquid reservoir part 510, as described above. Accordingly, the pressure sensor 137 is immersed in the in-tank liquid L having been filtered through the filter 12a of the liquid inlet-outlet part 506. The in-tank liquid L rises through the measuring fine tube 13b of the flow rate measuring part 508 and is introduced into the space G of the liquid reservoir part 510, and finally, the position of the liquid surface of the liquid in the liquid reservoir part 510 and the position of the liquid surface LS of the in-tank liquid outside the leak detection device become equal to each other. If the liquid surface LS of the in-tank liquid fluctuates, the liquid surface of the liquid in the liquid reservoir part 510 also fluctuates, and with this fluctuation of the liquid surface, namely, change of liquid level, flow of the liquid is formed in the measuring fine tube 13b.

Next, the leak detection operations, i.e., operations of CPU, in this embodiment are described. In this embodiment, kerosene is used as the in-tank liquid.

Fig. 42 is a timing view showing a relationship between a voltage Q applied to a thin film heating element from a pulse voltage generation circuit and a voltage output S of a leak detection circuit.

From the CPU, a single pulse voltage having a width t1 is applied at a given time interval t2, based on a clock. This single pulse voltage has a pulse width t1 of, for example, 2 to 10 seconds and a pulse height Vh of, for example, 1.5 to 4 V.

Through the above voltage application, heat is generated by the thin film heating element. With the thus generated heat, the measuring fine tube 13b and the liquid inside the tube are heated, and the heat is transferred to the surroundings. The influence of this heating reaches the thin film temperature detectors, and the temperatures of the thin film temperature detectors are changed. In the case where the flow rate of the liquid in the measuring fine tube 13b is zero, the temperature changes of the two temperature detectors are equivalent to each other if contribution of the temperature transfer due to convection is ignored.

However, in the case where the liquid surface of the in-tank liquid is lowered as in the leak of the in-tank liquid from the tank, the liquid is led out of the liquid reservoir part 510, allowed to pass through the measuring fine tube 13b and is led into the tank outside the detection device from the liquid inlet-outlet part 506, so that the liquid in the measuring fine tube 13b flows downward.

By virtue of this, the heat from the thin film heating element is transferred more to the thin film temperature detector of the temperature sensor 134 on the lower side than to the thin film temperature detector of the temperature sensor on the upper side.

Thus, there occurs a difference between the temperatures detected by the two thin film temperature detectors, and the changes of resistance values of these thin film temperature detectors become different from each other. In Fig. 42, a change of a voltage VT1 applied to the thin film temperature detector of the temperature sensor 133 and a change of a voltage VT2 applied to the thin film temperature detector of the temperature sensor 134 are shown. Thus, the output of the differential amplifier, namely, voltage output S of the leak detection circuit, varies as shown in Fig. 42.

In Fig. 43, a specific example of a relationship between a voltage Q applied to the thin film heating element from a pulse voltage generation circuit and a voltage output S of the leak detection circuit is shown. In this example, the single pulse voltage has a pulse height Vh of 2 V and a pulse width t1 of 5 seconds, and the voltage output S[F] is obtained by changing the liquid level change rate F (mm/h).

In the CPU, according to the application of a single pulse voltage to the thin film heating element of the heater 135 by the pulse voltage generation circuit, a difference (S0-S) between the voltage output S of the leak detection circuit and its initial value S0 (i.e., at the time of beginning of single pulse voltage application) is integrated in a given period of time t3 after the beginning of the single pulse voltage application. The resulting integrated value ∫(S0-S)dt corresponds to the region of oblique lines in Fig. 42, and is a flow rate-corresponding value that corresponds to the flow rate of the liquid in the measuring fine tube 13b. The given period of time t3 is, for example, 20 to 150 seconds.

In Fig. 44, specific examples of relationships between a liquid level change rate corresponding to the flow rate F of the liquid in the measuring fine tube 13b and the integrated value ∫(S0-S)dt are shown.

In these examples, the given period of time t3 to obtain the integrated value is 30 seconds, and relationships at three temperatures different from one another were obtained. It can be seen that there are favorable linear relationships between the liquid level change rate and the integrated value ∫(S0-S)dt in the region of a liquid level change rate of not more than 1.5 mm/h.

In these examples, favorable linear relationships are exhibited in the region of a liquid level change rate of not more than 1.5 mm/h, but by properly setting the ratio of the measuring fine tube sectional area to the measuring tube sectional area, the length of the measuring fine tube, etc., it becomes possible to obtain favorable liner relationships in the region of a liquid level change rate of not more than 20 mm/h.

Such a typical relationship between the integrated value ∫(S0-S)dt and the liquid level change rate can be stored as a calibration curve in the memory, in advance. Therefore, by performing conversion based on the integrated value ∫(S0-S)dt that is a flow rate-corresponding value calculated by the use of the output of the leak detection circuit with making reference to the stored contents of the memory, leak of the in-tank liquid can be obtained as a liquid level change rate. If a liquid level change rate that is lower than a certain value (e.g., 0.01 mm/h) is obtained, it is considered to be in the range of measurement error, and judgment of no leak can be made.

Strictly speaking, the relationship between the integrated value ∫(S0-S)dt and the liquid level change rate varies depending on the temperature of the liquid, as shown in Fig. 44. Then, calibration curves showing the relationship between the integrated value ∫(S0-S)dt and the liquid level change rate given at plural temperatures are stored in the memory, and based on the temperature (actually measured temperature) measured by the third temperature sensor and using the calibration curve which has been stored in the memory and is given at the temperature nearest to the actually measured temperature, conversion of the integrated value ∫(S0-S)dt to the liquid level change rate can be carried out. According to this method, leak detection of higher accuracy becomes possible.

This leak detection (micro leak detection) is carried out repeatedly at an interval of a proper period of time t2.

The proper period of time t2 is, for example, 40 seconds to 5 minutes (longer than the integration time t3).

In the CPU, further, the liquid level-corresponding output P inputted from the pressure sensor 137 through the A/D converter can be immediately converted to a liquid level p. This conversion relates to a specific gravity r of the liquid, and can be carried out using the following formula (1):

$$p = P/(rg)$$

wherein P is a pressure value measured by the pressure sensor 137, p is a liquid level based on the position of the height of the pressure sensor 137, and g is an acceleration of gravity.

The value of the liquid level p is based on the height of the pressure sensor 137, but by taking into consideration the height of the metering port 492 of the tank 490 and the distance between the position to mount the leak detection device onto the metering port and the pressure sensor 137, the output can be converted to a liquid level value against the tank itself. From the CPU, a liquid level detection signal exhibiting the results of the liquid level detection is outputted.

The specific gravity r of the liquid is detected in the following manner. In Fig. 46, flow of the specific gravity detection is shown.

The detection of a specific gravity of the liquid is carried out each time the liquid is injected into the tank for replenishing. After injection of the liquid, a proper period of time for calming the liquid surface is allowed to pass, and then the detection is started by an external input or the like. At this time, the pulse electrical conduction to the heater 135 of the flow rate sensor part is started (if the pulse electrical conduction has been already performed, it is continued).

The air passage 16a is closed by the on-off valve 138 (S1), then the liquid is allowed to stand for a proper period of time (e.g., 2 to 5 minutes) to calm the liquid surface (S2), thereafter the above measurement of an integrated value ∫(S0-S)dt is carried out plural times (e.g., 5 times) (S3), then the mean value of the resulting plural integrated values ∫(S0-S)dt is calculated (S4), and from the resulting mean value, a specific gravity r is calculated using a specific gravity calibration curve (S5).

The specific gravity calibration curve can be obtained by performing measurement of an integrated value ∫(S0-S)dt with respect to liquids of the same kind having various specific gravities already known (e.g., fuel oils including kerosene), similarly to the above, and the specific gravity calibration curve is stored in the memory in advance. It is also possible that in S3, measurement of an integrated value ∫(S0-S)dt is performed once, S4 is omitted, and in S5, the value obtained in the above measurement of one time is used as the mean value.

Then, whether the specific gravity r obtained as above is in the range of not less than 0.7 and not more than 0.95 is judged (S6). This judgment is made in order to judge whether the liquid is a fuel oil or not. In the case where judgment of $0.7 \le r \le 0.95$ is made, the liquid is regarded as a fuel oil, and this r value is stored as a specific gravity of the existing in-tank liquid in the memory (S7).

On the other hand, in the case where judgment that the r value is out of the range of $0.7 \le r \le 0.95$ is made in S6, whether 3 cycles of S3 to S5 have been continuously performed or not is judged (S8). If judgment that 3 cycles of S3 to S5 have been continuously performed is made, error processing to finally confirm that the liquid is not a fuel oil is carried out (S9). Based on this, the CPU can give a proper warning signal to the outside. On the other hand, if judgment that 3 cycles of S3 to S5 have not been continuously performed is made, S3 and the subsequent steps are carried out. It is also possible that S8 is omitted and S9 is carried out just after S6.

After S7 or S9, the air passage 16a is opened by the on-off valve 138 (S10) to complete the specific gravity detection.

In the CPU, the value of liquid level p obtained as above is stored in the memory at regular intervals of a certain period of time tt, e.g., 2 to 10 seconds, and each time of storing, a difference between the p value of this time and the p value of the previous time is calculated, and the resulting difference is stored as a value of time change rate p' of liquid level in the memory.

In Fig. 45, a specific example of a relationship between the liquid level change rate and the time change rate P' of the liquid level-corresponding output P is shown. In the region of a liquid level change rate of not more than 150 mm/h, there is a favorable linear relationship between the liquid level change rate and the time change rate P' of the liquid level-corresponding output, and therefore, it can be seen that the liquid level change rate and the liquid level time change rate p' favorably correspond to each other. In this example, a favorable linear relationship is exhibited in the region of a liquid level change rate of not more than 150 mm/h, but it is possible to obtain a favorable linear relationship in the region of a liquid level change rate of up to 200 mm/h.

Accordingly, leak of the in-tank liquid can be obtained as the magnitude of the time change rate p' of the liquid level p measured by the pressure sensor 137.

By the way, strictly speaking, the specific gravity r of the liquid varies according to the temperature of the liquid. Correspondingly to this, the following processing can be carried out in the specific gravity detection described above.

That is to say, as the specific gravity calibration curve, a specific gravity calibration curve obtained at a reference temperature TR (e.g., 15°C) (reference temperature specific gravity calibration curve) is used. In the preparation of the reference temperature specific gravity calibration curve, the specific gravity value r[TR] at the reference temperature TR can be calculated from the following formula (2) based on the specific gravity r[TA] that is detected at the liquid temperature TA.

$$r[TR] = r[TA]+0.00071(TA-TR) \quad -2$$

In the formula (2), the factor 0.00071 is a factor in the case where the liquid is a fuel oil.

The corrected specific gravity value r'[TX] at the present temperature TX can be calculated from the following formula (3) with the proviso that the temperature measured by the third temperature sensor 136 in the specific gravity detection of the detection target liquid described above referring to Fig. 46 is TX and the specific gravity value obtained by conversion using the reference temperature specific gravity calibration curve is r[TX].

$$r'[TX] = r[TX]-0.00071(TX-TR) \quad -3$$

In the formula (3), the factor 0.00071 is a factor in the case where the liquid is a fuel oil.

By performing conversion to a liquid level using the above-obtained corrected specific gravity value r'[TX] as the specific gravity value r of the formula (1), leak detection of higher accuracy becomes possible.

Such leak detection using a pressure sensor as above can cover a wider range of a liquid level change rate as compared with the aforesaid micro leak detection. On the other hand, the micro leak detection can perform measurement of high accuracy in the region of a micro liquid level change rate as compared with the leak detection using a pressure sensor.

By the way, the liquid level change in the tank 490 occurs also when the liquid is injected into the tank through the liquid injection port 494 or also when the liquid is supplied to the outside from the liquid supply port 498. In these cases, however, the rate of rising or falling of the liquid level in the tank 490 generally is considerably higher than the liquid level change rate or the liquid level time change rate.

Therefore, the CPU performs the following processing in connection with leak.

(1) When the value of the liquid level time change rate p' is in a given range (e.g., 10 to 100 mm/h) in the leak detection using a pressure sensor, the result of the leak detection using a pressure sensor is outputted as a leak detection signal.

(2) When the value of the liquid level time change rate p' is less than the lower limit of the above given range (e.g., less than 10 mm/h) in the leak detection using a pressure sensor, the result of the micro leak detection is outputted as a leak detection signal.

(3) When the value of the liquid level time change rate p' is more than the upper limit of the above given range (e.g., more than 100 mm/h) in the leak detection using a pressure sensor, judgment that there is a cause other than leak, such as injection of liquid or supply of liquid, is made, and a leak detection signal is not outputted. In this embodiment, further, the CPU can stop the first leak detection for the subsequent given period of time tm in the case of (3), namely, the case where the value of the liquid level time change rate p' is more than the upper limit of the above given range in the leak detection using a pressure sensor.

The given period of time tm for which the leak detection is stopped is preferably a little longer than the time for calming the liquid surface LS after injection of the liquid into the tank from the outside or after supply of the liquid to the outside from the tank, and can be, for example, 10 to 60 minutes. Particularly during the given period of time tm, the CPU can stop the operations of the pulse voltage generation circuit and the leak detection circuit. According to this, power consumption can be reduced.

The liquid level change rate or the liquid level time change rate relates to leak quantity (leak quantity per unit time). That is to say, a value obtained by multiplying the liquid level change rate or the liquid level time change rate by the horizontal sectional area of the interior of the tank at the liquid level corresponds to the leak quantity of the liquid. Therefore, the shape of the tank (i.e., relation between the position of the height and the horizontal sectional area of the interior of the tank) is stored in the memory in advance, and making reference to the stored contents of the memory, the leak quantity of the in-tank liquid can be calculated based on the liquid level and the leak (liquid level change rate or liquid level time change rate) detected as above.

When the horizontal sectional area of the interior of the tank (i.e., shape of the tank) is constant independent of the height as in the shape of a vertical cylinder shown in Fig. 40, the liquid level change rate or the liquid level time change rate and the leak quantity have a simple proportional relation. Therefore, the leak quantity can be readily calculated by multiplying the liquid level change rate or the liquid level time change rate by a proportional constant corresponding to the horizontal sectional area of the interior of the tank, irrespective of the value of the liquid level. That is to say, in this case, the leak detected by the device of this embodiment is substantially equal to that based on the leak quantity.

Fig. 47 is an exploded perspective view showing another embodiment using the fluid identification device of the invention as a liquid level detection device. Fig. 48 is a partly omitted sectional view of Fig. 47. Fig. 49 is a view showing a situation in which the fluid identification device of the invention is installed in a tank.

As shown in Fig. 49, at the top of a NOx decomposition urea aqueous solution tank 520 for constituting an exhaust gas purification system that is loaded on, for example, an automobile, an opening 522 is formed, and to the opening, a liquid level detection device 523 of the invention is fitted. The urea aqueous solution tank 520 is provided with an inlet pipe 524 through which the urea aqueous solution is injected and an outlet pipe 526 through which the urea aqueous solution is taken out.

The outlet pipe 526 is connected to the tank at the height position near the bottom of the urea aqueous solution 520, and is connected to a urea aqueous solution sprayer (not shown) through a urea aqueous solution supply pump 110. By the urea aqueous solution sprayer disposed just before an exhaust gas purification catalyst device in the exhaust system, spraying of the urea aqueous solution is carried out.

The liquid level detection device has an identification sensor part 528, a pressure sensor 530 and a supporting part 532. To one end (lower end) of the supporting part 532, the identification sensor part 528 is set, and at the other end (upper end) of the supporting part 532, a mounting part 4a for mounting the supporting part onto the tank opening 522 is installed.

In Fig. 47 and Fig. 48, the symbol 2a designates a base, each of 2b and 2c designates an O-ring, 4a designates a mounting part, 21 designates an indirect-heated concentration detection part, each of 21c and 22c designates a metallic fin, each of 21e and 22e designates an outer electrode terminal, 24 designates a measuring target liquid inlet passage, 540 designates a circuit board, 542 designates a cap member, each of 544, 546 and 548 designates a wiring, 550 designates a connector, and 532 designates a supporting part.

Fig. 50 is a flow chart showing a process of liquid level detection by a microcomputer.

By the pressure sensor 530, a liquid pressure P of a urea aqueous solution is detected, and the detected liquid pressure value is inputted into the microcomputer, and based on the value, the microcomputer calculates a temporary liquid level value H on the assumption that the urea aqueous solution is a urea aqueous solution having a given density, e.g., water having a urea concentration of zero and a density of 1 (ST1).

In this calculation, the following relational formula (3) obtained from a relationship (shown in Fig. 13) between the liquid pressure P (kPa) measured in advance by the pressure sensor 530 and the liquid level (temporary liquid level value) H (cm) can be used.

$$H = 0.0041 \cdot P2 + 10.181 \cdot P$$

On the other hand, the urea concentration value C obtained by the use of the identification sensor part 528 as above is inputted into the microcomputer. Based on the value, the microcomputer calculates a density value r of the urea aqueous solution having the urea concentration value C (ST2). The density r can be calculated from the following relational formula (4) obtained from a relationship between a change of the urea concentration C (wt%) and a change of the density r (g/cm3) of the urea aqueous solution.

$$r = 7.450E(-6) \cdot C2 + 2.482E(-3) \cdot C + 1,000 \quad -4$$

Then, using the temporary liquid level value H and the density r of the urea aqueous solution obtained as above, the liquid level H' (cm) is calculated from the following relational formula (5) (ST3).

$$H' = r \cdot H$$

In the above manner, detection of liquid pressure and identification of concentration, and calculation of liquid level based on them can be carried out accurately and rapidly. The routine of the liquid level detection based on the concentration identification can be properly carried out at the time of starting of automobile engine or periodically or on demand from the driver or automobile (later-described ECU) side or at the time of key-OFF of automobile, and the liquid level of the urea aqueous solution in the urea tank can be watched in a desired manner.

A signal indicating the concentration and the liquid level obtained as above is outputted to an output buffer circuit through a D/A converter (not shown), similarly to the embodiment of Fig. 6, and from the output buffer circuit, the signal is outputted, as an analogue output, to a main computer (ECU, not shown) that performs control of combustion in automobile engine. An analogue output voltage value corresponding to the liquid temperature is also outputted to the main computer (ECU). On the other hand, the signal indicating the concentration and the liquid level can be taken out as a digital output and inputted into equipments that perform displaying, alarming and other operations, when needed.

Further, when lowering of the temperature of the urea aqueous solution down to a temperature in the vicinity of its freezing temperature (about -13°C) is detected based on the liquid temperature-corresponding output value T inputted from the liquid temperature detection part 534, warning can be given.

Fig. 64 is a schematic sectional view showing an embodiment using the fluid identification device of the invention as a device for measuring a quantity of ammonia generated.

The device 1 for measuring a quantity of ammonia generated, which is shown in Fig. 64, basically has the same constitution as that of the liquid type identification device 1 in the embodiment shown in Fig. 1 to Fig. 14. Therefore, the same constitutional parts are given the same reference numerals, and detailed descriptions thereof are omitted. The numeral 301 designates a terminal pin.

As previously described, the liquid type-corresponding first voltage value V01 is mainly influenced by thermal conductivity of the liquid, and the liquid type-corresponding second voltage value V02 is mainly influenced by kinematic viscosity of the liquid. Therefore, the liquid type-corresponding first voltage value V01 is referred to a "thermal conductivity-corresponding voltage value V01" hereinafter, and the liquid type-corresponding second voltage value V02 is referred to as a "kinematic viscosity-corresponding voltage value V02" hereinafter.

The thermal conductivity-corresponding voltage value V01 and the kinematic viscosity-corresponding voltage value V02 vary according as the urea concentration and the ammonium formate concentration of the measuring target liquid US vary.

In the present embodiment, the quantity of ammonia generated from a mixed solution is measured by utilizing a phenomenon that the relationship between the thermal conductivity-corresponding voltage value V01 and the kinematic viscosity-corresponding voltage value V02 varies depending on the urea concentration and the ammonium formate concentration of a mixed solution and by measuring the urea concentration and the ammonium formate concentration. That is to say, the thermal conductivity-corresponding voltage value V01 and the kinematic viscosity-corresponding voltage value V02 are influenced by liquid properties different from each other, i.e., thermal conductivity and kinematic viscosity, and the relationship therebetween varies depending on the urea concentration and the ammonium formate concentration, so that the following concentration detection becomes possible.

That is to say, in the embodiment of the invention, with respect to several urea aqueous solutions (reference urea aqueous solutions) having a Y/X ratio of 0 (Y (wt%): ammonium formate concentration, X (wt%): urea concentration), i.e., an ammonium formate concentration of 0%, and having known urea concentrations, first calibration curves showing a relationship between the thermal conductivity-corresponding voltage value V01 and the kinematic viscosity-corresponding voltage value V02 are obtained in advance, and with respect to several mixed solutions having a Y/X ratio of c0 (constant) (Y (wt%): ammonium formate concentration, X (wt%): urea concentration), second calibration curves showing a relationship between the thermal conductivity-corresponding voltage value V01 and the kinematic viscosity-corresponding voltage value V02 are obtained in advance. Then, these calibration curves are stored in a memory means of the microcomputer 72. Examples of the first and the second calibration curves are shown in Fig. 65.

It is assumed that with respect to the measuring target liquid US that is a mixed solution having a urea concentration of Xa% and an ammonium formate concentration of Ya%, a thermal conductivity-corresponding voltage value V01a and a kinematic viscosity-corresponding voltage value V02a have been obtained.

If the liquid is a solution containing urea only (urea solution), the kinematic viscosity-corresponding voltage value should have become V02b when the thermal conductivity-corresponding voltage value is V01a, as shown in Fig. 65. Such disagreement of the combination of the thermal conductivity-corresponding voltage value V01 and the kinematic viscosity-corresponding voltage value V02 with the first calibration curve indicates that the solution contains not only urea but also ammonium formate.

From the first and the second calibration curves obtained in advance, it can be seen that when the thermal conductivity-corresponding voltage value is V01a, the temporary first kinematic viscosity-corresponding voltage value becomes V02b in the case of Y/X=0, and the temporary second kinematic viscosity-corresponding voltage value becomes V02c in the case of Y/X=c0.

Then, the value of Y/X=c, at which the thermal conductivity-corresponding voltage value is V01a and the kinematic viscosity-corresponding voltage value becomes V02a, is calculated using a proportional operation. That is to say, the value is determined by the following formula.

$$c=c0(V02a-V02b)/V02c$$

The first and the second calibration curves of Fig. 65 vary depending on the liquid temperature, and therefore, it is necessary that calibration curves corresponding to plural liquid temperatures should be obtained and stored in the memory means of the microcomputer 72 in advance and that the calibration curve used should be properly changed according to the liquid temperature.
If Y/X=c is ascertained, a value of X at which the thermal conductivity-corresponding voltage value V01 becomes V01a can be decided from the calibration curve (comparative curve) of the thermal conductivity-corresponding voltage value V01 and the urea concentration X in the case of Y/X=c, said calibration curve having been stored in advance. Further, according to X thus decided, a value of Y is also decided.
The calibration curve (comparative curve) of the thermal conductivity-corresponding voltage value V01 and the urea concentration X in the case of Y/X=c may be set by performing interpolation from the calibration curves against several c values having been stored in advance.
From the thus calculated urea concentration X wt% and ammonium formate concentration Y wt% and the mixed solution quantity Q, the urea quantity A=Q×X/100 and the ammonium formate quantity B=Q×Y/100 in the mixed solution are calculated. The mixed solution quantity Q may be mass (unit: kg, g or the like) or volume (unit: cc, 1 or the like)
Using four parameters of the urea concentration X wt%, the ammonium formate concentration Y wt%, the urea quantity A and the ammonium formate quantity B, the quantity of ammonia generated can be calculated from the following formula.

$$\text{Quantity of ammonia generated } G = X\times A+Y\times B$$

Fig. 66 is a schematic sectional view showing another embodiment using the fluid identification device of the invention as a device for measuring a quantity of ammonia generated, and this device is further equipped with a differential pressure sensor 300 in addition to the identification sensor module 2. As the differential pressure sensor 300, a differential pressure sensor having been used in the past is employable.
The device 1 for measuring a quantity of ammonia generated, which is shown in Fig. 66, basically has the same constitution as that of the liquid type identification device 1 in the embodiment shown in Fig. 1 to Fig. 14. Therefore, the same constitutional parts are given the same reference numerals, and detailed descriptions thereof are omitted.
As shown in Fig. 66, a terminal pin 31 of the above differential pressure sensor 300 is connected to a circuit of a circuit board 41a.
When a liquid pressure at a first inlet 300a of the differential sensor 300 is designated by p1, a liquid pressure at a second inlet 300b thereof is designated by p2, a difference in height between the first inlet 300a and the second inlet 300b is designated by L, and a density of the identification target liquid is designated by r, there is a relationship of p1-p2=rxL, and therefore, a density-corresponding voltage value V03 that is an electrical output dependent on r can be measured by the differential sensor 300.
In the same manner as previously described, with respect to several urea aqueous solutions (reference urea aqueous solutions) having a Y/X ratio of 0 (Y (wt%): ammonium formate concentration, X (wt%): urea concentration), i.e., an ammonium formate concentration of 0%, and having known urea concentrations, thermal conductivity-corresponding voltage values V01 are measured using the identification sensor module 2, and third calibration curves showing a relationship between the thermal conductivity-corresponding voltage value V01 and the density-corresponding voltage value V03 are obtained in advance. Then, with respect to several mixed solutions having a Y/X ratio of c0 (constant) (Y (wt%): ammonium formate concentration, X (wt%): urea concentration), thermal conductivity-corresponding voltage values V01 are measured using the identification sensor module 2, and fourth calibration curves showing a relationship between the thermal conductivity-corresponding voltage value V01 and the density-corresponding voltage value V03 are obtained in advance. These calibration curves are stored in a memory means of the microcomputer 72. Examples of the third and the fourth calibration curves are shown in Fig. 67.
In this embodiment, it is unnecessary to obtain the kinematic viscosity-corresponding voltage value V02, so that the application of pulse voltage may be finished after the lapse of the first period of time that is a relatively short period of time from the beginning of voltage application to the heating element. That is to say, the first period of time may be a pulse voltage application time. By virtue of this, the measuring time can be shortened.
It is assumed that with respect to the measuring target liquid US that is a mixed solution having a urea concentration of Xa% and an ammonium formate concentration of Ya%, a thermal conductivity-corresponding voltage value V01a and a density-corresponding voltage value V03a have been obtained.

If the liquid is a solution containing urea only (urea solution), the density-corresponding voltage value should have become V03b when the thermal conductivity-corresponding voltage value is V01a, as shown in Fig. 67. Such disagreement of the combination of the thermal conductivity-corresponding voltage value V01 and the density-corresponding voltage value V03 with the third calibration curve indicates that the solution contains not only urea but also ammonium formate.

From the third and the fourth calibration curves obtained in advance, it can be seen that when the thermal conductivity-corresponding voltage value is V01a, the density-corresponding voltage value becomes V03b in the case of Y/X=0, and the density-corresponding voltage value becomes V03c in the case of Y/X=c0.

Then, the value of Y/X=c, at which the thermal conductivity-corresponding voltage value is V01a and the density-corresponding voltage value becomes V03a, is calculated using a proportional operation. That is to say, the value is determined by the following formula.

$$c=c0(V03a-V03b)/V03c$$

The third and the fourth calibration curves of Fig. 67 vary depending on the liquid temperature, and therefore, it is necessary that calibration curves corresponding to plural liquid temperatures should be obtained in advance and that the calibration curve used should be properly changed according to the liquid temperature.

Then, from the value of Y/X=c, a value of X and a value of Y at which the thermal conductivity-corresponding voltage value V01 becomes V01a are decided, whereby the quantity of ammonia generated can be calculated in the same manner as described above.

Preferred embodiments of the present invention have been described above, but it should be construed that the invention is in no way limited to those embodiments, and various modifications can be made without departing from the objects of the invention, for example, pulse voltage P, the number of sampling times, etc. can be properly modified.

## Claims

1. An identification sensor module containing, in an container,
a fluid detection part which has a temperature detector for fluid detection and a heating element disposed in the vicinity of the temperature detector for fluid detection and which is disposed on the identification target fluid side,
a fluid detection circuit connected to the fluid detection part, and
an identification operation part which performs identification of an identification target fluid based on an output of the fluid detection circuit.

2. An identification sensor module containing, in an container,
a fluid detection part which has a temperature detector for fluid detection and a heating element disposed in the vicinity of the temperature detector for fluid detection and which is disposed on the identification target fluid side,
a fluid detection circuit connected to the fluid detection part, and
an identification operation part which performs identification of an identification target fluid based on an output of the fluid detection circuit,
wherein the fluid detection part has a chip for fluid detection comprising the temperature detector for fluid detection and a lead one end of which is electrically connected to the chip for fluid detection and the other end of which is connected to a substrate of the fluid detection circuit, and
the lead has a spring structure.

3. The identification sensor module as claimed in claim 2, wherein the other end of the lead connected to a substrate of the fluid detection circuit is constructed so as to penetrate the substrate from the front surface to the back surface and so as to project from the back surface.

4. The identification sensor module as claimed in claim 2 to 3, wherein in the fluid detection part,
the chip for fluid detection is embedded in a synthetic resin mold, and
one end of the lead is connected to the synthetic resin mold.

5. The identification sensor module as claimed in claim 4, wherein the spring structure of the lead is constructed so as to bias the synthetic resin mold toward the inner surface of the container.

**6.** The identification sensor module as claimed in any one of claims 2 to 5, wherein the spring structure of the lead comprises a plate spring.

**7.** The identification sensor module as claimed in any one of claims 1 to 6, wherein the lead has a first linear portion close to the one end, a second linear portion located on the extension of the first linear portion and close to the other end, and a bent portion located between the first linear portion and the second linear portion.

**8.** An identification sensor module containing, in an container,
a fluid detection part having a temperature detector for fluid detection,
a fluid detection circuit comprising the temperature detector for fluid detection, and
an identification operation part which performs identification of an identification target fluid based on an output of the fluid detection circuit,
wherein the container is provided with a protruded portion which protrudes toward the outside of the container correspondingly to the position at which the fluid detection part is disposed.

**9.** The identification sensor module as claimed in claim 8, wherein the shape of the protruded portion is any one of circular, elliptical, elongated circular and rectangular shapes.

**10.** The identification sensor module as claimed in claim 1 or 2, wherein a depressed portion is formed on the interior side of the container correspondingly to the protruded portion.

**11.** The identification sensor module as claimed in claim 10, wherein the shape of the depressed portion is any one of circular, elliptical, elongated circular and rectangular shapes.

**12.** The identification sensor module as claimed in claim 10 or 11, wherein the fluid detection part is disposed in contact with the inner surface of the depressed portion.

**13.** The identification sensor module as claimed in any one of claims 10 to 12, wherein the depth of the depressed portion is larger than a smaller dimension between dimensions of the depressed portion in the direction crossing the depth direction at right angles.

**14.** The identification sensor module as claimed in any one of claims 10 to 13, wherein the fluid detection part has a chip for fluid detection comprising the temperature detector for fluid detection, a synthetic resin mold in which the chip for fluid detection is embedded, and a lead one end of which is connected to the synthetic resin mold and is electrically connected to the chip for fluid detection, and
the synthetic resin mold is disposed in the depressed portion.

**15.** The identification sensor module as claimed in any one of claims 10 to 14, wherein the fluid detection part is disposed in such a manner that the chip for fluid detection is in contact with the inner surface of the depressed portion.

**16.** The identification sensor module as claimed in claim 15, wherein the chip for fluid detection is in contact with the side surface of the depressed portion.

**17.** The identification sensor module as claimed in any one of claims 10 to 16, wherein the other end of the lead is connected to a substrate of the fluid detection circuit, and the lead has a spring structure which biases the synthetic resin mold toward the bottom surface of the depressed portion.

**18.** The identification sensor module as claimed in claim 17, wherein the spring structure of the lead is constructed so as to bias the synthetic resin mold toward the bottom surface of the depressed portion.

**19.** The identification sensor module as claimed in claim 17 or 18, wherein the spring structure comprises a plate spring.

**20.** The identification sensor module as claimed in any one of claims 14 to 19, wherein the lead has:

a first linear portion close to the one end,
a second linear portion located on the extension of the first linear portion and close to the other end, and
a bent portion located between the first linear portion and the second linear portion.

**21.** The identification sensor module as claimed in any one of claims 8 to 20, wherein the fluid detection part has a temperature detector for fluid detection and a heating element disposed in the vicinity of the temperature detector for fluid detection.

**22.** An identification sensor module comprising a fluid detection part which has a temperature detector for fluid detection and a heating element disposed in the vicinity of the temperature detector for fluid detection and which is disposed on the identification target fluid side and a fluid temperature detection part which has a temperature detector for fluid temperature detection, said fluid detection part and said fluid temperature detection part being disposed so as to be side by side apart from each other at a given distance in the horizontal direction to the fluid level of an identification target fluid.

**23.** The identification sensor module as claimed in claim 22, wherein to the fluid detection part is connected a fluid detection circuit, and an identification operation part which performs identification of an identification target fluid based on an output of the fluid detection circuit is installed.

**24.** The identification sensor module as claimed in claim 22 or 23, wherein a difference in position in the vertical direction between the fluid detection part and the fluid temperature detection part which are disposed so as to be side by side apart from each other at a given distance in the horizontal direction is not more than 2 mm.

**25.** The identification sensor module as claimed in any one of claims 1 to 24, wherein the fluid detection part is disposed in contact with the container on the identification target fluid side in such a manner that the fluid detection part is not exposed from the container on the identification target fluid side.

**26.** The identification sensor module as claimed in any one of claims 1 to 24, wherein the fluid detection part is disposed in such a manner that at least a part of the fluid detection part is exposed from the container on the identification target fluid side.

**27.** The identification sensor module as claimed in claim 26, wherein the exposed portion of the fluid detection part is covered with a hydrophilic film or a filter.

**28.** The identification sensor module as claimed in any one of claims 1 to 27, wherein the container is a container, and the container is constituted of a container main body located on the identification target fluid side and a cap located on the opposite side to the identification target fluid side.

**29.** The identification sensor module as claimed in claim 28, wherein the container main body is made of a metal.

**30.** The identification sensor module as claimed in any one of claims 1 to 29, wherein a part of the fluid detection circuit and the identification operation part are incorporated into an IC.

**31.** The identification sensor module as claimed in any one of claims 1 to 30, wherein the fluid detection part is constructed by embedding a thin film chip for fluid detection, which is obtained by forming a temperature detector for fluid detection made of a thin film on a chip substrate, in a synthetic resin mold so that one surface of the thin film chip may be exposed, and
the thin film chip for fluid detection is disposed so that the one surface thereof may be located on the identification target fluid side of the container.

**32.** The identification sensor module as claimed in any one of claims 1 to 31, wherein in the container, a fluid temperature detection part having a temperature detector for fluid temperature detection is contained,
the fluid detection circuit is connected to the temperature detector for fluid temperature detection, and
the fluid temperature detection part is disposed in the identification target fluid.

**33.** The identification sensor module as claimed in claim 32, wherein the fluid temperature detection part is disposed in contact with the container on the identification target fluid side in such a manner that the fluid temperature detection part is not exposed from the container on the identification target fluid side.

**34.** The identification sensor module as claimed in claim 32, wherein the fluid temperature detection part is disposed in such a manner that at least a part of the fluid temperature detection part is exposed from the container on the identification target fluid side.

**35.** The identification sensor module as claimed in claim 34, wherein the exposed portion of the fluid temperature detection part is covered with a hydrophilic film or a filter.

**36.** The identification sensor module as claimed in claim 35, wherein the fluid temperature detection part is constructed by embedding a thin film chip for fluid temperature detection, which is obtained by forming a temperature detector for fluid temperature detection made of a thin film on a chip substrate, in a synthetic resin mold so that one surface of the thin film chip may be exposed, and
the thin film chip for fluid temperature detection is disposed so that the one surface thereof may be located on the identification target fluid side of the container.

**37.** A fluid identification device having the identification sensor module of any one of claims 1 to 36.

**38.** The fluid identification device as claimed in claim 37, wherein a measuring target fluid inlet part whose both ends are open is formed through the region close to the fluid detection part and the fluid temperature detection part, and a difference in position in the vertical direction between the fluid detection part and the fluid temperature detection part in a measuring target fluid inlet passage is not more than 2 mm.

**39.** The fluid identification device as claimed in claim 37 or 38, wherein the identification sensor module is installed in a waterproof case, and
the identification sensor module is disposed in such a manner that the container on the fluid detection part side is exposed from the waterproof case on the identification target fluid side.

**40.** The fluid identification device as claimed in claim 39, wherein the identification sensor module is disposed in such a manner that the container on the fluid detection part side protrudes from the waterproof case on the identification target fluid side.

**41.** The fluid identification device as claimed in claim 40 or 41, wherein the container is a container, the container is constituted of a container main body located on the identification target fluid side and a cap located on the opposite side to the identification target fluid side, and a joint between the container main body and the cap is disposed inside the waterproof case.

**42.** The fluid identification device as claimed in any one of claims 39 to 41, wherein the waterproof case has a cover member that covers the container on the identification target fluid side, and inside the cover member, a passage of the identification target fluid is formed.

**43.** The fluid identification device as claimed in any one of claims 39 to 42, wherein a fluid level sensor module for detecting a fluid level of an identification target fluid is installed in the waterproof case.

**44.** The fluid identification device as claimed in any one of claims 39 to 43, wherein a power circuit part is encased in the waterproof case.

**45.** The fluid identification device as claimed in any one of claims 39 to 44, wherein a waterproof wiring extends out of the waterproof case.

**46.** The fluid identification device as claimed in any one of claims 37 to 45, wherein the identification of the identification target fluid is at least one of fluid type identification, concentration identification, identification of presence of fluid, identification of fluid temperature, flow rate identification, detection of leak of fluid and fluid level identification.

**47.** The fluid identification device as claimed in any one of claims 37 to 46, wherein the identification target fluid is any one of a hydrocarbon type liquid, an alcohol type liquid and a urea aqueous solution.

**48.** The fluid identification device as claimed in claim 46, which is constructed so that detection of a flow rate of the fluid may be performed based on an electrical property value of the temperature detector for fluid detection and an electrical property value of the temperature detector for fluid temperature detection, and identification of a fluid type may be performed by measuring electrical conductivity between the fluid detection part and the fluid temperature detection part.

**49.** The fluid identification device as claimed in claim 46, which is constructed so as to perform identification of a fluid type of the identification target fluid by:

disposing the fluid identification device in a fluid type identification chamber,
applying a pulse voltage to the heating element of the fluid detection part for a given period of time to heat the identification target fluid, which is temporarily staying in the fluid type identification chamber, by means of the heating element of the fluid detection part, and
identifying the fluid type of the identification target fluid by a voltage output difference corresponding to the temperature difference between the initial temperature of the temperature detector for fluid detection and the peak temperature thereof.

**50.** The fluid identification device as claimed in claim 46, which has:

a main passage through which the identification target fluid flows,
a sub-passage diverged from the main passage,
the fluid identification device installed in the sub-passage,
a sub-passage on-off valve which is installed in the sub-passage and controls flow of the identification target fluid to the fluid identification device, and
a control device to control the fluid identification device and the sub-passage on-off valve,

wherein the control device is constructed so as to perform control in such a manner that:

in the identification of the identification target fluid, the control device closes the sub-passage on-off valve to allow the identification target fluid to temporarily stay in the fluid identification device and identifies the identification target fluid, and
in the detection of a flow rate of the identification target fluid, the control device opens the sub-passage on-off valve to allow the identification target fluid to flow to the fluid identification device and detects the flow rate of the identification target fluid.

**51.** The fluid identification device as claimed in claim 46, which has:

a fluid identification detection chamber in which the identification target fluid is allowed to temporarily stay,
the identification sensor module of the fluid identification device, which is placed in the fluid identification detection chamber, and
a flow control plate which is placed in the fluid identification detection chamber and surrounds the identification sensor module.

**52.** The fluid identification device as claimed in claim 46, which is constructed so as to perform identification of a concentration of the identification target fluid by:

disposing the fluid identification device in a fluid type identification chamber,
applying a pulse voltage to the heating element of the fluid detection part for a given period of time to heat the identification target fluid, which is temporarily staying in the fluid type identification chamber, by means of the heating element of the fluid detection part, and
identifying the concentration of the identification target fluid by a voltage output difference corresponding to the temperature difference between the initial temperature of the temperature detector for fluid detection and the peak temperature thereof.

**53.** The fluid identification device as claimed in claim 46, which is placed in a measuring fine tube at the lower end of which the identification target fluid in a tank is introduced or discharged, and performs detection of leak of the identification target fluid from the tank by:

obtaining an output corresponding to a difference between temperatures detected by the temperature detector for fluid detection in the fluid detection part and the temperature detector for fluid temperature detection in the fluid temperature detection part,
detecting a specific gravity of the identification target fluid in the tank based on a flow rate-corresponding value corresponding to a flow rate of the identification target fluid that is calculated using the above-obtained output, measuring a fluid level of the identification target fluid by the fluid level sensor module using the resulting specific gravity value, and detecting leak of the identification target fluid from the tank based on the magnitude of a time change rate of the fluid level.

**54.** The fluid identification device as claimed in claim 46, which is constructed so as to perform detection of a fluid level of the identification target fluid by:

detecting a fluid pressure of the identification target fluid by the fluid level sensor module and calculating a temporary fluid level value based on the fluid pressure on the assumption that the identification target fluid is a fluid of a given density,
applying a pulse voltage to the heating element of the fluid detection part for a given period of time to identify a concentration of the identification target fluid by means of the heating element of the fluid detection part,
obtaining a density value of the identification target fluid based on the relationship between the identified concentration and the density of the identification target fluid, and
calculating the fluid level of the identification target fluid based on the temporary fluid level value and the density value.

**55.** A device for measuring a quantity of ammonia generated, which is a device equipped with the fluid identification device of any one of claims 37 to 45 and for measuring a quantity of ammonia generated from an identification target liquid comprising urea water, ammonium formate water or mixed water thereof, wherein the quantity of ammonia generated is measured by:

using a sensor equipped with a heating element and a temperature detector disposed in the vicinity of the heating element,
applying a pulse voltage to the heating element for a given period of time to heat the measuring target liquid by means of the heating element,
then, by an electrical output corresponding to electrical resistance of the temperature detector, measuring a thermal conductivity-corresponding output value which is an electrical output of the temperature detector dependent on the thermal conductivity of the measuring target liquid,
measuring a density-corresponding output value which is an electrical output dependent on the density of the measuring target liquid, using a differential pressure sensor,
calculating a urea concentration X% by weight and an ammonium formate concentration Y% by weight of the measuring target liquid, from the relationship between the thermal conductivity-corresponding output value and the density-corresponding output value,
calculating a urea quantity A and an ammonium formate quantity B contained in the measuring target liquid, from the concentration and the quantity of the measuring target liquid, and
determining the quantity of ammonia generated, by the following formula:

$$\text{Quantity of ammonia generated} = X \times A + Y \times B$$

**56.** A device for measuring a quantity of ammonia generated, which is a device equipped with the fluid identification device of any one of claims 37 to 45 and for measuring a quantity of ammonia generated from an identification target liquid comprising urea water, ammonium formate water or mixed water thereof, wherein the quantity of ammonia generated is measured by:

using a sensor equipped with a heating element and a temperature detector disposed in the vicinity of the heating element,
applying a pulse voltage to the heating element for a given period of time to heat the measuring target liquid by means of the heating element,
then, by an electrical output corresponding to electrical resistance of the temperature detector, measuring a thermal conductivity-corresponding output value which is an electrical output of the temperature detector dependent on the thermal conductivity of the measuring target liquid,
measuring a density-corresponding output value which is an electrical output dependent on the density of the measuring target liquid, using a differential pressure sensor,
calculating a urea concentration X% by weight and an ammonium formate concentration Y% by weight of the measuring target liquid, from the relationship between the thermal conductivity-corresponding output value and the density-corresponding output value,
calculating a urea quantity A and an ammonium formate quantity B contained in the measuring target liquid, from the concentration and the quantity of the measuring target liquid, and
determining the quantity of ammonia generated, by the following formula:

$$\text{Quantity of ammonia generated} = X \times A + Y \times B$$

**57.** A method for measuring a quantity of ammonia generated, which is a method for measuring a quantity of ammonia generated from an identification target liquid comprising urea water, ammonium formate water or mixed water thereof, using the fluid identification device of any one of claims 37 to 45, and comprises:

using a sensor equipped with a heating element and a temperature detector disposed in the vicinity of the heating element,
applying a pulse voltage to the heating element for a given period of time to heat the measuring target liquid by means of the heating element,
then, by an electrical output corresponding to electrical resistance of the temperature detector, measuring a thermal conductivity-corresponding output value which is an electrical output of the temperature detector dependent on the thermal conductivity of the measuring target liquid, and measuring a kinematic viscosity-corresponding output value which is an electrical output of the temperature detector dependent on the kinematic viscosity of the measuring target liquid,
calculating a urea concentration X% by weight and an ammonium formate concentration Y% by weight of the measuring target liquid, from the relationship between the thermal conductivity-corresponding output value and the kinematic viscosity-corresponding output value,
calculating a urea quantity A and an ammonium formate quantity B contained in the measuring target liquid, from the concentration and the quantity of the measuring target liquid, and
determining the quantity of ammonia generated, by the following formula:

$$\text{Quantity of ammonia generated} = X \times A + Y \times B$$

**58.** A method for measuring a quantity of ammonia generated, which is a method for measuring a quantity of ammonia generated from a measuring target liquid comprising urea water, ammonium formate water or mixed water thereof, using the fluid identification device of any one of claims 17 to 24, and comprises:

using a sensor equipped with a heating element and a temperature detector disposed in the vicinity of the heating element,
applying a pulse voltage to the heating element for a given period of time to heat the measuring target liquid by means of the heating element,
then, by an electrical output corresponding to electrical resistance of the temperature detector, measuring a thermal conductivity-corresponding output value which is an electrical output of the temperature detector dependent on the thermal conductivity of the measuring target liquid,
measuring a density-corresponding output value which is an electrical output dependent on the density of the measuring target liquid, using a differential pressure sensor,
calculating a urea concentration X% by weight and an ammonium formate concentration Y% by weight of the measuring target liquid, from the relationship between the thermal conductivity-corresponding output value and the density-corresponding output value,
calculating a urea quantity A and an ammonium formate quantity B contained in the measuring target liquid, from the concentration and the quantity of the measuring target liquid, and
determining the quantity of ammonia generated, by the following formula:

$$\text{Quantity of ammonia generated} = X \times A + Y \times B$$

**59.** A fluid identification method comprising performing identification of an identification target fluid using the fluid identification device of any one of claims 37 to 45.

[Fig. 1]

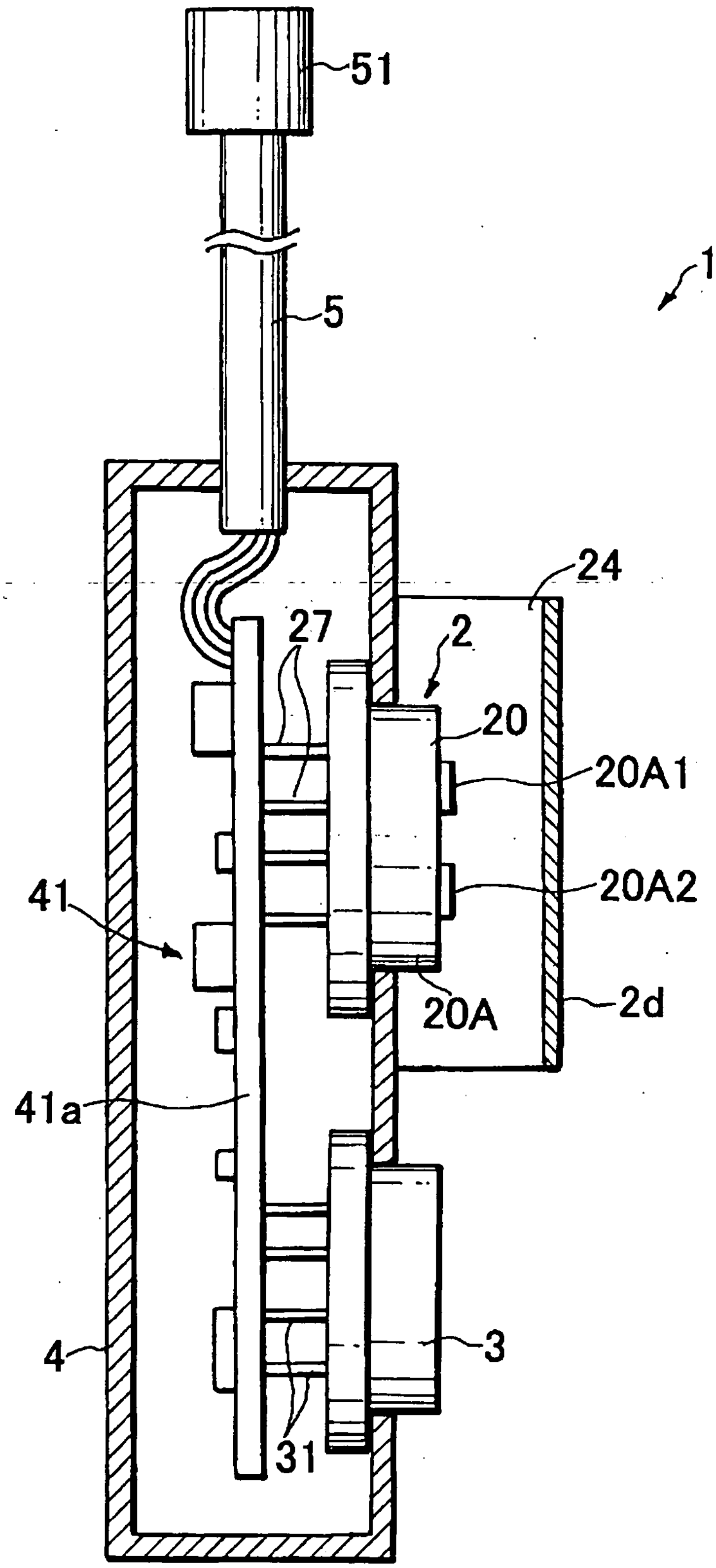

[Fig. 2]

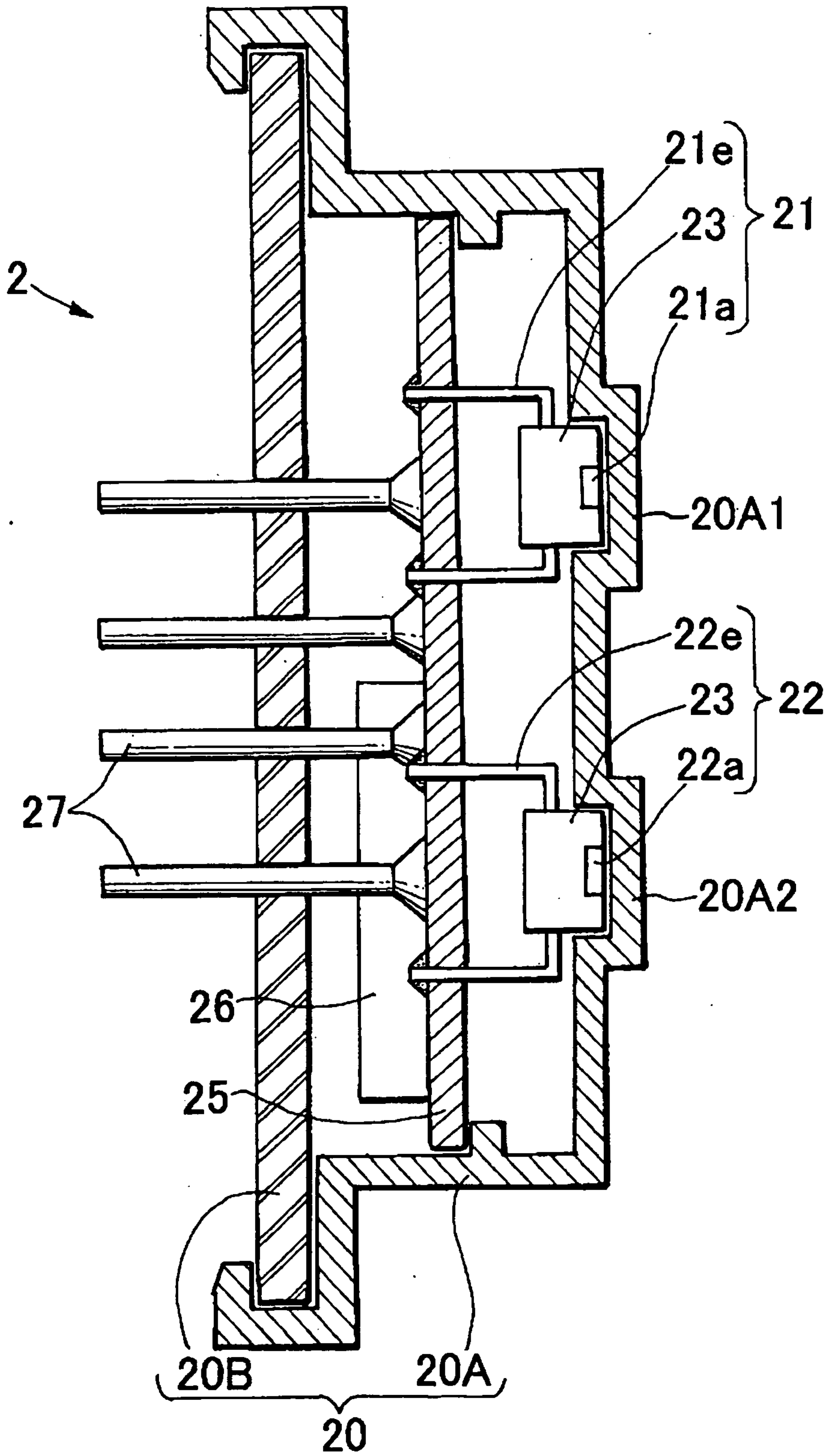

[Fig. 3]

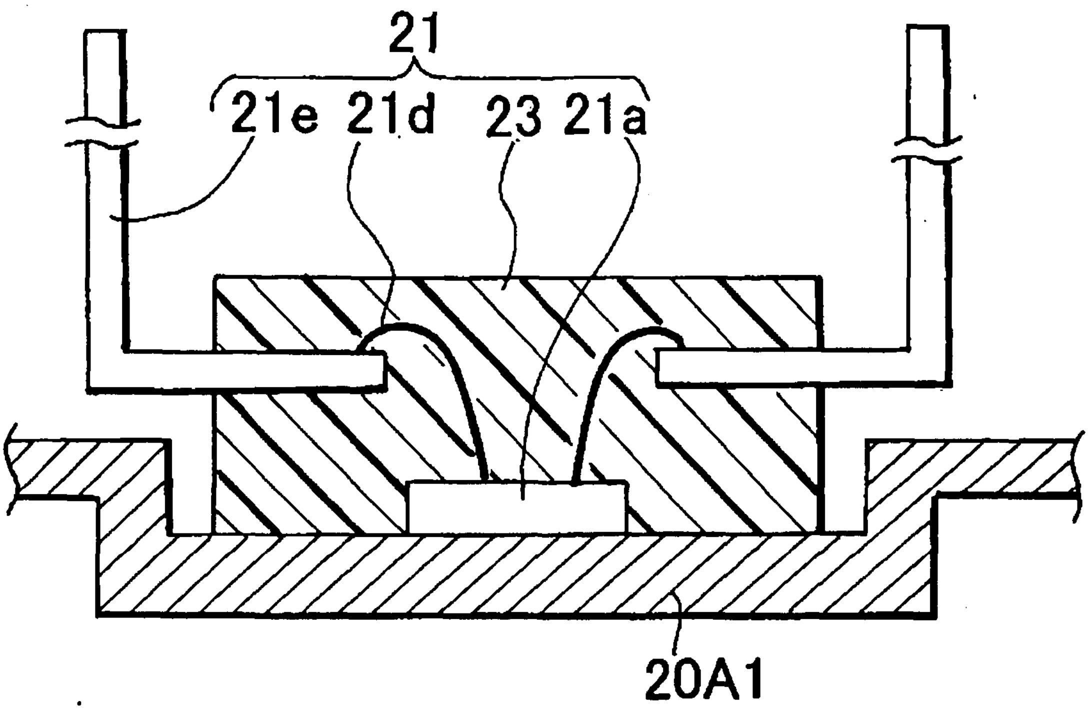

[Fig. 4]

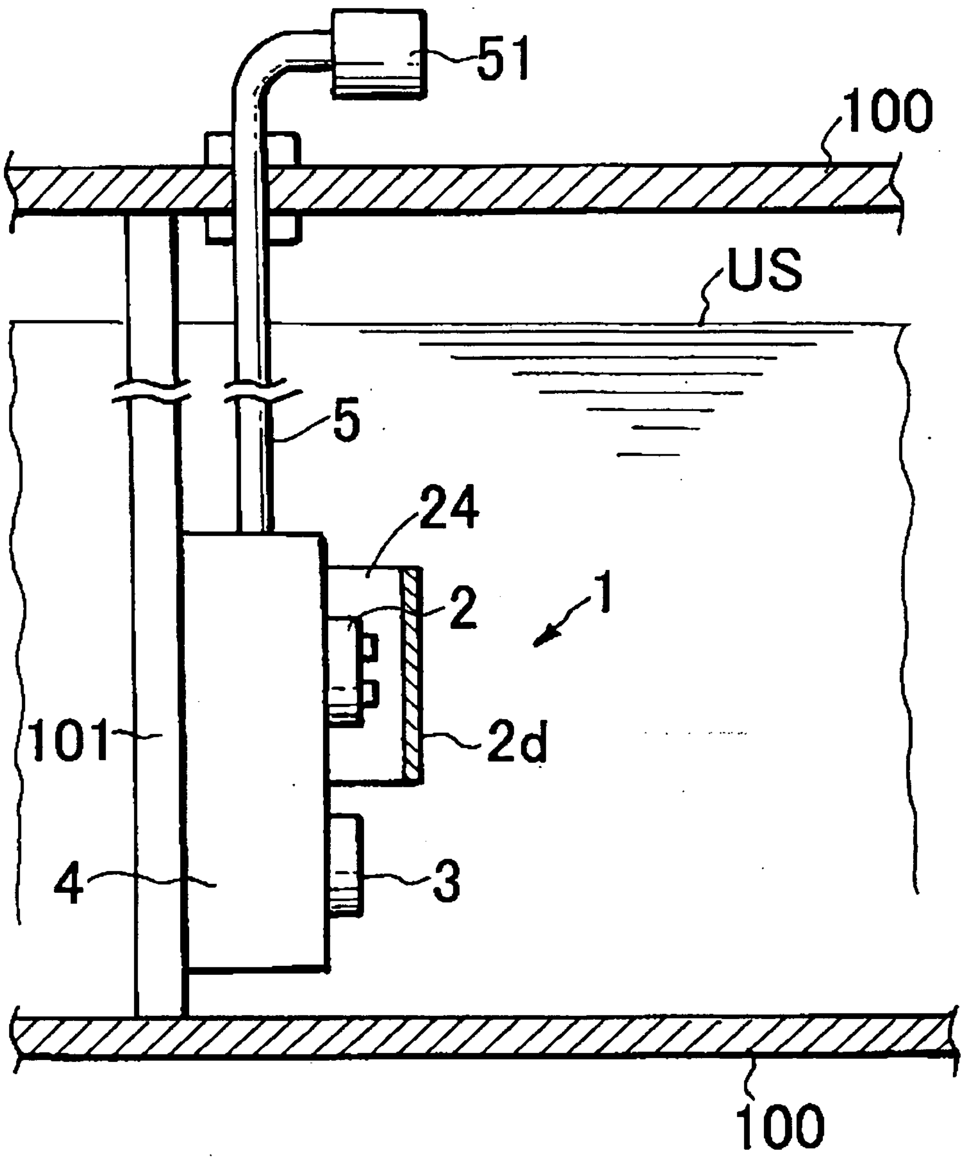

[Fig. 5]

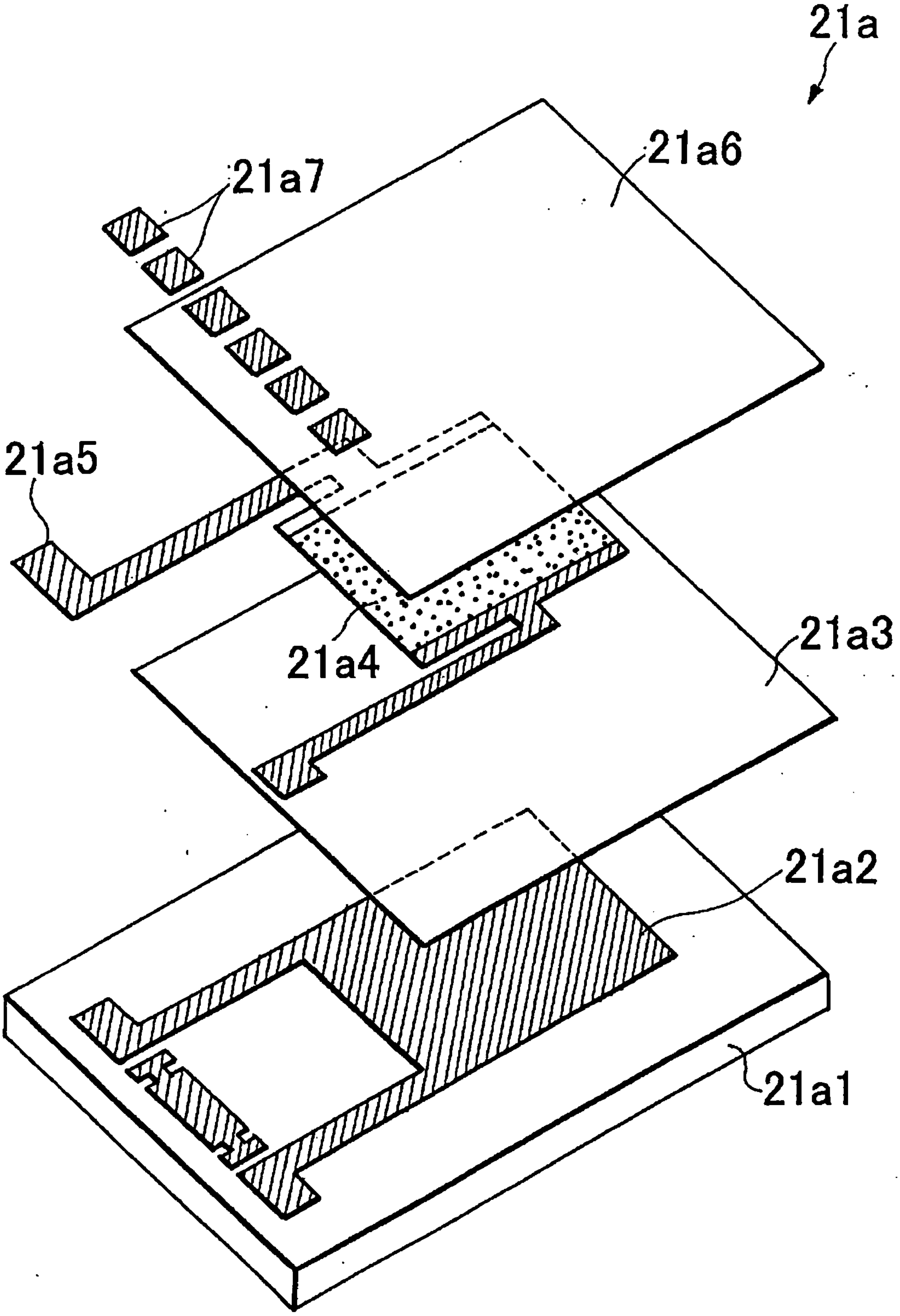

[Fig. 6]

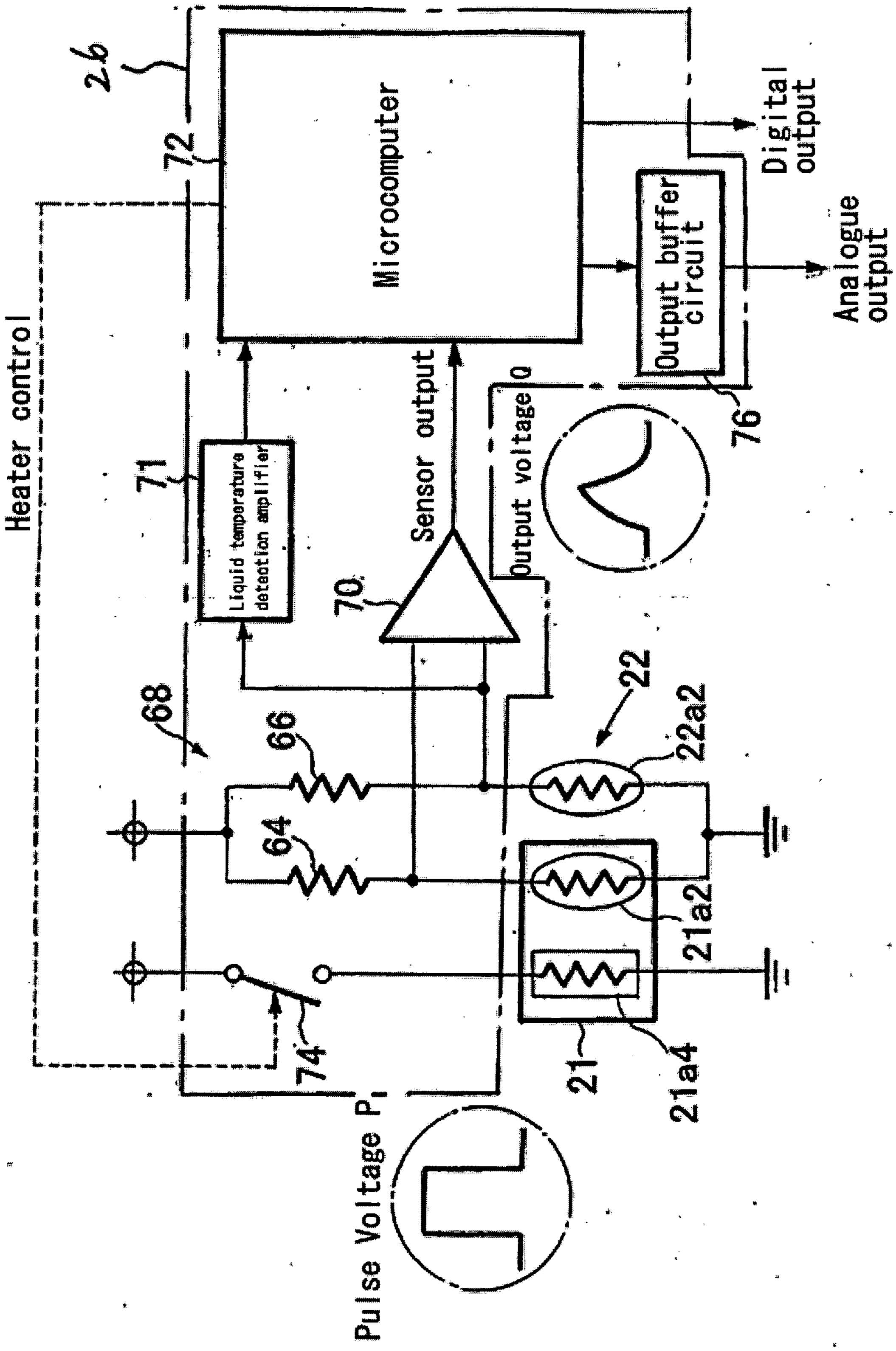

26
72
Microcomputer
Digital output
Output buffer circuit
Analogue output
Heater control
71
Liquid temperature detection amplifier
Sensor output
Output voltage Q
76
70
68
66
64
22
22a2
21a2
74
21
21a4
Pulse Voltage P

[Fig. 7]

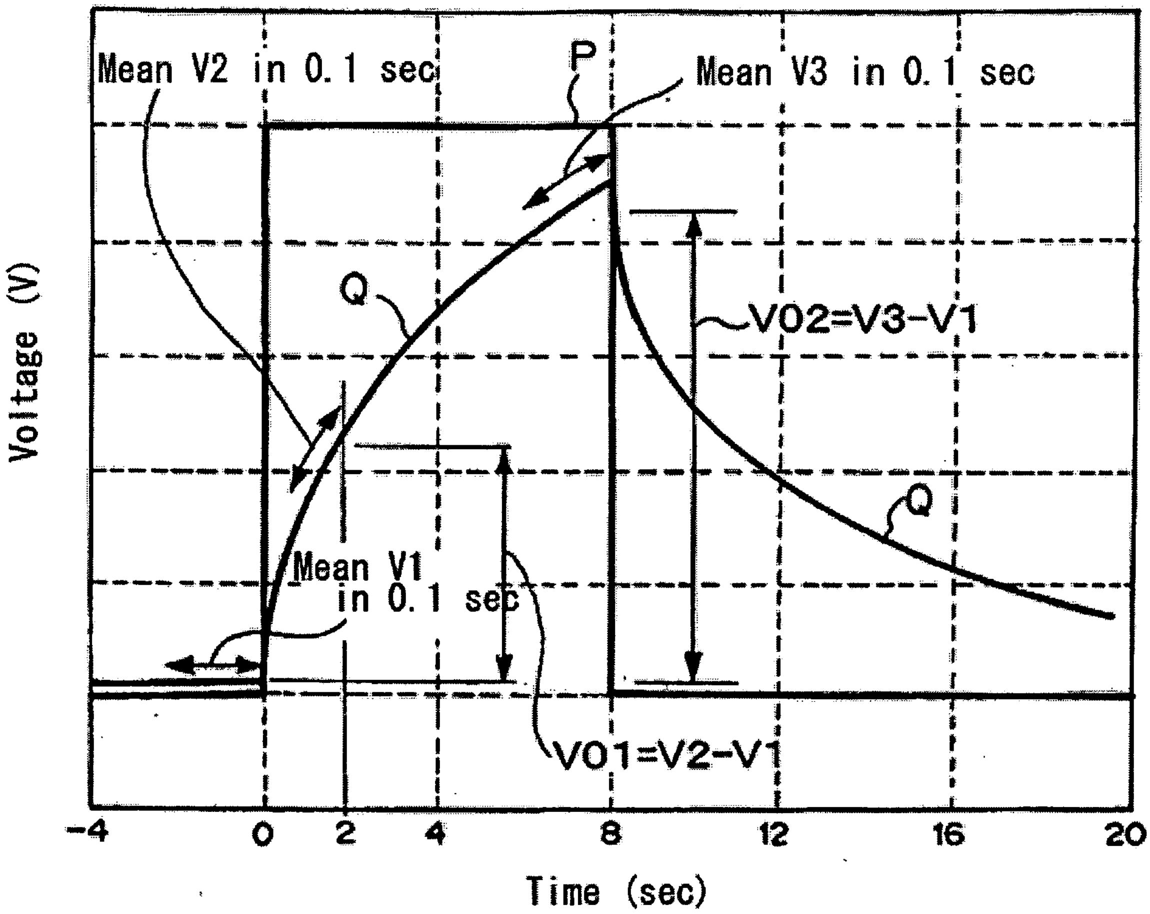
Mean V2 in 0.1 sec
P
Mean V3 in 0.1 sec
Q
V02=V3-V1
Q
Mean V1
in 0.1 sec
V01=V2-V1
Voltage (V)
-4
0
2
4
8
12
16
20
Time (sec)

[Fig. 8]

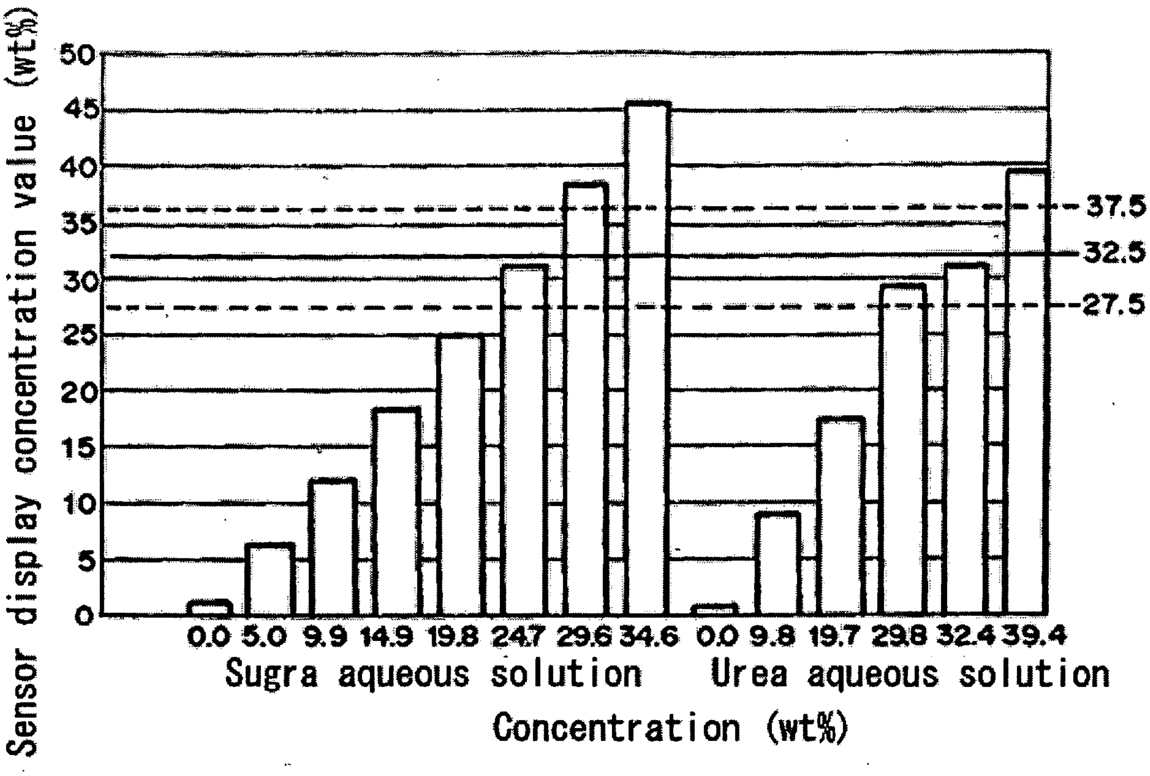
Sensor display concentration value (wt%)
0
5
10
15
20
25
30
35
40
45
50
37.5
32.5
27.5
0.0 5.0 9.9 14.9 19.8 24.7 29.6 34.6 0.0 9.8 19.7 29.8 32.4 39.4
Sugra aqueous solution
Urea aqueous solution
Concentration (wt%)

[Fig. 9]

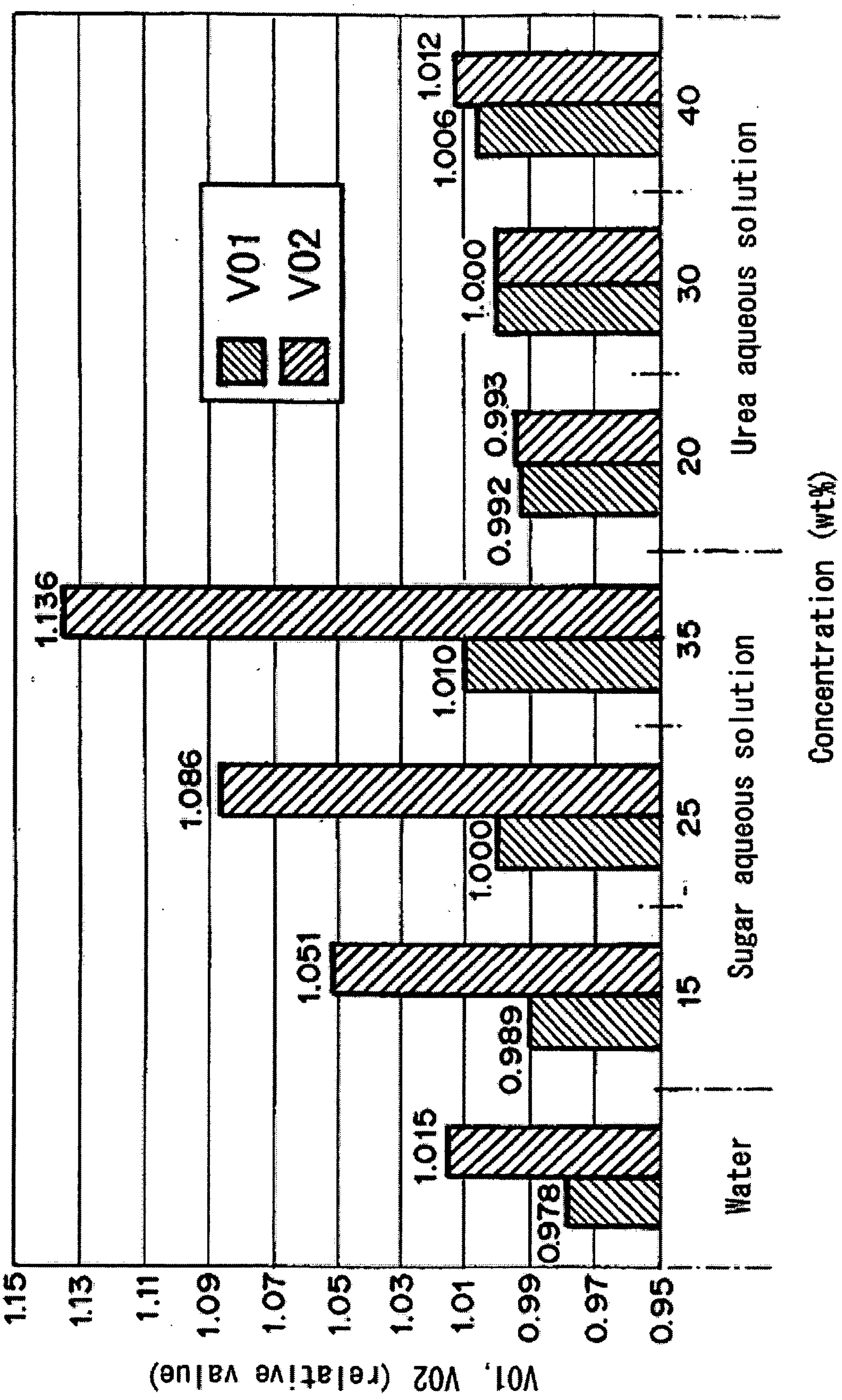
V01
V02
1.015
0.978
Water
0.989
1.051
15
1.000
1.086
25
1.010
1.136
35
Sugar aqueous solution
0.992
0.993
20
1.000
30
1.006
1.012
40
Urea aqueous solution
Concentration (wt%)
V01, V02 (relative value)
0.95
0.97
0.99
1.01
1.03
1.05
1.07
1.09
1.11
1.13
1.15

[Fig. 10]

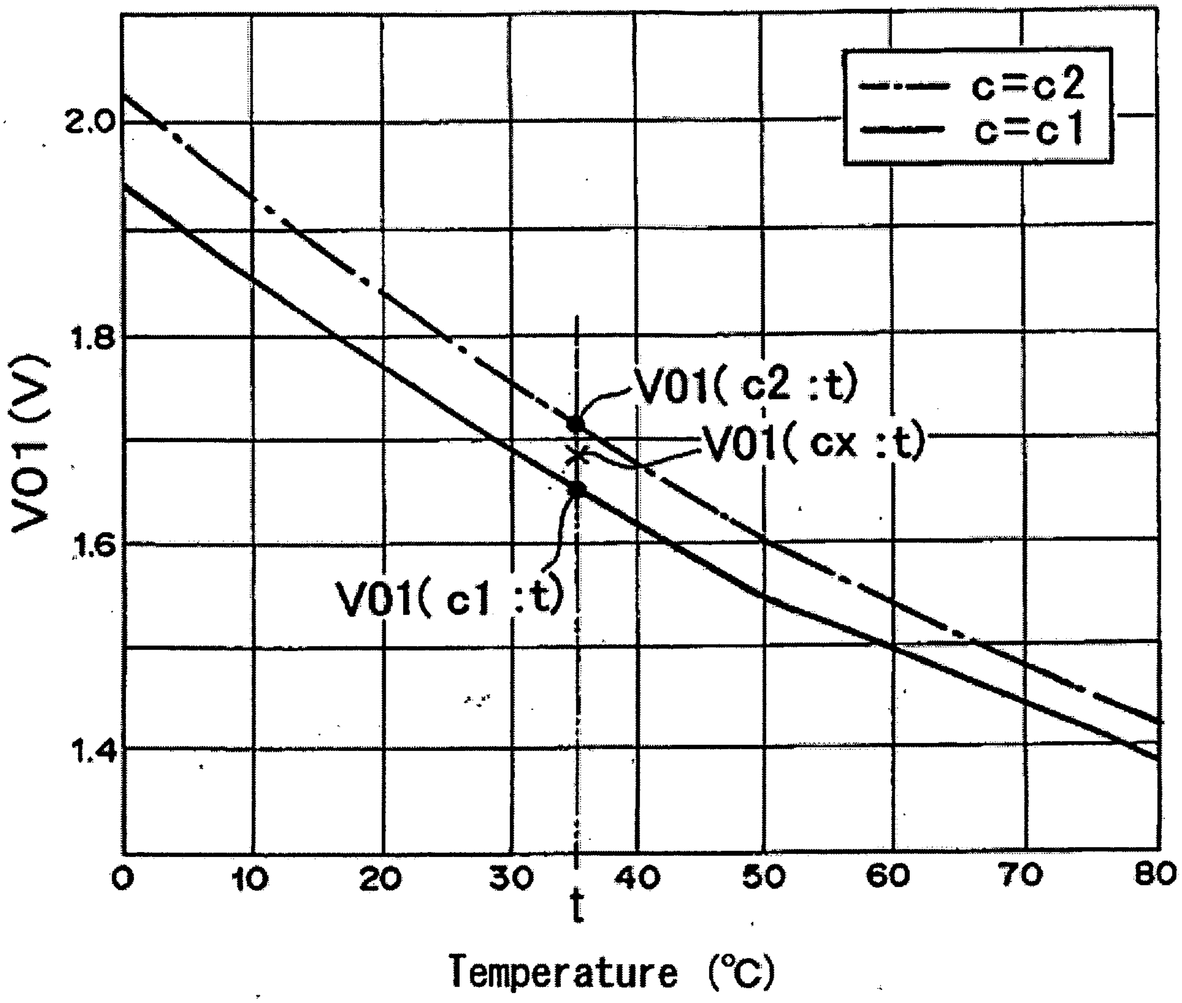
c=c2
c=c1
2.0
1.8
1.6
1.4
V01 (V)
V01( c2 :t)
V01( cx :t)
V01( c1 :t)
0
10
20
30
40
50
60
70
80
t
Temperature (℃)

[Fig. 11]

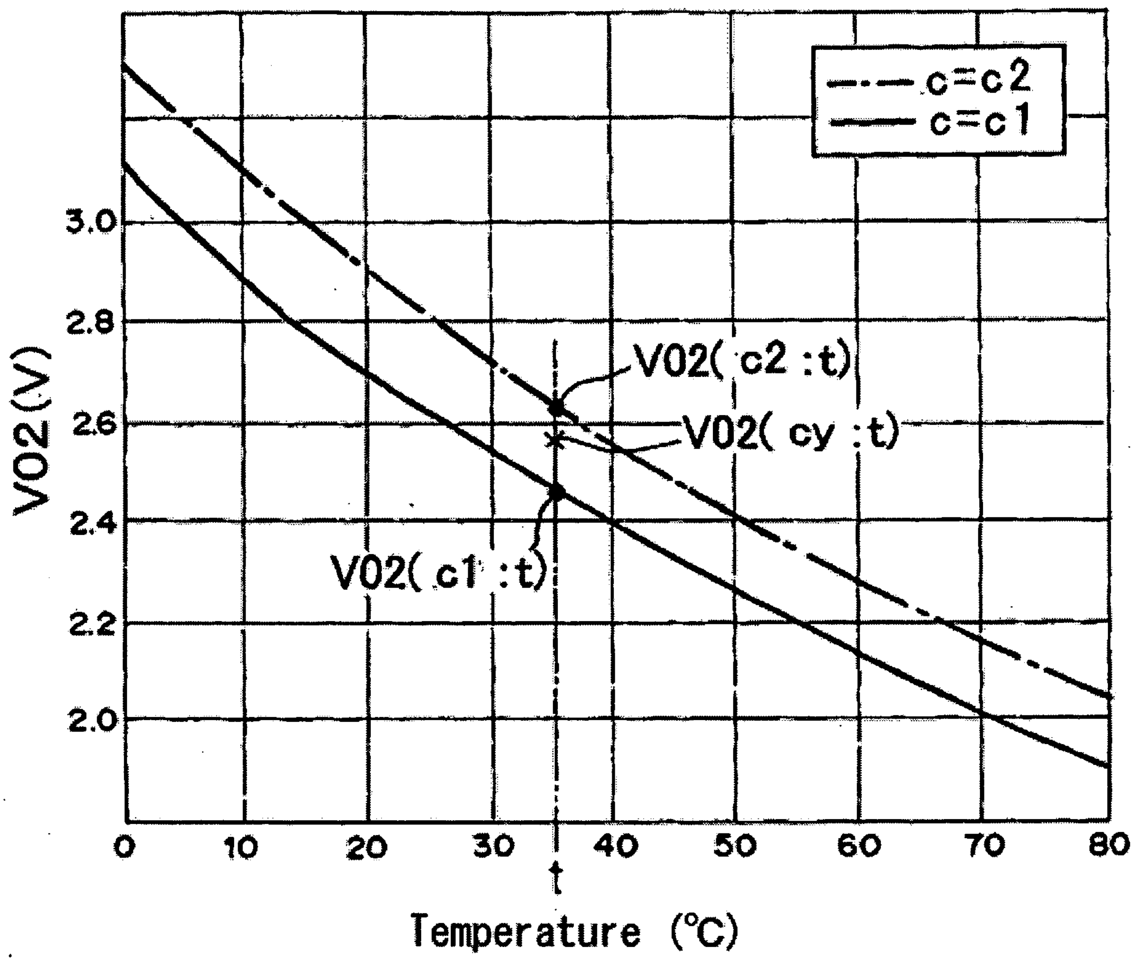
c=c2
c=c1
3.0
2.8
2.6
2.4
2.2
2.0
V02(V)
V02( c2 :t)
V02( cy :t)
V02( c1 :t)
0
10
20
30
40
50
60
70
80
t
Temperature (°C)

[Fig. 12]

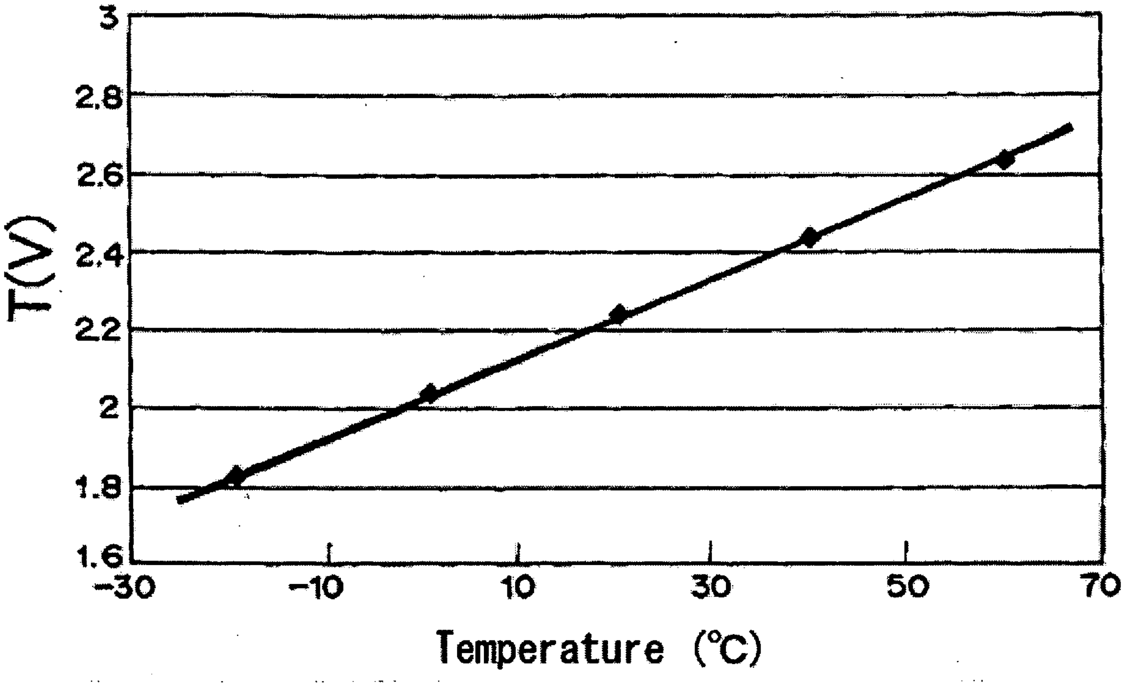

[Fig. 13]

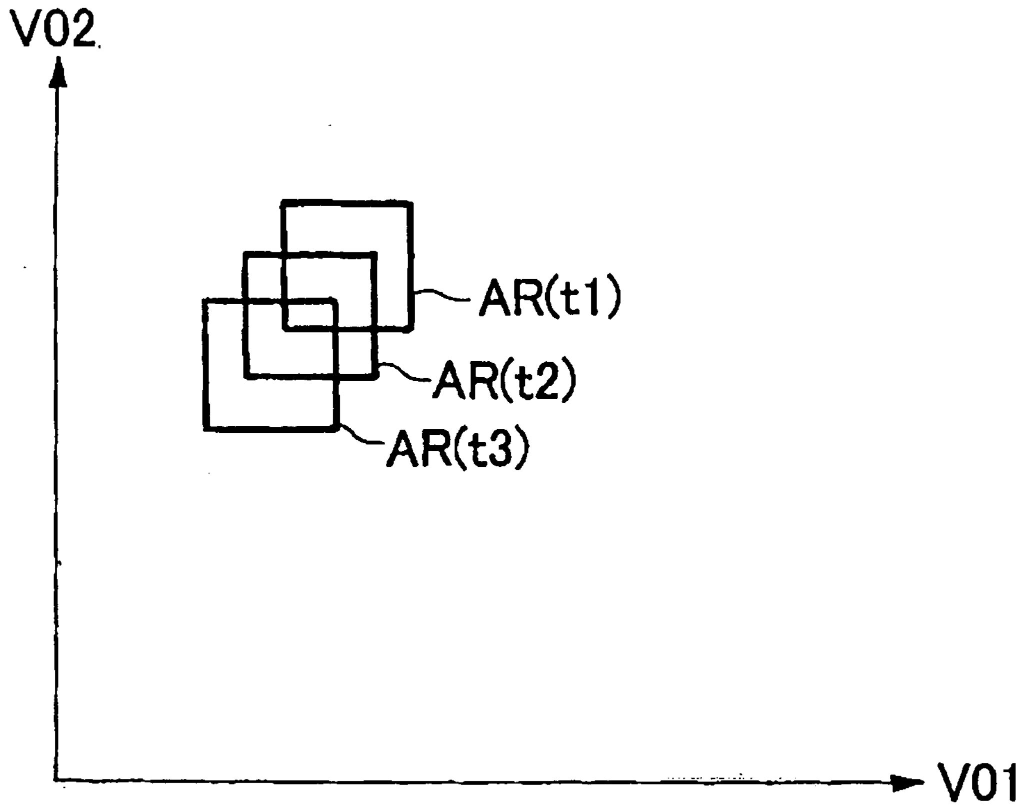

[Fig. 14]

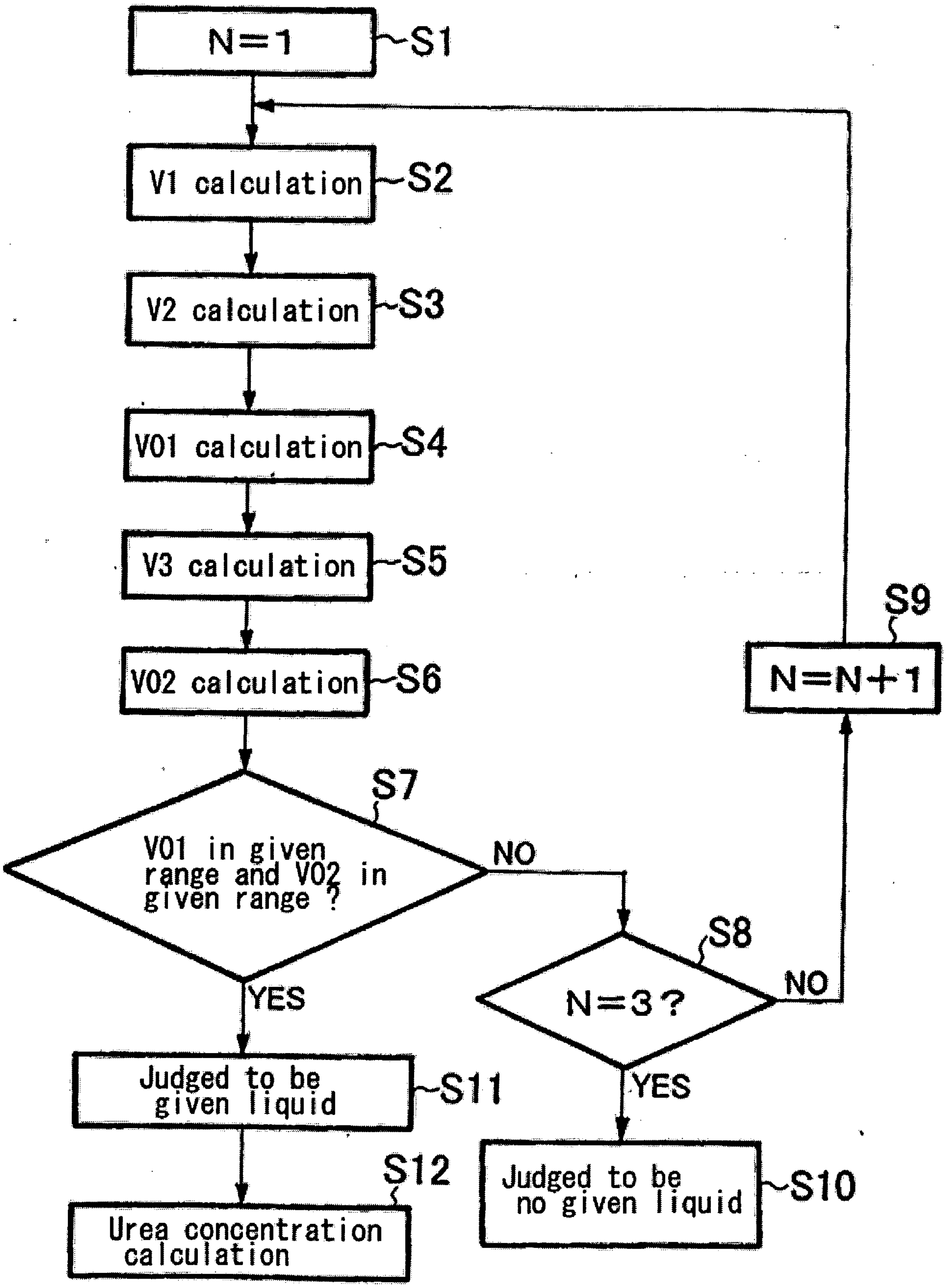
N=1
S1
V1 calculation
S2
V2 calculation
S3
V01 calculation
S4
V3 calculation
S5
V02 calculation
S6
V01 in given range and V02 in given range ?
S7
NO
YES
N=3?
S8
NO
YES
N=N+1
S9
Judged to be given liquid
S11
Urea concentration calculation
S12
Judged to be no given liquid
S10

[Fig. 15]

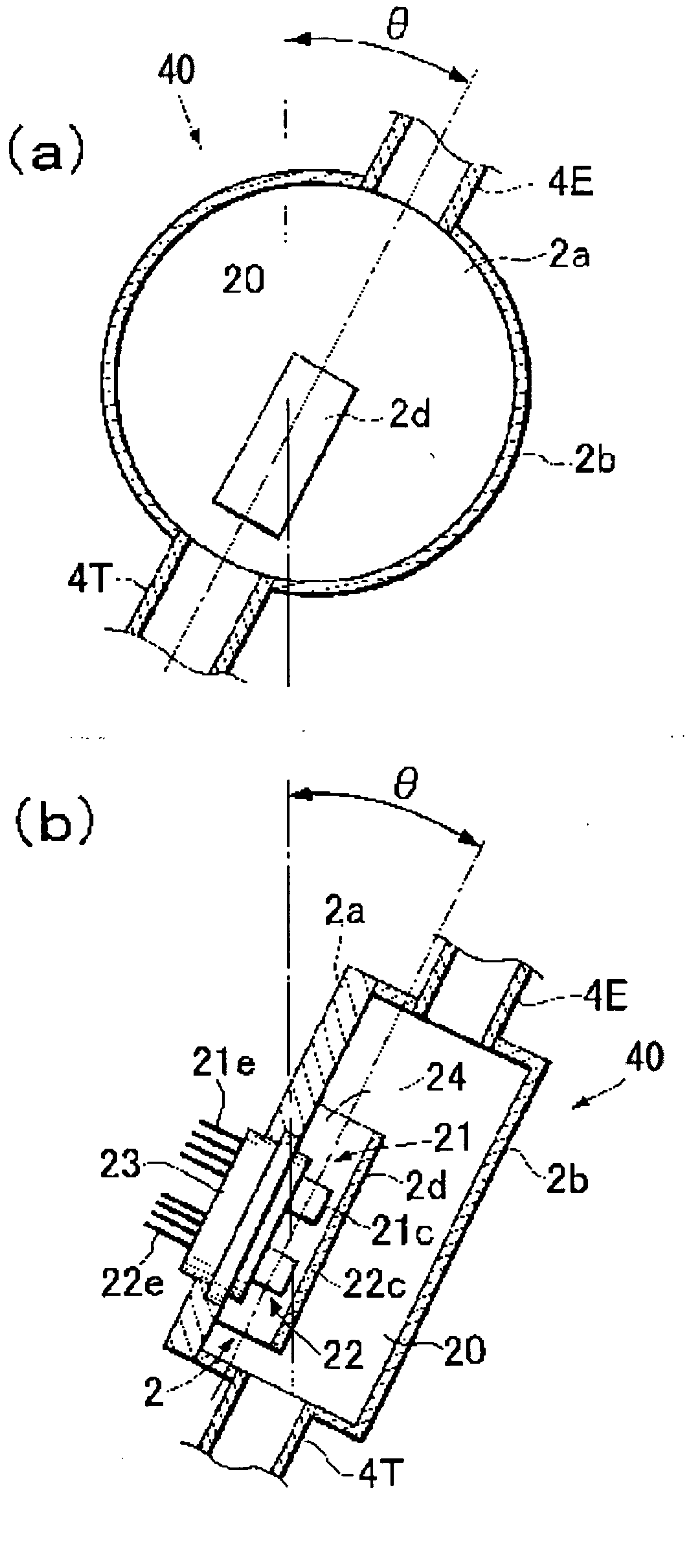
(a)
θ
40
4E
2a
20
2d
2b
4T
(b)
θ
2a
4E
40
21e
24
21
23
2d
2b
21c
22e
22c
22
20
2
4T

[Fig. 16]

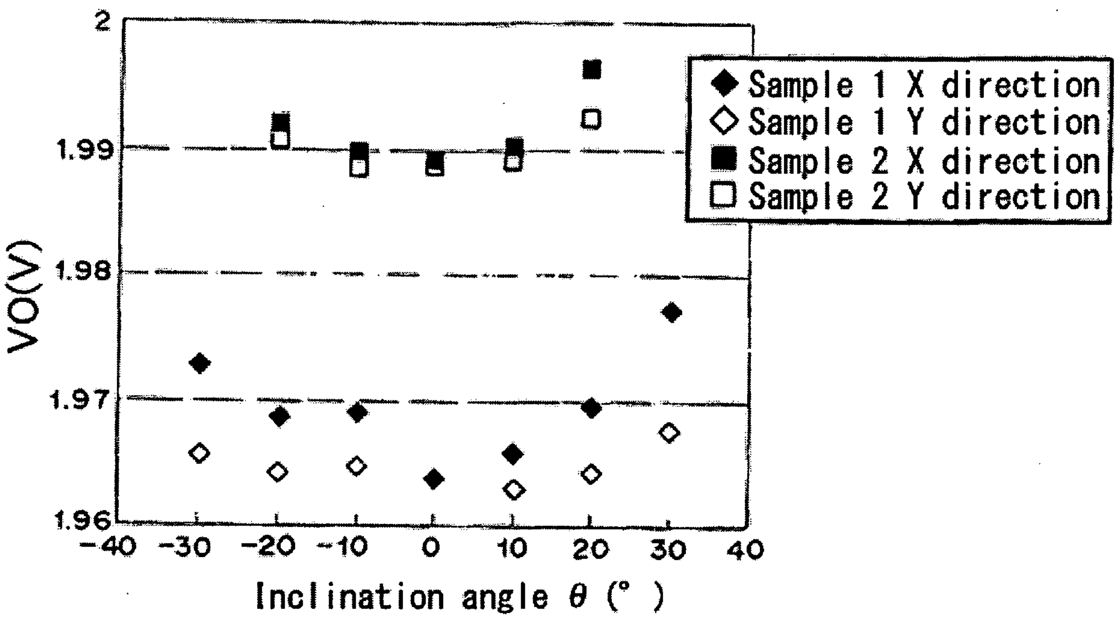
2
1.99
1.98
1.97
1.96
VO(V)
-40 -30 -20 -10 0 10 20 30 40
Inclination angle θ (° )
Sample 1 X direction
Sample 1 Y direction
Sample 2 X direction
Sample 2 Y direction

[Fig. 17]

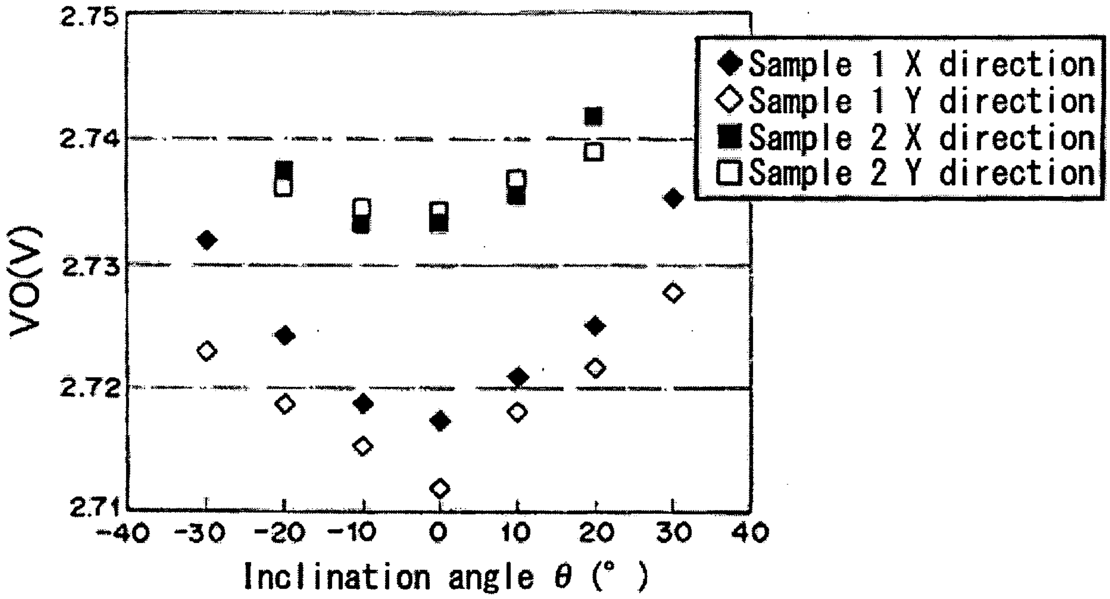
2.75
2.74
2.73
2.72
2.71
VO(V)
-40 -30 -20 -10 0 10 20 30 40
Inclination angle θ (° )
Sample 1 X direction
Sample 1 Y direction
Sample 2 X direction
Sample 2 Y direction

[Fig. 18]

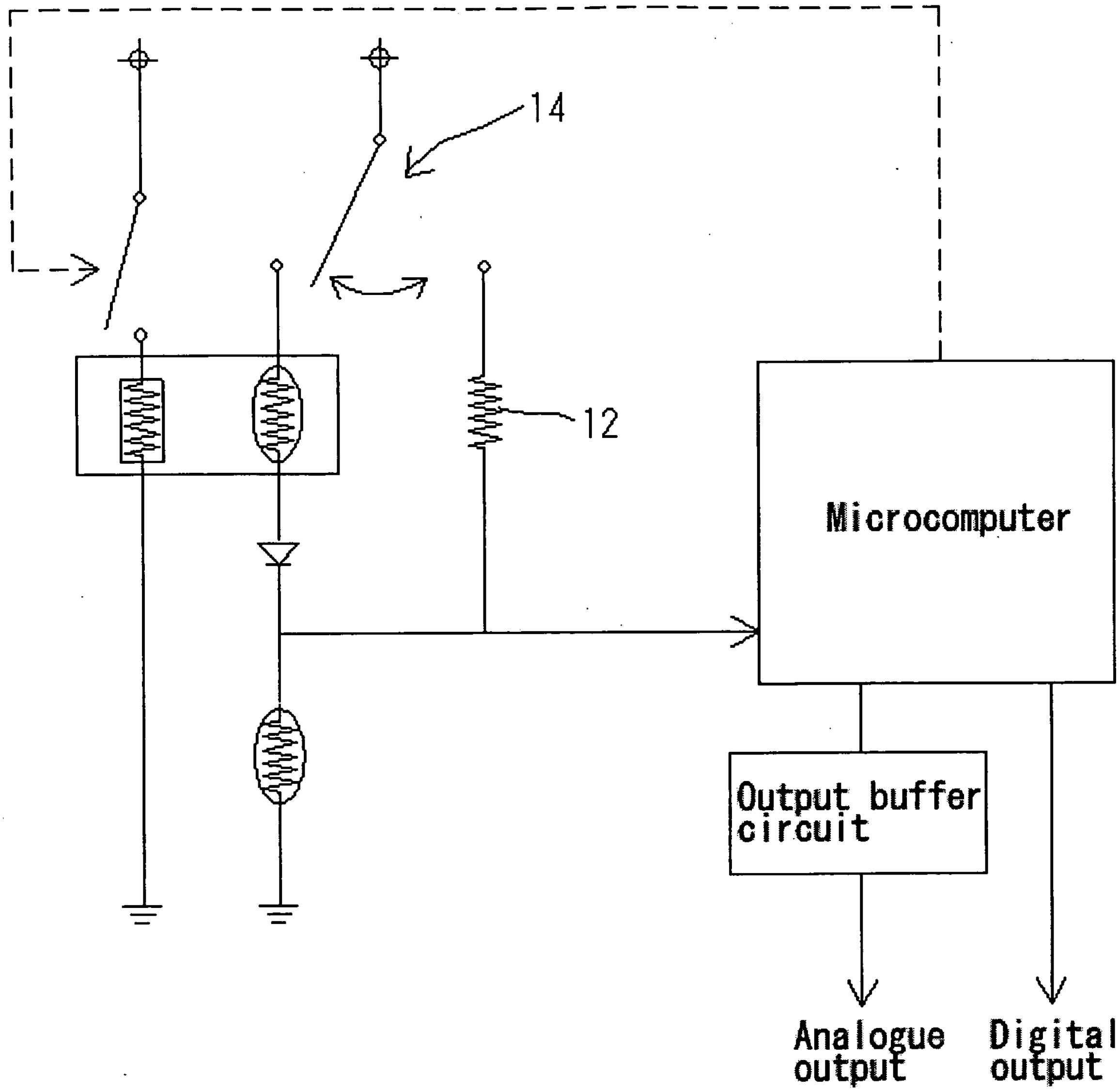
14
12
Microcomputer
Output buffer circuit
Analogue output
Digital output

[Fig. 19]

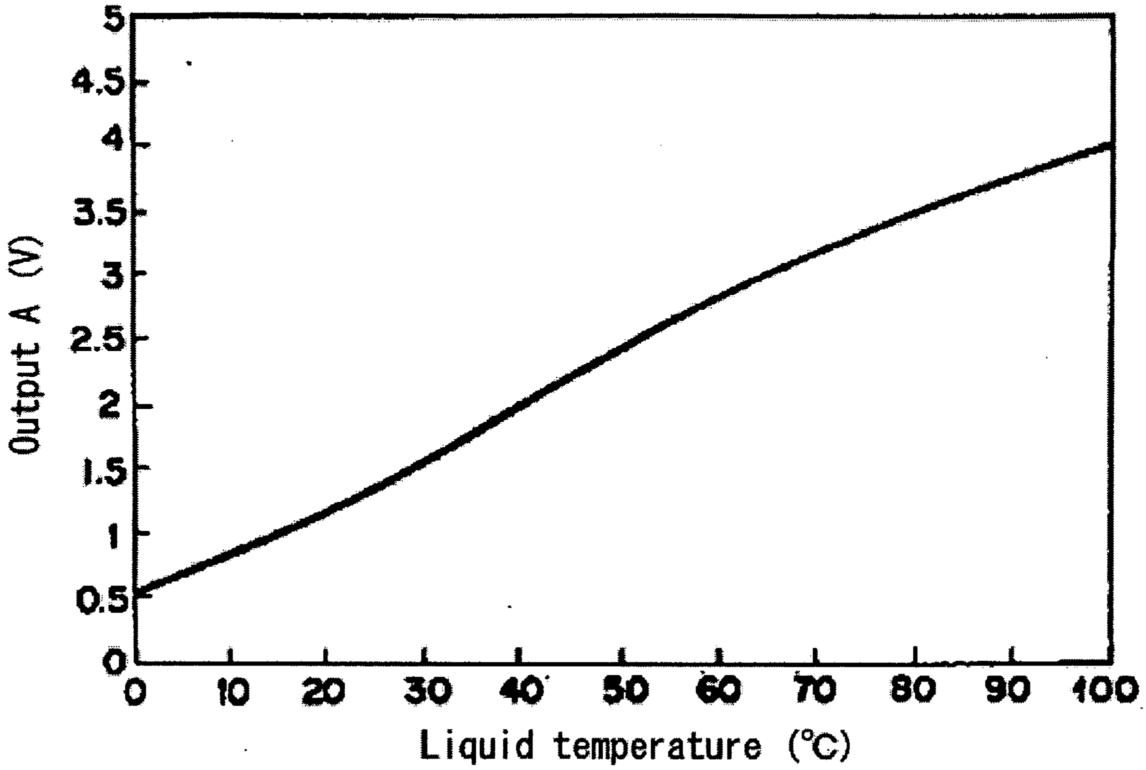
5
4.5
4
3.5
3
2.5
2
1.5
1
0.5
0
Output A (V)
0
10
20
30
40
50
60
70
80
90
100
Liquid temperature (℃)

[Fig. 20]

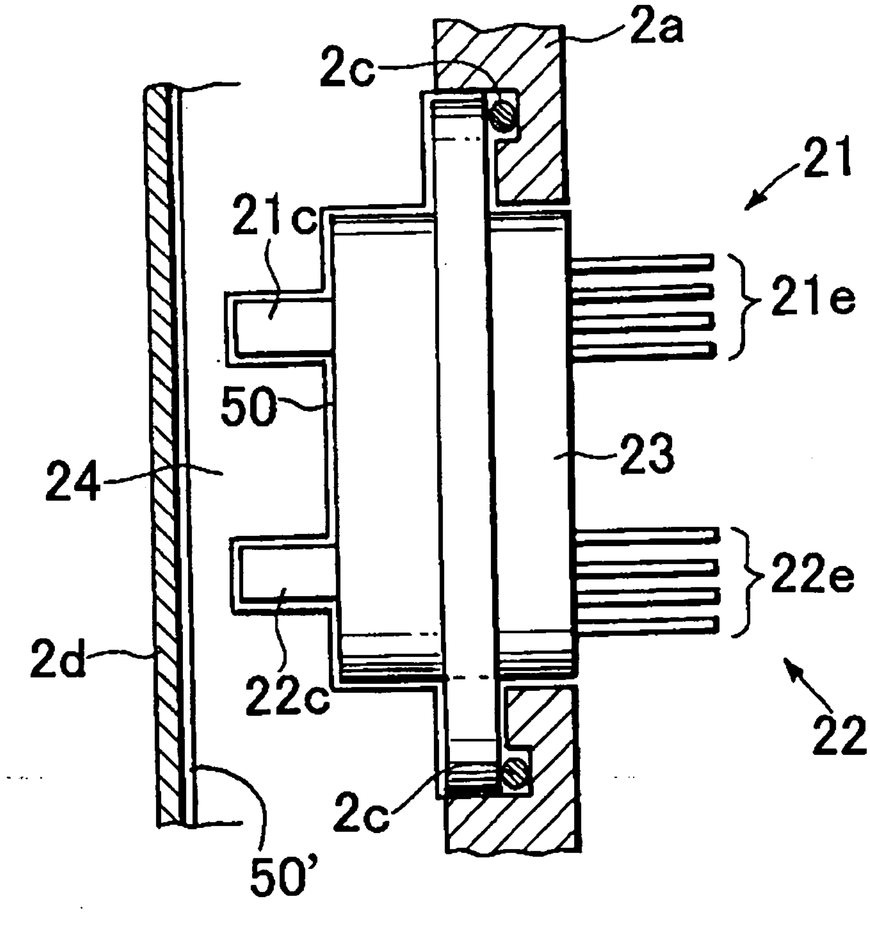

[Fig. 21]

(A)

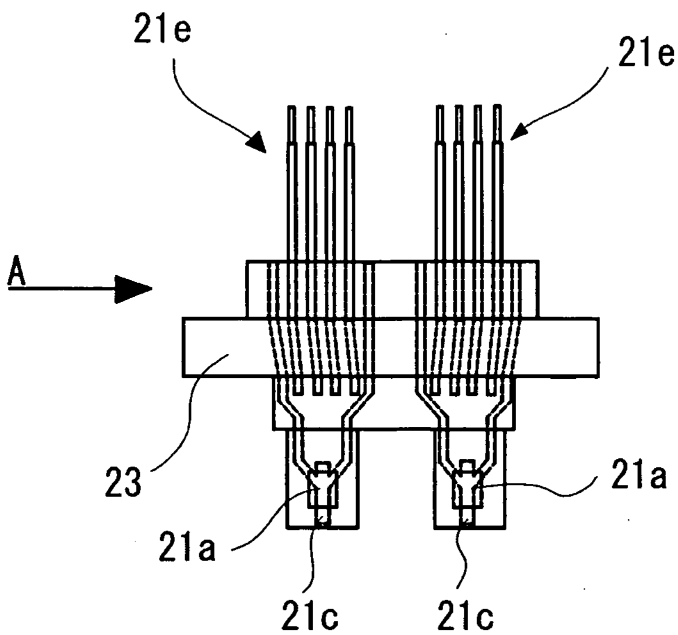


(B)

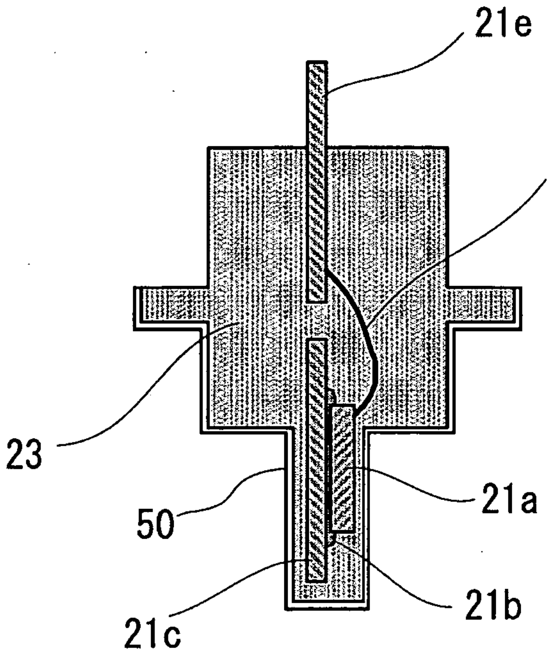

[Fig. 22]

(A)

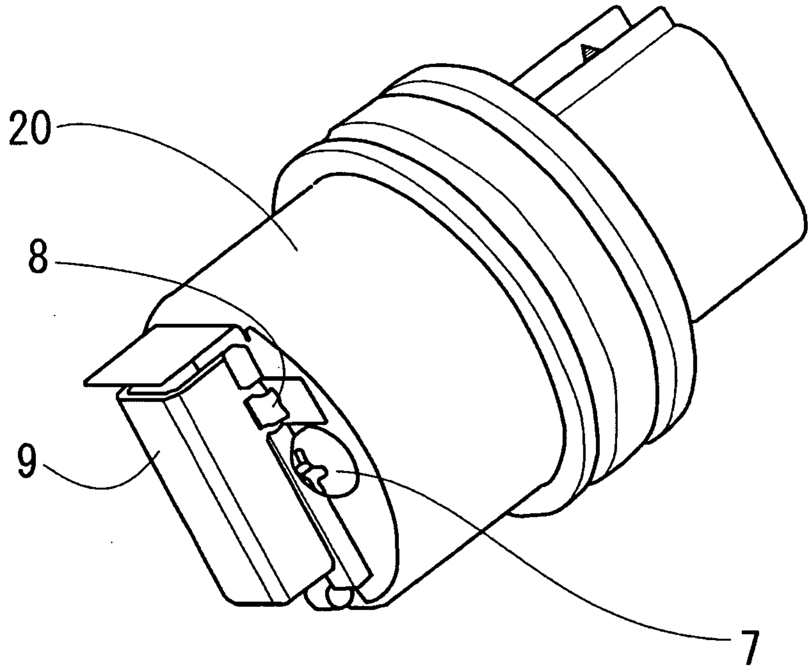


(B)

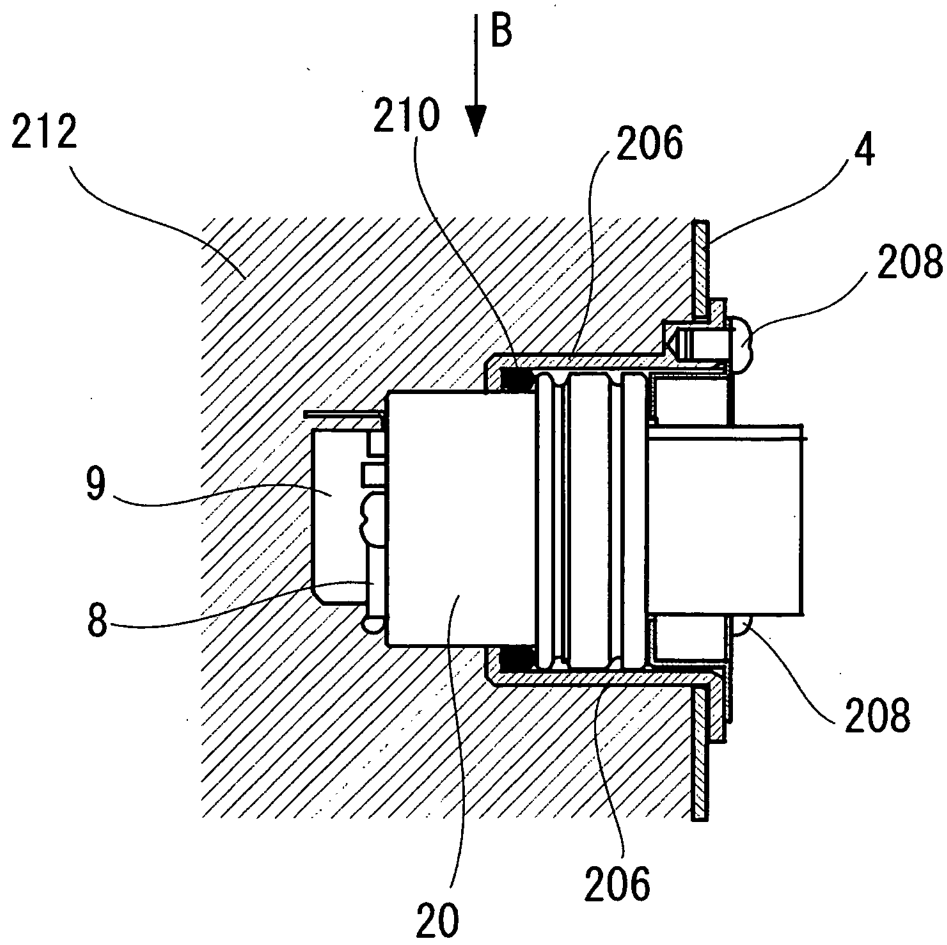

[Fig. 23]

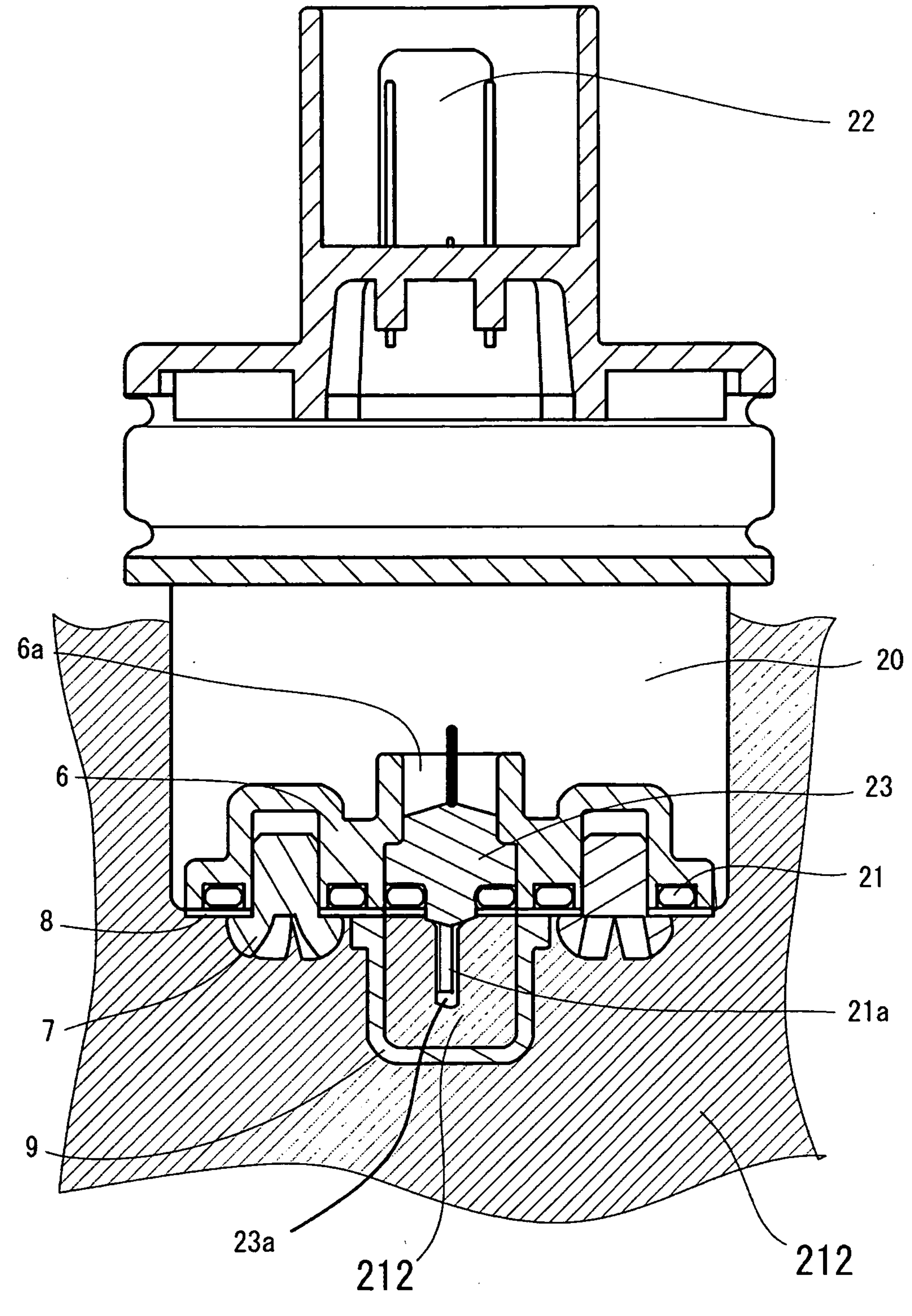

[Fig. 24]

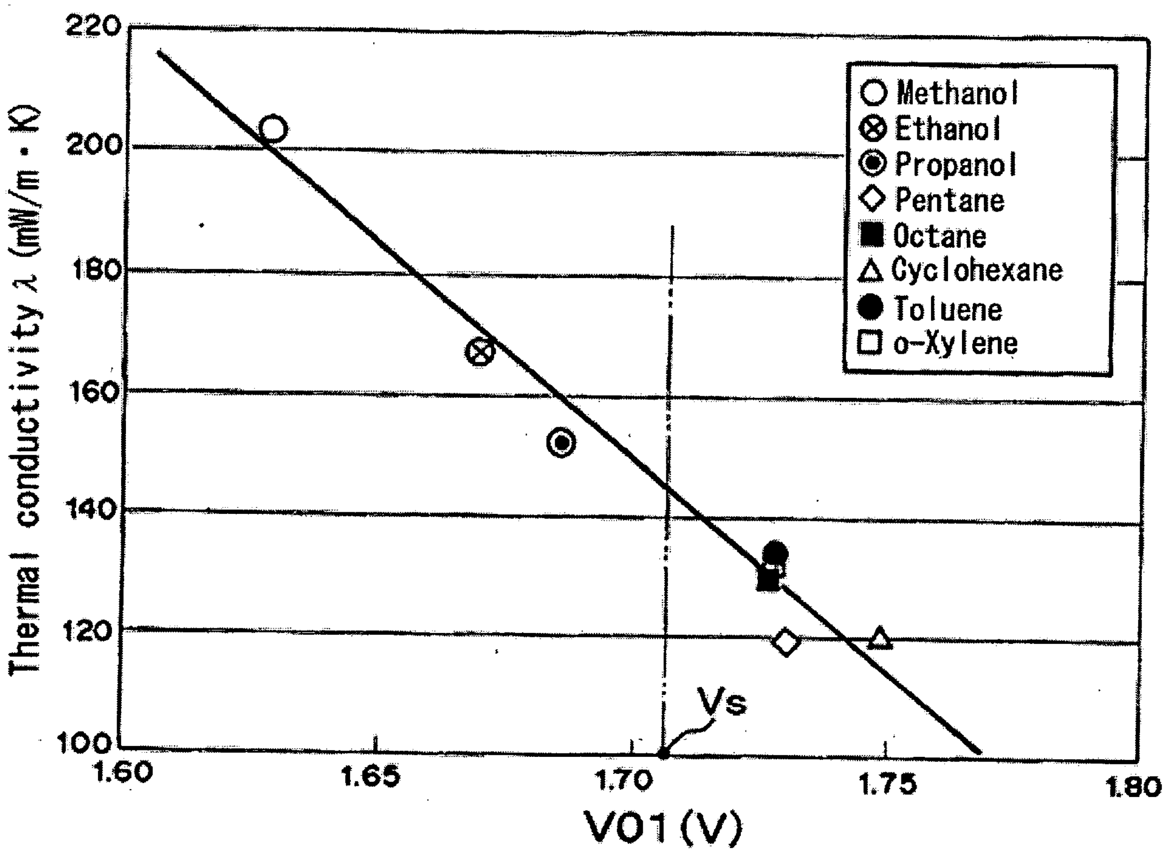
Thermal conductivity λ (mW/m・K)
220
200
180
160
140
120
100
1.60
1.65
1.70
1.75
1.80
V01 (V)
Vs
Methanol
Ethanol
Propanol
Pentane
Octane
Cyclohexane
Toluene
o-Xylene

[Fig. 25]

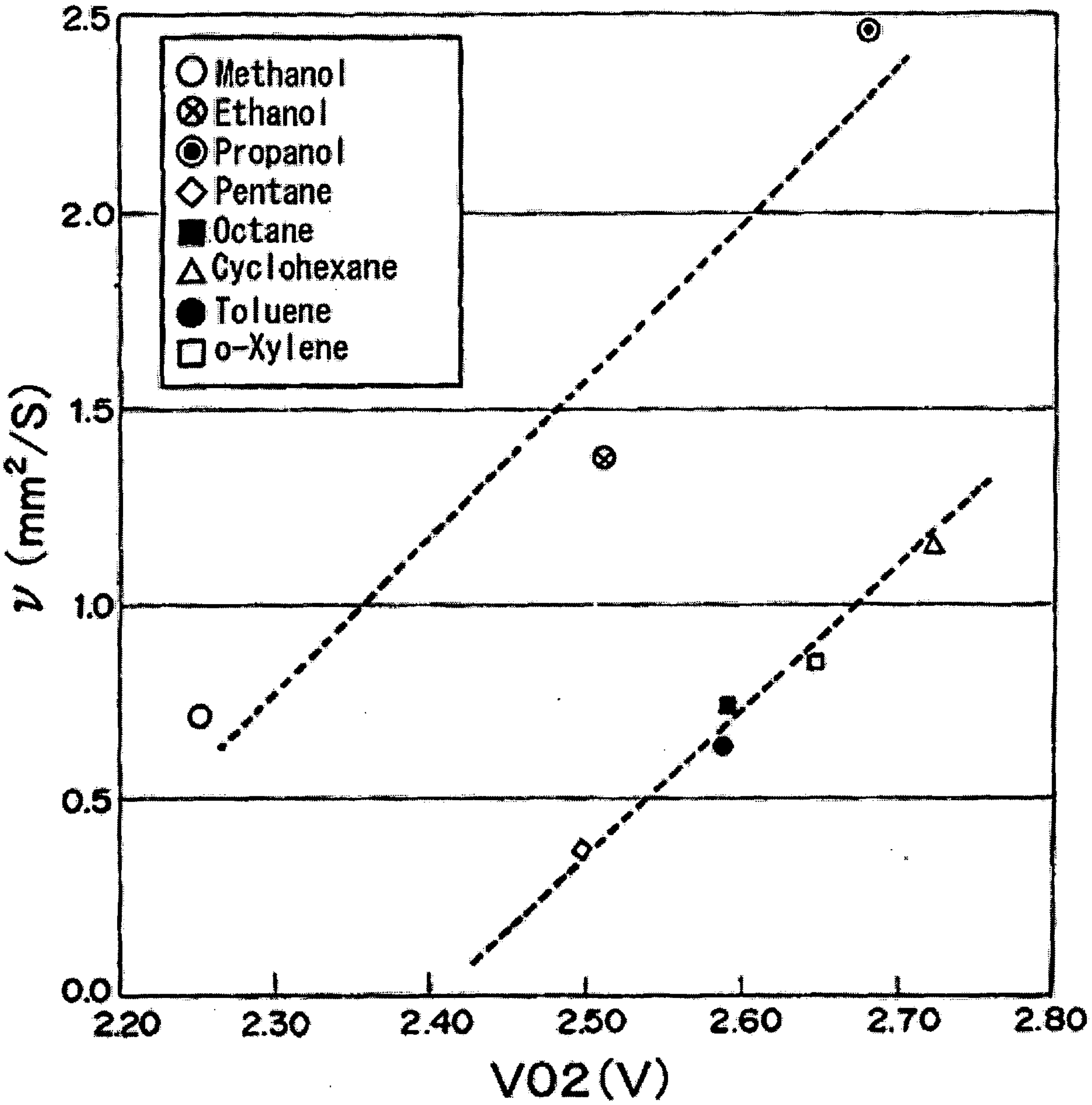
Methanol
Ethanol
Propanol
Pentane
Octane
Cyclohexane
Toluene
o-Xylene
ν (mm²/S)
0.0
0.5
1.0
1.5
2.0
2.5
2.20
2.30
2.40
2.50
2.60
2.70
2.80
V02(V)

[Fig. 26]

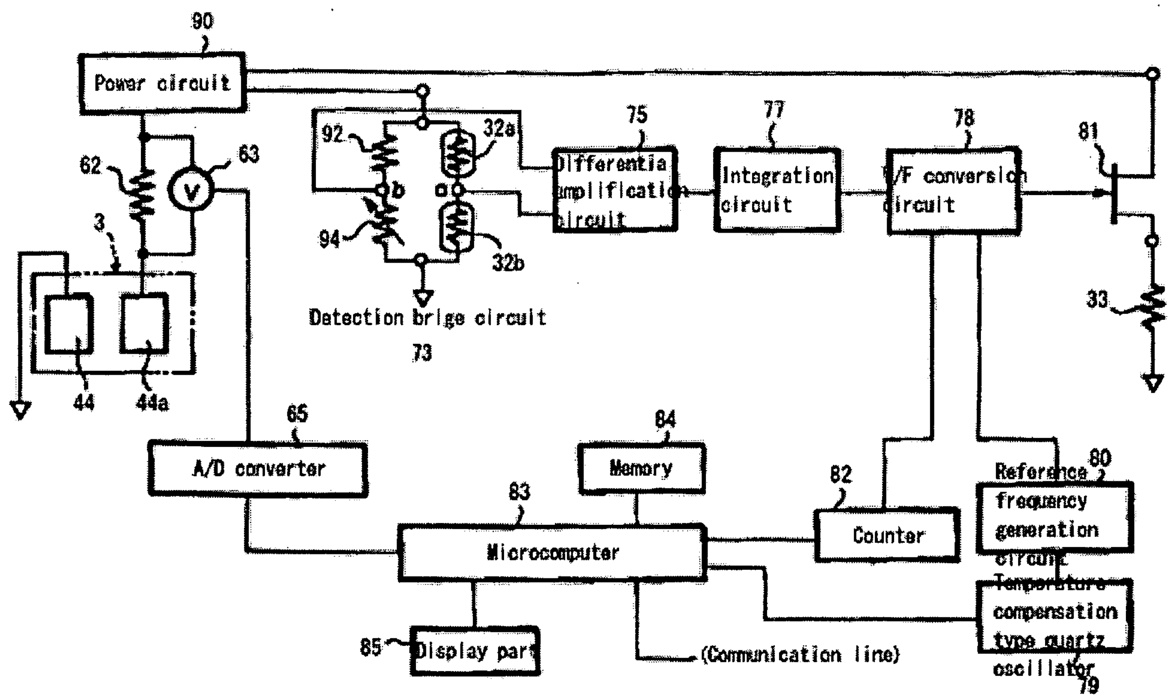
90
Power circuit
62
63
V
3
44
44a
92
94
b
32a
32b
Detection brige circuit
73
75
Differentia amplification circuit
77
Integration circuit
78
V/F conversion circuit
81
33
65
A/D converter
84
Memory
83
Microcomputer
82
Counter
80
Reference frequency generation circuit
Temperature compensation type quartz oscillator
79
85
Display part
(Communication line)

[Fig. 27]

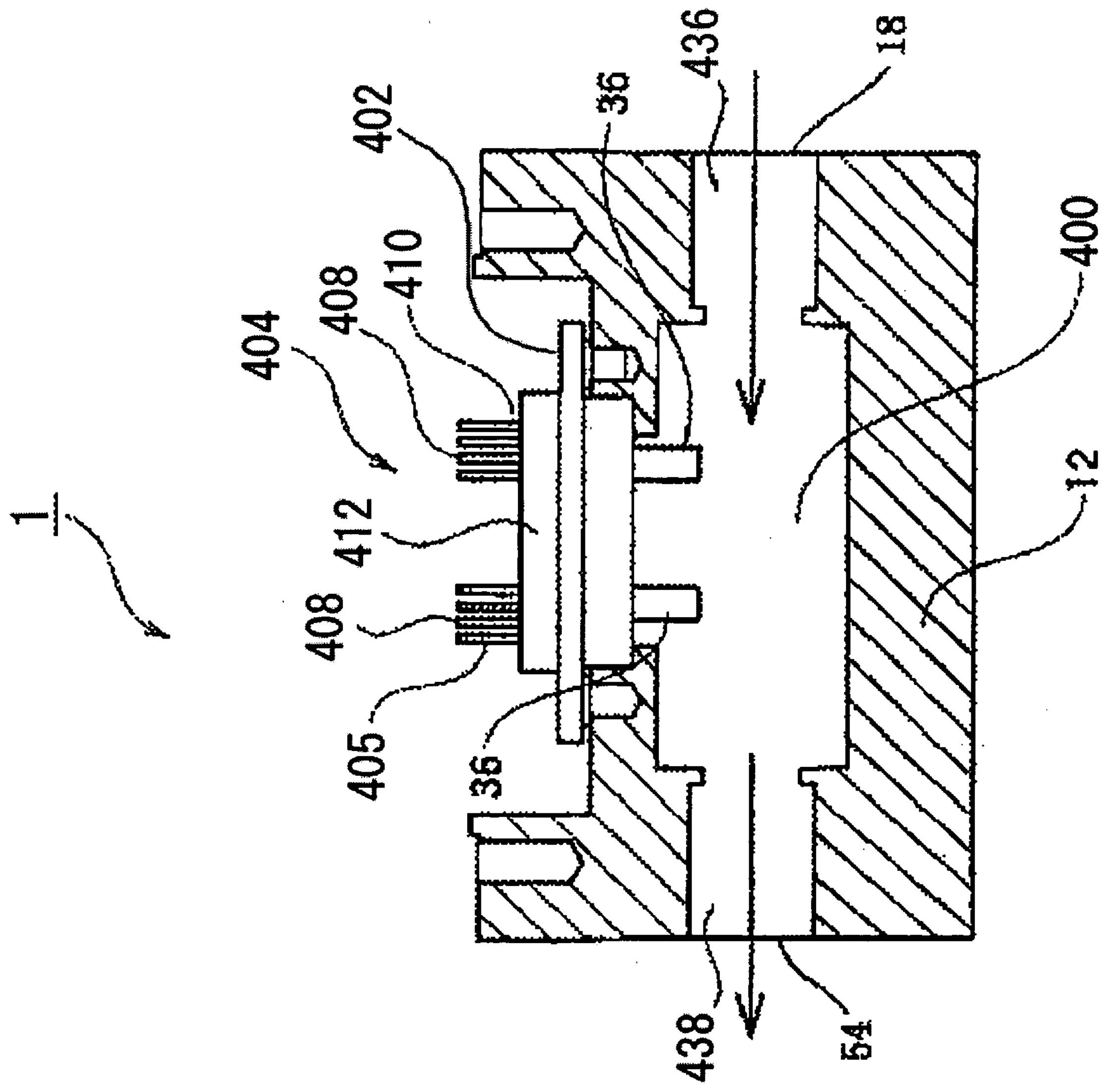

[Fig. 28]

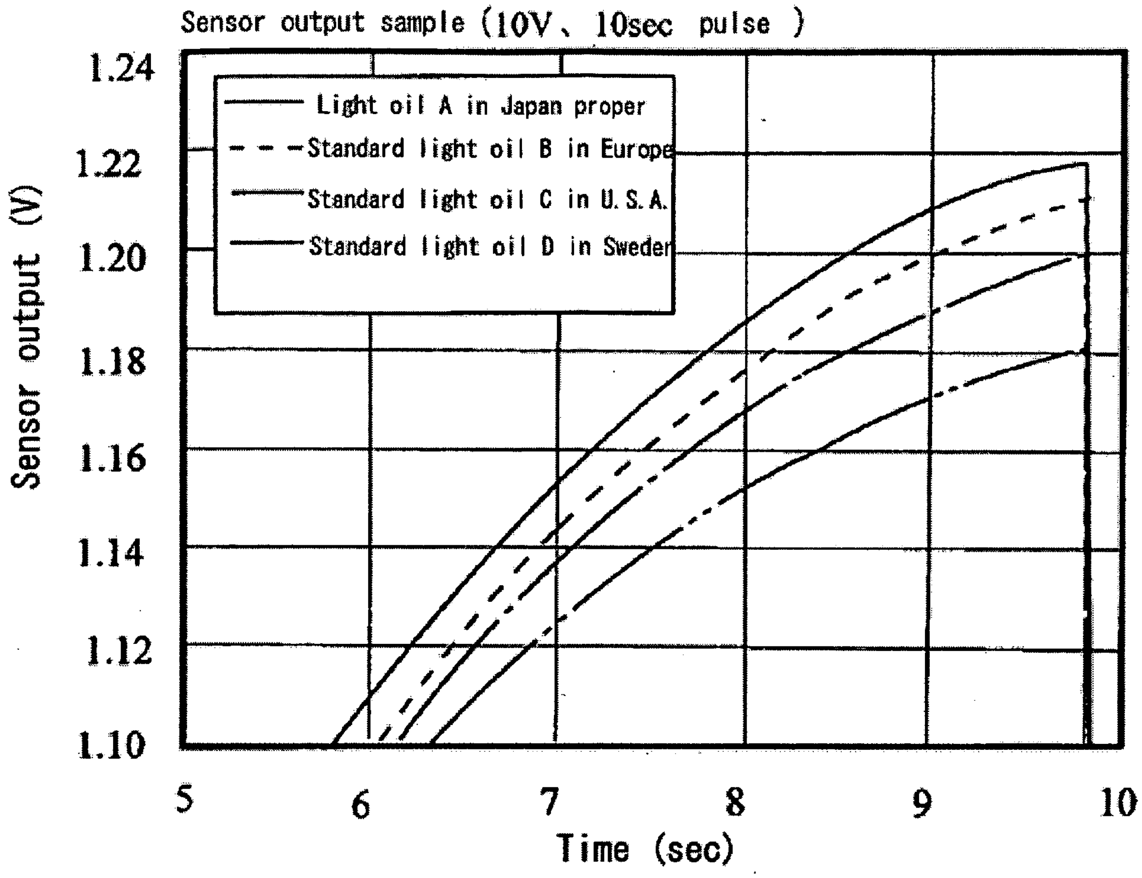
Sensor output sample (10V、10sec pulse )
1.24
1.22
1.20
1.18
1.16
1.14
1.12
1.10
Sensor output (V)
Light oil A in Japan proper
Standard light oil B in Europe
Standard light oil C in U.S.A.
Standard light oil D in Sweden
5
6
7
8
9
10
Time (sec)

[Fig. 29]

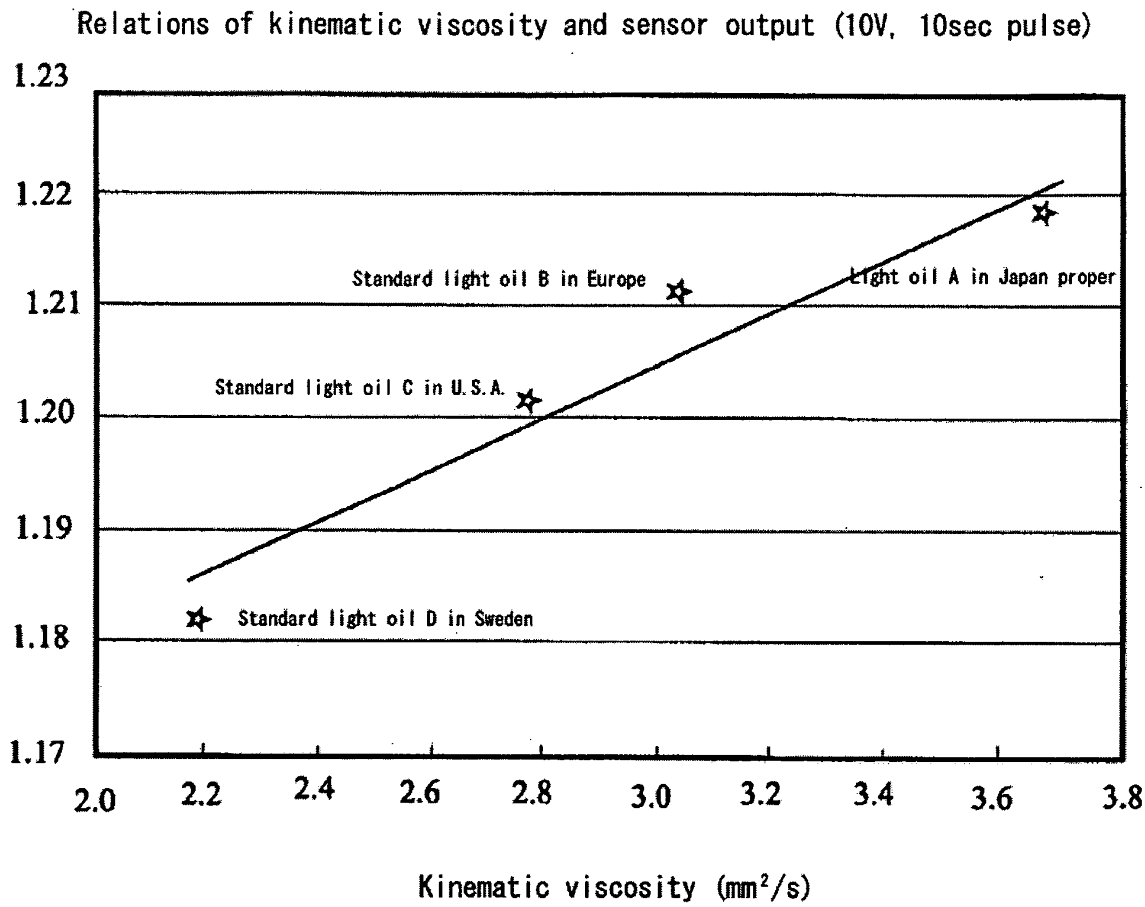
Relations of kinematic viscosity and sensor output (10V, 10sec pulse)
1.23
1.22
1.21
1.20
1.19
1.18
1.17
2.0
2.2
2.4
2.6
2.8
3.0
3.2
3.4
3.6
3.8
Standard light oil B in Europe
Light oil A in Japan proper
Standard light oil C in U.S.A.
Standard light oil D in Sweden
Kinematic viscosity (mm²/s)

[Fig. 30]

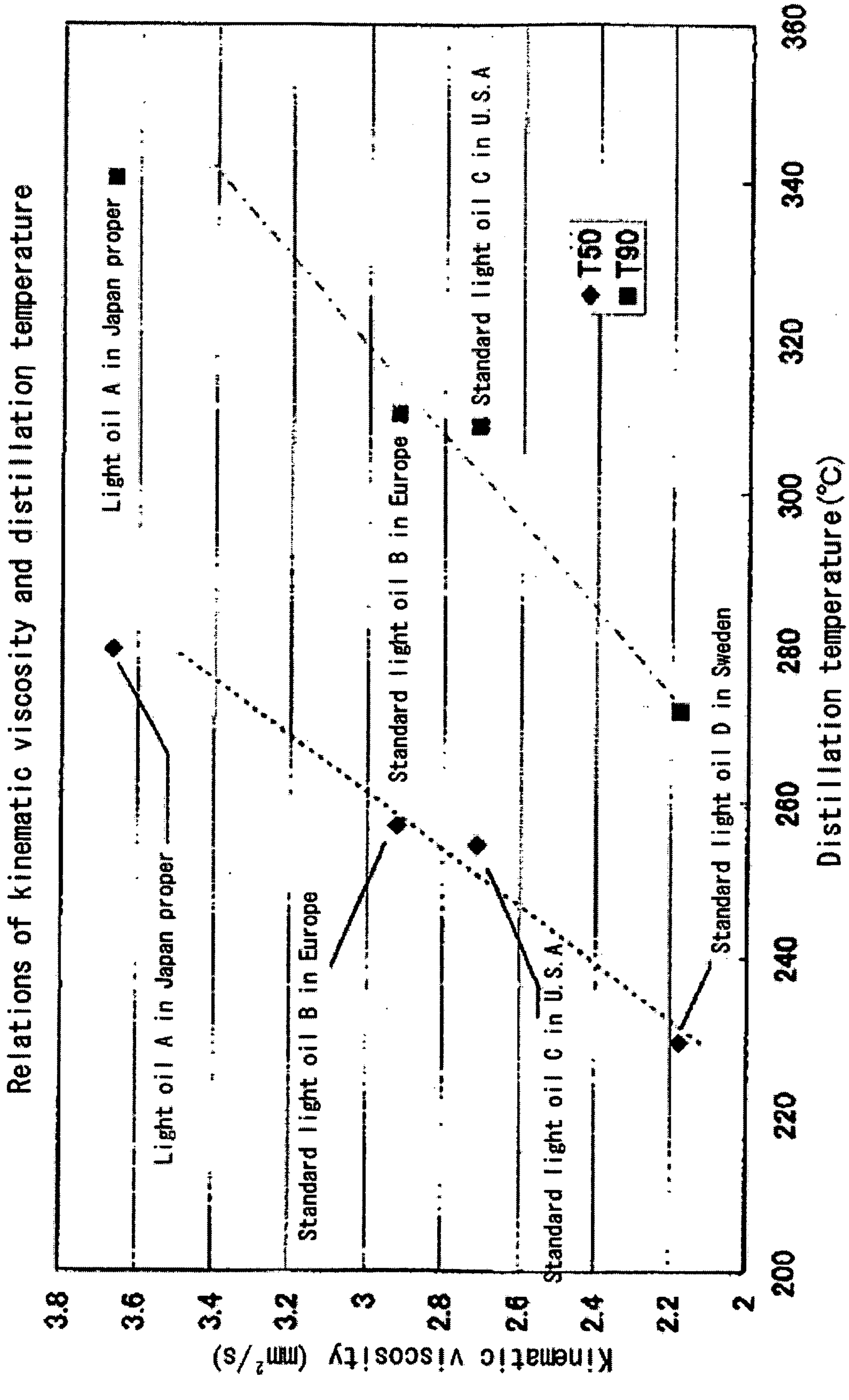
Relations of kinematic viscosity and distillation temperature
Kinematic viscosity (mm²/s)
3.8
3.6
3.4
3.2
3
2.8
2.6
2.4
2.2
2
Distillation temperature (°C)
200
220
240
260
280
300
320
340
360
Light oil A in Japan proper
Standard light oil B in Europe
Standard light oil C in U.S.A
Standard light oil D in Sweden
T50
T90

[Fig. 31]

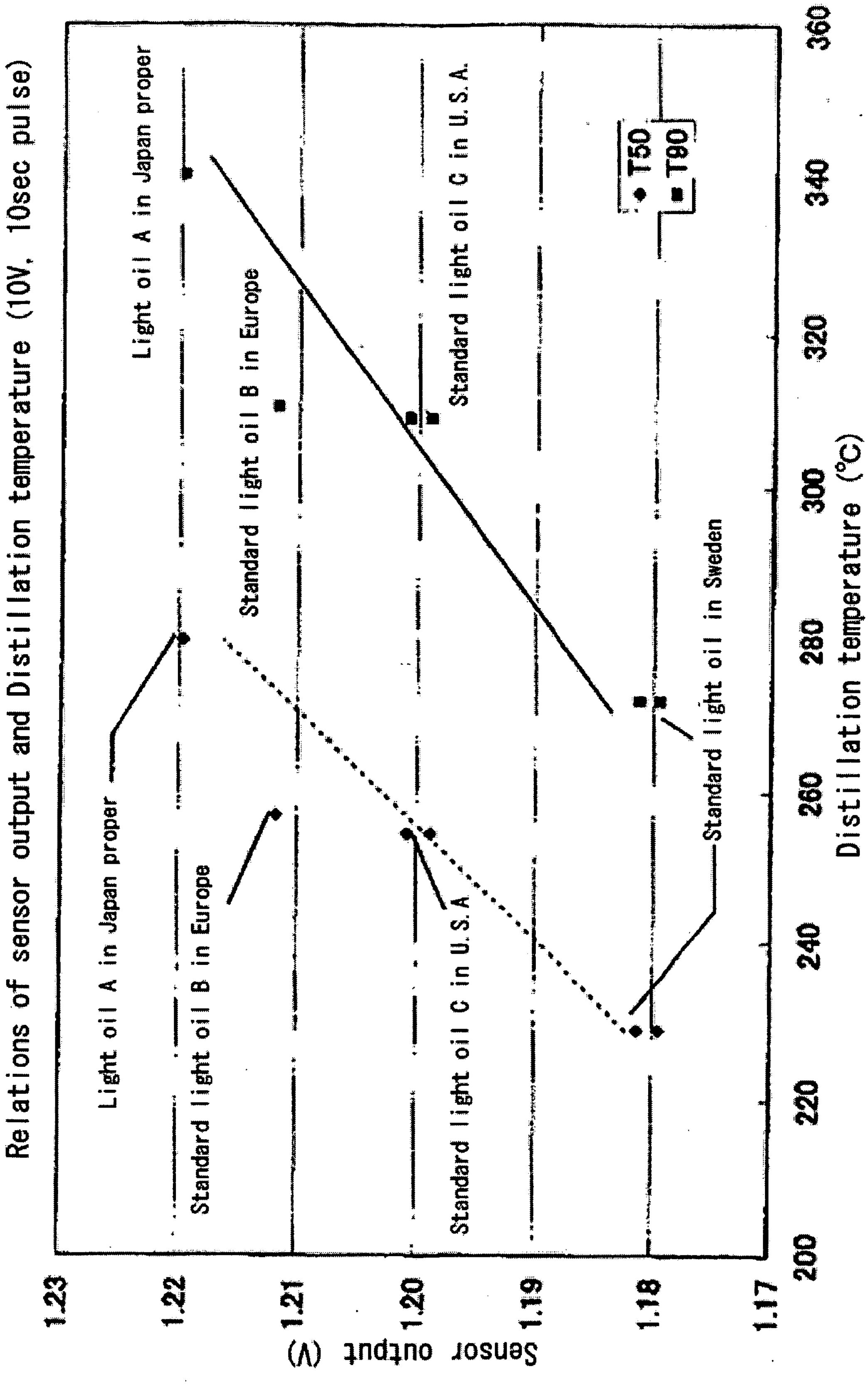
Relations of sensor output and Distillation temperature (10V, 10sec pulse)
Sensor output (V)
1.23
1.22
1.21
1.20
1.19
1.18
1.17
Distillation temperature (°C)
200
220
240
260
280
300
320
340
360
Light oil A in Japan proper
Standard light oil B in Europe
Standard light oil C in U.S.A.
Standard light oil in Sweden
Light oil A in Japan proper
Standard light oil B in Europe
Standard light oil C in U.S.A.
Standard light oil in Sweden
T50
T90

[Fig. 32]

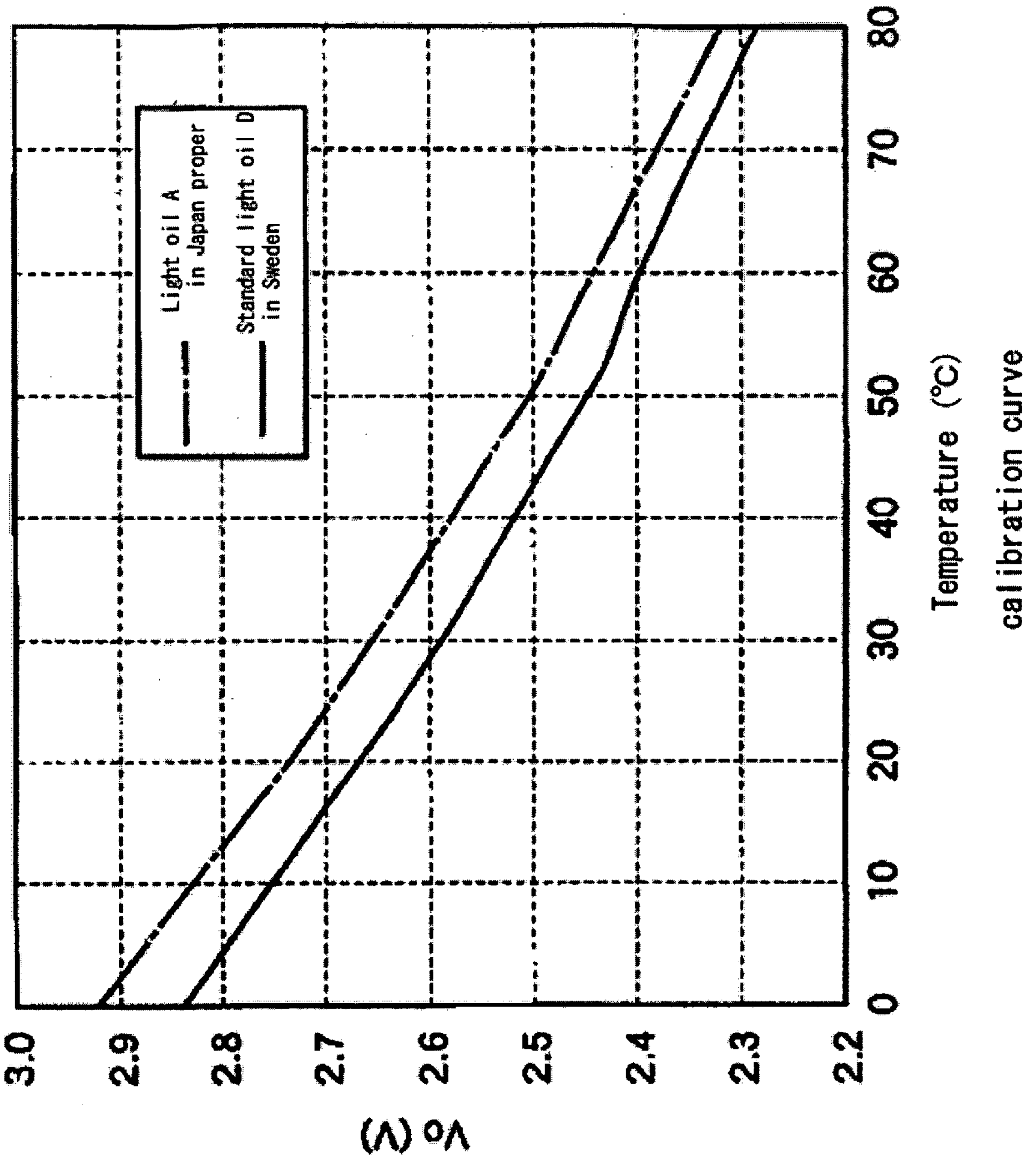
Light oil A in Japan proper
Standard light oil D in Sweden
Vo (V)
3.0
2.9
2.8
2.7
2.6
2.5
2.4
2.3
2.2
0
10
20
30
40
50
60
70
80
Temperature (℃)
calibration curve

[Fig. 33]

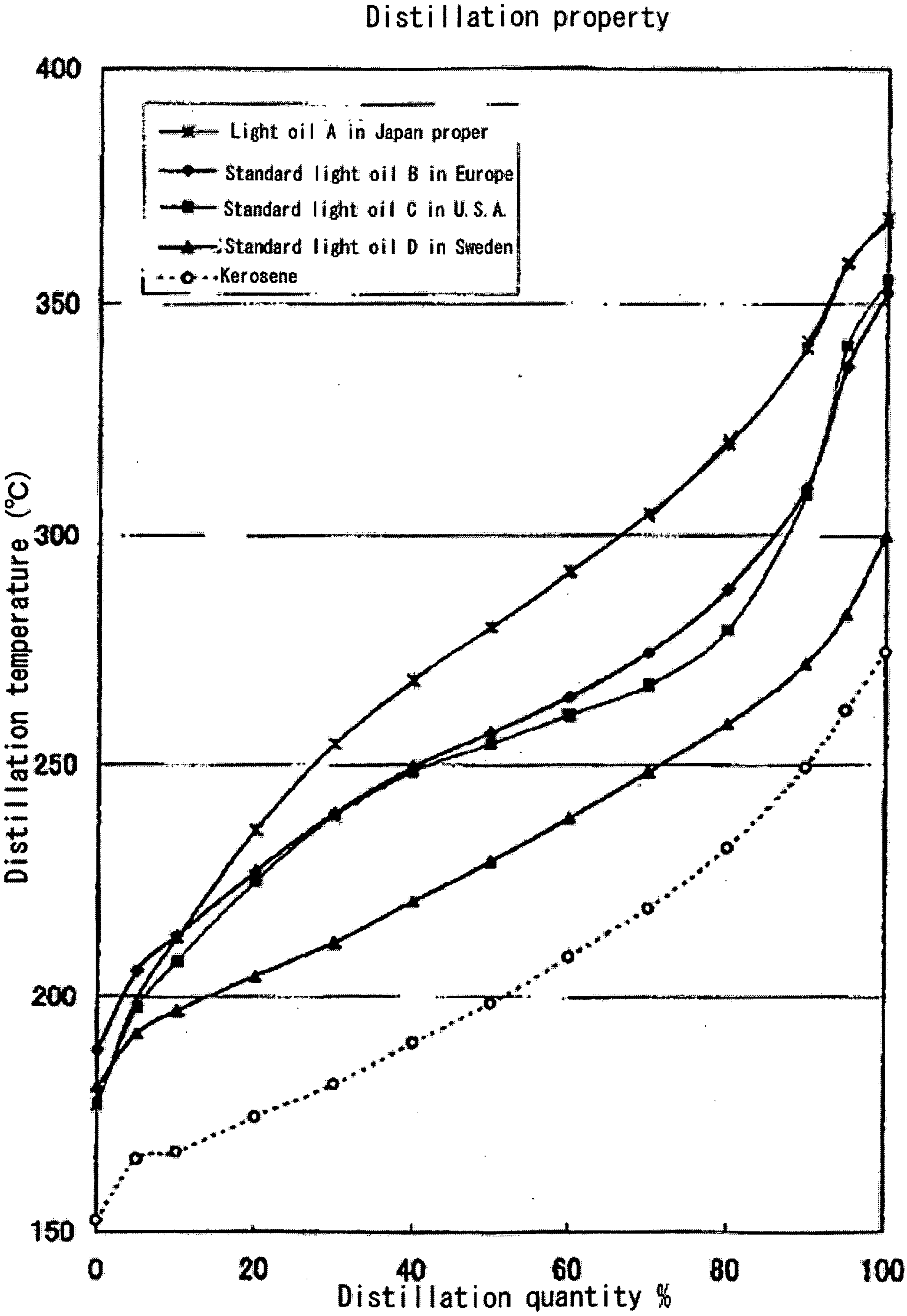
Distillation property
Light oil A in Japan proper
Standard light oil B in Europe
Standard light oil C in U.S.A.
Standard light oil D in Sweden
Kerosene
Distillation temperature (°C)
400
350
300
250
200
150
0
20
40
60
80
100
Distillation quantity %

[Fig. 34]

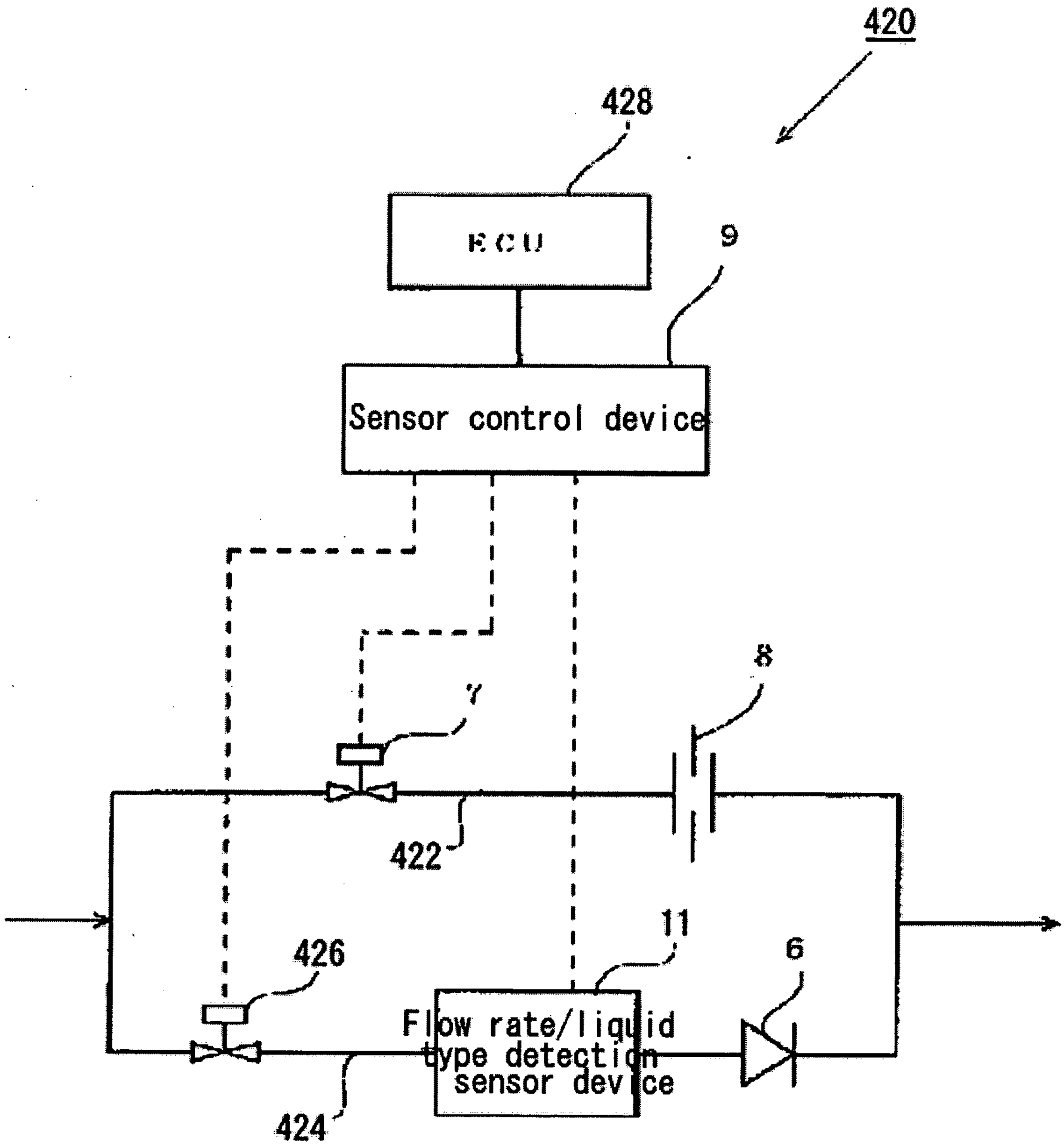
420
428
ECU
9
Sensor control device
8
7
422
11
426
6
Flow rate/liquid type detection sensor device
424

[Fig. 35]

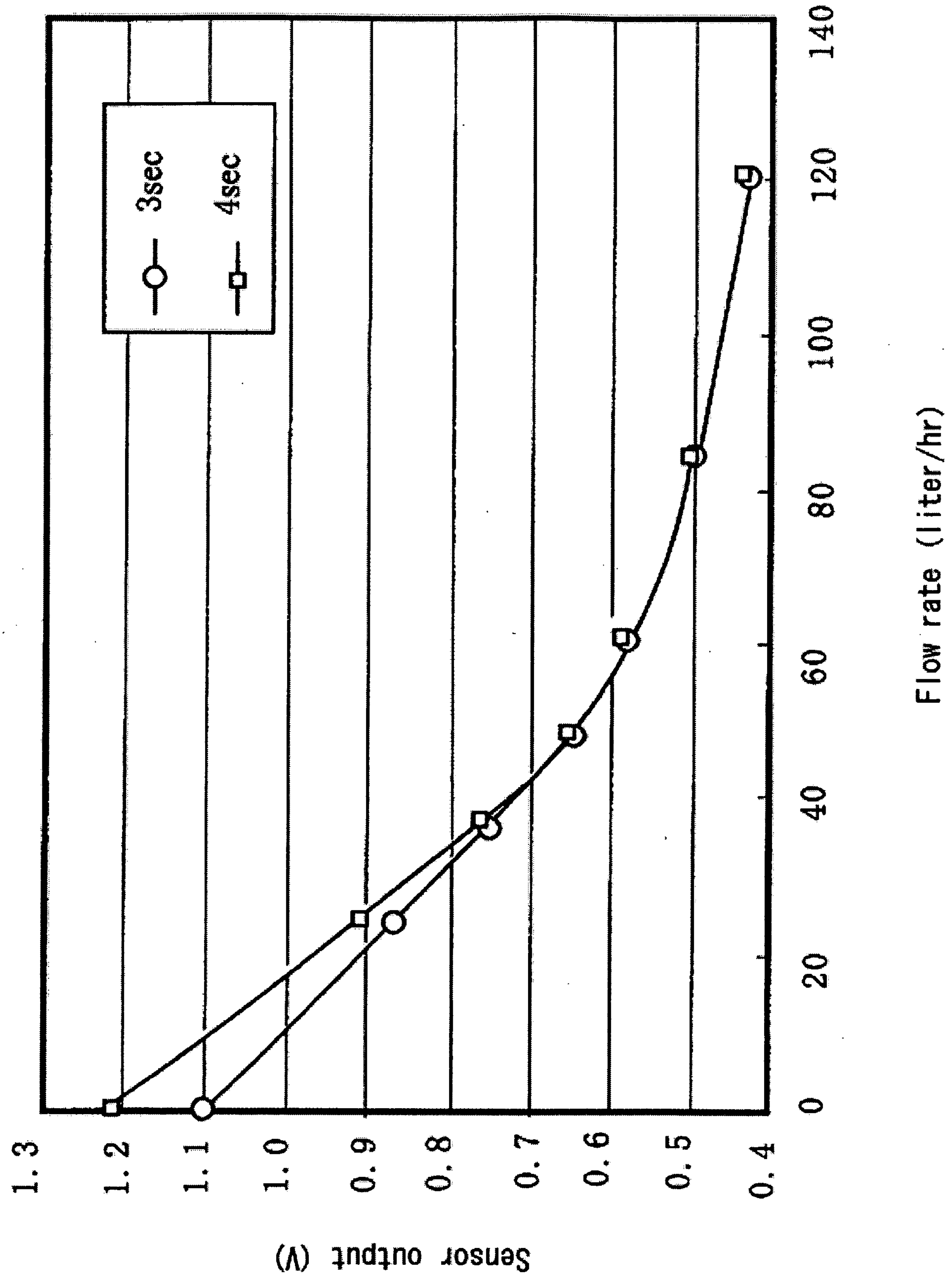
3sec
4sec
Sensor output (V)
1.3
1.2
1.1
1.0
0.9
0.8
0.7
0.6
0.5
0.4
Flow rate (liter/hr)
0
20
40
60
80
100
120
140

[Fig. 36]

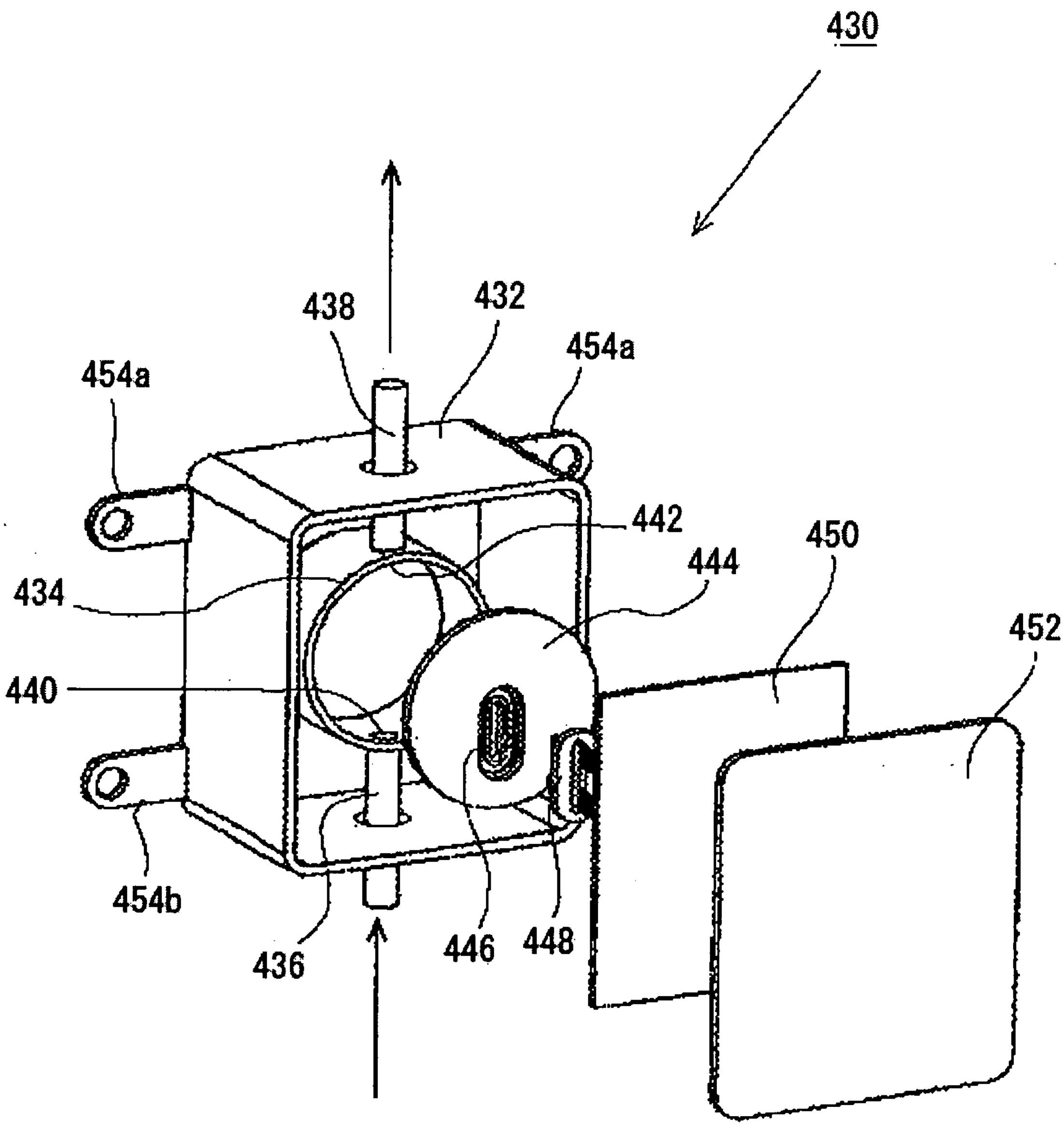

[Fig. 37]

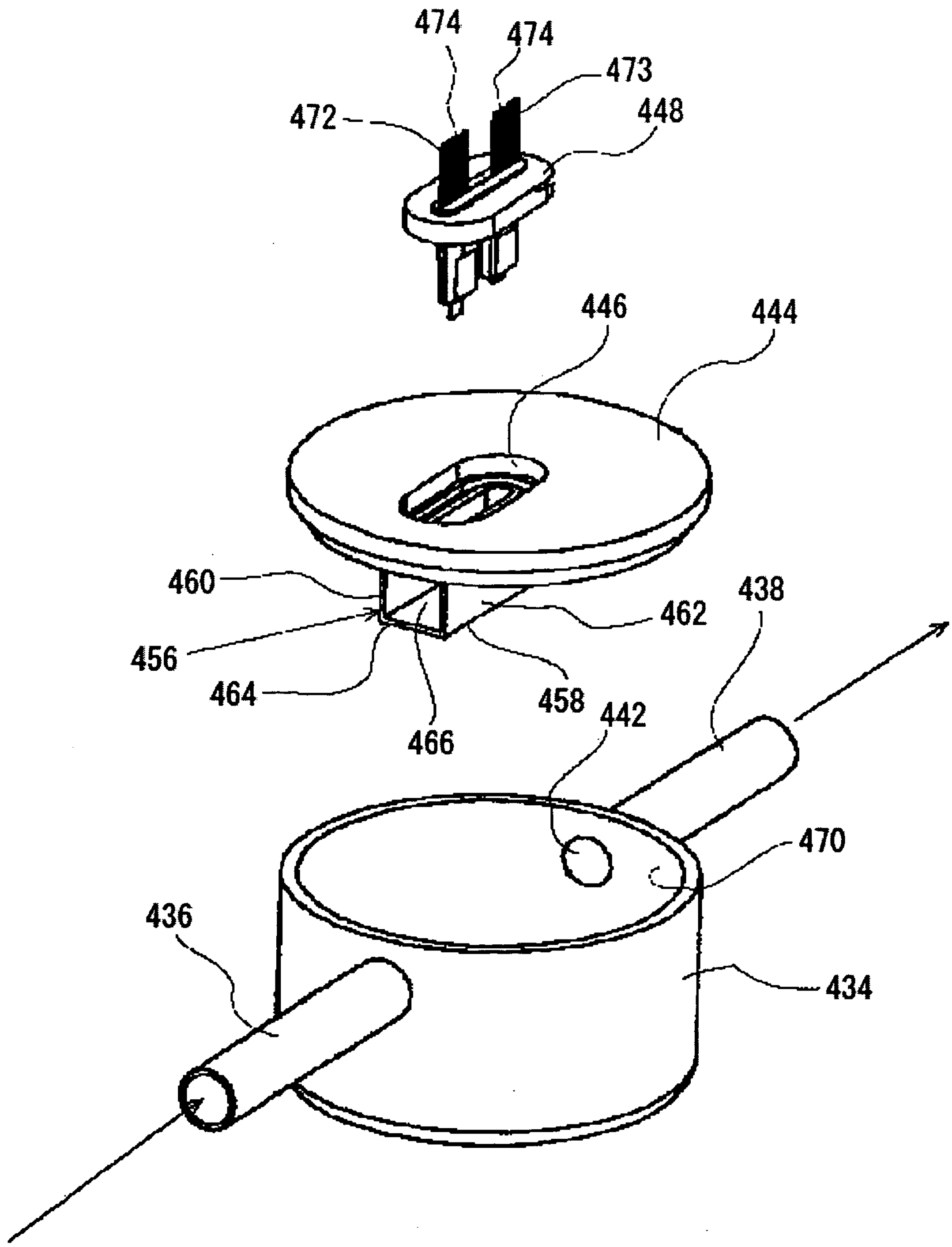
474
474
473
472
448
446
444
460
462
438
456
464
458
442
466
470
436
434

[Fig. 38]

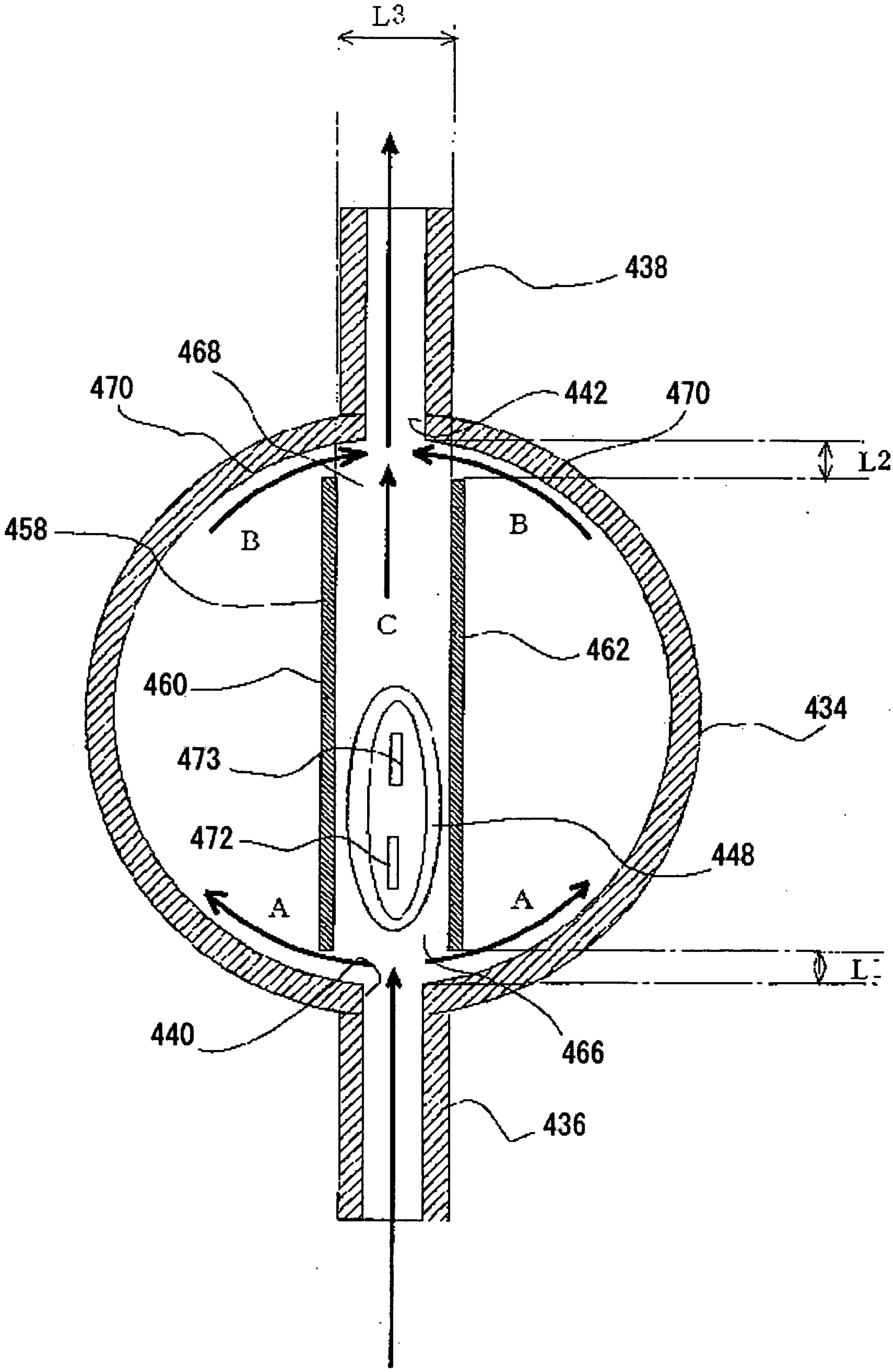
L3
438
468
470
442
470
L2
458
B
B
C
462
460
434
473
472
448
A
A
440
466
436

[Fig. 39]

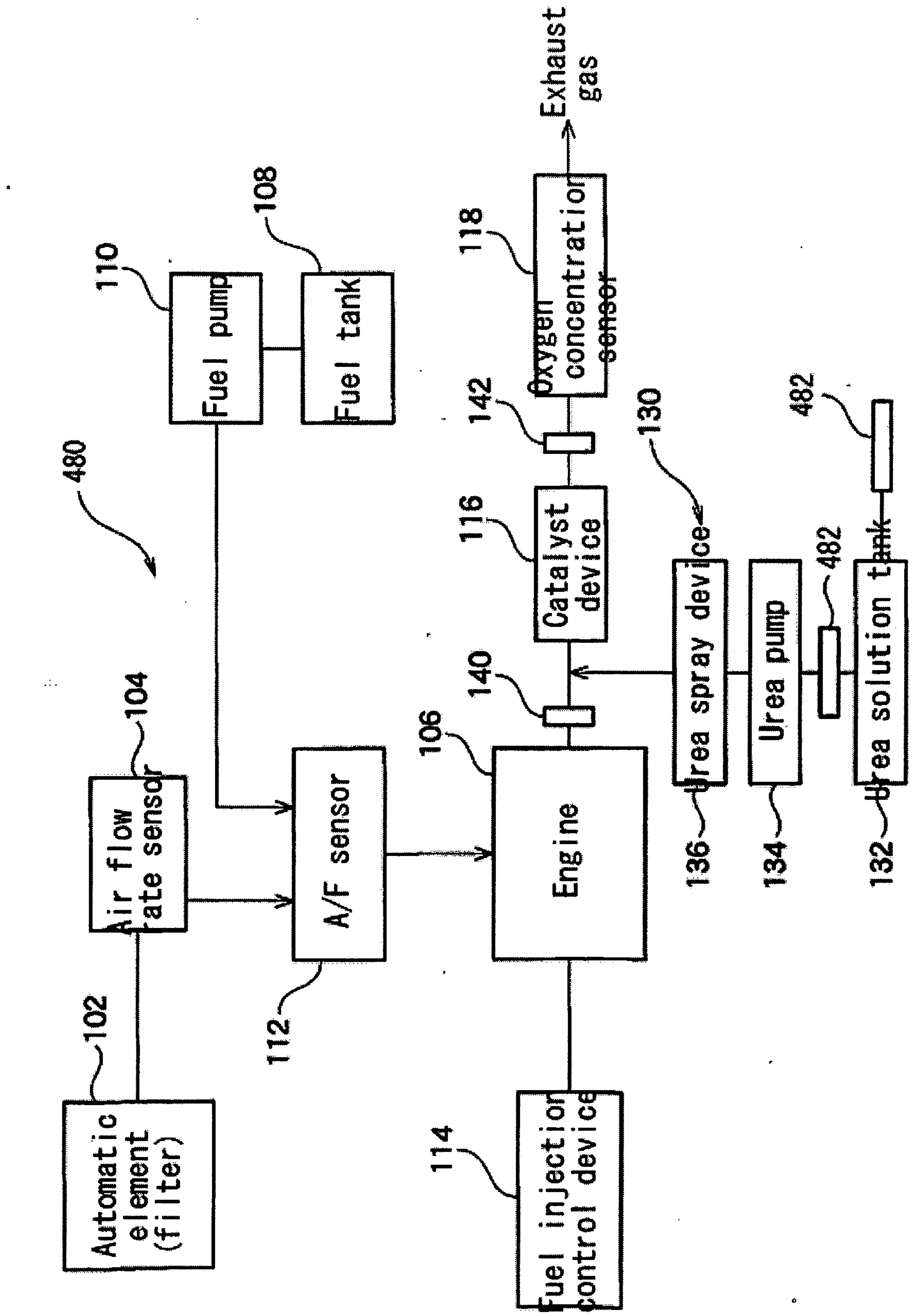
480
Fuel pump
110
Fuel tank
108
Oxygen concentration sensor
118
Exhaust gas
142
Catalyst device
116
140
130
Urea spray device
136
Urea pump
134
482
Urea solution tank
132
482
Air flow rate sensor
104
A/F sensor
112
Engine
106
Automatic element (filter)
102
Fuel injection control device
114

[Fig. 40]

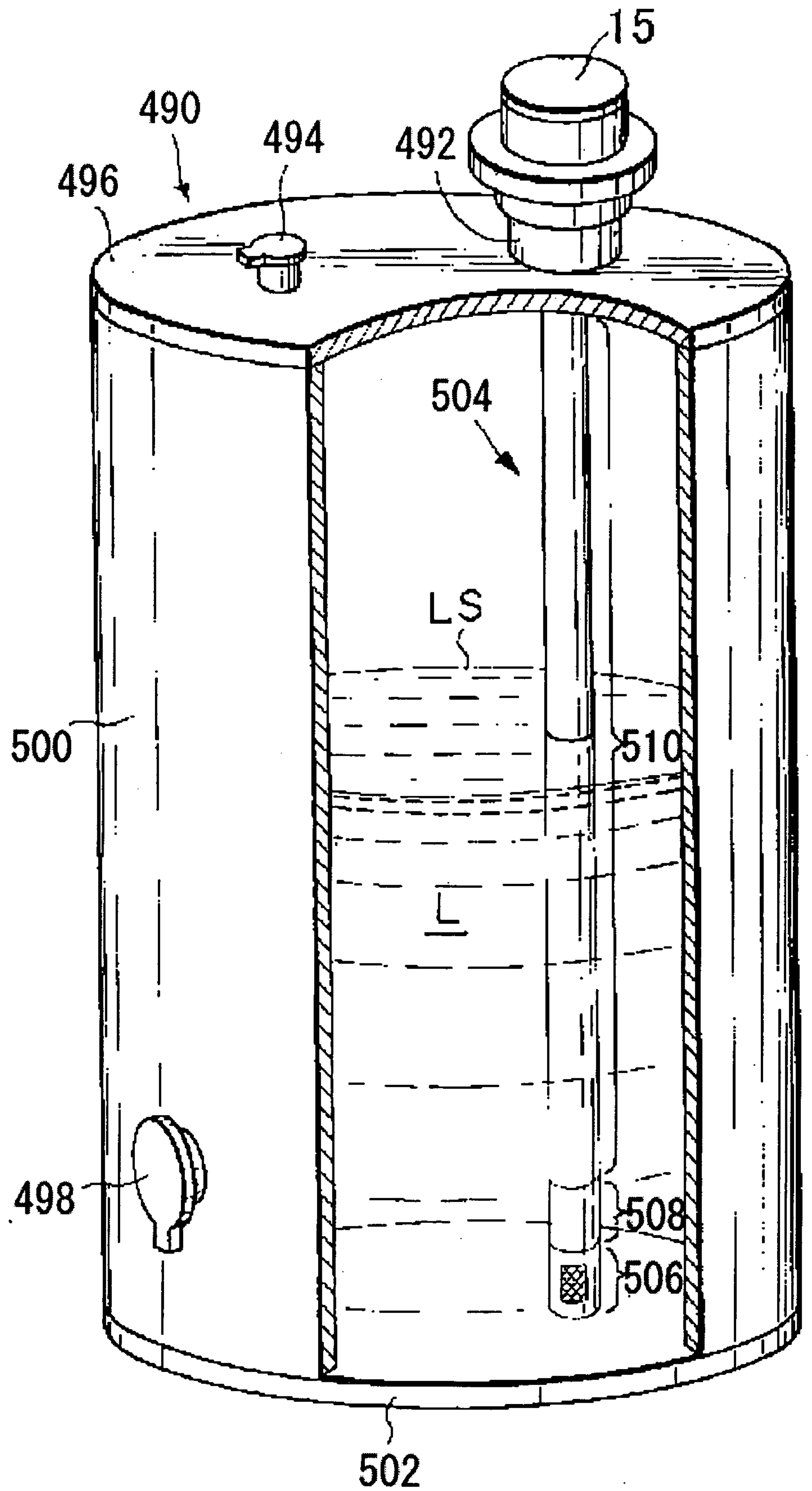

[Fig. 41]

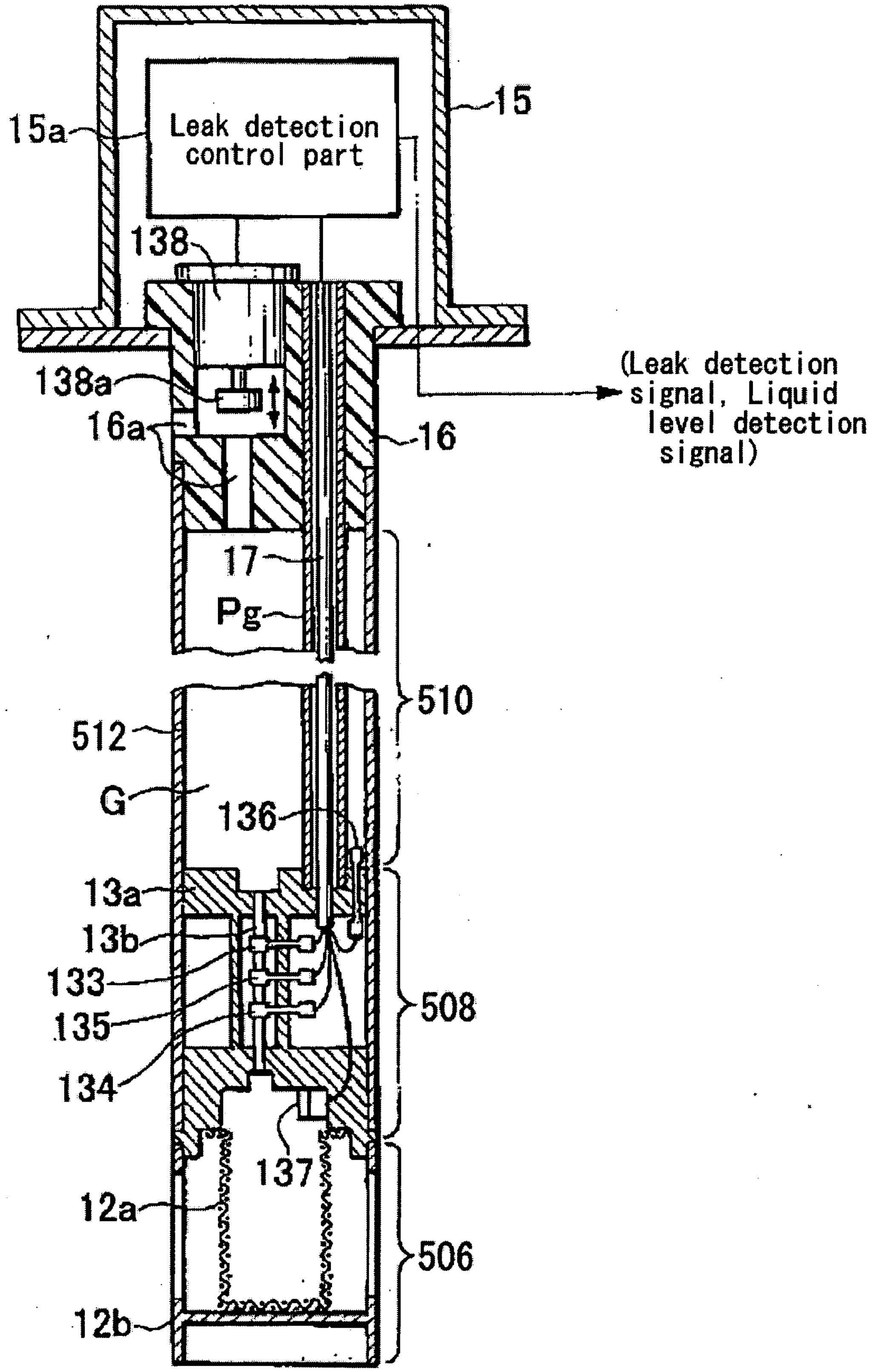
15a
Leak detection control part
15
138
138a
16a
16
(Leak detection signal, Liquid level detection signal)
17
Pg
510
512
G
136
13a
13b
133
135
134
508
137
12a
506
12b

[Fig. 42]

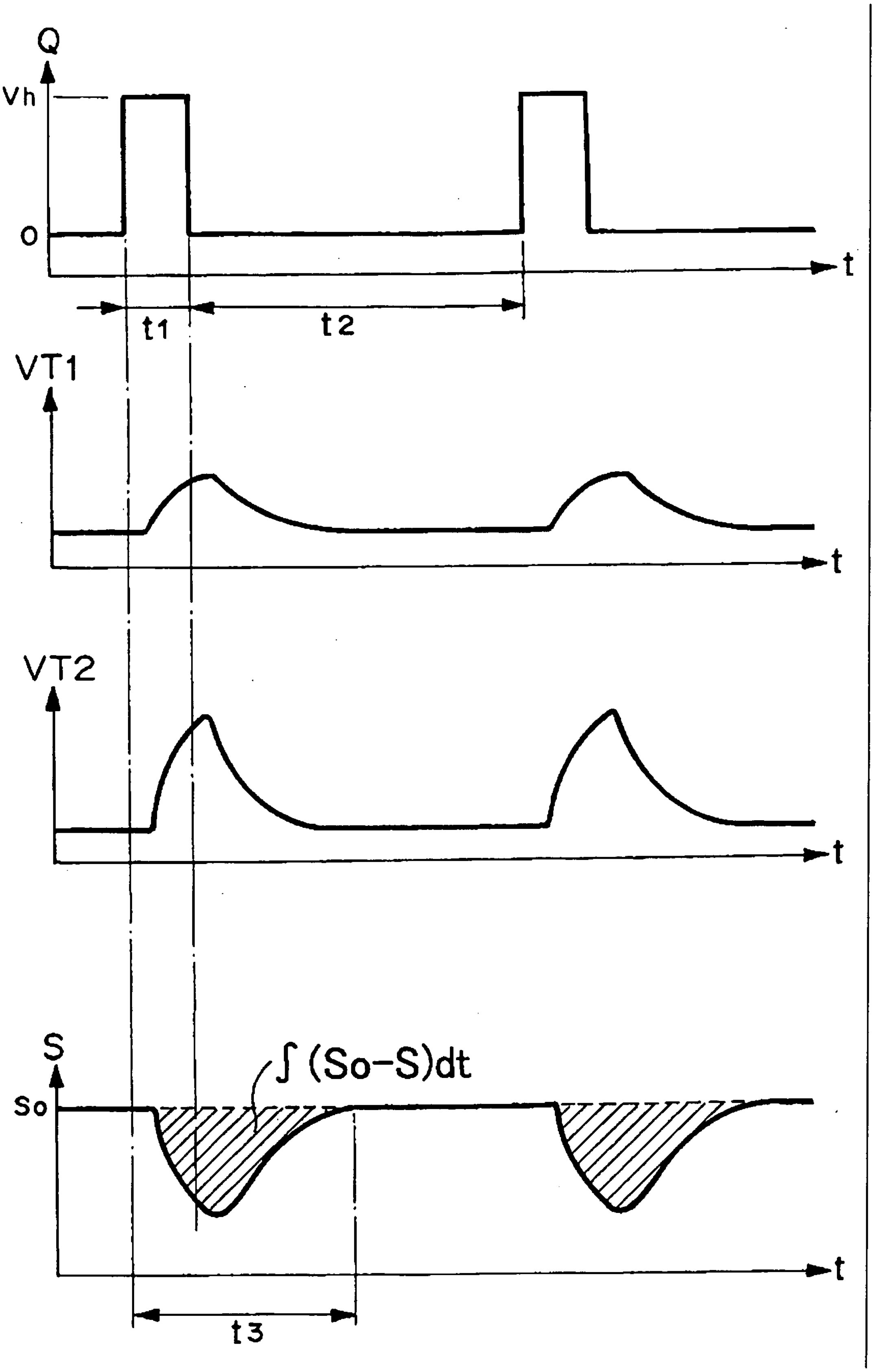

[Fig. 43]

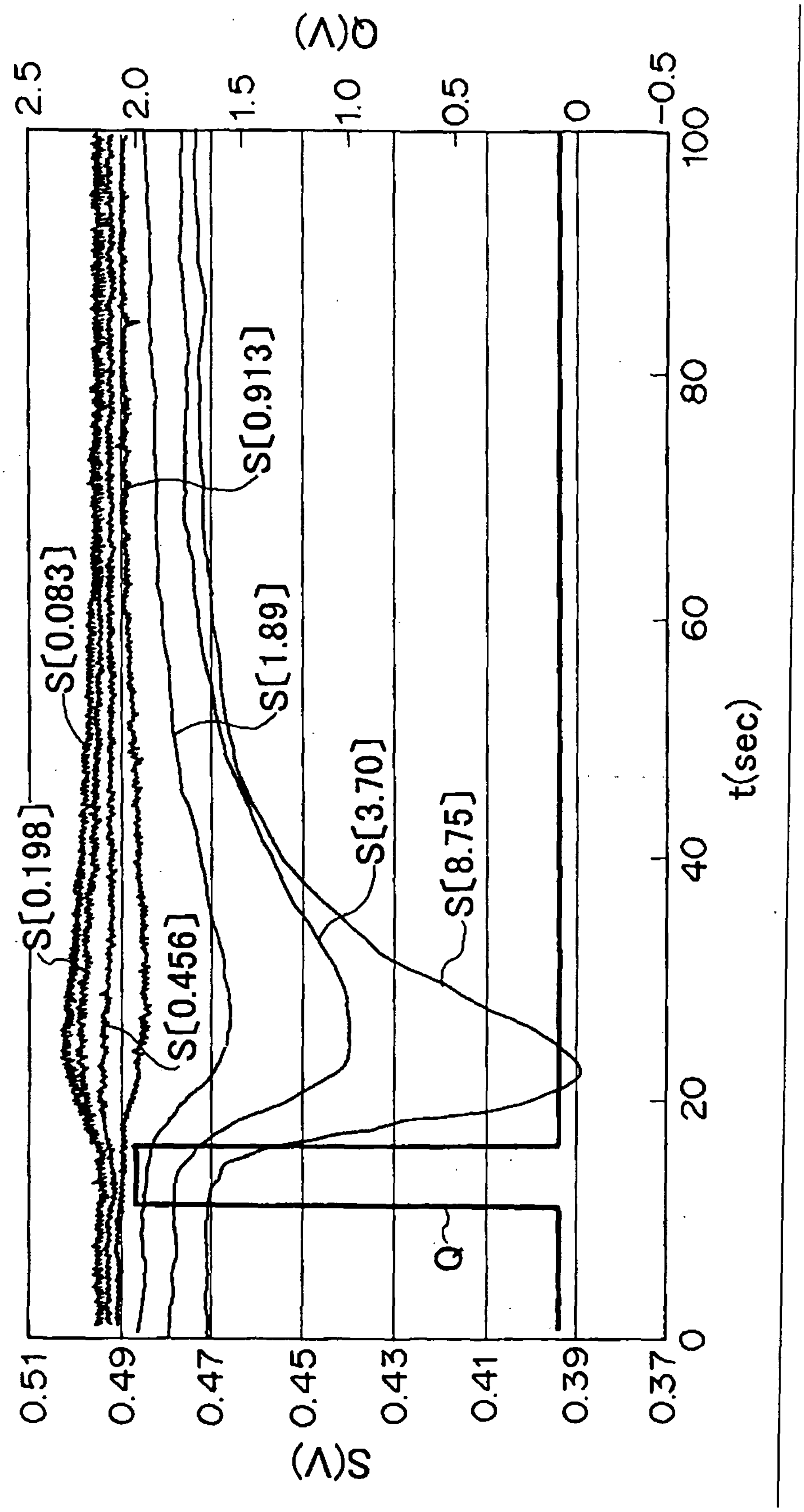
S[0.198]
S[0.083]
S[0.456]
S[0.913]
S[1.89]
S[3.70]
S[8.75]
Q
t(sec)
S(V)
Q(V)
0.51
0.49
0.47
0.45
0.43
0.41
0.39
0.37
2.5
2.0
1.5
1.0
0.5
0
-0.5
0
20
40
60
80
100

[Fig. 44]

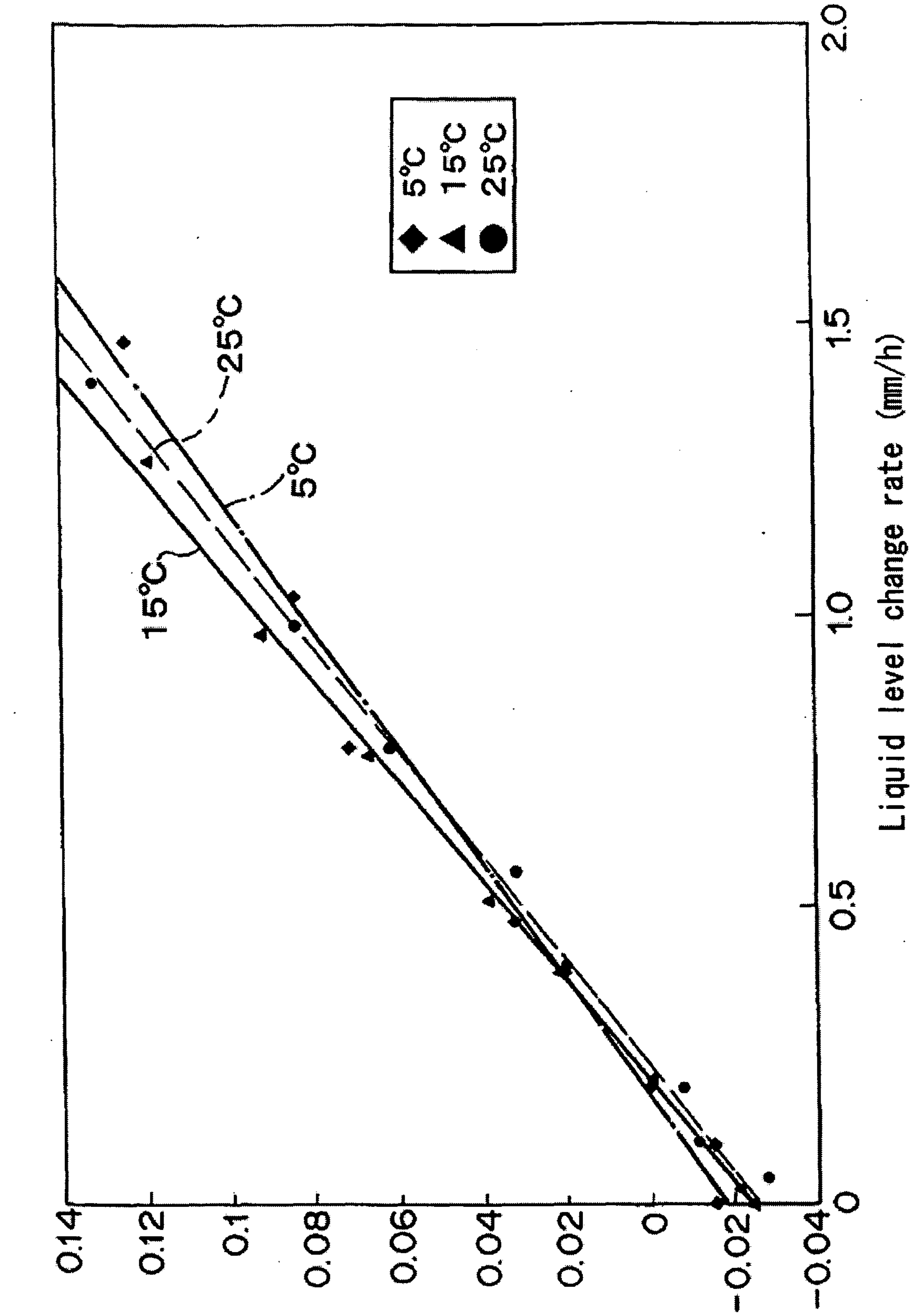
∫(So−S)dt
0.14
0.12
0.1
0.08
0.06
0.04
0.02
0
−0.02
−0.04
0
0.5
1.0
1.5
2.0
Liquid level change rate (mm/h)
5°C
15°C
25°C
5°C
15°C
25°C

[Fig. 45]

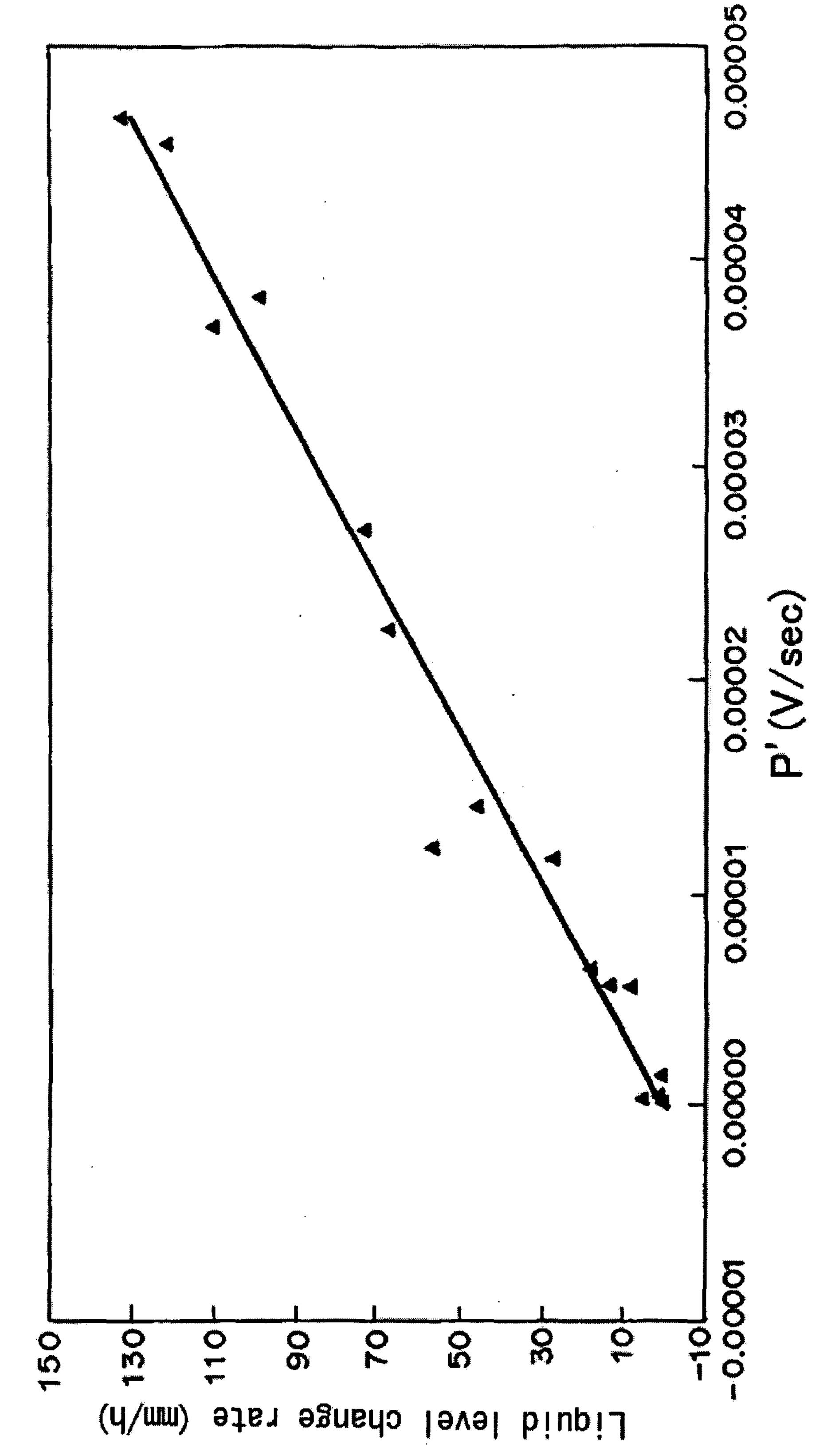
Liquid level change rate (mm/h)
150
130
110
90
70
50
30
10
−10
−0.00001
0.00000
0.00001
0.00002
0.00003
0.00004
0.00005
P' (V/sec)

[Fig. 46]

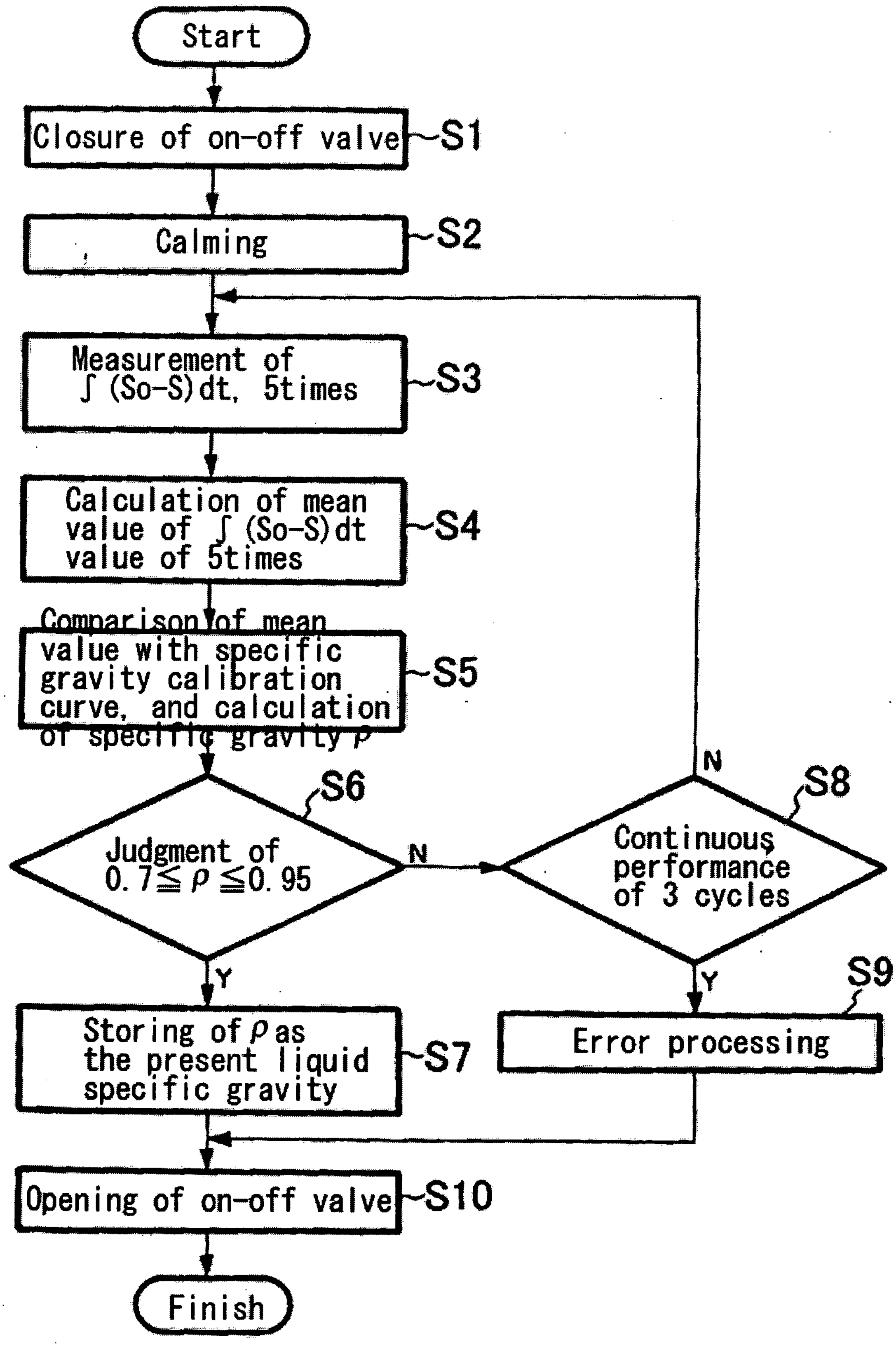
Start
Closure of on-off valve
S1
Calming
S2
Measurement of
∫(So-S)dt, 5times
S3
Calculation of mean
value of ∫(So-S)dt
value of 5times
S4
Comparison of mean
value with specific
gravity calibration
curve, and calculation
of specific gravity ρ
S5
Judgment of
0.7≦ρ≦0.95
S6
N
Y
Continuous
performance
of 3 cycles
S8
N
Y
Error processing
S9
Storing of ρ as
the present liquid
specific gravity
S7
Opening of on-off valve
S10
Finish

[Fig. 47]

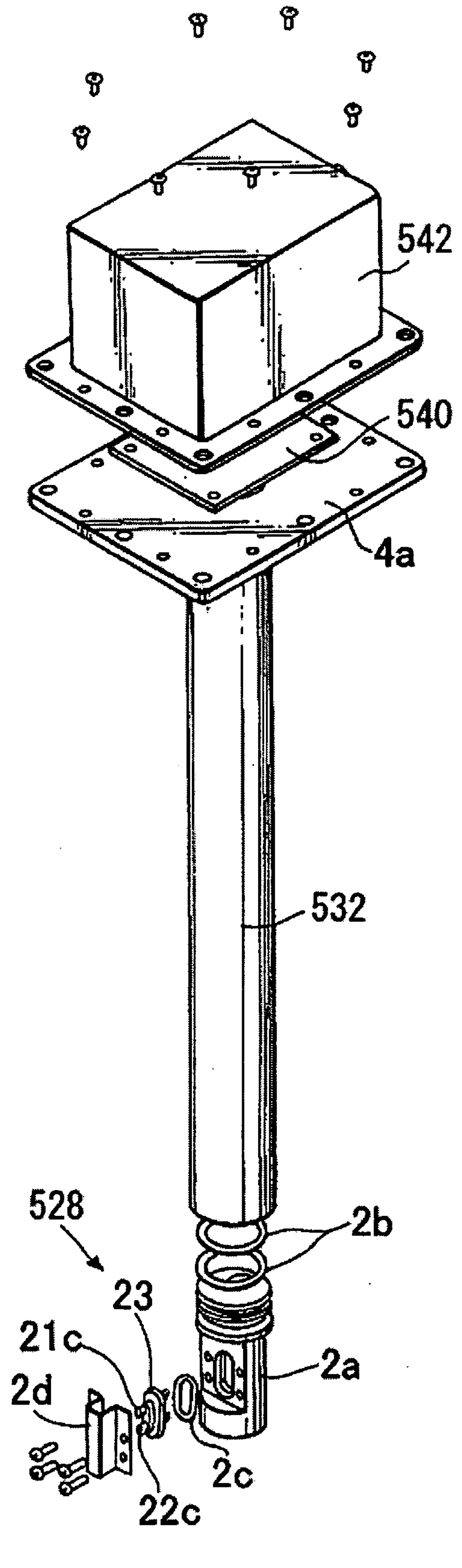

[Fig. 48]

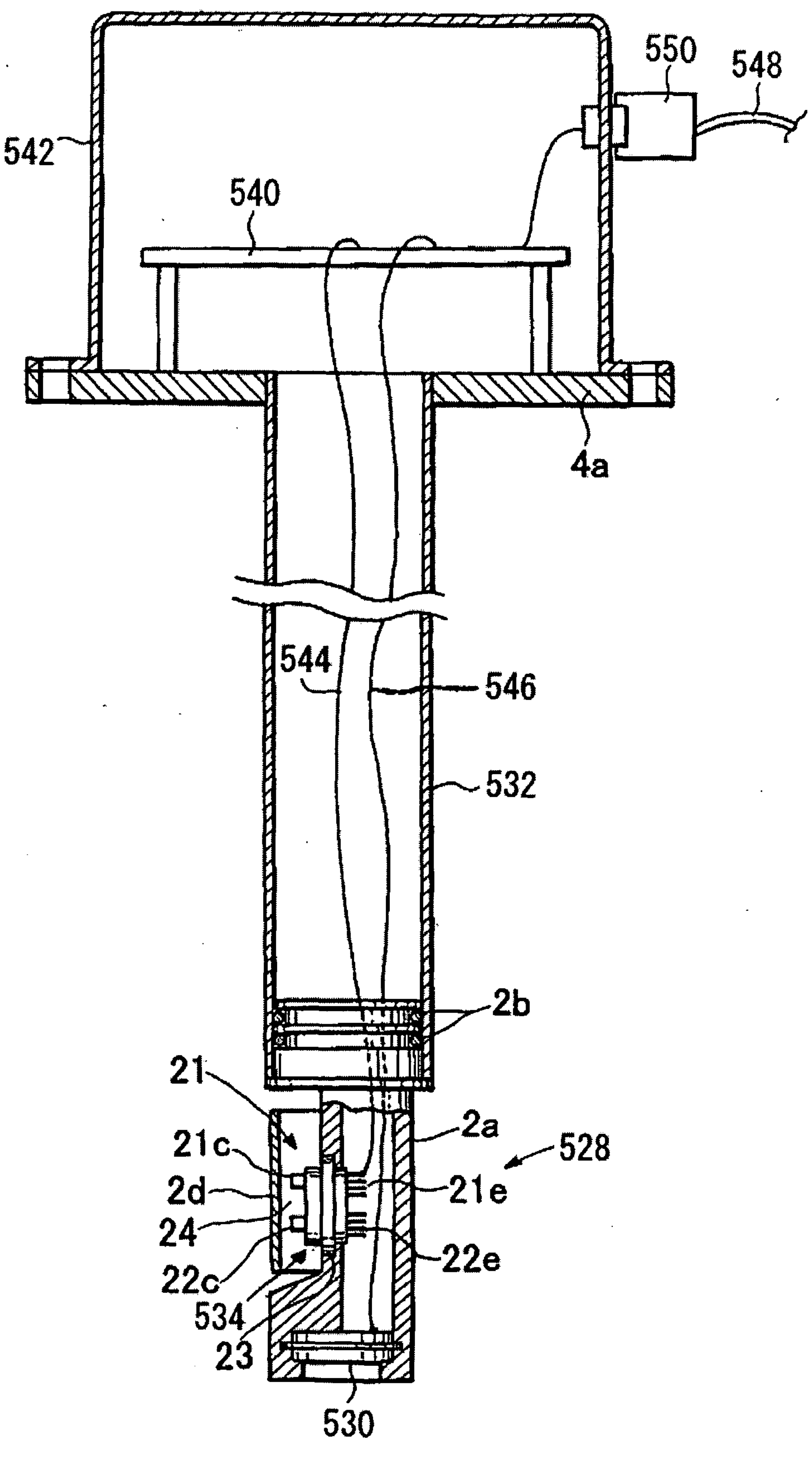
550
548
542
540
4a
544
546
532
2b
21
21c
2a
528
21e
2d
24
22e
22c
534
23
530

[Fig. 49]

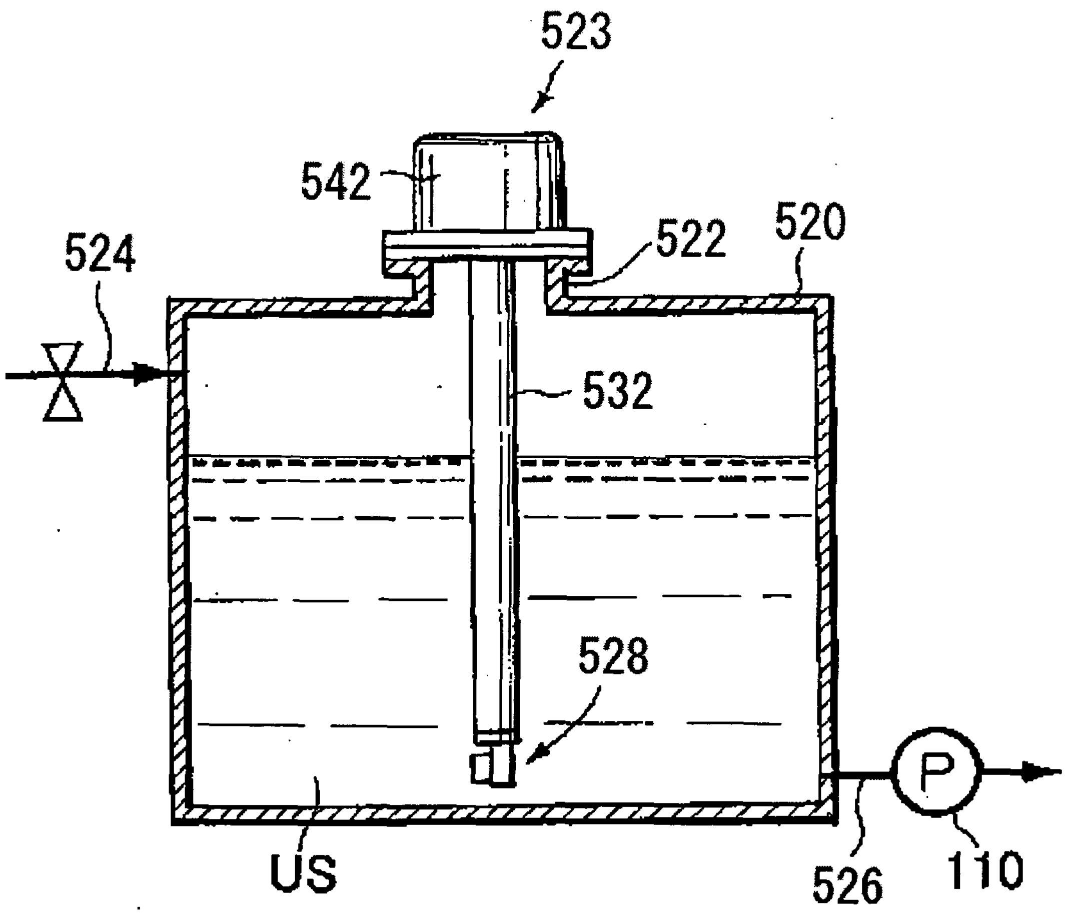

[Fig. 50]

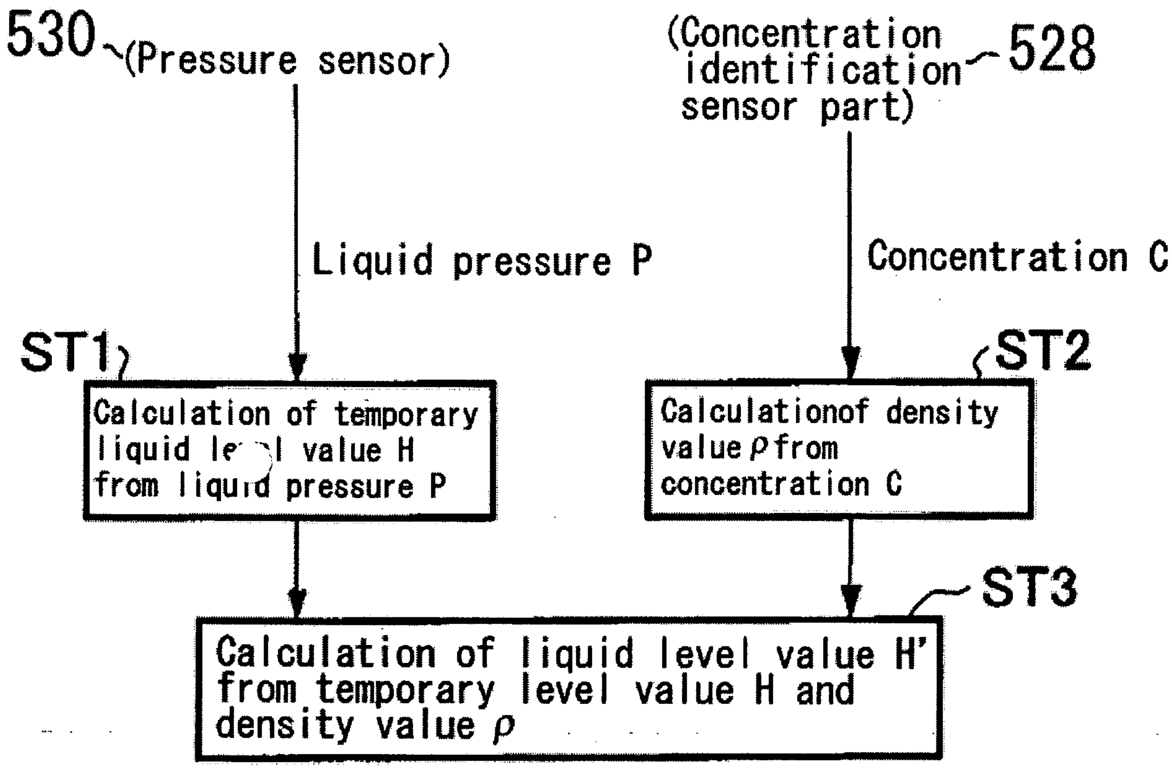
530 (Pressure sensor)
(Concentration identification sensor part) 528
Liquid pressure P
Concentration C
ST1
ST2
Calculation of temporary liquid level value H from liquid pressure P
Calculationof density value ρ from concentration C
ST3
Calculation of liquid level value H' from temporary level value H and density value ρ

[Fig. 51]

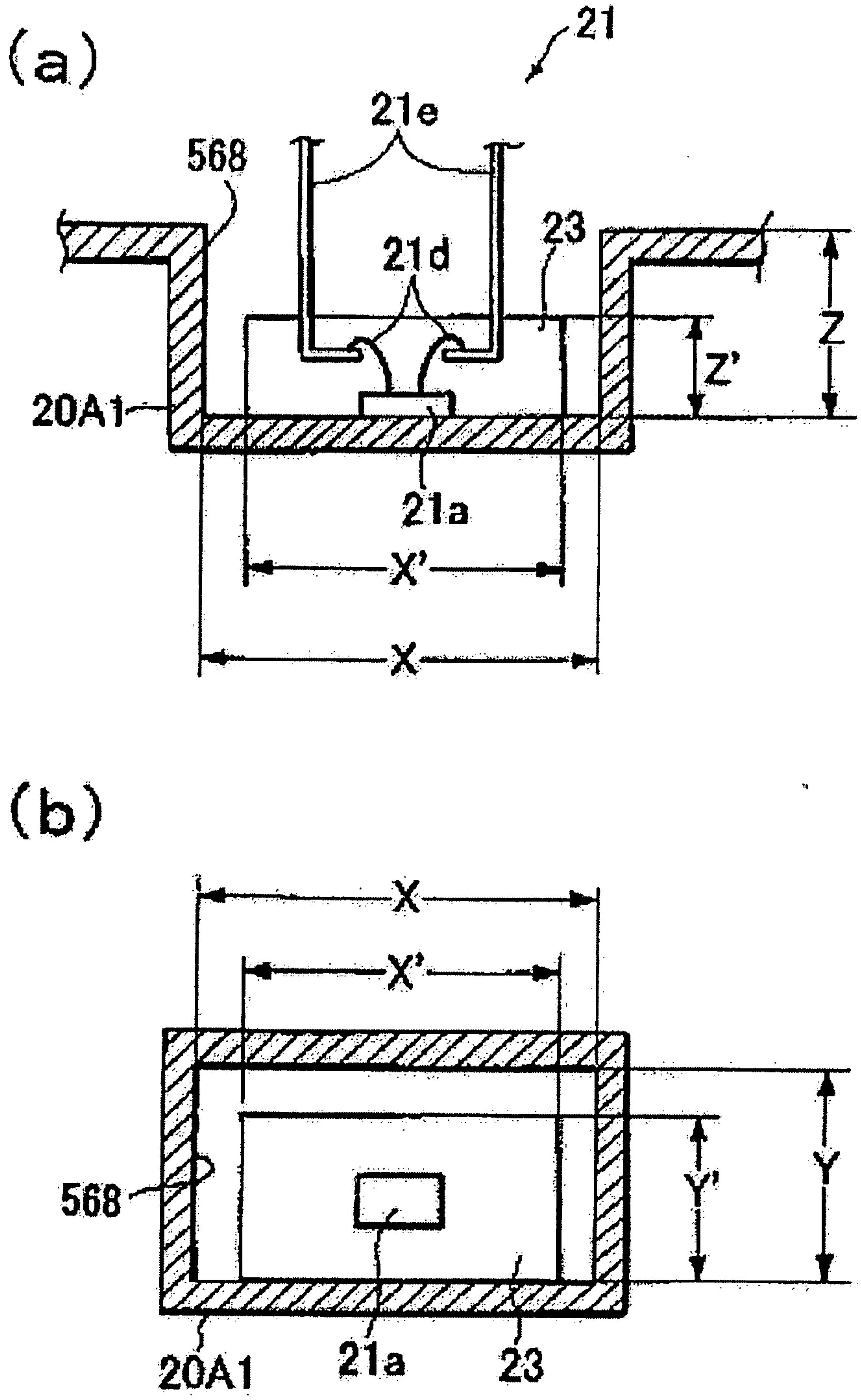

[Fig. 52]

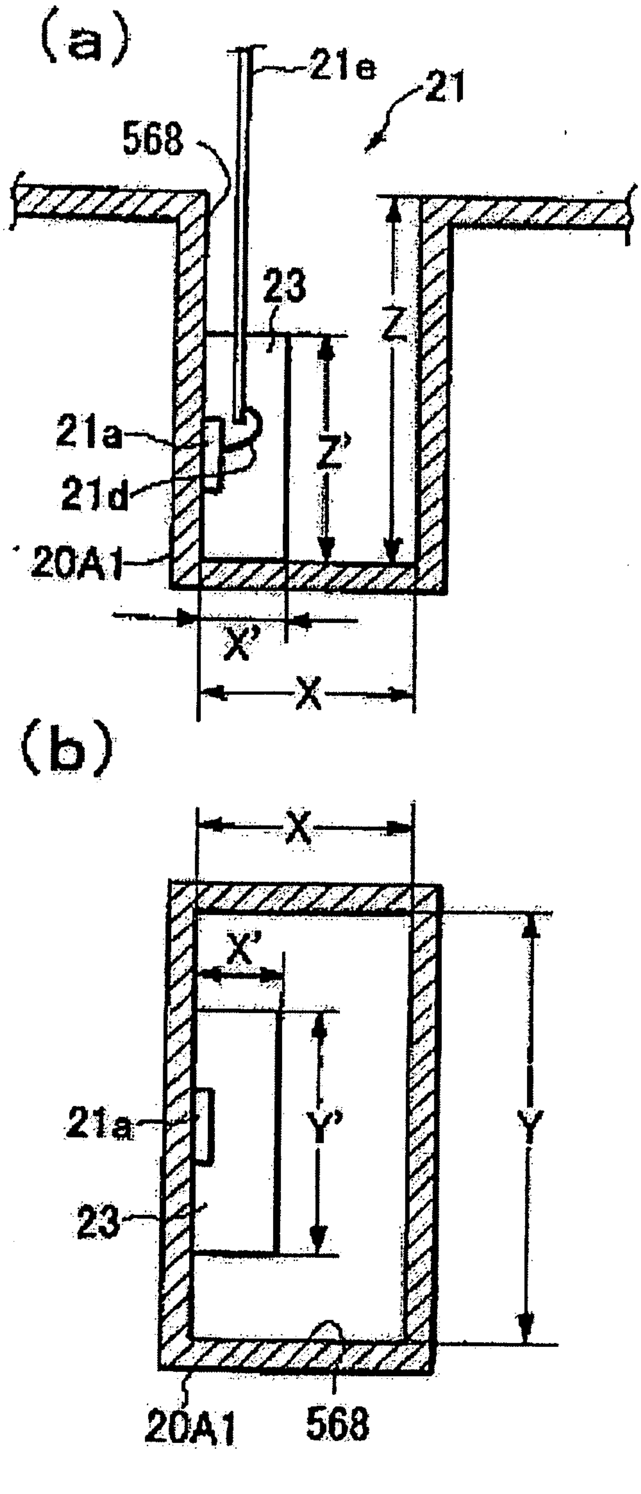

[Fig. 53]

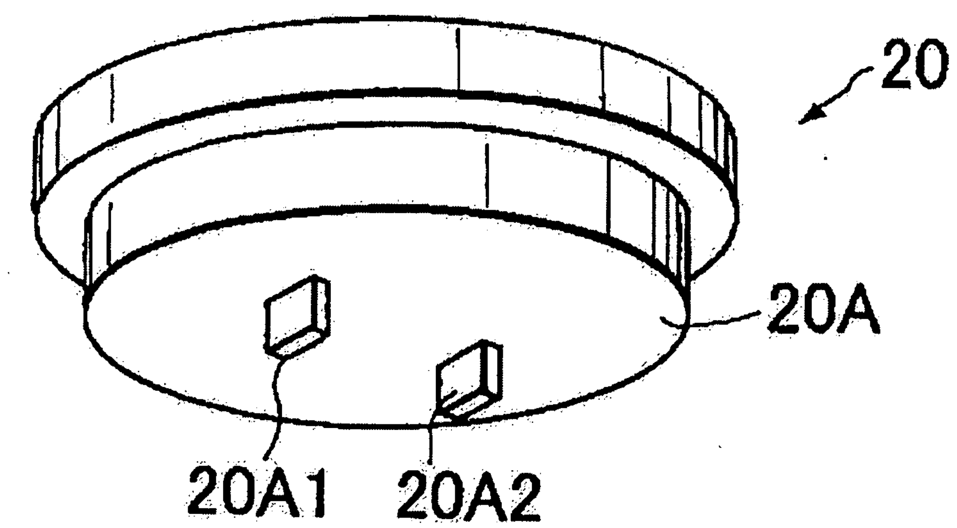

[Fig. 54]

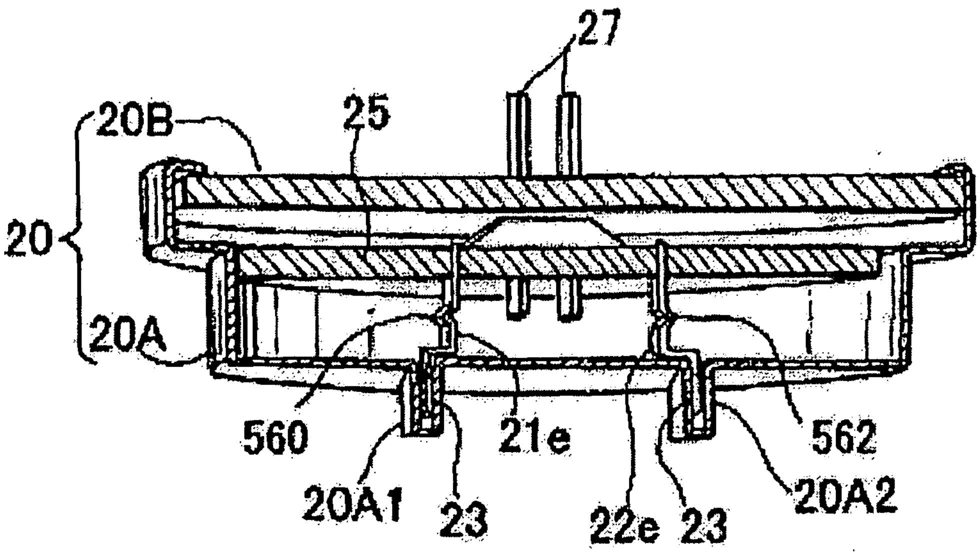

[Fig. 55]

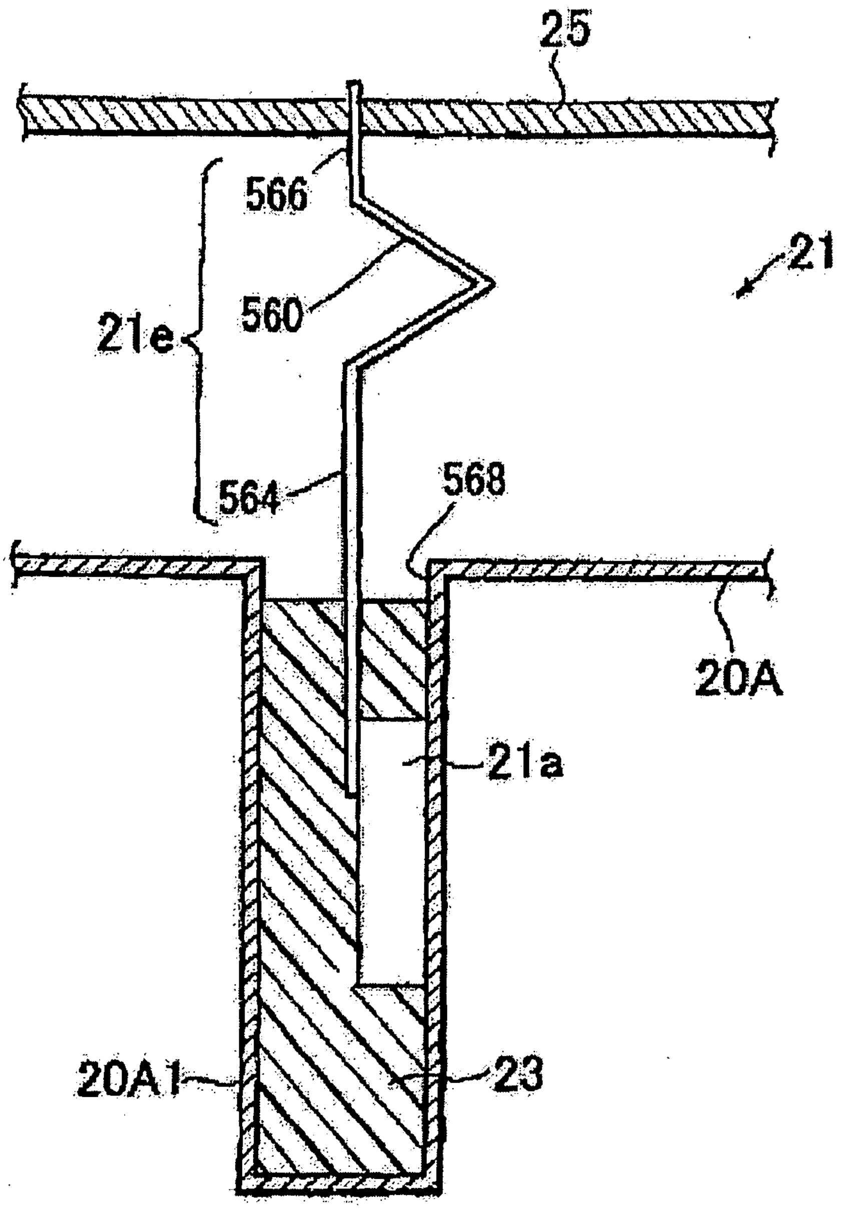

[Fig. 56]

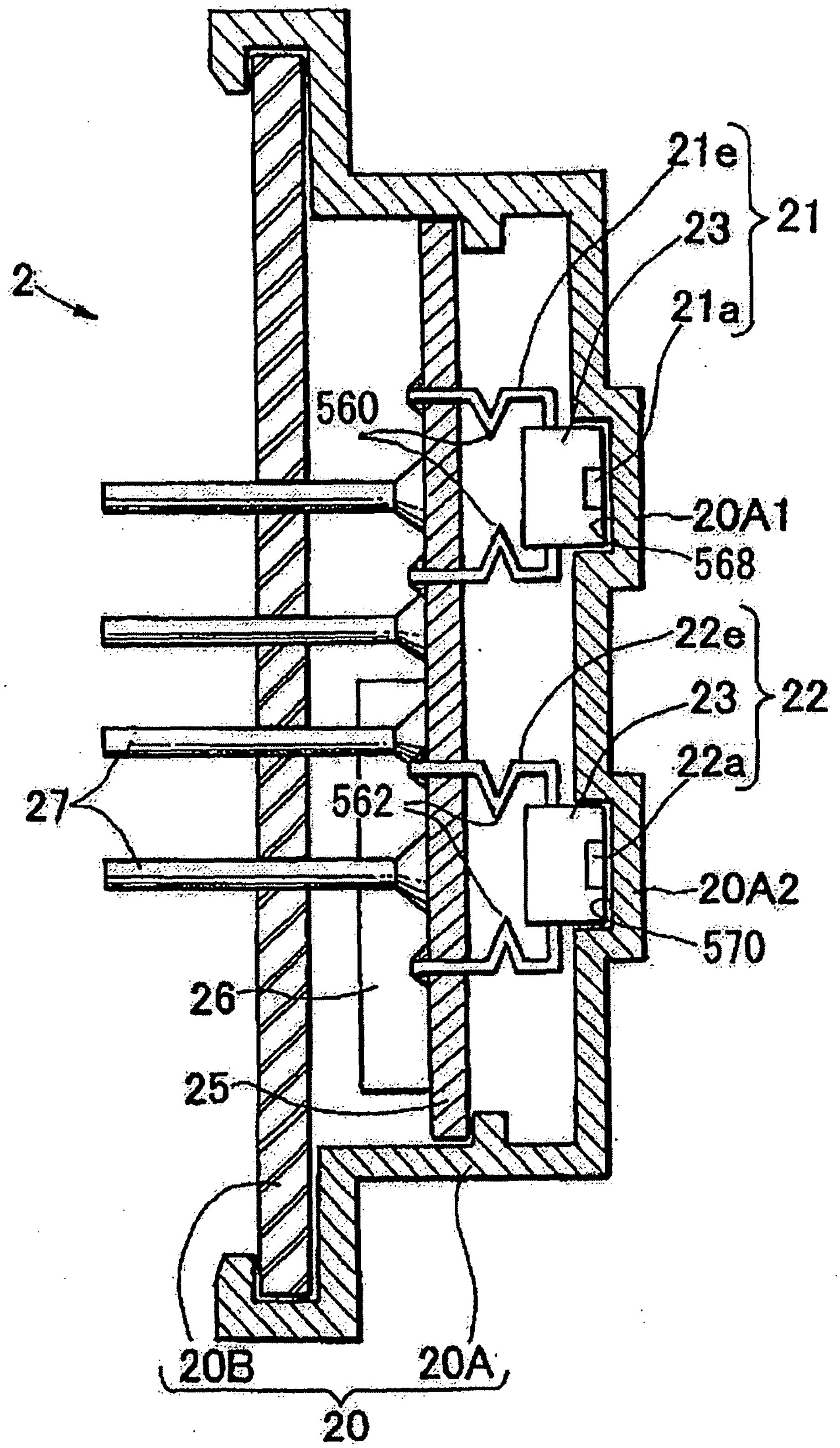
21e
23
21
21a
2
560
20A1
568
22e
23
22
22a
562
27
20A2
570
26
25
20B
20A
20

[Fig. 57]

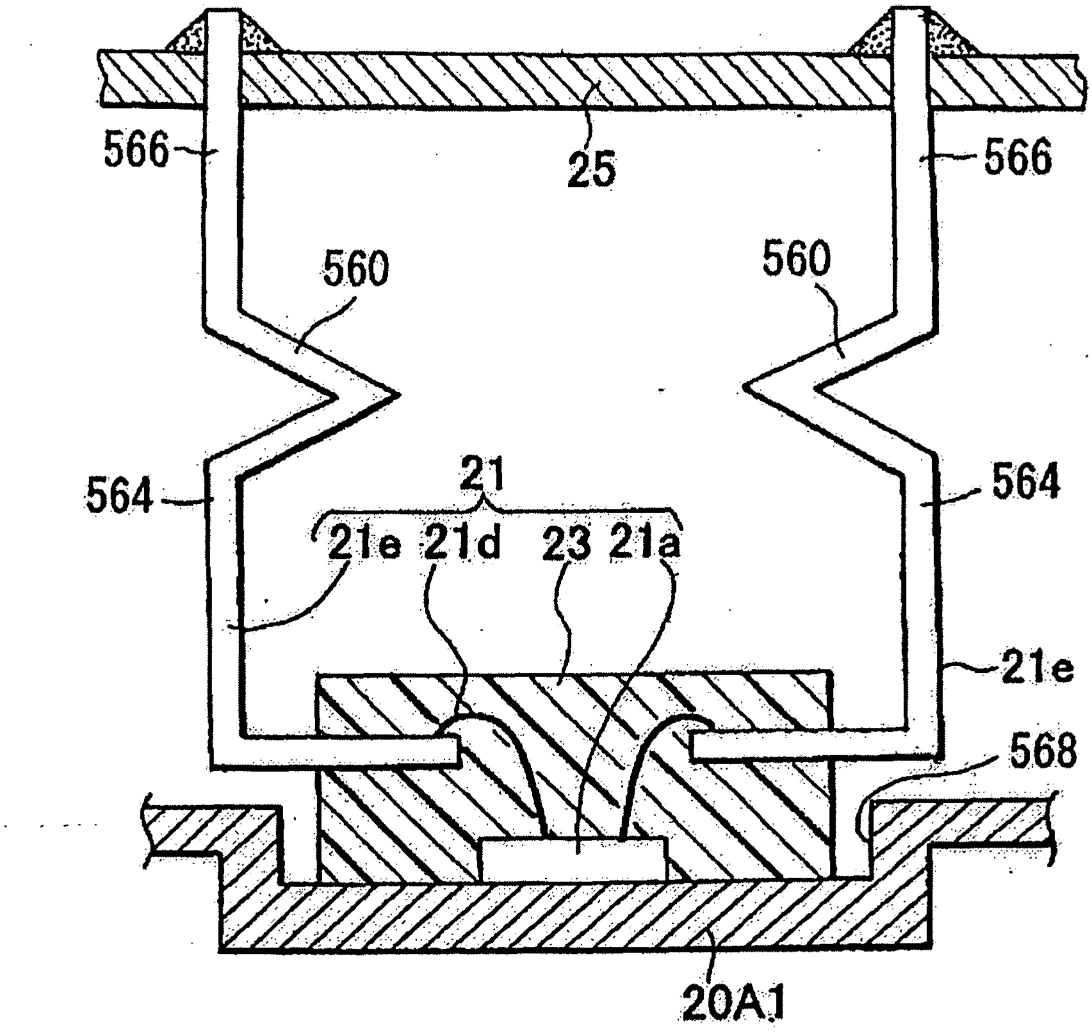

[Fig. 58]

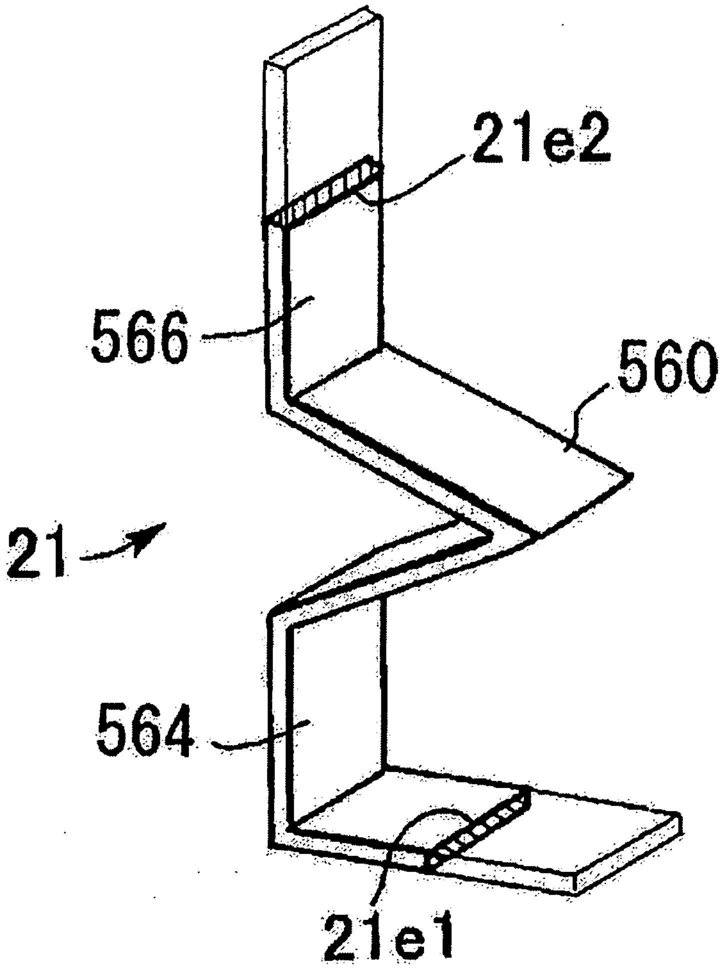

[Fig. 59]

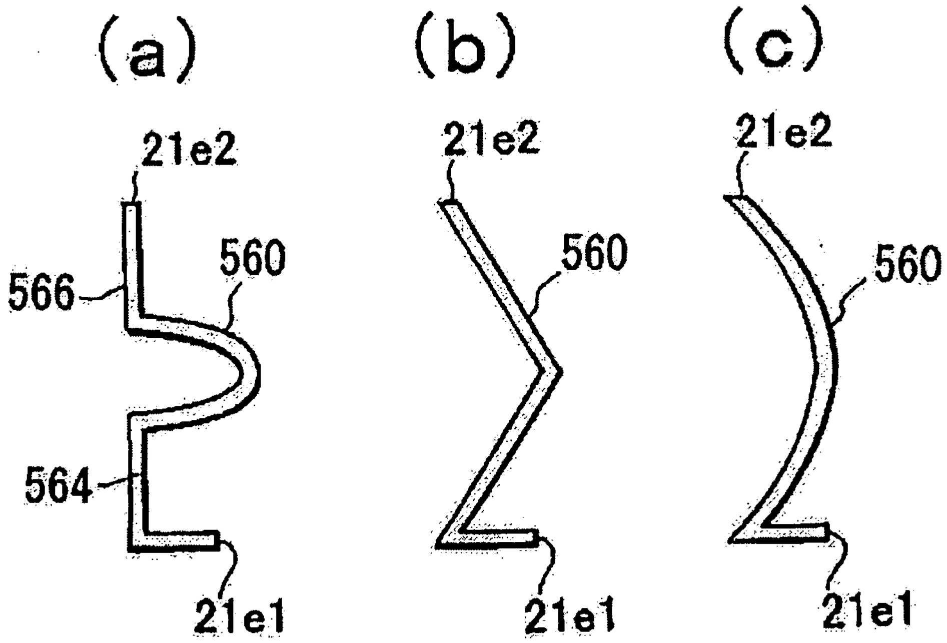

[Fig. 60]

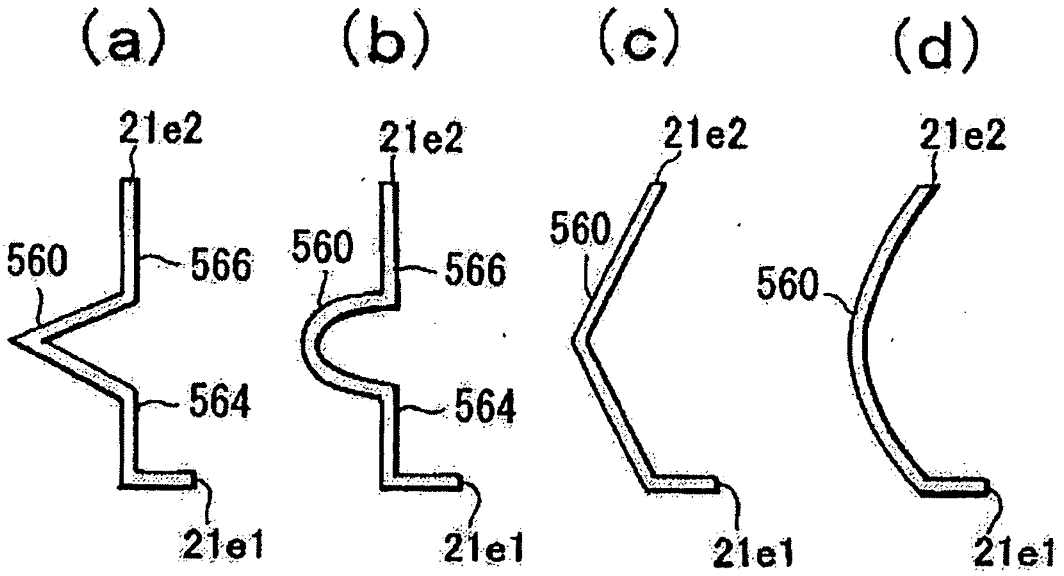
(a)
(b)
(c)
(d)
21e2
560
566
564
21e1
21e2
560
566
564
21e1
21e2
560
21e1
21e2
560
21e1

[Fig. 61]

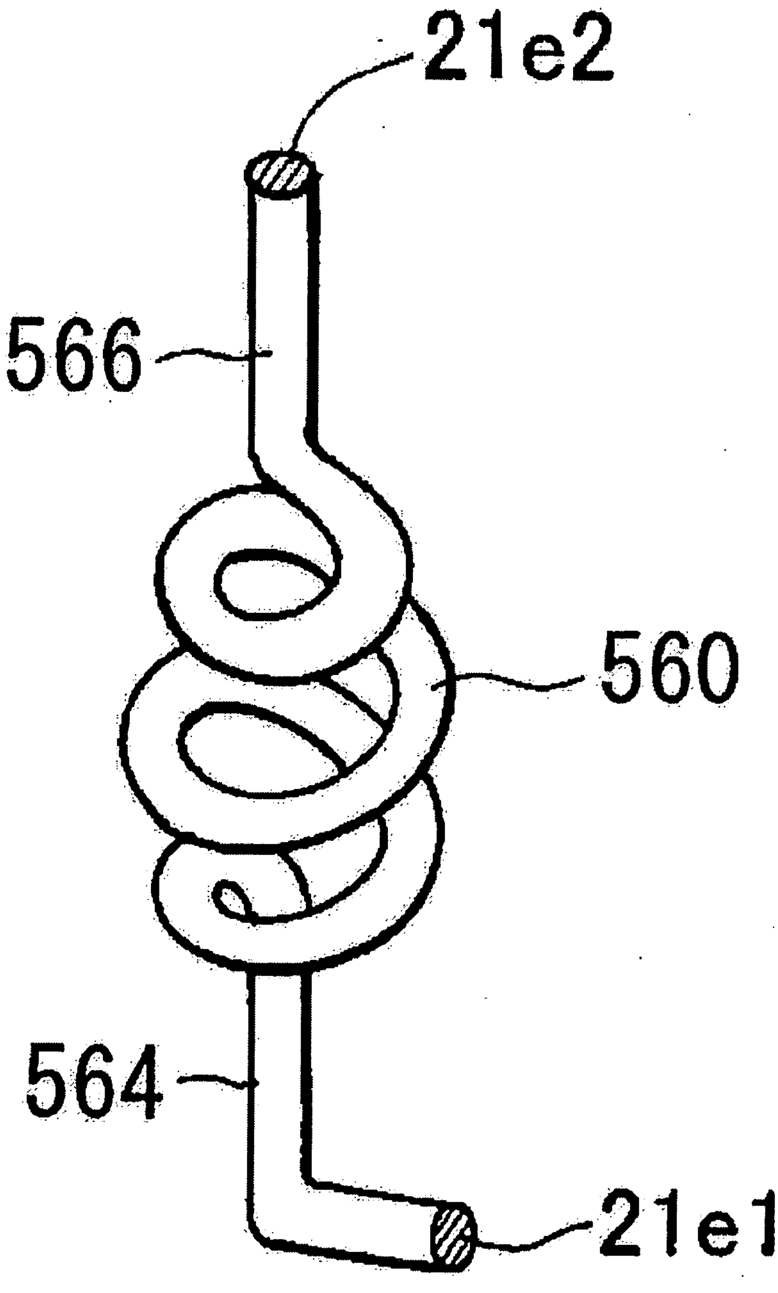

[Fig. 62]

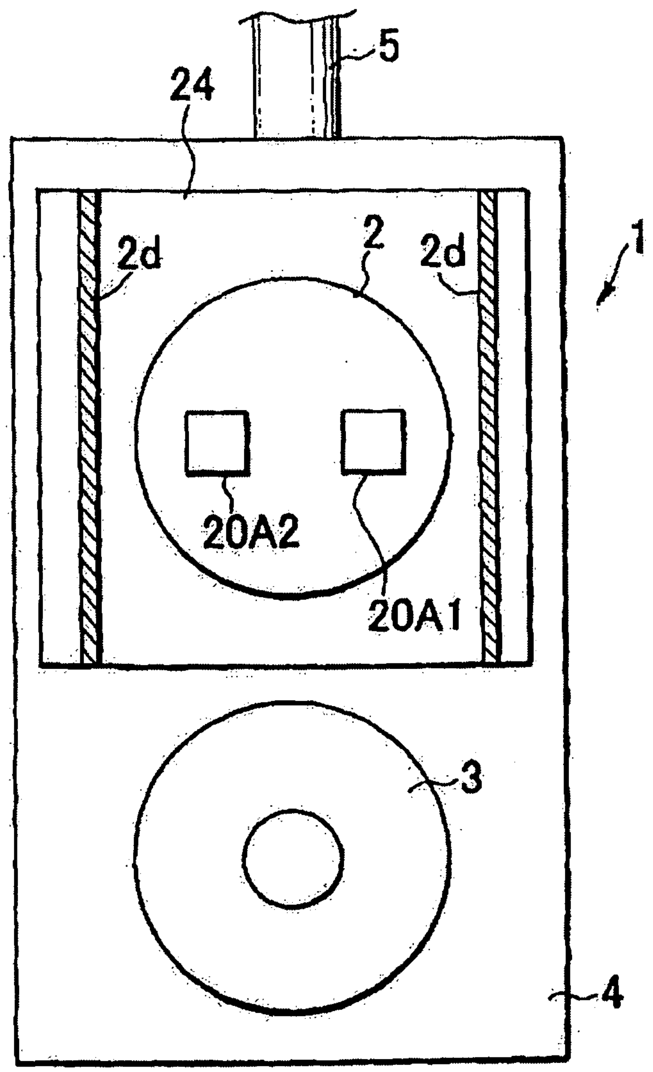

[Fig. 63]

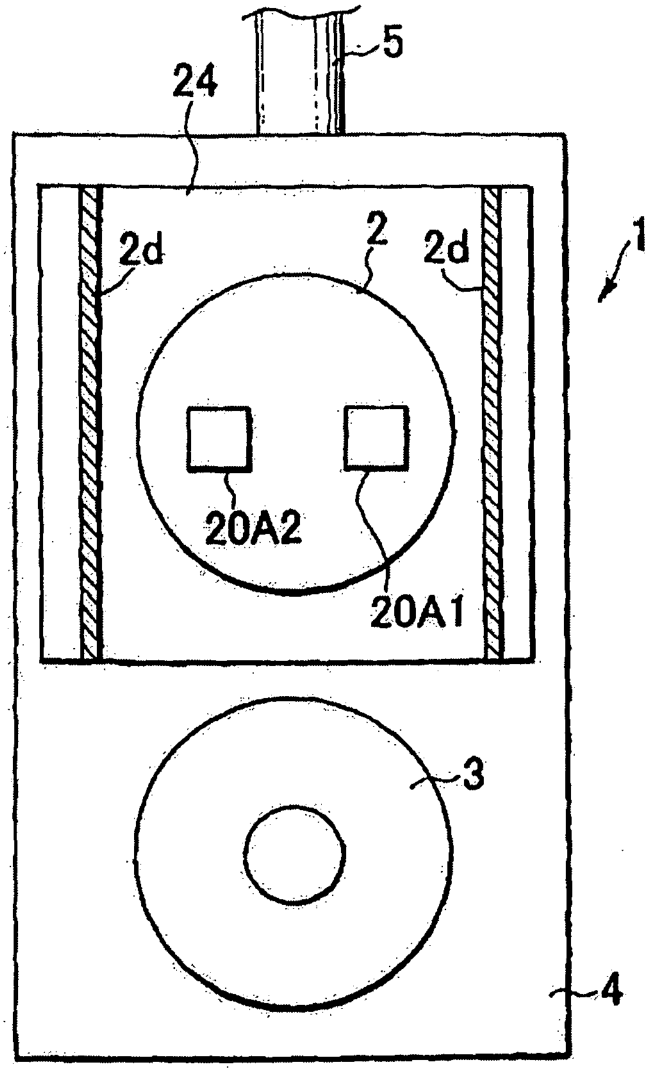

[Fig. 64]

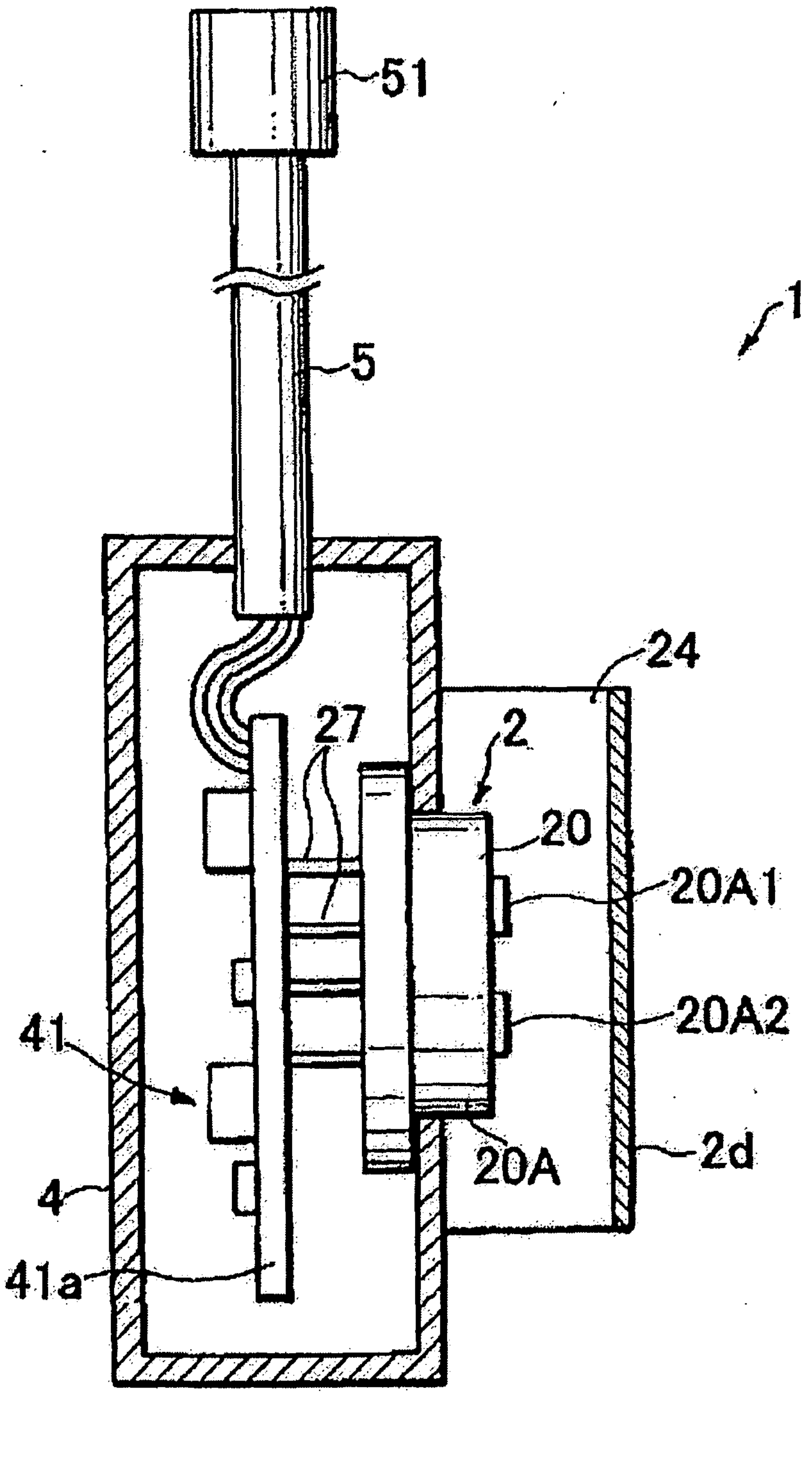

[Fig. 65]

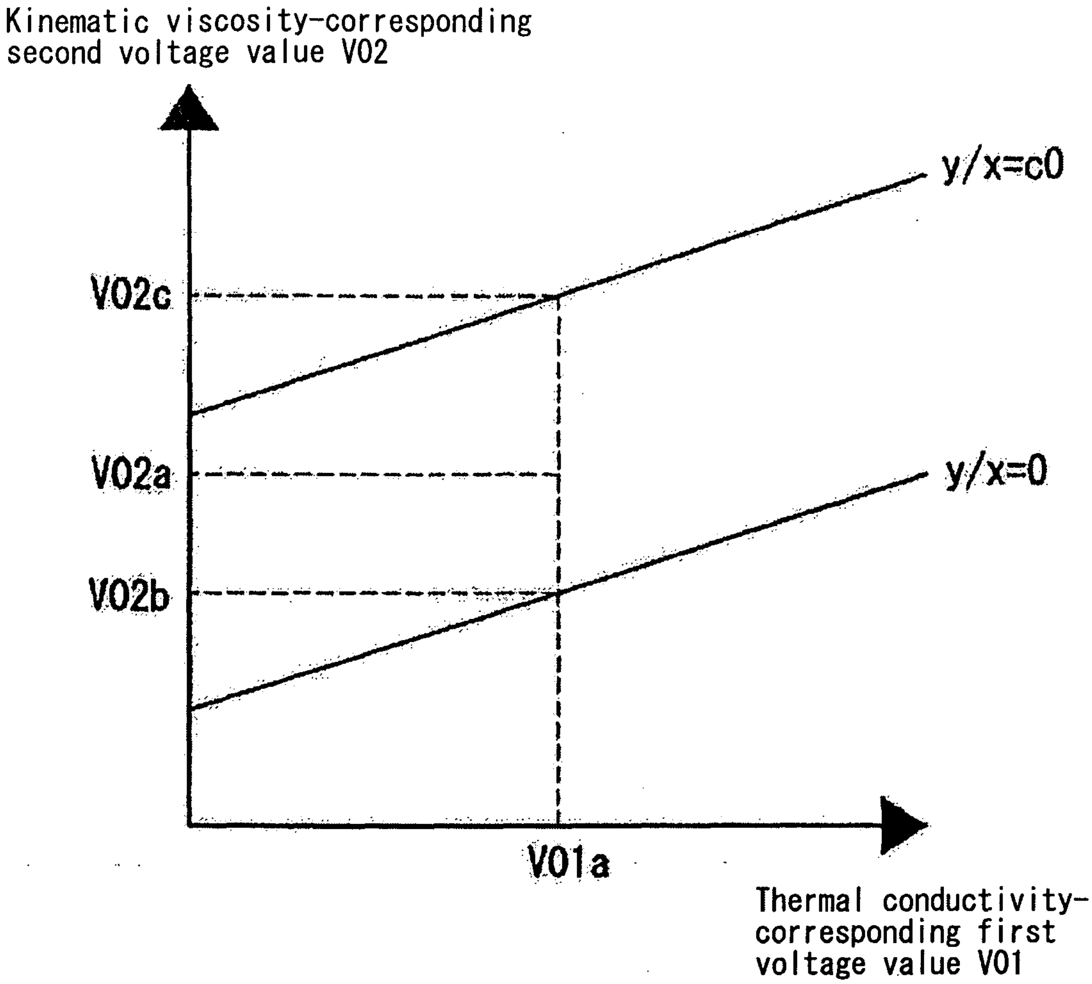
Kinematic viscosity-corresponding second voltage value V02
y/x=c0
V02c
V02a
y/x=0
V02b
V01a
Thermal conductivity-corresponding first voltage value V01

[Fig. 66]

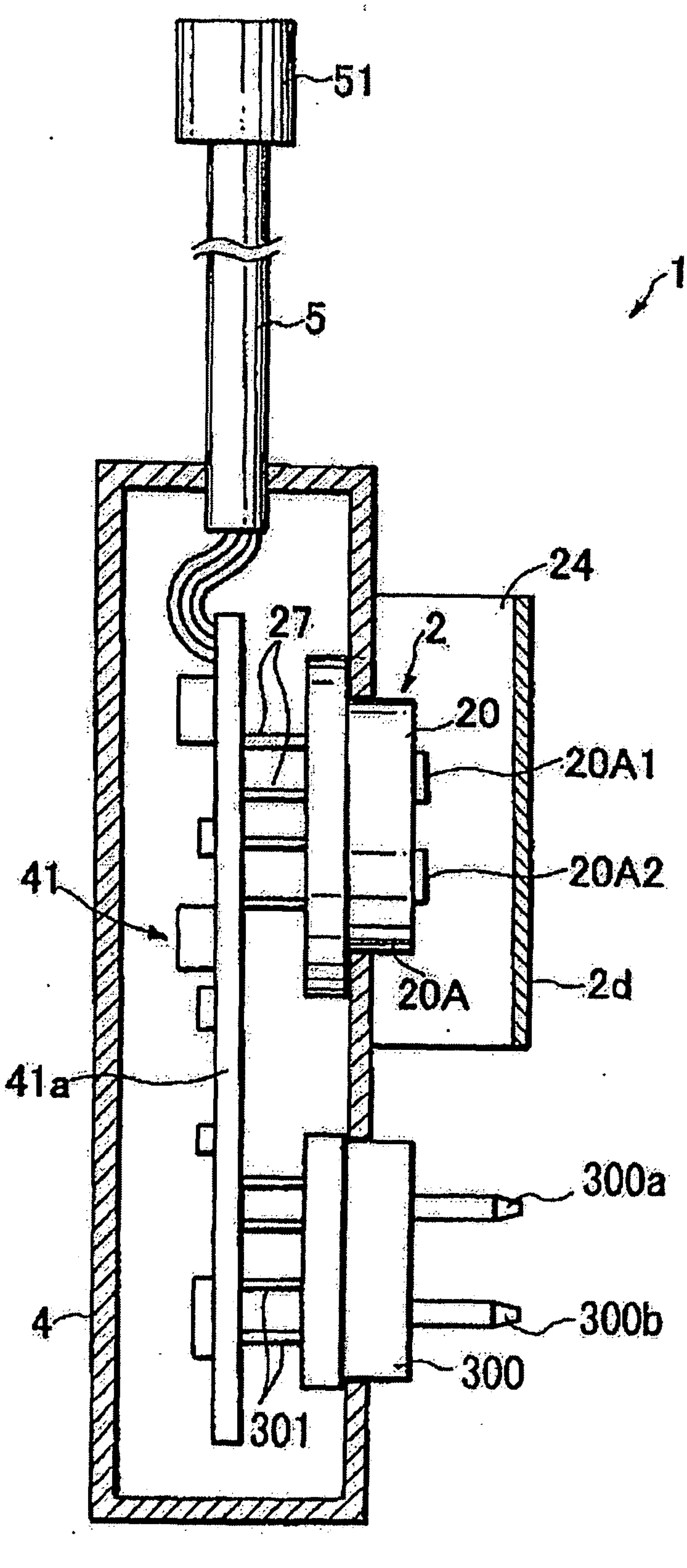

[Fig. 67]

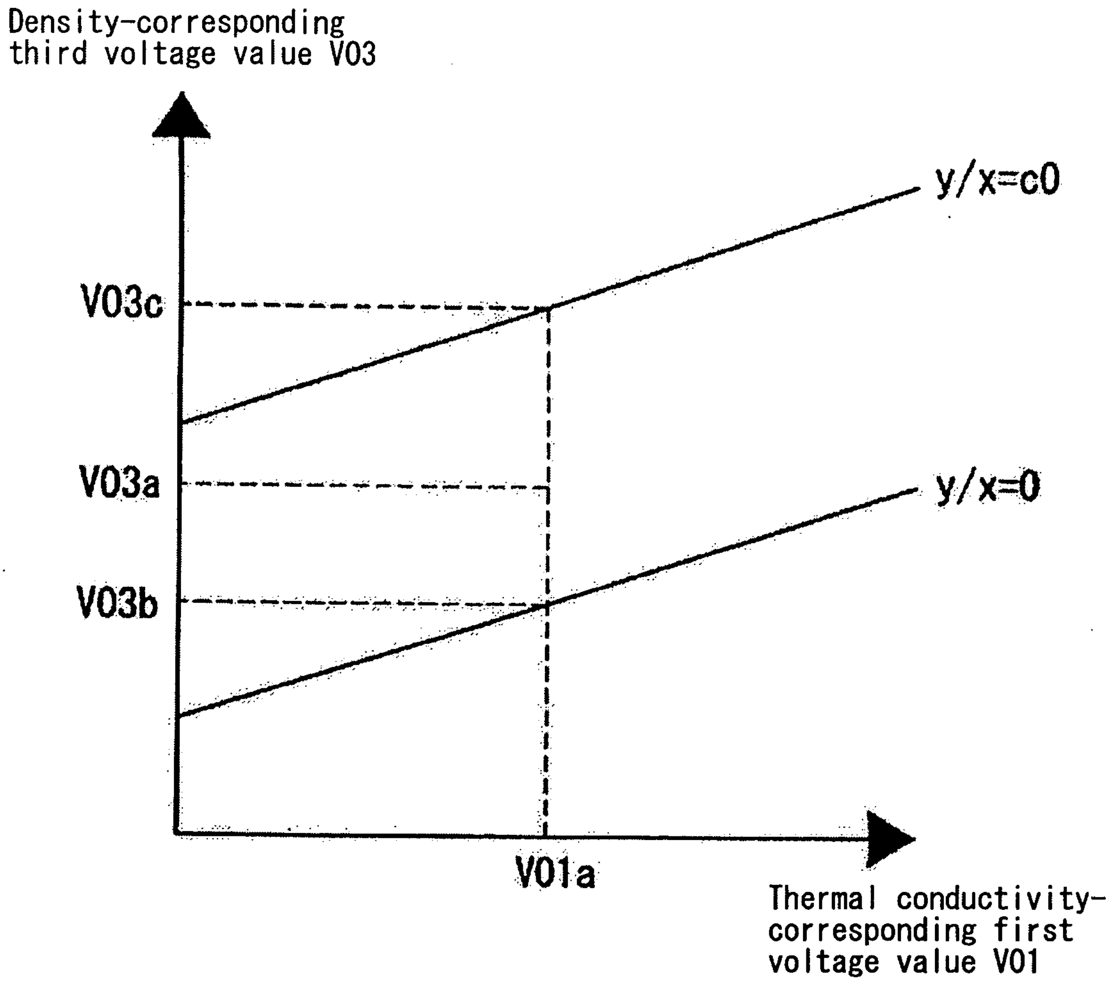
Density-corresponding third voltage value V03
y/x=c0
V03c
V03a
y/x=0
V03b
V01a
Thermal conductivity-corresponding first voltage value V01

**INTERNATIONAL SEARCH REPORT**

International application No. PCT/JP2007/051526

A. CLASSIFICATION OF SUBJECT MATTER
*G01N25/18*(2006.01)i

According to International Patent Classification (IPC) or to both national classification and IPC

B. FIELDS SEARCHED

Minimum documentation searched (classification system followed by classification symbols)
G01N25/00-G01N25/72, G01F23/00, G01F23/14-G01F23/28

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br><br><br>Y<br>A | JP 11-153561 A (Mitsui Mining & Smelting Co., Ltd.),<br>08 June, 1999 (08.06.99),<br>Claims 1 to 18; Par. Nos. [0009], [0028]; Figs. 1 to 15<br>(Family: none) | 1,22-25, 28-33,37, 46-47,59<br>38-45,48-53<br>2-21,26-27, 34-36,54-58 |
| X | WO 2006/008920 A1 (Mitsui Mining & Smelting Co., Ltd.),<br>26 January, 2006 (26.01.06),<br>Claim 1<br>(Family: none) | 26-27,34-36 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>26 April, 2007 (26.04.07) | Date of mailing of the international search report<br>15 May, 2007 (15.05.07) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2007/051526

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2005-337969 A (Mitsui Mining & Smelting Co., Ltd.), 08 December, 2005 (08.12.05), Claims 1 to 11; Fig. 2 & WO 2005/116620 A1 | 38-45 |
| Y | JP 2005-43126 A (Mitsui Mining & Smelting Co., Ltd.), 17 February, 2005 (17.02.05), Claims 1, 7; Par. No. [0189] & US 2006/213263 A1 & EP 1653227 A1 & WO 2005/005971 A1 | 48-52 |
| Y | JP 2005-134312 A (Mitsui Mining & Smelting Co., Ltd.), 26 May, 2005 (26.05.05), Claim 1 & WO 2005/043104 A1 | 53 |
| P,A | JP 2006-119088 A (Mitsui Mining & Smelting Co., Ltd.), 11 May, 2006 (11.05.06), Claims 1 to 11 & WO 2006/046457 A1 | 54 |

Form PCT/ISA/210 (continuation of second sheet) (April 2005)

**INTERNATIONAL SEARCH REPORT**

International application No.
PCT/JP2007/051526

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The inventions of claims 1, 37, 59 relate to an identification sensor module including a fluid detection unit, a fluid detection circuit, and an identification calculation unit contained in a vessel.

The inventions of claims 2-7 relate to an identification sensor module in which a lead has a spring structure.

The inventions of claims 8-21 relate to an identification sensor module having an expanding portion expanding toward outside the vessel.

The inventions of claims 22-36 relate to an arrangement interval between the fluid detection unit and the fluid temperature detection unit in the horizontal direction. (Continued to extra sheet)

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (April 2005)

INTERNATIONAL SEARCH REPORT

International application No.
PCT/JP2007/051526

Continuation of Box No.III of continuation of first sheet(2)

The invention of claim 38 relates to an arrangement interval between the fluid detection unit and the fluid temperature detection unit in the vertical direction.

The inventions of claims 39-45 relate to an identification sensor module mounted on a waterproof case.

The inventions of claims 46-54 relate to the identification contents of the fluid identification.

The inventions of claims 55-58 relate to measurement of an amount of ammonia generated by the fluid to be identified.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11153561 A
- JP 2005337969 A
- JP 11118566 A
- JP 2003185522 A